# EUROPEAN PATENT APPLICATION

(11) **EP 1 739 173 A1**
(43) Date of publication of application: **03.01.2007**
(21) Application number: 05727480.5
(22) Date of filing: 24.03.2005
(51) Int. Cl.: C12N 15/09, A61K 39/395, A61K 45/00, A61K 48/00, A61P 35/00, C07K 14/82, C12Q 1/68, G01N 33/53, G01N 33/574

(54) **KIT FOR SOLID CANCER DIAGNOSIS AND MEDICINE FOR SOLID CANCER THERAPY**

(30) Priority: 29.03.2004 JP 2004095732
(71) Applicant: Medical & Biological Laboratories Co., Ltd., Nagoya-shi, Aichi 460-0002 (JP); Shimada, Hideaki, 2720812 (JP)
(72) Inventor: SHIMADA, Hideaki, Ichikawa-shi, Chiba 2720812 (JP); TOMONAGA, Takeshi, Chiba-shi, Chiba 2600033 (JP); HIWASA, Takaki, Chiba-shi, Chiba 2600808 (JP); MATSUSHITA, Kazuyuki, Chiba-shi, Chiba 2600851 (JP); OCHIAI, Takenori, Chiba-shi, Chiba 2600034 (JP); NOMURA, Fumio, Chiba-shi, Chiba 2640033 (JP); TAKIGUCHI, Masaki, Funabashi-shi, Chiba 2740824 (JP)
(74) Representative: Vossius & Partner
(86) International application number: PCT/JP2005/006222
(87) International publication number: WO 2005/093063

(57) **Abstract**

The present invention provides novel solid cancer antigenic proteins, and diagnostic kits for solid cancer and therapeutic agents for solid cancer based on the antigenic proteins. Specifically, the present invention provides a human solid cancer antigenic polypeptide having an amino acid sequence selected from the group consisting of SEQ ID NOS: 2, 4, 6, 8, 10, 12, 14, 16, 18, 20, 22, 24, 26, 28, 30, 32, 34, 36, 38, 40, 42, 44, 48, 50, 52, 54, 56, 59, 61, 63, 65, 67, 69, 71, 73, and 75.

## Description

### Technical Field

The present invention relates to diagnostic kits for solid cancer and medicaments for preventing or treating solid cancer.

### Background Art

All solid cancers are characterized by the presence of malignant tumors. Examples thereof include esophageal cancer, gastric cancer, lung cancer, kidney cancer, thyroid cancer, parotid gland cancer, head and neck cancer, soft tissue and bone sarcoma, urinary tract cancer, bladder cancer, uterine cancer, liver cancer, breast cancer, ovarian cancer, and fallopian tube cancer. Thus, in particular, treatment of progressive solid cancer is difficult, resulting in death in many cases. Therefore, early tumor detection is the most important objective of therapeutic measures against solid cancer.

Hitherto, tumor markers such as CEA and CA19-9 have been reported and used for diagnosis of the solid cancers listed above and for the prognosis thereof However, with any tumor marker, the percentage of positive results is as low as 20% to 30%. In particular, during the early stage of cancer, negative results are obtained when using most such markers. In addition, since poor therapeutic outcomes are obtained in the case of the progressive solid cancers listed above, early detection of cancer is the most effective. Thus, novel and effective tumor markers have been expected to be discovered.

In addition, molecular biological diagnostic methods using antigenic protein markers are disclosed in JP Patent Publication No. 7-51065 A (1995), WO00/060073, and JP Patent Publication No. 2000-511536 A, for example. In addition, a SEREX method (serological identification of antigens by recombinant expression cloning) whereby proteins produced using mRNA of tumor cells obtained from a cancer patient are subjected to screening with the patient's autoserum has been reported (Proc. Natl. Acad. Sci. USA 92: 11810-11813, 1995 and US Patent No. 5,698,396). Further, it has been reported that the aforementioned SEREX method was used to isolate cancer antigens recognized by IgG antibodies in the cases of malignant melanoma, kidney cancer, esophageal cancer, colorectal cancer, and lung cancer, for example (Int. J. Cancer 72: 965-971, 1997; Cancer Res. 58: 1034-1041, 1998; Int. J. Cancer 29: 652-658, 1998; Int. J. Oncol. 14: 703-708, 1999; Cancer Res. 56: 4766-4772, 1996; Hum. Mol. Genet 6: 33-39, 1997). Further, JP Patent Publication No. 2001-333782 A discloses malignant melanoma antigenic proteins identified by the SEREX method, DNA sequences encoding the proteins, and a method for diagnosing malignant melanoma using such proteins and DNA sequences. However, in order to improve diagnostic accuracy with regard to solid cancer, it is essential to prepare as many protein markers having high antigenicity as possible so as to use them in combination.

Meanwhile, as therapeutic methods for solid cancer, surgical extraction of cancer tissue, systemic administration of anticancer drugs, and the like have been carried out. However, as described above, in the case of progressive solid cancer, such therapeutic methods have had few effects. Even when solid cancer is found during the early stage, these therapeutic methods impose heavy physical burdens on patients, which has been problematic.

### Disclosure of the Invention

As described above, it has been pointed out that the molecular biological diagnostic method using cancer tissue-specific antigenic protein markers is effective as a method for early diagnosis of solid cancer. Thus, several new antigenic protein markers have been suggested. However, in order to improve diagnostic accuracy with regard to solid cancer, it is essential to prepare as many protein markers having high antigenicity as possible so as to use them in combination.

In addition, since such antigenic protein markers are dominantly expressed in solid cancer tissue, the markers are expected to be applied to therapeutic methods of selectively targeting cancer tissue.

Thus, it is an objective of the present invention to provide novel solid cancer antigenic proteins, diagnostic kits for solid cancer, and therapeutic agents for solid cancer based on the antigenic proteins.

As a result of intensive studies to attain above objectives, the present inventors have found novel antigenic polypeptides that are specific to human solid cancer, and have found that solid cancer can be diagnosed and that solid cancer can be prevented or treated with the use of the expression of such antigenic polypeptides. Thus, this has led to the completion of the present invention.

That is, the present invention relates to a human solid cancer antigenic polypeptide having an amino acid sequence selected from the group consisting of SEQ ID NOS: 2, 4, 6, 8, 10, 12, 14, 16, 18, 20, 22, 24, 26, 28, 30, 32, 34, 36, 38, 40, 42, 44, 48, 50, 52, 54, 56, 59, 61, 63, 65, 67, 69, 71, 73, and 75.

The present invention also relates to a polynucleotide encoding the human solid cancer antigenic polypeptide.

Also, the present invention relates to a polynucleotide having a nucleotide sequence selected from the group consisting of SEQ ID NOS: 1, 3, 5, 7, 9, 11, 13, 15, 17, 19, 21, 23, 25, 27, 29, 31, 33, 35, 37, 39, 41, 43, 45, 46, 47, 49, 51, 53, 55, 57, 58, 60, 62, 64, 66, 68, 70, 72, and 74.

The present invention further relates to a diagnostic kit for solid cancer comprising a means of detecting the expression of at least one human solid cancer antigenic polypeptide in a sample derived from a subject, characterized in that the human solid cancer antigenic polypeptide has an amino acid sequence selected from the group consisting of SEQ ID NOS: 2, 4, 6, 8, 10, 12, 14, 16, 18, 20, 22, 24, 26, 28, 30, 32, 34, 36, 38, 40, 42, 44, 48, 50, 52, 54, 56, 59, 61, 63, 65, 67, 69, 71, 73, and 75.

The present invention also relates to a diagnostic kit for solid cancer comprising a means of detecting the expression of at least one human solid cancer antigenic polypeptide in a sample derived from a subject, characterized in that the human solid cancer antigenic polypeptide is encoded by a polynucleotide having a nucleotide sequence selected from the group consisting of SEQ ID NOS: 1, 3, 5, 7, 9, 11, 13, 15, 17, 19, 21, 23, 25, 27, 29, 31, 33, 35, 37, 39, 41, 43, 45, 46, 47, 49, 51, 53, 55, 57, 58, 60, 62, 64, 66, 68, 70, 72, and 74.

Examples of the means of detecting the expression of the human solid cancer antigenic polypeptide(s) include the solid cancer antigenic polypeptide or a partial peptide thereof, an antibody against the solid cancer antigenic polypeptide, and a primer or probe comprising polynucleotide consisting of the entire or a partial sequence of a polynucleotide encoding the solid cancer antigenic polypeptide or a complementary sequence thereof.

In addition, the means of detecting the expression of the human solid cancer antigenic polypeptide(s) may be immobilized on a solid phase and/or labeled.

Examples of the solid cancer include colorectal cancer, esophageal cancer, gastric cancer, and breast cancer.

Examples of the sample include serum, blood, hemocytes, and tissue.

The present invention also relates to a medicament for preventing or treating solid cancer comprising a means of inhibiting the functions or expression of at least one human solid cancer antigenic polypeptide, characterized in that the human solid cancer antigenic polypeptide has an amino acid sequence selected from the group consisting of SEQ ID NOS: 2, 4, 6, 8, 10, 12, 14, 16, 18, 20, 22, 24, 26, 28, 30, 32, 34, 36, 38, 40, 42, 44, 48, 50, 52, 54, 56, 59, 61, 63, 65, 67, 69, 71, 73, and 75.

The present invention further relates to a medicament for preventing or treating solid cancer comprising a means of inhibiting the functions or expression of at least one human solid cancer antigenic polypeptide, characterized in that the human solid cancer antigenic polypeptide is encoded by a polynucleotide having a nucleotide sequence selected from the group consisting of SEQ ID NOS: 1, 3, 5, 7, 9, 11, 13, 15, 17, 19, 21, 23, 25, 27, 29, 31, 33, 35, 37, 39, 41, 43, 45, 46, 47, 49, 51, 53, 55, 57, 58, 60, 62, 64, 66, 68, 70, 72, and 74.

Examples of the means of inhibiting the functions or expression of the human solid cancer antigenic polypeptide(s) include an antibody against the solid cancer antigenic polypeptide, a means capable of inhibiting transcription of a gene encoding the solid cancer antigenic polypeptide, and a means capable of inhibiting translation of a gene encoding the solid cancer antigenic polypeptide.

Further, the present invention relates to a medicament for preventing or treating solid cancer comprising a gene encoding a prophylactic or therapeutic agent for solid cancer and a means of targeting to human solid cancer.

Examples of the means of targeting to human solid cancer include an antibody against a solid cancer antigenic polypeptide and a nucleotide sequence of an expression control region of a polynucleotide encoding a solid cancer antigenic polypeptide.

### Brief Description of the Drawings

Fig. 1 A shows results of a comparison in terms of the specific expression of an antigenic protein between cancer tissue and normal tissue of a colorectal cancer patient.
Fig. 1 B shows results of a comparison in terms of the specific expression of an antigenic protein between cancer tissue and normal tissue of a colorectal cancer patient.
Fig. 2 shows results of Western blotting analysis showing reactivity of an antigenic polypeptide expressed by a polynucleotide having the nucleotide sequence set forth in SEQ ID NO: 41 and an antibody in the serum of a patient.
Fig. 3 shows results of Western blotting analysis showing reactivity of an antigenic polypeptide expressed by a polynucleotide having the nucleotide sequence set forth in SEQ ID NO: 43 and an antibody in the serum of a patient.
Fig. 4 shows results of Western blotting analysis showing reactivity of an antigenic polypeptide expressed by a polynucleotide having the nucleotide sequence set forth in SEQ ID NO: 45 and an antibody in the serum of a patient.
Fig. 5 shows results of Western blotting analysis showing reactivity of an antigenic polypeptide expressed by a polynucleotide having the nucleotide sequence set forth in SEQ ID NO: 46 and an antibody in the serum of a patient.
Fig. 6 shows results of Western blotting analysis showing reactivity of an antigenic polypeptide expressed by a polynucleotide having the nucleotide sequence set forth in SEQ ID NO: 47 and an antibody in the serum of a patient.
Fig. 7 shows results of Western blotting analysis showing reactivity of an antigenic polypeptide expressed by a polynucleotide having the nucleotide sequence set forth in SEQ ID NO: 49 and an antibody in the serum of a patient.
Fig. 8 shows results of Western blotting analysis showing reactivity of an antigenic polypeptide expressed by a polynucleotide having the nucleotide sequence set forth in SEQ ID NO: 51 and an antibody in the serum of a patient.
Fig. 9 shows results of Western blotting analysis showing reactivity of an antigenic polypeptide expressed by a polynucleotide having the nucleotide sequence set forth in SEQ ID NO: 53 and an antibody in the serum of a patient.
Fig. 10 shows results of Western blotting analysis showing reactivity of an antigenic polypeptide expressed by a polynucleotide having the nucleotide sequence set forth in SEQ ID NO: 55 and an antibody in the serum of a patient.
Fig. 11 shows results of Western blotting analysis showing reactivity of an antigenic polypeptide expressed by a polynucleotide having the nucleotide sequence set forth in SEQ ID NO: 57 and an antibody in the serum of a patient.
Fig. 12 shows results of Western blotting analysis showing reactivity of an antigenic polypeptide expressed by a polynucleotide having the nucleotide sequence set forth in SEQ ID NO: 58 and an antibody in the serum of a patient.
Fig. 13 shows results of Western blotting analysis showing reactivity of an antigenic polypeptide expressed by a polynucleotide having the nucleotide sequence set forth in SEQ ID NO: 60 and an antibody in the serum of a patient.
Fig. 14 shows results of Western blotting analysis showing reactivity of an antigenic polypeptide expressed by a polynucleotide having the nucleotide sequence set forth in SEQ ID NO: 62 and an antibody in the serum of a patient.
Fig. 15 shows results of Western blotting analysis showing reactivity of an antigenic polypeptide expressed by a polynucleotide having the nucleotide sequence set forth in SEQ ID NO: 64 and an antibody in the serum of a patient.
Fig. 16 shows results of Western blotting analysis showing reactivity of an antigenic polypeptide expressed by a polynucleotide having the nucleotide sequence set forth in SEQ ID NO: 66 and an antibody in the serum of a patient.
Fig. 17 shows results of Western blotting analysis showing reactivity of an antigenic polypeptide expressed by a polynucleotide having the nucleotide sequence set forth in SEQ ID NO: 68 and an antibody in the serum of a patient.
Fig. 18 shows results of Western blotting analysis showing reactivity of an antigenic polypeptide expressed by a polynucleotide having the nucleotide sequence set forth in SEQ ID NO: 70 and an antibody in the serum of a patient.
Fig. 19 shows results of Western blotting analysis showing reactivity of an antigenic polypeptide expressed by a polynucleotide having the nucleotide sequence set forth in SEQ ID NO: 72 and an antibody in the serum of a patient.
Fig. 20 shows results of Western blotting analysis showing reactivity of an antigenic polypeptide expressed by a polynucleotide having the nucleotide sequence set forth in SEQ ID NO: 74 and an antibody in the serum of a patient.

### Best Mode for Carrying Out the Invention

Hereafter, the present invention will be described in detail. The present application claims a priority from Japanese Patent Application No. 2004-95732 filed on March 29, 2004, and the entire contents disclosed in the specification and/or drawings thereof are hereby incorporated.

### 1. Novel human solid cancer antigenic polypeptides

The present invention is based on novel antigenic polypeptides that are specific to human solid cancer. With the consent of colorectal cancer patients, the present inventors extracted proteins from normal tissue and cancer tissue of surgical samples taken from the patients, followed by analysis via two-dimensional electrophoresis (e.g., Electrophoresis 22: 3019-3025, 2001). Thus, we have found 20 types of antigenic polypeptides (set forth in numbers 1 to 20 in table 1) that are specifically expressed in solid cancer cells and that have not been known to have the function as tumor markers (see Example 1). In addition, serum obtained from esophageal cancer, gastric cancer, colorectal cancer, and breast cancer patients with their consent was analyzed by a SEREX method (Proc. Natl. Acad. Sci. USA 92: 11810-11813, 1995; US Patent No. 5,698,396). Accordingly, we have also found antigenic polypeptides (set forth in numbers 21 to 39 in table 1) against 19 types of specific antibodies that exist exclusively in serum of esophageal cancer, gastric cancer, colorectal cancer, and breast cancer patients (see Example 2). These solid cancer antigenic polypeptides are shown in table 1.

**Table 1**

| No. | Name | Registered number | Nucleotide sequence | Amino acid sequence | Clone name Clone name |
|---|---|---|---|---|---|
| 1 | Malate dehydrogenase 2 | NP_005909, NM_005918 | 1 | 2 | |
| 2 | Tropomyosin 4 | NP_003281, NM_003290 | 3 | 4 | |
| 3 | FK506 binding protein 4 | NP_002005, NM_002014 | 5 | 6 | |
| 4 | Chaperonin-containing TCP1, subunit 6A | NP_001753, NM_001762 | 7 | 8 | |
| 5 | Serine protease inhibitor, clade H, collagen binding protein 1 | NP_001226, NM_001235 | 9 | 10 | |
| 6 | Sulfide dehydrogenase-like | NP_067022, NM_021199 | 11 | 12 | |
| 7 | Hydroxysteroid(17-β)dehydrogenase 4 | NP_000405, NM_000414 | 13 | 14 | |
| 8 | Stress-induced-phosphoprotein 1 | N_006810, NM_006819 | 15 | 16 | |
| 9 | Heterogeneous nuclear ribonucleoprotein L | NP_001524, NM_001533 | 17 | 18 | |
| 10 | Heterogeneous nuclear ribonucleoprotein U | NP_004492, NM_004501 | 19 | 20 | |
| 11 | Matrine 3 | NP_061322, NM 018834 | 21 | 22 | |
| 12 | Annexin A3 | NP_005130, NM_005139 | 23 | 24 | |
| 13 | PTK9L protein tyrosine kinase 9-like | NP_009215, NM_007284 | 25 | 26 | |
| 14 | splicing factor, arginine/serine-rich 1 | NP_008855, NM_006924 | 27 | 28 | |
| 15 | Thiosulfate sulphurtransferase | NP 003303, NM_003312 | 29 | 30 | |
| 16 | S-adenosylhomocysteine hydrolase | NP_000678, NM_000687 | 31 | 32 | |
| 17 | GDP-mannose 4,6-dehydratase | NP_001491, NM_001500 | 33 | 34 | |
| 18 | Hydroxyacyl dehydrogenase, subunit A | NP_000173, NM_000182 | 35 | 36 | |
| 19 | Prolyl-4-hydroxylase β subunit | NP_000909, NM_000918 | 37 | 38 | |
| 20 | Peroxyredoxin 5 | NP_036226, NM_012094 | 39 | 40 | |
| 21 | Progesterone receptor membrane component 2 | NM_006320 | 41 | 42 | K35-1-1 |
| 22 | MAP kinase interacting serine/threonine kinase 2 | NM_199054 | 43 | 44 | K30-1-1 |
| 23 | EST: 601191782F1 | BE264462 | 45 | | 12N3-1 |
| 24 | EST: 602301679F1 | BG032310 | 46 | | 1201-1 |
| 25 | Additional sex combs-like 1 | NM 015338 | 47 | 48 | 14A1-1-1 |
| 26 | Forkhead box A1 | NM_004496 | 49 | 50 | 18G3-1 |
| 27 | Retinoic acid induced 16 | NM_022749 | 51 | 52 | 19C1-1 |
| 28 | RIKEN cDNA 5730528L13-like gene | NM_080655 | 53 | 54 | 19F1-1 |
| 29 | Lysine tRNA synthase | BC004132 | 55 | 56 | 19F1-2 |
| 30 | EST: AGENCOURT_15657942 | CF597227 | 57 | | 6BD3-1 |
| 31 | KDEL endoplasmic reticulum protein retention receptor 1 | NM_006801 | 58 | 59 | 14H1-2-1 |
| 32 | Lysosomal associated protein transmembrane 4 beta | NM_018407 | 60 | 61 | 18B2-1 |
| 33 | Protein phosphatase 1, catalytic subunit, α isoform | NM_002708 | 62 | 63 | 18G1-1 |
| 34 | Peroxyredoxin 3 | NM_006793 | 64 | 65 | 20J4-1 |
| 35 | Aldo-keto reductase family 1, member C3 | NM 003739 | 66 | 67 | 19M2 |
| 36 | Ubiquitin-conjugating enzyme E2I | BC000744 | 68 | 69 | 10Q3-1 |
| 37 | Phosphatidic acid phosphatase, Type 2C | NM_003712 | 70 | 71 | 14A1-1-2 |
| 38 | Beta-catenin interacting protein 1 | NM_020248 | 72 | 73 | 14B1-2-1 |
| 39 | Sorting nexin 15 | NM_147777 | 74 | 75 | 14H2-1-1 |

Note that, in accordance with the present invention, the term "polypeptide(s)" means molecules such as proteins and peptides, which are composed of a plurality of amino acid residues that are bound to one another via an amide bond (peptide bond). Also, the term "polynucleotide" means a molecule in which a plurality of phosphate esters of nucleosides each having a purine or pyrimidine that is bind to a sugar via a β-N-glycoside bond (ATPs, GTPs, CTPs, or UTPs; or dATPs, dGTPs, dCTPs, or dTTPs) are bound to each other.

In addition, the present invention encompasses nucleotide sequences derived from the nucleotide sequences listed in table 1 by addition, deletion, or substitution of one to several nucleotides. In addition, based on such nucleotide variation, the present invention also encompasses amino acid sequences derived from the amino acid sequences listed in table 1 by addition, deletion, or substitution of one to several amino acid residues.

Further, the term "serum antibody" means an IgG antibody that exists in the serum of a solid cancer patient and that binds to a solid cancer antigenic polypeptide. Moreover, the term "antibody" means a polyclonal or monoclonal antibody prepared by using a solid cancer antigenic polypeptide or a partial fragment thereof as an immunogen.

Other terms and concepts used in the present invention will be defined in more detail in the following embodiments and Examples. In addition, based on known literature and the like, persons skilled in the art would be readily and reliably able to use various techniques so as to carry out the present invention, except for techniques that are clearly described herein with references. For instance, preparation of pharmaceuticals for the preparation of the medicaments of the present invention is described in Remington's Pharmaceutical Sciences, 18th Edition, ed. A. Gennaro, Mack Publishing Co., Easton, PA, 1990. Also, genetic engineering techniques and molecular biological techniques used in the present invention are described in, for example, Sambrook and Maniatis, Molecular Cloning-A Laboratory Manual, Cold Spring Harbor Laboratory Press, New York, 1989 and Ausubel, F. M. et al., Current Protocols in Molecular Biology, John Wiley & Sons, New York, N.Y, 1995.

The term "solid cancer antigenic polypeptide" refers to a polypeptide expressed by any of the 39 genes or ESTs (Expressed Sequence Tags) shown in table 1. These gene products are known to have various functions; however, their specific expression in solid cancer has not been known. Herein, in accordance with the present invention, an expression product of an EST is defined as a "polypeptide encoded by a polynucleotide comprising the nucleotide sequence set forth in SEQ ID NO: 45, 46, or 57."

Moreover, as described in Examples below, genes and peptides set forth in SEQ ID NOS: 1 to 40 are identified via two-dimensional electrophoresis, and genes, ESTs, and peptides set forth in SEQ ID NOS: 41 to 75 are identified via the SEREX method.

### 2. Diagnostic kits for solid cancer

As described above, solid cancer antigenic polypeptides shown in table 1 are specifically expressed in human solid cancer. Thus, it becomes possible to diagnose a subject as having human solid cancer by detecting the expression of such a solid cancer antigenic polypeptide in a sample derived from the subject.

A diagnostic kit for solid cancer of the present invention (hereafter sometimes referred to as "the present diagnostic kit for solid cancer") includes a means of detecting the expression of at least one human solid cancer antigenic polypeptide in a sample derived from a subject.

The present diagnostic kit for solid cancer is a reagent kit used to make a diagnosis of solid cancer. A variety of such kits are commercially available in accordance with types of test components. Also, the present diagnostic kit for solid cancer may comprise components used in publicly known kits, except that a means of detecting the expression of a human solid cancer antigenic polypeptide is used (e.g., solid cancer antigenic polypeptides, antibodies, primers, and probes).

Further, with the use of the present diagnostic kit for solid cancer, it is possible to diagnose a subject as having solid cancer. Examples of solid cancer include, but are not limited to, colorectal cancer, esophageal cancer, gastric cancer, lung cancer, kidney cancer, thyroid cancer, parotid gland cancer, head and neck cancer, soft tissue and bone sarcoma, urinary tract cancer, bladder cancer, uterine cancer, liver cancer, breast cancer, ovarian cancer, and fallopian tube cancer. Preferably, the kit can be used for diagnosis of colorectal cancer, esophageal cancer, gastric cancer, or breast cancer.

Herein, examples of a means of detecting the expression of a solid cancer antigenic polypeptide, the protein expression thereof, the antibody expression thereof, and the gene expression thereof include:
(1) an antibody against the solid cancer antigenic polypeptide;
(2) a solid cancer antigenic polypeptide; and
(3) a probe or primer designed based on a polynucleotide encoding the solid cancer antigenic polypeptide.
Hereafter, these means will be described in detail.

### (1) Antibody against a solid cancer antigenic polypeptide

An antibody against a solid cancer antigenic polypeptide is able to bind to a solid cancer antigenic polypeptide expressed in cancer. Thus, by detecting the reaction between the antibody and a solid cancer antigenic polypeptide in a sample, it is possible to diagnose whether or not the sample is derived from a cancer patient or a subject at high risk of developing solid cancer.

An antibody against a solid cancer antigenic polypeptide can be a polyclonal or monoclonal antibody. Examples thereof include an entire molecule and Fab, F(ab')₂, and Fv fragments that can bind to the epitope of a solid cancer antigenic polypeptide. For instance, in the case of a polyclonal antibody, such antibody can be obtained by immunizing an animal with an immunogen such as an antigenic polypeptide or a partial fragment thereof and recovering from the serum of the animal. Alternatively, such antibody can be prepared by administering the expression vector for eukaryotic cells described above into the muscle or skin of an animal via injection or a gene gun, and collecting the serum. Examples of such animal include mice, rats, rabbits, goats, and chickens.

Further, the monoclonal antibody can be produced in accordance with a known method for producing monoclonal antibodies ("Monoclonal Antibody (Tan-Clone Kotai)," cowritten by Hideaki Nagamune and Hiroshi Terada, Hirokawa Shoten, 1990; "Monoclonal Antibody" James W. Goding, third edition, Academic Press, 1996).

Furthermore, an antibody against a solid cancer antigenic polypeptide can be an antibody labeled with a labeling substance. The details of such labeled antibody are described above.

When the expression of a solid cancer antigenic polypeptide in a sample derived from a subject is detected using an antibody against a solid cancer antigenic polypeptide and diagnosis of human solid cancer is carried out, the sample derived from the subject is examined for the presence or absence of an antibody against a solid cancer antigenic polypeptide or an antigenic polypeptide that binds to a labeled antibody thereof. If the antigenic polypeptide is detected in the sample, the subject is determined to be a solid cancer patient or a subject at high risk of developing solid cancer. That is, the antibody or labeled antibody used herein specifically binds to an antigenic polypeptide expressed in a solid cancer cell. Thus, a sample comprising an antigenic polypeptide that is bound to an antibody can be determined as a sample of a solid cancer patient or a subject at high risk of developing solid cancer. In such case, preferably 2 or more, more preferably 5 or more, even more preferably 10 or more, most preferably 15 to 39 antibodies are examined in terms of binding with antigenic polypeptides in a sample. Such sample to be used is not particularly limited as long as a solid cancer antigenic polypeptide is expressed therein. Examples thereof include blood, hemocytes (e.g., mononuclear cells), and tissue.

In another embodiment, a method wherein an antibody and an antigenic polypeptide are allowed to bind to each other in a liquid phase system is used. For instance, a labeled antibody is allowed to come into contact with a sample such that the labeled antibody and an antigenic polypeptide bind to each other. Then, such conjugate is separated such that a signal from the label is detected in a manner similar to that described above.

In another method of diagnosis in a liquid phase system, an antibody (primary antibody) against a solid cancer antigenic polypeptide is allowed to come into contact with a sample such that a primary antibody and the antigenic polypeptide bind to each other. Then, a labeled antibody (secondary antibody) is allowed to bind to the thus obtained conjugate such that the signal from the label of the conjugate comprising the three components is detected. Alternatively, in order to intensify the signal, an unlabeled secondary antibody is allowed to bind to a conjugate of an antibody and an antigenic polypeptide such that a labeling substance may be allowed to bind to the secondary antibody. For instance, such binding of a labeling substance to a secondary antibody can be carried out in a manner such that a secondary antibody and a labeling substance are preliminarily biotinylated and avidinylated, respectively. Also, an antibody (tertiary antibody) that recognizes a partial region of a secondary antibody (e.g., Fc region) is used for labeling so that the tertiary antibody is allowed to bind to the secondary antibody. In addition, as a primary antibody and a secondary antibody, a monoclonal antibody can be used. Alternatively, either the primary antibody or the secondary antibody may be a polyclonal antibody. Separation of such conjugate from a liquid phase and signal detection can be carried out in a manner similar to that described above.

In another embodiment, a method for examining binding between an antibody and an antigenic polypeptide in a solid phase system is used. The method involving a solid phase system is preferable in terms of detection of minute amounts of antigenic polypeptides and facilitation of operations. That is, in accordance with the method involving a solid phase system, antibodies (primary antibodies) against solid cancer antigenic polypeptides are immobilized on a solid phase (e.g., a resin plate, a membrane, or beads), antigenic polypeptides are allowed to bind to the thus immobilized antibodies, unbound peptides are removed by washing, labeled antibodies (secondary antibodies) are allowed to bind to conjugates of the antibody and the antigenic polypeptide remaining on the plate, for example, and signals of the secondary antibodies are detected. This method is a so-called "sandwich method." Such method is widely used as "ELISA (enzyme linked immunosorbent assay)" using an enzyme as a marker. In the method, the both primary antibody and the secondary antibody may be monoclonal antibodies. Alternatively, either the primary antibody or the secondary antibody may be a polyclonal antibody. Signal detection can be carried out in a manner similar to that described above.

### (2) Solid cancer antigenic polypeptide

Since solid cancer antigenic polypeptides are expressed by cancer cells, the serum of a patient having cancer contains an antibody (serum antibody) against an expressed solid cancer antigenic polypeptide. Thus, by examining the reaction between a solid cancer antigenic polypeptide and a serum antibody, the expression of a solid cancer antigenic polypeptide in a subject can be detected. Examples of such solid cancer antigenic polypeptide that can be used include the antigenic polypeptides shown in table 1 and partial peptides thereof. Herein, the term "antigenic polypeptide" means the antigenic polypeptides shown in table 1, and also partial peptides of the antigenic polypeptides that comprise at least 6 amino acids, preferably 6 to 500 amino acids, and more preferably 8 to 50 amino acids.

These antigenic polypeptides can be prepared by preparing RNA via *in vitro* transcription from a recombinant expression vector comprising a polynucleotide having a nucleotide sequence listed in table 1, for example, carrying out *in vitro* translation using the RNA as a template, and expressing an antigenic peptide *in vitro.* Alternatively, when a recombinant expression vector is introduced into a prokaryotic cell such as *E coli.* or *Bacillus subtilis,* or a eukaryotic cell such as yeast, an insect cell, or a mammalian cell such that a transformed cell is produced, it is possible to allow such transformed cell to express an antigenic polypeptide.

When an antigenic polypeptide is expressed via *in vitro* translation, a polynucleotide encoding an antigenic polypeptide is inserted into a vector comprising an RNA polymerase promoter to construct a recombinant expression vector. Then, the obtained vector is added to an *in vitro* translation system such as a rabbit reticulocyte lysate or a wheat germ extract that contains an RNA polymerase corresponding to the promoter. Thus, an antigenic polypeptide can be produced *in vitro.* Examples of an RNA polymerase promoter include T7, T3, and SP6. Examples of a vector comprising such RNA polymerase promoter include pKA1, pCDM8, pT3/T7 18, pT7/3 19, and pBluescript II.

When an antigenic polypeptide is allowed to be expressed in an organism such as *E coli.,* an expression vector is produced by ligating a polynucleotide to a vector having a replication origin that is replicable in a microorganism, a promoter, a ribosome binding site, a DNA cloning site, and a terminator. A host cell is then transformed with the expression vector, and the thus obtained transformant is subjected to culture. Thus, an antigenic polypeptide encoded by the polynucleotide can be expressed using microorganisms. In such case, it is also possible to express an antigenic polypeptide as a fusion protein with another protein. Examples of an *E coli.* expression vector include pUC, pBluescript II, a pET expression system, and a pGEX expression system.

When an antigenic polypeptide is allowed to be expressed in a eukaryotic cell, a recombinant vector is produced by inserting a polynucleotide encoding an antigenic polypeptide into a eukaryotic cell expression vector having a promoter, a splicing region, a poly(A) addition site, and the like. Then, the recombinant vector is introduced into a eukaryotic cell. Accordingly, the antigenic polypeptide can be expressed in the transformed eukaryotic cell. Examples of such expression vector include pKA1, pCDM8, pSVK3, pMSG, pSVL, pBK-CMV, pBK-RSV, EBV vector, pRS, pcDNA3, pMSG, and pYES2. In addition, when pIND/V5-His, pFLAG-CMV-2, pEGFP-N1, pEGFP-C1, and the like are used as expression vectors, it is also possible to express an antigenic polypeptide as a fusion protein to which various types of tags such as a His tag, a FLAG tag, a myc tag, an HA tag, and GFP have been added. Examples of such eukaryotic cell that is generally used include cultured mammalian cells such as monkey kidney cells COS7 and Chinese hamster ovary (CHO) cells, budding yeast, fission yeast, silkworm cells, and Xenopus oocytes. In addition, any eukaryotic cell may be used as long as it can express an antigenic polypeptide. When an expression vector is introduced into a eukaryotic cell, known methods such as an electroporation method, a calcium phosphate method, a liposome method, and a DEAE-dextran method may be used.

When isolation and purification of an antigenic polypeptide of interest from a culture product is carried out following expression of the antigenic polypeptide using prokaryotic cells or eukaryotic cells, known separation techniques can be used in combination. Examples thereof include treatment using a denaturing agent such as urea or a surfactant, ultrasonication, enzymatic digestion, salting-out or solvent precipitation, dialysis, centrifugation, ultrafiltration, gel filtration, SDS-PAGE, isoelectric focusing, ion exchange chromatography, hydrophobic chromatography, affinity chromatography, and reversed phase chromatography.

In addition, recombinant antigenic polypeptides obtained via the methods described above include a fusion protein of such polypeptide and any other protein. For instance, such fusion protein comprises glutathione S-transferase (GST) or green-fluorescent protein (GFP). Further, peptides expressed in transformed cells are sometimes subjected to various forms of post-translational modification in the cells. Thus, modified peptides can be used as antigenic polypeptides. Examples of such post-translational modification include removal of N-terminal methionine, N-terminal acetylation, glycosylation, partial hydrolysis due to intracellular protease, myristoylation, isoprenylation, and phosphorylation.

In order to detect the expression of a solid cancer antigenic polypeptide in a sample derived from a subject using solid cancer antigenic polypeptides, the presence or absence of at least one serum antibody in a sample of a subject that binds to a solid cancer antigenic polypeptide is examined. If the serum antibody is found in the serum of the subject, the subject is determined to be a solid cancer patient or a subject at high risk of developing solid cancer. Specifically, solid cancer antigenic polypeptides bind to serum antibodies (IgG) derived from a solid cancer patient. Thus, when such polypeptides are allowed to react with the serum of a subject, the serum sample is found to contain serum antibodies that are bound to the polypeptides, the sample can be determined to be derived from a solid cancer patient or a subject at high risk of developing solid cancer. In such case, 2 or more, preferably 5 or more, more preferably 10 or more, and most preferably 15 to 39 antigenic polypeptides are examined in terms of binding with antibodies. Further, other known solid cancer markers (e.g., CEA, Cyfra, and SCC-Ag) can be used in combination. Moreover, the sample may be a serum antibody-containing sample, which in turn indicates serum.

Specifically, diagnosis using the present diagnostic kit for solid cancer is carried out by allowing serum of a subject to come into contact with, for example, solid cancer antigenic polypeptides in the diagnostic kit for solid cancer so as to allow the solid cancer antigenic polypeptides to react with IgG antibodies in the serum of the subject in a liquid phase. Further, labeled IgG antibodies that specifically bind to serum IgG antibodies are subjected to reaction such that signals of the labeled IgG antibodies can be detected. Examples of labels used in labeled antibodies include enzymes, radioisotopes, and fluorescent dyes. Such enzymes are not particularly limited as long as they satisfy conditions such as a large turnover number, stability even while being bound to antibodies, and capacity to cause color development specifically to a substrate. Examples of the enzymes that can be used include enzymes used in usual enzyme immunoassay (EIA) such as peroxidase, β-galactosidase, alkaline phosphatase, glucose oxidase, acetylcholine esterase, glucose-6-phosphodehydrogenase, and malate dehydrogenase. Also, enzyme inhibitors, coenzymes, and the like can be used. Binding between these enzymes and antibodies can be carried out by known methods using crosslinkers such as maleimide compounds. As the substrate, known substances can be used depending on the type of enzyme used. For instance, when peroxidase is used as the enzyme, 3,3',5,5'-tetramethylbenzene can be used, and when alkaline phosphatase is used as the enzyme, paranitrophenol or the like can be used.

When such enzyme is used, enzyme activity is determined by adding a substrate that causes color development as a result of degradation via enzymatic action so as to optically measure the amount of the substrate degraded. Then, the obtained enzyme activity is converted into an amount of binding antibody such that the amount of antibody is calculated based on comparison with a standard.

Examples of radioisotopes that can be used include ¹²⁵I and ³H, which are used in general radioimmunoassay (RIA). When such a radioisotope is used, the radiation dose of the radioisotope is measured using a scintillation counter or the like.

Examples of fluorescent dyes that can be used include fluorescein isothiocyanate (FITC) and tetramethylrhodamine isothiocyanate (TRITC), which are used in general fluorescent antibody methods. When such a fluorescent dye is used, the fluorescence level may be measured using a measuring apparatus equipped with a fluorescence microscope.

Further, examples of labeled antibodies include antibodies to which metals such as manganese and iron are bound. By carrying out *in vivo* administration of such antibodies to which metals are bound so as to measure the metals via MRI or the like, the presence of serum antibodies, or the expression of solid cancer antigenic polypeptides, can be detected.

Signal detection can be carried out by employing Western blotting analysis, for example. Alternatively, a conjugate of an antigenic polypeptide, a serum antibody, and a labeled IgG antibody is separated by a known separation means (e.g., chromatography, a salting out, an alcohol precipitation, an enzymatic method, or a solid phase method) such that signals of the labeled IgG antibody may be detected.

In addition, at least one antigenic polypeptide is immobilized on a solid phase (e.g., plates, membranes, and beads) such that binding between serum antibodies of a subject and the antigenic polypeptides can be examined on the solid phase. By immobilizing antigenic polypeptides on a solid phase, it becomes possible to readily remove unbound labeled binding molecules. In particular, in accordance with a protein array method wherein a membrane on which several tens of different types of antigenic polypeptides are immobilized is used, different types of a plurality of antibodies can be analyzed in terms of expression in a short time using about 0.01 ml of the serum of a subject.

### (3) Primers or probes

The present diagnostic kit for solid cancer may comprise a primer or probe that comprises polynucleotide consisting of the entire or a partial sequence of a polynucleotide encoding a solid cancer antigenic polypeptide shown in table 1 or a complementary sequence thereof. The primer or probe specifically binds to mRNA of the antigenic polypeptide expressed in the sample of a subject or cDNA synthesized from the mRNA. Thus, it is possible to detect the expression of a gene encoding an antigenic polypeptide in a sample, which in turn indicates the expression of the antigenic polypeptide.

Such primer and probe can be designed in accordance with techniques known by persons skilled in the art based on nucleotide sequences of polynucleotides encoding antigenic polypeptides, which are set forth in SEQ ID NOS:1, 3, 5, 7, 9, 11, 13, 15, 17, 19, 21, 23, 25, 27, 29, 31, 33, 35, 37, 39, 41, 43, 45, 46, 47, 49, 51, 53, 55, 57, 58, 60, 62, 64, 66, 68, 70, 72, and 74. The following remarks relate to the designing of such primer and probe.

The length of the primer that substantially have its function is preferably 10 or more nucleotides, more preferably 16 to 50 nucleotides, and even more preferably 20 to 30 nucleotides. In addition, the length of the probe that substantially have its function is preferably 10 or more nucleotides, more preferably 16 to 50 nucleotides, and even more preferably 20 to 30 nucleotides.

Upon design, it is preferable to confirm the melting temperature of a primer or probe (Tm). The "Tm" means the temperature at which 50% of an arbitrary polynucleotide chain forms a hybrid with the complementary strand thereof. When template DNA or RNA and a primer or probe anneal or hybridize by forming a duplex, the temperature for annealing or hybridization must be optimized. Meanwhile, when the temperature is excessively decreased, nonspecific reaction takes place. Thus, the temperature is preferably as high as possible. Therefore, the Tm of the primer or probe to be designed is an important factor when carrying out amplification reaction or hybridization. The Tm can be confirmed with the use of known software for designing a primer or probe. Examples of such software that can be used in the present invention include Oligo™ (National Bioscience Inc., (U.S.A.)) and GENETYX (Software Development Co., Ltd. (Japan)). In addition, the Tm can be confirmed by manual calculation without using such software. In such case, calculating formulae based on the nearest neighbor base pair model (nearest neighbor method), the Wallace method, the GC% method, and the like can be used. In accordance with the present invention, the average Tm is preferably about 45°C to 55°C.

Another example of conditions under which a primer or probe can be specifically anneals or hybridizes involves GC content or the like. Such condition has been known by persons skilled in the art.

The primer and the probe designed as described above can be prepared in accordance with methods known by persons skilled in the art. Further, as known by persons skilled in the art, such primer or probe may contain a sequence such as an additional sequence known as a tag sequence in addition to the sequence for annealing or hybridization. Also, the aforementioned primer or probe to which such additional sequence has been added is within the scope of the present invention.

When expression of solid cancer antigenic polypeptides in a sample derived from a subject is detected, the aforementioned primer and/or probe are used in an amplification reaction or a hybridization reaction, such that the amplification product or hybridization product thereof is detected.

Examples of such sample of interest include feces, blood, and hemocytes (e.g., mononuclear cells). In addition, when carrying out an amplification reaction or a hybridization reaction, a nucleic acid to be tested is prepared from a sample derived from a subject, in general. The test nucleic acid may be DNA or RNA, as long as it is nucleic acid. DNA or RNA can be extracted using methods known in the art according to need. For instance, upon DNA extraction, a method of carrying out phenol extraction and ethanol precipitation and a method using glass beads can be used. Also, upon RNA extraction, a guanidine-cesium chloride ultracentrifugation method, a hot phenol method, an acid guanidinium thiocyanate-phenol-chloroform (AGPC) method, or the like can be used. With the use of a sample or test nucleic acid prepared as described above, an amplification reaction and/or a hybridization reaction described below is carried out.

Expression of a solid cancer antigenic polypeptide in a sample can be detected by carrying out amplification with the use of a primer and test nucleic acid as a template and detecting the specific amplification reaction.

Amplification methods are not particularly limited. However, examples thereof include known methods utilizing principles of polymerase chain reaction (PCR) methods such as PCR, LAMP (loop-mediated isothermal amplification), ICAN (isothermal and chimeric primer-initiated amplification of nucleic acids), RCA (rolling circle amplification), LCR (ligase chain reaction), and SDA (strand displacement amplification). Amplification is carried out until an amplification product can be detected.

For instance, upon PCR, a nucleotide sequence between a pair of primers is synthesized based on a template, DNA to be tested, using DNA polymerase. Upon PCR, amplified fragments can be exponentially amplified with the repetition of a cycle consisting of denaturation, annealing, and synthesis. Optimal conditions for PCR can readily be determined by persons skilled in the art.

In addition, upon RT-PCR, cDNA is produced via reverse transcriptase reaction using RNA to be tested as a template, followed by PCR using a pair of primers and the thus prepared cDNA that serves as a template.

In addition, with the use of amplification techniques such as quantitative PCR including competitive PCR and real-time PCR, quantitative detection can be achieved.

In order to detect whether or not the aforementioned amplification reaction is a specific amplification reaction, known methods whereby amplification products obtained via an amplification reaction can be specifically recognized can be used. For instance, a specific amplification reaction can be detected using agarose gel electrophoresis whereby amplification of amplified fragments in given sizes is confirmed.

Alternatively, labels such as radioisotopes, fluorescent substances, or luminescent substances are allowed to act on dNTP that is incorporated during an amplification reaction such that such label can be detected. Examples of radioisotopes that can be used include ³²P, ¹²⁵I, and ³⁵S. Examples of fluorescent substances that can be used include fluorescein (FITC), sulforhodamine (SR), and tetramethylrhodamine (TRITC). In addition, examples of luminescent substances that can be used include luciferin.

For instance, the label type and the method for introducing a label are not particularly limited. Thus, conventionally known methods can be used. Examples of the method for introducing a label include a random prime method using a radioisotope.

As a method for observing an amplification product into which labeled dNTP has been incorporated, any methods for detecting aforementioned labels known in the art can be used. For instance, when a radioisotope is used as a label, the radioactivity can be measured using a liquid scintillation counter, a γ-counter, or the like. When fluorescence is used as a label, the fluorescence can be detected using a fluorescence microscope, a fluorescent plate reader, or the like.

When a specific amplification reaction is detected as described above, a gene encoding a solid cancer antigenic polypeptide is expressed in a sample, which in turn indicates the expression of a solid cancer antigenic polypeptide. Thus, a subject whose sample shows antigenic polypeptide expression is determined to be a solid cancer patient or subject at high risk of developing solid cancer.

In addition, a sample or test nucleic acid is subjected to a hybridization reaction using a probe, followed by detection of the specific binding (hybrid). Thus, the expression of solid cancer antigenic polypeptides can be detected.

The hybridization reaction must be carried out under stringent conditions that allow a probe to specifically bind to a polynucleotide derived from a solid cancer antigenic polypeptide. Such stringent conditions have been known in the art and thus are not particularly limited. The stringent conditions include a sodium concentration of 10 to 300 mM and preferably 20 to 100 mM and a temperature of 25°C to 70°C and preferably 42°C to 55°C.

When carrying out hybridization, it is possible to add appropriate labels such as fluorescence labels (e.g., fluorescein and rhodamine), radioactive labels (e.g., ³²P), enzyme labels (e.g., alkaline phosphatase and horseradish peroxidase), and biotin labels to a probe. Thus, the present diagnostic kit for solid cancer comprises probes to which labels as descried above have been attached.

Detection using labeled probes includes allowing a sample or test nucleic acid that has been prepared from the sample to come into contact with a probe so as to achieve hybridization. The expression "so as to achieve hybridization" indicates that detection is carried out in an environment (temperature and salt concentration) in which specific binding occurs under the stringent conditions described above. Specifically, a sample or test nucleic acid is immobilized on an adequate solid phase such as a slide glass, a membrane, or a microtiter. Then, labeled probes are added thereto. Thus, a sample or test nucleic acid is allowed to come into contact with the probes such that hybridization reaction is carried out, followed by removal of the nonhybridized probes. Accordingly, the label of the probe that has hybridized with the sample or test nucleic acid is detected. Detection of the label indicates that solid cancer antigenic polypeptides are expressed in the sample. Therefore, a subject whose sample shows expression of an antigenic polypeptide is diagnosed as being a solid cancer patient or a subject at high risk of developing solid cancer.

In addition, when the concentration of the label is determined to be an index, it is also possible to carry out quantitative detection. Examples of a detection method using labeled probes include Southern hybridization, Northern hybridization, and FISH (fluorescent *in situ* hybridization).

Further, when the present diagnostic kit for solid cancer is used to make a diagnosis, the expression level of solid cancer antigenic polypeptides in a sample derived from a subject is measured. If the expression level of at least one solid cancer antigenic polypeptide is higher than that of a healthy individual, the subject is determined to be a solid cancer patient or subject at high risk of developing solid cancer. In terms of specific criteria for the expression level of a solid cancer antigenic polypeptide, the expression level of a subject is 10% or more, preferably 30% or more, more preferably 70% or more, and most preferably 100% or more higher than the expression level of a healthy individual.

### 3. Medicament for preventing or treating solid cancer

### 3.1. Inhibition of functions or expression of solid cancer antigenic polypeptide

Solid cancer antigenic polypeptides are specifically expressed in solid cancer. Thus, such expression very probably causes malignant cell transformation. Therefore, it is expected that therapeutic effects against malignant cell transformation or progress in such transformation can be obtained by inhibiting the functions or the expression of a solid cancer antigenic polypeptide.

Accordingly, a means of inhibiting the functions and the expression of at least one human solid cancer antigenic polypeptides described above is effective as a medicament for preventing and/or treating solid cancer.

Examples of such means of inhibiting the functions or the expression of a human solid cancer antigenic polypeptide include:
(1) an antibody against the solid cancer antigenic polypeptide;
(2) a means capable of inhibiting transcription of a gene encoding the solid cancer antigenic polypeptide; and
(3) a means capable of inhibiting translation of a gene encoding the solid cancer antigenic polypeptide.

### (1) Antibodies against solid cancer antigenic polypeptides

Antibodies against solid cancer antigenic polypeptides can inhibit activities of the antigenic polypeptides by specifically binding to solid cancer antigenic polypeptides in a subject. Thus, medicaments comprising antibodies against solid cancer antigenic polypeptides are effective for prevention or therapy of solid cancer.

### (2) Means capable of inhibiting transcription of a gene encoding a solid cancer antigenic polypeptide

Examples of a means of inhibiting transcription of a gene encoding a solid cancer antigenic polypeptide include expression vectors that can be used for substitution of transcriptional promoter regions of the genes in a subject with transcriptional repression-type promoters. In addition, as a means of inhibiting transcription of a gene encoding a solid cancer antigenic polypeptide, expression vectors may be used, with which a nucleotide sequence having transcriptional repression activity is inserted into a region involving translation of the gene. Design and preparation of such expression vectors have been known to persons skilled in the art.

### (3) Means capable of inhibiting translation of a gene encoding a solid cancer antigenic polypeptide

In addition, examples of a means of inhibiting translation of a gene encoding a solid cancer antigenic polypeptide include a method using a so-called antisense RNA. Specifically, a nucleic acid transcribing antisense RNA corresponding to mRNA of a gene is introduced as a plasmid or incorporated into a genome of a subject such that the antisense RNA is allowed to be overexpressed, resulting in inhibition of translation of mRNA of a gene encoding a solid cancer antigenic polypeptide. Techniques related to antisense RNA have been known to be used in cases where mammals and the like are used as hosts (Han et al. (1991) Proc. Natl. Acad. Sci. USA, 88, 4313-4317; Hackett et al. (2000) Plant Physiol., 124, 1079-86).

Further, it is also possible to utilize RNA interference so as to inhibit translation of a gene encoding a solid cancer antigenic polypeptide. Specifically, double-stranded RNA complementary to a nucleotide sequence of a gene encoding a target solid cancer antigenic polypeptide is introduced into a cell such that mRNA of an endogenous gene encoding a solid cancer antigenic polypeptide is degraded, resulting in specific inhibition of gene expression in the cell. Such technique has been found available in the cases of mammalian cells and the like (Hannon, GJ., Nature (2002) 418, 244-251 (review); JP Patent Publication No. 2002-516062 A; JP Patent Publication No. 8-506734 A (1996)).

### 3.2. Targeting to solid cancer

Since solid cancer antigenic polypeptides are specifically expressed in solid cancer, it becomes possible to allow therapeutic agents for solid cancer to act on cancer lesions with the use of a means of targeting to solid cancer based on such specific expression.

Thus, the means of targeting to solid cancer described above is also effective as a medicament for preventing and/or treating solid cancer. The medicament for preventing and/or treating solid cancer of the present invention includes a gene encoding a prophylactic or therapeutic agent for solid cancer and a means of targeting to human solid cancer.

Examples of such means of targeting to human solid cancer include:
(1) an antibody against a solid cancer antigenic polypeptide; and
(2) a nucleotide sequence of an expression control region of a polynucleotide encoding a solid cancer antigenic polypeptide.

### (1) Antibodies against solid cancer antigenic polypeptides

Antibodies against solid cancer antigenic polypeptides bind to the antigenic polypeptides that are specifically expressed in cancer cells. Thus, when known agents for solid cancer therapy (e.g., anticancer drugs or immunopotentiating agents) are linked to such antibodies so as to be administered to patients, it is possible to allow the agents for solid cancer therapy to specifically act on cancer cells.

### (2) Nucleotide sequences of expression control regions of polynucleotides encoding solid cancer antigenic polypeptides

Expression control regions of polynucleotides encoding solid cancer antigenic polypeptides (hereafter to be referred to as "promoter sequence(s)") are expression control regions of genes that are specifically expressed in solid cancer cells. Thus, when therapeutic genes are produced by ligating polynucleotides encoding agents for solid cancer therapy to a promoter sequence and are administered *in vivo,* it becomes possible to allow such therapeutic genes to be expressed in a cancer cell-specific manner. Examples of polynucleotides encoding substances having an anticancer effect or precursors thereof that can be used include DNA and cDNA of genes encoding p53, herpes simplex virus thymidine kinase, interleukin-2, -12, -17, -18, cytosine deaminase, uracil phosphoribosyltransferase, and the like. Moreover, such promoter sequences can be used in a therapeutic method wherein adenovirus and herpes virus are proliferated in a cancer-cell-specific manner such that cancer cells are degraded. That is, by inserting promoter sequence before the adenovirus E1A region, such adenovirus is allowed to specifically proliferate exclusively in cancer cells such that cancer cells are degraded.

### 3.3. Application and administration of medicament

Examples of solid cancer to which the medicament of the present invention is applied include, but are not limited to, colorectal cancer, esophageal cancer, gastric cancer, lung cancer, kidney cancer, thyroid cancer, parotid gland cancer, head and neck cancer, soft tissue and bone sarcoma, urinary tract cancer, bladder cancer, uterine cancer, liver cancer, breast cancer, ovarian cancer, and fallopian tube cancer. More specifically, such solid cancer is colorectal cancer, esophageal cancer, gastric cancer, or breast cancer.

It is possible to administer the medicament of the present invention for the purpose of preventing development of the solid cancer described above or preventing deterioration in terms of symptoms or alleviating symptoms of a patient having solid cancer or a patient who has been diagnosed as being at high risk of developing solid cancer.

When the aforementioned means is used as a medicament for treating or preventing solid cancer, it can be mixed with pharmaceutically acceptable carriers so as to be used as a pharmaceutical composition. In such case, an active ingredient of such medicament may be adequately adjusted so as to account for 1% to 90% of the carrier content.

Examples of administration routes of the medicament of the present invention include systemic administration such as intravenous or intra-arterial administration, in general. Further, it is preferable to carry out local administration such as local injection to primary cancer lesions or metastatic lesions that can be predicted based on the cancer type.

The dose of the medicament of the present invention varies depending on age, sex, symptoms, administration routes, administration frequencies, and formulations. These may be adequately adjusted by persons skilled in the art or physicians.

The present invention will be hereafter described in detail with reference to the following examples, although the scope of the present invention is not limited thereto.

### [Example 1] Identification of colorectal cancer antigenic polypeptides via two-dimensional electrophoresis

### [1] Material and methods

With the consent of patients (6 cases), frozen specimens of cancer tissue and non-cancer tissue of each patient were separately collected immediately after colorectal cancer extraction. These frozen specimens were kept at -80°C. An adequate amount of each frozen specimen was homogenized with a solution containing 9.5 M Urea, 2% CHAPS, 1% DTT, and complete protease inhibitor cocktail tablets (Roche). The resultant was centrifuged using an ultracentrifuge (Hitachi) at 100,000 g, followed by extraction of the supernatant (protein solution) thereof. Then, protein concentration was determined based on absorbance.

Proteins obtained from cancer tissue and non-cancer tissue (400 µg each) were subjected to two-dimensional electrophoresis for separation. The first-dimensional electrophoresis and the second-dimensional electrophoresis were carried out via agarose isoelectric focusing electrophoresis and 12% or 6% to 10% Tris/Glycine SDS polyacrylamide gel electrophoresis, respectively. The thus separated proteins were stained with Coomassie Brilliant Blue R250, followed by detection of spots indicating an increased protein expression level in cancer tissue compared with that in non-cancer tissue. Then, gel containing the spots was excised, followed by digestion of proteins contained in the gel sections using trypsin (Roche). The thus obtained peptides were collected and subjected to amino acid sequence determination using an ion-trap mass spectrometer (LCQ DECA XP, ThermoQuest).

### [2] Results

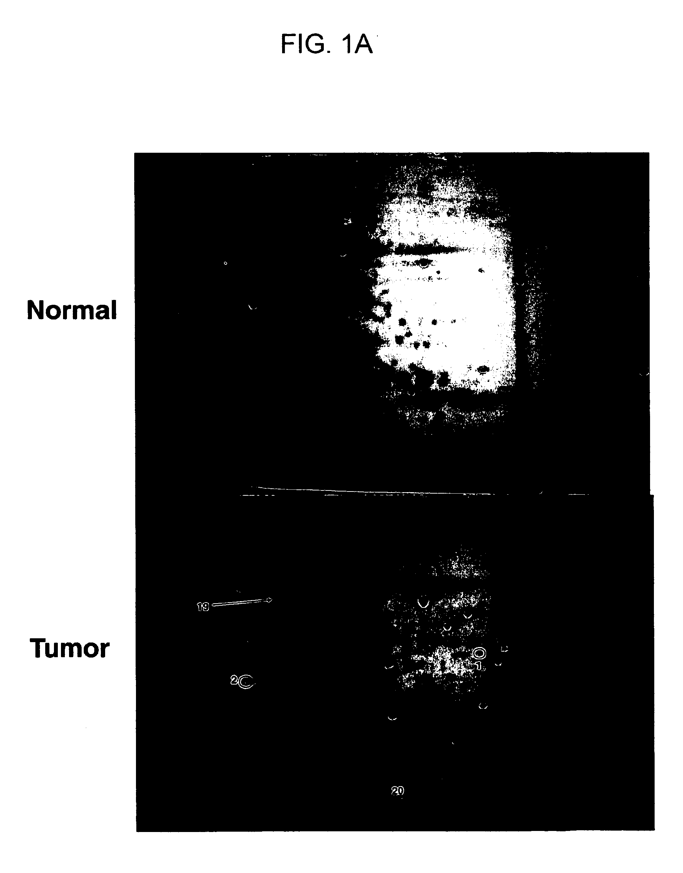

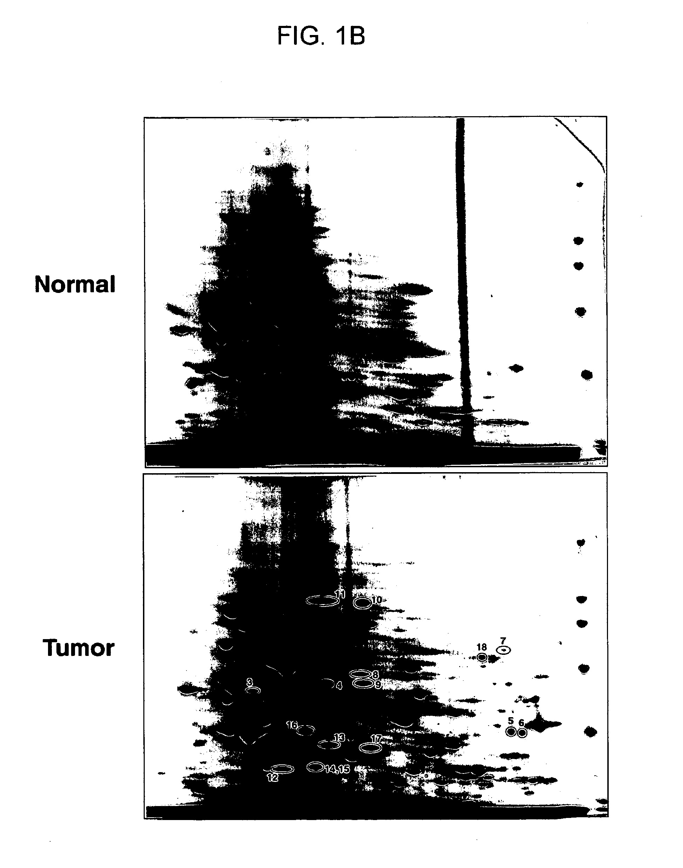

The results are shown in figs. 1A and 1B. The comparison between cancer tissue (Tumor) and normal tissue (Normal) of colorectal cancer patients (6 cases) confirmed specific expression of proteins in the cancer tissue in 4 to 6 cases. The expressed proteins had amino acid sequences set forth in SEQ ID NOS: 2, 4, 6, 8, 10, 12, 14, 16, 18, 20, 22, 24, 26, 28, 30, 32, 34, 36, 38, and 40. In figs 1A and 1B, numbers assigned to encircled proteins correspond to antigenic polypeptides denoted by the numbers of 1 to 20 in table 1.

Nucleotide sequences encoding these proteins are set forth in SEQ ID NOS: 1, 3, 5, 7, 9, 11, 13, 15, 17, 19, 21, 23, 25, 27, 29, 31, 33, 35, 37, and 39, respectively. The respective genes are shown in table 1.

### [Example 2] Identification of solid cancer antigenic polypeptides by a SEREX method

### [1] Construction of cDNA library

A human esophageal-cancer-derived cell line T.Tn was cultured in DMEM medium containing a 10% fetal bovine-serum supplemented with kanamycin (100 µg/ml). Then, total RNA (250 µg) was isolated from these cultured cells via a guanidinium thiocyanate-phenol-chloroform extraction method, followed by 2 repetitions of poly(A) selection using oligo-dT (Oligotex-dT30 super, TAKARA). Thus, mRNA was purified. With the use of the thus obtained mRNA (5.7 µg), a cDNA library of each cell was constructed. Single-stranded cDNA was synthesized using a *Xho*I linker primer and 5-methyl dCTP. Double-stranded cDNA having blunt ends was synthesized using T4 DNA polymerase based on the single-stranded cDNA. To both ends of the double-stranded cDNA, a linker containing restriction enzyme sites (*Eco*RI/λZAPII) was added. The obtained cDNA fragment was inserted into a bacteriophage (Stratagene). Then, a cDNA library of each cancer cell comprising about 1.8 × 10⁶ clones was constructed.

### [2] Screening of cDNA library

Phage vectors of the above-prepared cDNA libraries of cancer cells were allowed to infect *E coli.* XL1-Blue. Then, plaque was formed on an NZY agarose plate. Each infected *E coli.* cell was treated with 10 mM IPTG, resulting in expression induction. Thus, peptides encoded by the various cDNAs were expressed. These peptides were transferred to a nitrocellulose filter (NitroBind, Osmonics). The filter was washed with TBS (0.5% Tween20-containing TBS (10 mM Tris-HCl, 150 mM NaCl; pH 7.5)) such that bacteriophages that had adsorbed thereto were removed. Thereafter, nonspecific reaction was suppressed using TBS-Tween containing 1% albumin. The filter was subjected to reaction with serum of patients with esophageal cancer, gastric cancer, colorectal cancer, and breast cancer at room temperature for 2 hours.

The serum isolated from each patient was kept at -80°C and diluted 500-fold immediately before use with a TBS-Tween solution (TBS-Tween containing 0.5% polyoxyethylene sorbitan monolaurate) that contained 1% by weight albumin. The thus diluted serum was mixed with an *E coli.* lysate at a ratio of 1:5. The resultant was allowed to stand at 4°C for 8 hours, followed by centrifugation at 15,000 g for 20 minutes. Then, the supernatant thereof was recovered and used. In addition, untreated serum was diluted 2000-fold and used according to need.

Each serum was subjected to reaction with the aforementioned nitrocellulose filter on which expressed peptides had been blotted at room temperature for 10 to 20 hours. Thus, polypeptides with which serum antibodies had reacted were identified. Specifically, alkaline-phosphatase-labeled anti human IgG-F (ab')₂ goat antibodies (Jachson) were diluted 5000-fold and used as secondary antibodies during the reaction. Label signals were detected via enzymatic chromogenic reaction using nitroblue tetrazolium (Wako) and 5-bromo-4-chloro-3-indolyl phosphate (Wako). Colonies corresponding to chromogenic-reaction-positive were collected from the agarose plate and dissolved in SM buffer (100 mM NaCl, 10 mM MgSO₄, and 50 mM Tris-HCl; pH 7.5). Until chromogenic-reaction-positive colonies became united, secondary screening and tertiary screening were repeated as described above. Thus, phage clones that reacted with serum IgG of 5 patients were subjected to screening such that positive clones were isolated.

### [3] Identification of novel antigens

With the use of the obtained positive clones, insert DNA was replicated by PCR. The resulting product was subjected to sequencing using a Big Dye DNA Sequencing Kit (ABI) and ABI Prism (Perkin Elmer). As a result of searching based on existing databases, in addition to antigenic polypeptides that were expression products of known cancer-related genes, 19 types of novel antigenic polypeptides that reacted with serum antibodies of a plurality of patients were identified. Table 2 shows antibody prevalences among cancer patients to these novel antigenic polypeptides.

**Table 2**

| No. | Clone name | Name | Registered number | Antibody prevalence (%) | | | | | |
|---|---|---|---|---|---|---|---|---|---|
| | | | | Esophageal cancer patient | Early esophageal cancer patient | Colorectal cancer patient | Gastric cancer patient | Breast cancer patient | Healthy individual |
| 1 | K35-1-1 | Progesterone receptor membrane component 2 | NM_006320 | 10 | 13 | 0 | 20 | 0 | 10 |
| 2 | K30-1-1 | MAP kinase interacting serine/threonine kinase 2 | NM_199054 | 0 | 13 | 10 | 20 | 20 | 5 |
| 3 | 12N3-1 | EST: 601191782F1 | BE264462 | 5 | 0 | 0 | 0 | 0 | 0 |
| 4 | 1201-1 | EST: 602301679F1 | BG032310 | 15 | 0 | 10 | 30 | 0 | 0 |
| 5 | 14A1-1-1 | Additional sex combs-like 1 | NM_015338 | 15 | 13 | 10 | 10 | 0 | 6 |
| 6 | 18G3-1 | Forkhead box A1 | NM_004496 | 10 | 0 | 0 | 0 | 20 | 0 |
| 7 | 19C1-1 | Retinoic acid induced 16 | NM_022749 | 5 | 0 | 0 | 0 | 10 | 0 |
| 8 | 19F1-1 | RIKEN cDNA 5730528L13-like gene | NM_080655 | 5 | 0 | 40 | 10 | 10 | 5 |
| 9 | 19F1-2 | Lysine tRNA synthase | BC004132 | 0 | 13 | 20 | 20 | 20 | 0 |
| 10 | 6BD3-1 | EST: AGENCOURT_15657942 | CF597227 | 14 | | | | | 0 |
| 11 | 14H1-2-1 | KDEL endoplasmic reticulum protein retention receptor 1 | NM_006801 | 10 | | | | | 0 |
| 12 | 18B2-1 | Lysosomal associated protein transmembrane 4 beta | NM_018407 | 10 | | | | | 0 |
| 13 | 18G1-1 | Protein phosphatase 1,catalytic subunit, a isoform | NM_002708 | 10 | | | | | |
| 14 | 20J4-1 | Peroxyredoxin 3 | NM_006793 | 14 | | | | | 0 |
| 15 | 19M2 | Aldo-keto reductase family 1, member C3 | NM_003739 | 38 | | | | | 0 |
| 16 | 10Q3-1 | Ubiquitin-conjugating enzyme E2I | BC000744 | 33 | | | | | 0 |
| 17 | 14A1-1-2 | Phosphatidic acid phosphatase, Type 2C | NM_003712 | 40 | | | | | 21 |
| 18 | 14B1-2-1 | Beta-catenin interacting protein 1 | NM_020248 | 33 | | | | | 17 |
| 19 | 14H2-1-1 | Sorting nexin 15 | NM_147777 | 11 | | | | | 4 |

Polynucleotide (cDNA) sequences that encode amino acids of these 19 types of novel antigenic polypeptides have nucleotide sequences set forth in SEQ ID NOS: 41, 43, 45, 46, 47, 49, 51, 53, 55, 57, 58, 60, 62, 64, 66, 68, 70, 72, and 74, respectively. In addition, nucleotide sequences set forth in SEQ ID NOS: 41, 43, 47, 49, 51, 53, 55, 58, 60, 62, 64, 66, 68, 70, 72, and 74 have amino acid sequences set forth in SEQ ID NOS: 42, 44, 48, 50, 52, 54, 56, 59, 61, 63, 65, 67, 69, 71, 73, and 75, respectively.

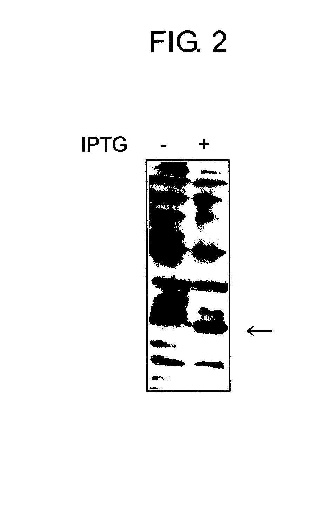

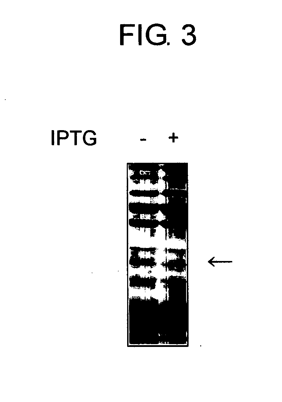

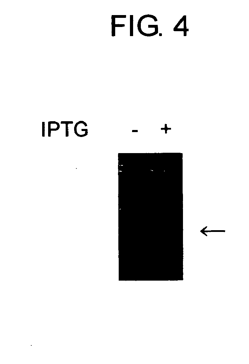

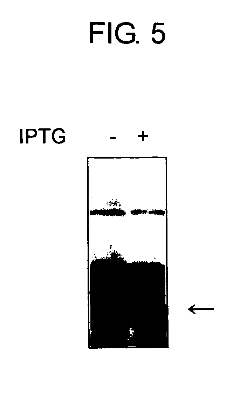

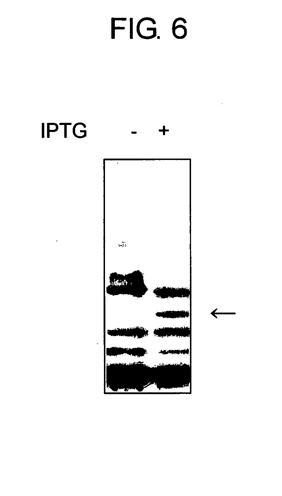

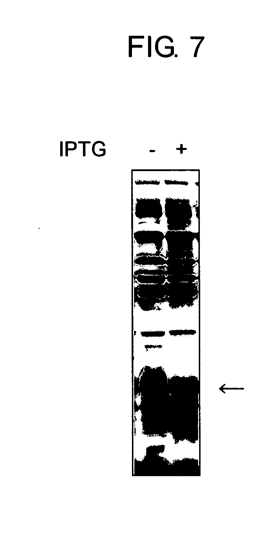

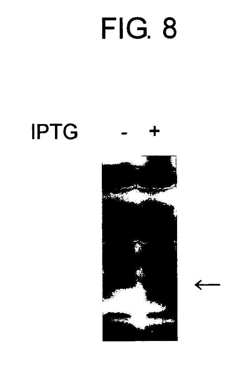

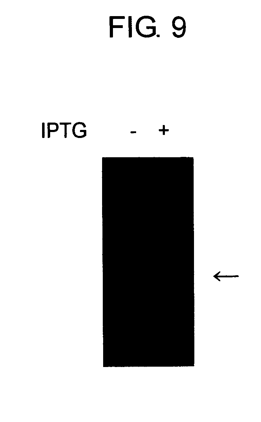

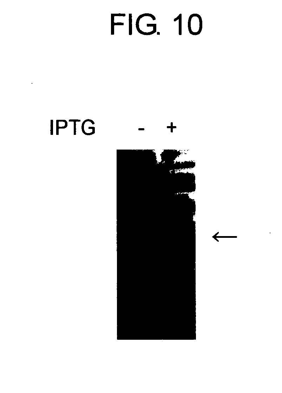

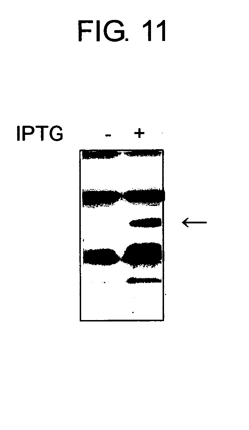

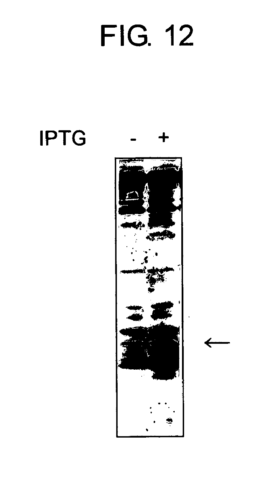

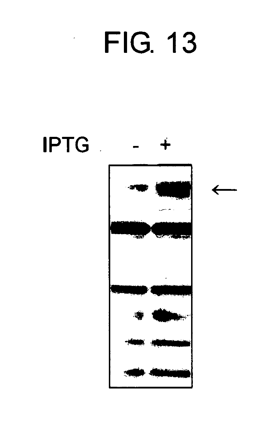

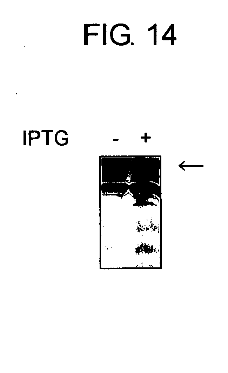

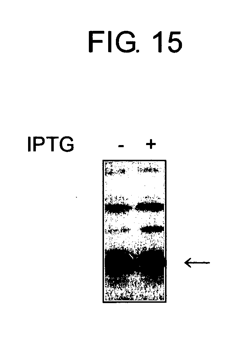

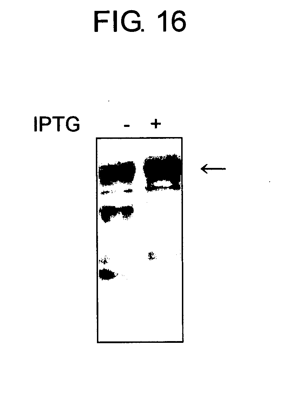

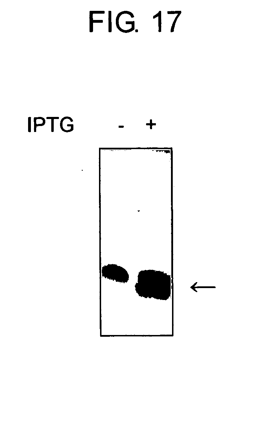

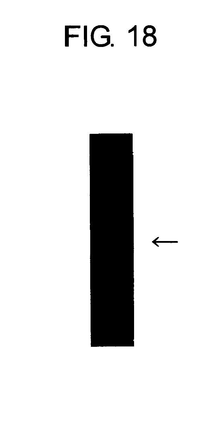

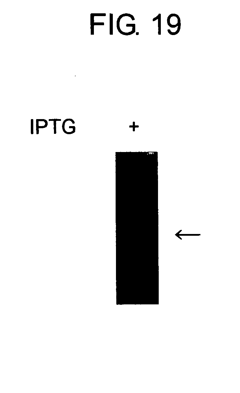

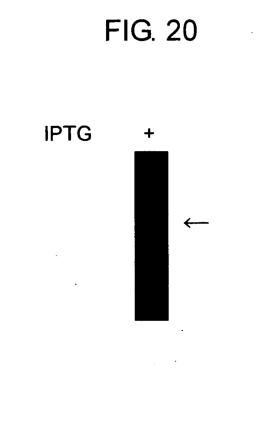

Figs. 2 to 20 show the results of Western blotting analysis that was carried out to examine binding reactions involving these 19 types of novel antigenic polypeptides and serum antibodies of the patients. In figs. 2 to 20, arrows indicate polypeptides that underwent specific reaction with the serum antibodies of the patients. The polypeptides were detected in the IPTG-treated *E coli.* extract; however, they were not detected in the untreated *E coli.* extract. Thus, the polypeptides were confirmed to be derived from cDNA that had been introduced thereinto.

All publications, patents, and patent applications cited herein are incorporated herein by reference in their entirety.

### Industrial Applicability

According to the diagnostic kit for solid cancer of the present invention, solid cancer can be diagnosed with high accuracy and it is useful for early diagnosis of solid cancer. In addition, with the use of the medicament for preventing or treating solid cancer of the present invention, it becomes possible to carry out a therapy whereby selective targeting of solid cancer is carried out.

## Claims

1. A human solid cancer antigenic polypeptide having an amino acid sequence selected from the group consisting of SEQ ID NOS: 2, 4, 6, 8, 10, 12, 14, 16, 18, 20, 22, 24, 26, 28, 30, 32, 34, 36, 38, 40, 42, 44, 48, 50, 52, 54, 56, 59, 61, 63, 65, 67, 69, 71, 73, and 75.

2. A polynucleotide encoding the human solid cancer antigenic polypeptide according to claim 1.

3. A polynucleotide having a nucleotide sequence selected from the group consisting of SEQ ID NOS: 1, 3, 5, 7, 9, 11, 13, 15, 17, 19, 21, 23, 25, 27, 29, 31, 33, 35, 37, 39, 41, 43, 45, 46, 47, 49, 51, 53, 55, 57, 58, 60, 62, 64, 66, 68, 70, 72, and 74.

4. A diagnostic kit for solid cancer comprising a means of detecting the expression of at least one human solid cancer antigenic polypeptide in a sample derived from a subject, **characterized in that** the human solid cancer antigenic polypeptide has an amino acid sequence selected from the group consisting of SEQ ID NOS: 2, 4, 6, 8, 10, 12, 14, 16, 18, 20, 22, 24, 26, 28, 30, 32, 34, 36, 38, 40, 42, 44, 48, 50, 52, 54, 56, 59, 61, 63, 65, 67, 69, 71, 73, and 75.

5. A diagnostic kit for solid cancer comprising a means of detecting the expression of at least one human solid cancer antigenic polypeptide in a sample derived from a subject, **characterized in that** the human solid cancer antigenic polypeptide is encoded by a polynucleotide having a nucleotide sequence selected from the group consisting of SEQ ID NOS: 1, 3, 5, 7, 9, 11, 13, 15, 17, 19, 21, 23, 25, 27, 29, 31, 33, 35, 37, 39, 41, 43, 45, 46, 47, 49, 51, 53, 55, 57, 58, 60, 62, 64, 66, 68, 70, 72, and 74.

6. The diagnostic kit for solid cancer according to claim 4 or 5, wherein the means of detecting the expression of the human solid cancer antigenic polypeptide is the solid cancer antigenic polypeptide or a partial peptide thereof.

7. The diagnostic kit for solid cancer according to claim 4 or 5, wherein the means of detecting the expression of the human solid cancer antigenic polypeptide is an antibody against the solid cancer antigenic polypeptide.

8. The diagnostic kit for solid cancer according to claim 4 or 5, wherein the means of detecting the expression of the human solid cancer antigenic polypeptide is a primer or probe comprising a polynucleotide consisting of the entire or a partial sequence of a polynucleotide encoding the solid cancer antigenic polypeptide or a complementary sequence thereof.

9. The diagnostic kit for solid cancer according to any one of claims 4 to 8, wherein the means of detecting the expression of the human solid cancer antigenic polypeptide is immobilized on a solid phase.

10. The diagnostic kit for solid cancer according to any one of claims 4 to 9, wherein the means of detecting the expression of the human solid cancer antigenic polypeptide is labeled.

11. The diagnostic kit for solid cancer according to any one of claims 4 to 10, wherein the solid cancer is selected from the group consisting of colorectal cancer, esophageal cancer, gastric cancer, and breast cancer.

12. The diagnostic kit for solid cancer according to any one of claims 4 to 11, wherein the sample is selected from the group consisting of serum, blood, hemocytes, and tissue.

13. A medicament for preventing or treating solid cancer comprising a means of inhibiting the functions or expression of at least one human solid cancer antigenic polypeptide, **characterized in that** the human solid cancer antigenic polypeptide has an amino acid sequence selected from the group consisting of SEQ ID NOS: 2, 4, 6, 8, 10, 12, 14, 16, 18, 20, 22, 24, 26, 28, 30, 32, 34, 36, 38, 40, 42, 44, 48, 50, 52, 54, 56, 59, 61, 63, 65, 67, 69, 71, 73, and 75.

14. A medicament for preventing or treating solid cancer comprising a means of inhibiting the functions or expression of at least one human solid cancer antigenic polypeptide, **characterized in that** the human solid cancer antigenic polypeptide is encoded by a polynucleotide having a nucleotide sequence selected from the group consisting of SEQ ID NOS: 1, 3, 5,7,9, 11, 13, 15, 17, 19, 21, 23, 25, 27, 29, 31, 33, 35, 37, 39, 41, 43, 45, 46, 47, 49, 51, 53, 55, 57, 58, 60, 62, 64, 66, 68, 70, 72, and 74.

15. The medicament according to claim 13 or 14, wherein the means of inhibiting the functions or expression of the human solid cancer antigenic polypeptide is an antibody against the solid cancer antigenic polypeptide.

16. The medicament according to claim 13 or 14, wherein the means of inhibiting the functions or expression of the human solid cancer antigenic polypeptide is a means capable of inhibiting transcription of a gene encoding the solid cancer antigenic polypeptide.

17. The medicament according to claim 13 or 14, wherein the means of inhibiting the functions or expression of the human solid cancer antigenic polypeptide is a means capable of inhibiting translation of a gene encoding the solid cancer antigenic polypeptide.

18. A medicament, for preventing or treating solid cancer comprising a gene encoding a prophylactic or therapeutic agent for solid cancer and a means of targeting to human solid cancer.

19. The medicament according to claim 18, wherein the means of targeting to human solid cancer is an antibody against a solid cancer antigenic polypeptide.

20. The medicament according to claim 18, wherein the means of targeting to human solid cancer is a nucleotide sequence of an expression control region of a polynucleotide encoding a solid cancer antigenic polypeptide.


SEQUENCE LISTING

<110> SHIMADA, Hideaki
      TOMONAGA, Takeshi
      HIWASA, Takaki
      MATSUSHITA, Kazuyuki
      OCHIAI, Takenori
      NOMURA, Fumio

<120> Human solid tumor-specific proteins

<130> PH-2406PCT

<150> JP 2004-095732
<150> 2004-03-29

<160> 75

<170> PatentIn version 3.1

<210> 1
<211> 1321
<212> DNA
<213> Homo sapiens

<220>
<221> CDS
<222> (87)..(1103)
<223>

<400> 1
   ggcgctgggc agtgtggagg tcgttggagt cacttccgcg tcaccagctc ctgtgcctgc      60
   cagtcggtgc ccctcccgct ccagcc atg ctc tcc gcc ctc gcc cgg cct gcc 113
   Met Leu Ser Ala Leu Ala Arg Pro Ala
   1 5
   agc gct gct ctc cgc cgc agc ttc agc acc tcg gcc cag aac aat gct      161
   Ser Ala Ala Leu Arg Arg Ser Phe Ser Thr Ser Ala Gln Asn Asn Ala
   10                  15                  20                  25
   aaa gta gct gtg cta ggg gcc tct gga ggc atc ggg cag cca ctt tca      209
   Lys Val Ala Val Leu Gly Ala Ser Gly Gly Ile Gly Gln Pro Leu Ser
                   30                  35                  40
   ctt ctc ctg aag aac agc ccc ttg gtg agc cgc ctg acc ctc tat gat      257
   Leu Leu Leu Lys Asn Ser Pro Leu Val Ser Arg Leu Thr Leu Tyr Asp
               45                  50                  55
   atc gcg cac aca ccc gga gtg gcc gca gat ctg agc cac atc gag acc      305
   Ile Ala His Thr Pro Gly Val Ala Ala Asp Leu Ser His Ile Glu Thr
           60                  65                  70
   aaa gcc gct gtg aaa ggc tac ctc gga cct gaa cag ctg cct gac tgc        353
   Lys Ala Ala Val Lys Gly Tyr Leu Gly Pro Glu Gln Leu Pro Asp Cys
       75                  80                  85
   ctg aaa ggt tgt gat gtg gta gtt att ccg gct gga gtc ccc aga aag        401
   Leu Lys Gly Cys Asp Val Val Val Ile Pro Ala Gly Val Pro Arg Lys
   90                  95                  100                 105
   cca ggc atg acc cgg gac gac ctg ttc aac acc aat gcc acg att gtg        449
   Pro Gly Met Thr Arg Asp Asp Leu Phe Asn Thr Asn Ala Thr Ile Val
                   110                 115                 120
   gcc acc ctg acc gct gcc tgt gcc cag cac tgc ccg gaa gcc atg atc        497
   Ala Thr Leu Thr Ala Ala Cys Ala Gln His Cys Pro Glu Ala Met Ile
               125                 130                 135
   tgc gtc att gcc aat ccg gtt aat tcc acc atc ccc atc aca gca gaa        545
   Cys Val Ile Ala Asn Pro Val Asn Ser Thr Ile Pro Ile Thr Ala Glu
           140                 145                 150
   gtt ttc aag aag cat gga gtg tac aac ccc aac aaa atc ttc ggc gtg        593
   Val Phe Lys Lys His Gly Val Tyr Asn Pro Asn Lys Ile Phe Gly Val
       155                 160                 165
   acg acc ctg gac atc gtc aga gcc aac acc ttt gtt gca gag ctg aag        641
   Thr Thr Leu Asp Ile Val Arg Ala Asn Thr Phe Val Ala Glu Leu Lys
   170                 175                 180                 185
   ggt ttg gat cca gct cga gtc aac gtc cct gtc att ggt ggc cat gct        689
   Gly Leu Asp Pro Ala Arg Val Asn Val Pro Val Ile Gly Gly His Ala
                   190                 195                 200
   ggg aag acc atc atc ccc ctg atc tct cag tgc acc ccc aag gtg gac        737
   Gly Lys Thr Ile Ile Pro Leu Ile Ser Gln Cys Thr Pro Lys Val Asp
               205                 210                 215
   ttt ccc cag gac cag ctg aca gca ctc act ggg cgg atc cag gag gcc        785
   Phe Pro Gln Asp Gln Leu Thr Ala Leu Thr Gly Arg Ile Gln Glu Ala
           220                 225                 230
   ggc acg gag gtg gtc aag gct aaa gcc gga gca ggc tct gcc acc ctc        833
   Gly Thr Glu Val Val Lys Ala Lys Ala Gly Ala Gly Ser Ala Thr Leu
       235                 240                 245
   tcc atg gcg tat gcc ggc gcc cgc ttt gtc ttc tcc ctt gtg gat gca        881
   Ser Met Ala Tyr Ala Gly Ala Arg Phe Val Phe Ser Leu Val Asp Ala
   250                 255                 260                 265
   atg aat gga aag gaa ggt gtt gtg gaa tgt tcc ttc gtt aag tca cag 929
   Met Asn Gly Lys Glu Gly Val Val Glu Cys Ser Phe Val Lys Ser Gln
   270 275 280
   gaa acg gaa tgt acc tac ttc tcc aca ccg ctg ctg ctt ggg aaa aag 977
   Glu Thr Glu Cys Thr Tyr Phe Ser Thr Pro Leu Leu Leu Gly Lys Lys
   285 290 295
   ggc atc gag aag aac ctg ggc atc ggc aaa gtc tcc tct ttt gag gag 1025
   Gly Ile Glu Lys Asn Leu Gly Ile Gly Lys Val Ser Ser Phe Glu Glu
   300 305 310
   aag atg atc tcg gat gcc atc ccc gag ctg aag gcc tcc atc aag aag 1073
   Lys Met Ile Ser Asp Ala Ile Pro Glu Leu Lys Ala Ser Ile Lys Lys
   315 320 325
   ggg gaa gat ttc gtg aag acc ctg aag tga gccgctgtga cgggtggcca 1123
   Gly Glu Asp Phe Val Lys Thr Leu Lys
   330 335
   gtttccttaa tttatgaagg catcatgtca ctgcaaagcc gttgcagata aactttgtat 1183
   tttaatttgc tttggtgatg attactgtat tgacatcatc atgccttcca aattgtgggt 1243
   ggctctgtgg gcgcatcaat aaaagccgtc cttgatttta tttttcaagg tcccttctgt 1303
   aaaaaaaaaa aaaaaaaa 1321

<210> 2
<211> 338
<212> PRT
<213> Homo sapiens

<400> 2

Met Leu Ser Ala Leu Ala Arg Pro Ala Ser Ala Ala Leu Arg Arg Ser
1               5                   10                  15

Phe Ser Thr Ser Ala Gln Asn Asn Ala Lys Val Ala Val Leu Gly Ala
            20                  25                  30

Ser Gly Gly Ile Gly Gln Pro Leu Ser Leu Leu Leu Lys Asn Ser Pro
        35                  40                  45

Leu Val Ser Arg Leu Thr Leu Tyr Asp Ile Ala His Thr Pro Gly Val

50                    55                    60

Ala Ala Asp Leu Ser His Ile Glu Thr Lys Ala Ala Val Lys Gly Tyr
65                  70                  75                  80

Leu Gly Pro Glu Gln Leu Pro Asp Cys Leu Lys Gly Cys Asp Val Val
                85                  90                  95

Val Ile Pro Ala Gly Val Pro Arg Lys Pro Gly Met Thr Arg Asp Asp
            100                 105                 110

Leu Phe Asn Thr Asn Ala Thr Ile Val Ala Thr Leu Thr Ala Ala Cys
        115                 120                 125

Ala Gln His Cys Pro Glu Ala Met Ile Cys Val Ile Ala Asn Pro Val
    130                 135                 140

Asn Ser Thr Ile Pro Ile Thr Ala Glu Val Phe Lys Lys His Gly Val
145                 150                 155                 160

Tyr Asn Pro Asn Lys Ile Phe Gly Val Thr Thr Leu Asp Ile Val Arg
                165                 170                 175

Ala Asn Thr Phe Val Ala Glu Leu Lys Gly Leu Asp Pro Ala Arg Val
            180                 185                 190

Asn Val Pro Val Ile Gly Gly His Ala Gly Lys Thr Ile Ile Pro Leu
        195                 200                 205

Ile Ser Gln Cys Thr Pro Lys Val Asp Phe Pro Gln Asp Gln Leu Thr
    210                 215                 220

Ala Leu Thr Gly Arg Ile Gln Glu Ala Gly Thr Glu Val Val Lys Ala
225                 230                 235                 240

Lys Ala Gly Ala Gly Ser Ala Thr Leu Ser Met Ala Tyr Ala Gly Ala
                245                 250                 255

Arg Phe Val Phe Ser Leu Val Asp Ala Met Asn Gly Lys Glu Gly Val
            260                 265                 270

Val Glu Cys Ser Phe Val Lys Ser Gln Glu Thr Glu Cys Thr Tyr Phe
        275                 280                 285

Ser Thr Pro Leu Leu Leu Gly Lys Lys Gly Ile Glu Lys Asn Leu Gly
    290                 295                 300

Ile Gly Lys Val Ser Ser Phe Glu Glu Lys Met Ile Ser Asp Ala Ile
305                 310                 315                 320

Pro Glu Leu Lys Ala Ser Ile Lys Lys Gly Glu Asp Phe Val Lys Thr
                325                 330                 335

Leu Lys

<210> 3
<211> 2049
<212> DNA
<213> Homo sapiens

<220>
<221> CDS
<222> (51)..(797)
<223>

<400> 3
gagcccagcc gagcgtccgc cgctgcccgt gcgcctctgc gctccgcgcc atg gcc 56
Met Ala
1

ggc ctc aac tcc ctg gag gcg gtg aaa cgc aag atc cag gcc ctg cag 104
Gly Leu Asn Ser Leu Glu Ala Val Lys Arg Lys Ile Gln Ala Leu Gln
        5                   10                  15

cag cag gcg gac gag gcg gaa gac cgc gcg cag ggc ctg cag cgg gag 152
Gln Gln Ala Asp Glu Ala Glu Asp Arg Ala Gln Gly Leu Gln Arg Glu
    20                  25                  30

ctg gac ggc gag cgc gag cgg cgc gag aaa gct gaa ggt gat gtg gcc 200
Leu Asp Gly Glu Arg Glu Arg Arg Glu Lys Ala Glu Gly Asp Val Ala

35                  40                  45                  50

gcc ctc aac cga cgc atc cag ctc gtt gag gag gag ttg gac agg gct        248
Ala Leu Asn Arg Arg Ile Gln Leu Val Glu Glu Glu Leu Asp Arg Ala
                55                  60                  65

cag gaa cga ctg gcc acg gcc ctg cag aag ctg gag gag gca gaa aaa        296
Gln Glu Arg Leu Ala Thr Ala Leu Gln Lys Leu Glu Glu Ala Glu Lys
            70                  75                  80

gct gca gat gag agt gag aga gga atg aag gtg ata gaa aac cgg gcc        344
Ala Ala Asp Glu Ser Glu Arg Gly Met Lys Val Ile Glu Asn Arg Ala
        85                  90                  95

atg aag gat gag gag aag atg gag att cag gag atg cag ctc aaa gag        392
Met Lys Asp Glu Glu Lys Met Glu Ile Gln Glu Met Gln Leu Lys Glu
    100                 105                 110

gcc aag cac att gcg gaa gag gct gac cgc aaa tac gag gag gta gct        440
Ala Lys His Ile Ala Glu Glu Ala Asp Arg Lys Tyr Glu Glu Val Ala
115                 120                 125                 130

cgt aag ctg gtc atc ctg gag ggt gag ctg gag agg gca gag gag cgt        488
Arg Lys Leu Val Ile Leu Glu Gly Glu Leu Glu Arg Ala Glu Glu Arg
                135                 140                 145

gcg gag gtg tct gaa cta aaa tgt ggt gac ctg gaa gaa gaa ctc aag        536
Ala Glu Val Ser Glu Leu Lys Cys Gly Asp Leu Glu Glu Glu Leu Lys
            150                 155                 160

aat gtt act aac aat ctg aaa tct ctg gag gct gca tct gaa aag tat        584
Asn Val Thr Asn Asn Leu Lys Ser Leu Glu Ala Ala Ser Glu Lys Tyr
        165                 170                 175

tct gaa aag gag gac aaa tat gaa gaa gaa att aaa ctt ctg tct gac        632
Ser Glu Lys Glu Asp Lys Tyr Glu Glu Glu Ile Lys Leu Leu Ser Asp
    180                 185                 190

aaa ctg aaa gag gct gag acc cgt gct gaa ttt gca gag aga acg gtt        680
Lys Leu Lys Glu Ala Glu Thr Arg Ala Glu Phe Ala Glu Arg Thr Val
195                 200                 205                 210

gca aaa ctg gaa aag aca att gat gac ctg gaa gag aaa ctt gcc cag        728
Ala Lys Leu Glu Lys Thr Ile Asp Asp Leu Glu Glu Lys Leu Ala Gln
                215                 220                 225

gcc aaa gaa gag aac gtg ggc tta cat cag aca ctg gat cag aca cta        776
Ala Lys Glu Glu Asn Val Gly Leu His Gln Thr Leu Asp Gln Thr Leu
            230                 235                 240

aac gaa ctt aac tgt ata taa gcaaaacaga agagtcttgt tccaacagaa 827
Asn Glu Leu Asn Cys Ile
        245

actctggagc tccgtgggtc tttctcttct cttgtaagaa gttccttttg ttattgccat 887

cttcgctttg ctggaaatgt caagcaaatt atgaatacat gaccaaatat tttgtatcgg 947

agaagctttg agcaccagtt aaatctcatt ccttcccttt ttttttcaaa tggcaccagc 1007

tttttcagct ctcttatttt ttccttaagt agcatttatt cctaaggtag gcagggtatt 1067

tcctagtaag catactttct taagacggag gccatttggt tcctgggaga ataggcagcc 1127

ccacactttg aagaatacag accccagtat ctagtcgtgg atataattaa aacgctgaag 1187

accataacct tttgggtcaa ctgttggtca aactatagga gagaccaggg accatcacat 1247

gggtagggat tttccatcca gagccaataa aaggactggt gggggccggg ggtggctatt 1307

gtgggaagtc ataacccaca gatagatcaa cctaagaatc ctggcccttc tccactctcc 1367

accatgcagg acaaacatct tctcaagcag tcaacgtaga atgcttggga aatagtcata 1427

attacccaca tatagtaatt aatagatggt aattaattga tccttgatgt gatgttcttt 1487

tgcatatttc cttcattcta aagttgttcc ctggccggga gcgtttgctt tcgcctgtaa 1547

tcccaacact ttgggaggcc aggacagatc acttgaggtc aggagttcga gaccagccca 1607

gccaacatgg cgaaaccatg tctctactaa aaatacaaaa attatggtga cgcctgcctg 1667

tagtcccagc tactcgggag gctgaggcag gaggatcgct tgaacccagg aagtggagac 1727

tgcagtgagc cgatatcgca ccacagcgct ccagcctggt cgacagagtg agactccatc 1787

tcaagaaaaa ataaaaataa agttgttctc tgaagagcaa atgtctcatt ccagtaatga 1847

cccactcagc aggaatatgg tggagttcag tccaattcag gtcagccata tccaaaagac 1907

cacaagtcat tactaagttg agcaaaagag tttttatcta ttagcagaaa gggcctctct 1967

ggcagcagag attaaaaact ggcccaactt catttccata cttcagggaa cagcaaattg 2027

aggatttact tatctaggac tt 2049

<210> 4
<211> 248
<212> PRT

<213>  Homo sapiens

<400>  4

Met Ala Gly Leu Asn Ser Leu Glu Ala Val Lys Arg Lys Ile Gln Ala
1               5                   10                  15

Leu Gln Gln Gln Ala Asp Glu Ala Glu Asp Arg Ala Gln Gly Leu Gln
            20                  25                  30

Arg Glu Leu Asp Gly Glu Arg Glu Arg Arg Glu Lys Ala Glu Gly Asp
        35                  40                  45

Val Ala Ala Leu Asn Arg Arg Ile Gln Leu Val Glu Glu Glu Leu Asp
    50                  55                  60

Arg Ala Gln Glu Arg Leu Ala Thr Ala Leu Gln Lys Leu Glu Glu Ala
65                  70                  75                  80

Glu Lys Ala Ala Asp Glu Ser Glu Arg Gly Met Lys Val Ile Glu Asn
                85                  90                  95

Arg Ala Met Lys Asp Glu Glu Lys Met Glu Ile Gln Glu Met Gln Leu
            100                 105                 110

Lys Glu Ala Lys His Ile Ala Glu Glu Ala Asp Arg Lys Tyr Glu Glu
        115                 120                 125

Val Ala Arg Lys Leu Val Ile Leu Glu Gly Glu Leu Glu Arg Ala Glu
    130                 135                 140

Glu Arg Ala Glu Val Ser Glu Leu Lys Cys Gly Asp Leu Glu Glu Glu
145                 150                 155                 160

Leu Lys Asn Val Thr Asn Asn Leu Lys Ser Leu Glu Ala Ala Ser Glu
                165                 170                 175

Lys Tyr Ser Glu Lys Glu Asp Lys Tyr Glu Glu Glu Ile Lys Leu Leu
            180                 185                 190

Ser Asp Lys Leu Lys Glu Ala Glu Thr Arg Ala Glu Phe Ala Glu Arg
        195                 200                 205

Thr Val Ala Lys Leu Glu Lys Thr Ile Asp Asp Leu Glu Glu Lys Leu
    210                 215                 220

Ala Gln Ala Lys Glu Glu Asn Val Gly Leu His Gln Thr Leu Asp Gln
225                 230                 235                 240

Thr Leu Asn Glu Leu Asn Cys Ile
                245

<210>  5
<211>  2251
<212>  DNA
<213>  Homo sapiens

<220>
<221>  CDS
<222>  (154)..(1533)
<223>

<400>  5
gccgcgtgca gaggtgctca agcctcctcg cggtccgcag tcagtgccgc cgcgcccggc      60

ctcccgcacg ccccgcaggt agcgcccccg cccgcggccc agagtgcgct cgcgccggca     120

ccagctcccg gataaacggc gcgccgcgcg gag atg aca gcc gag gag atg aag      174
                                     Met Thr Ala Glu Glu Met Lys
                                     1               5

gcg acc gag agc ggg gcg cag tcg gcg ccg ctg ccc atg gag gga gtg      222
Ala Thr Glu Ser Gly Ala Gln Ser Ala Pro Leu Pro Met Glu Gly Val
        10                  15                  20

gac atc agc ccc aaa cag gac gaa ggc gtg ctg aag gtc atc aag aga      270
Asp Ile Ser Pro Lys Gln Asp Glu Gly Val Leu Lys Val Ile Lys Arg
    25                  30                  35

gag ggc aca ggt aca gag atg ccc atg att ggg gac cga gtc ttt gtc      318
Glu Gly Thr Gly Thr Glu Met Pro Met Ile Gly Asp Arg Val Phe Val
40                  45                  50                  55

cac tac act ggc tgg cta tta gat ggc aca aag ttt gac tcc agt ctg      366

His Tyr Thr Gly Trp Leu Leu Asp Gly Thr Lys Phe Asp Ser Ser Leu
                60                  65                  70

gat cgc aag gac aaa ttc tcc ttt gac ctg gga aaa ggg gag gtc atc 414
Asp Arg Lys Asp Lys Phe Ser Phe Asp Leu Gly Lys Gly Glu Val Ile
            75                  80                  85

aag gct tgg gac att gcc ata gcc acc atg aag gtg ggg gag gtg tgc 462
Lys Ala Trp Asp Ile Ala Ile Ala Thr Met Lys Val Gly Glu Val Cys
        90                  95                  100

cac atc acc tgc aaa cca gaa tat gcc tac ggt tca gca ggc agt cct 510
His Ile Thr Cys Lys Pro Glu Tyr Ala Tyr Gly Ser Ala Gly Ser Pro
    105                 110                 115

cca aag att ccc ccc aat gcc acg ctt gta ttt gag gtg gag ttg ttt 558
Pro Lys Ile Pro Pro Asn Ala Thr Leu Val Phe Glu Val Glu Leu Phe
120                 125                 130                 135

gag ttt aag gga gaa gat ctg acg gaa gag gaa gat ggc gga atc att 606
Glu Phe Lys Gly Glu Asp Leu Thr Glu Glu Glu Asp Gly Gly Ile Ile
                140                 145                 150

cgc aga ata cag act cgc ggt gaa ggc tat gct aag ccc aat gag ggt 654
Arg Arg Ile Gln Thr Arg Gly Glu Gly Tyr Ala Lys Pro Asn Glu Gly
            155                 160                 165

gct atc gtg gag gtt gca ctg gaa ggg tac tac aag gac aag ctc ttt 702
Ala Ile Val Glu Val Ala Leu Glu Gly Tyr Tyr Lys Asp Lys Leu Phe
        170                 175                 180

gac cag cgg gag ctc cgc ttt gag att ggc gag ggg gag aac ctg gat 750
Asp Gln Arg Glu Leu Arg Phe Glu Ile Gly Glu Gly Glu Asn Leu Asp
    185                 190                 195

ctg cct tat ggt ctg gag agg gcc att cag cgc atg gag aaa gga gaa 798
Leu Pro Tyr Gly Leu Glu Arg Ala Ile Gln Arg Met Glu Lys Gly Glu
200                 205                 210                 215

cat tcc atc gtg tac ctc aag ccc agc tat gct ttt ggc agt gtt ggg 846
His Ser Ile Val Tyr Leu Lys Pro Ser Tyr Ala Phe Gly Ser Val Gly
                220                 225                 230

aag gaa aag ttc caa atc cca cca aat gct gag ctg aaa tat gaa tta 894
Lys Glu Lys Phe Gln Ile Pro Pro Asn Ala Glu Leu Lys Tyr Glu Leu
            235                 240                 245

cac ctc aag agt ttt gaa aag gcc aag gag tct tgg gag atg aat tca 942
His Leu Lys Ser Phe Glu Lys Ala Lys Glu Ser Trp Glu Met Asn Ser
        250                 255                 260

gaa gag aag ctg gaa cag agc acc ata gtg aaa gag cgg ggc act gtg     990
Glu Glu Lys Leu Glu Gln Ser Thr Ile Val Lys Glu Arg Gly Thr Val
    265                 270                 275

tac ttc aag gaa ggt aaa tac aag caa gct tta cta cag tat aag aag     1038
Tyr Phe Lys Glu Gly Lys Tyr Lys Gln Ala Leu Leu Gln Tyr Lys Lys
280                 285                 290                 295

atc gtg tct tgg ctg gaa tat gag tct agt ttt tcc aat gag gaa gca     1086
Ile Val Ser Trp Leu Glu Tyr Glu Ser Ser Phe Ser Asn Glu Glu Ala
                300                 305                 310

cag aaa gca cag gcc ctt cga ctg gcc tct cac ctc aac ctg gcc atg     1134
Gln Lys Ala Gln Ala Leu Arg Leu Ala Ser His Leu Asn Leu Ala Met
            315                 320                 325

tgt cat ctg aaa cta cag gcc ttc tct gct gcc att gaa agc tgt aac     1182
Cys His Leu Lys Leu Gln Ala Phe Ser Ala Ala Ile Glu Ser Cys Asn
        330                 335                 340

aag gcc cta gaa ctg gac agc aac aac gag aag ggc ctc ttc cgc cgg     1230
Lys Ala Leu Glu Leu Asp Ser Asn Asn Glu Lys Gly Leu Phe Arg Arg
    345                 350                 355

gga gag gcc cac ctg gcc gtg aat gac ttt gaa ctg gca cgg gct gat     1278
Gly Glu Ala His Leu Ala Val Asn Asp Phe Glu Leu Ala Arg Ala Asp
360                 365                 370                 375

ttc cag aag gtc ctg cag ctc tac ccc aac aac aaa gcc gcc aag acc     1326
Phe Gln Lys Val Leu Gln Leu Tyr Pro Asn Asn Lys Ala Ala Lys Thr
                380                 385                 390

cag ctg gct gtg tgc cag cag cgg atc cga agg cag ctt gcc cgg gag     1374
Gln Leu Ala Val Cys Gln Gln Arg Ile Arg Arg Gln Leu Ala Arg Glu
            395                 400                 405

aag aag ctc tat gcc aat atg ttt gag agg ctg gct gag gag gag aac     1422
Lys Lys Leu Tyr Ala Asn Met Phe Glu Arg Leu Ala Glu Glu Glu Asn
        410                 415                 420

aag gcc aag gca gag gct tcc tca gga gac cat ccc act gac aca gag     1470
Lys Ala Lys Ala Glu Ala Ser Ser Gly Asp His Pro Thr Asp Thr Glu
    425                 430                 435

atg aag gag gag cag aag agc aac acg gca ggg agc cag tct cag gtg     1518
Met Lys Glu Glu Gln Lys Ser Asn Thr Ala Gly Ser Gln Ser Gln Val
440                 445                 450                 455

gag aca gaa gca tag cccctctcca ccagccctac tcctgcggct gcctgccccc     1573

Glu Thr Glu Ala

cagtctcccc actccaccct gttagttttg taaaaactga agaattttga gtgaattaga 1633

cctttatttt tctatctggt tggatggtgg ctttagggga agggggaaag gtgtaggctg 1693

ggggattgag gtggggaatc attttagctg gtgtcagccc ctcttccctt cctccattgc 1753

acatgaacat atgtccatcc atatatattc atcagaatgt taatttattt tgctccctct 1813

gttaggtcca ttttctaagg gtagaagagg caagtggtag ggatgaggtc tgataagaac 1873

ccagggtgga gagggagact cctgggcagc cgttttcctc atcctttccc tctcccagtc 1933

catttccaaa tgtggcctcc atgtgggtgc tagggacatg ggaaaaacca ctgctatgcc 1993

atttcttctc tctgttccct tcctcacccc cgacggtgtg gctgatgatg tcttctggtg 2053

tcatggtgac caccccctgt tccctgttct ggtatttccc ctgtcagttt cccctctcgg 2113

ccaggttgtg tcccaaaatc ccctcagcct cttctctgca cgttgctgaa ggtccaggct 2173

tgcctcaagt tccatgcttg agcaataaag tggaaacaat aaaacctggg tgtcagacaa 2233

aaaaaaaaaa aaaaaaaa 2251

<210> 6
<211> 459
<212> PRT
<213> Homo sapiens

<400> 6

Met Thr Ala Glu Glu Met Lys Ala Thr Glu Ser Gly Ala Gln Ser Ala
1               5                   10                  15

Pro Leu Pro Met Glu Gly Val Asp Ile Ser Pro Lys Gln Asp Glu Gly
            20                  25                  30

Val Leu Lys Val Ile Lys Arg Glu Gly Thr Gly Thr Glu Met Pro Met
        35                  40                  45

Ile Gly Asp Arg Val Phe Val His Tyr Thr Gly Trp Leu Leu Asp Gly
    50                  55                  60

Thr Lys Phe Asp Ser Ser Leu Asp Arg Lys Asp Lys Phe Ser Phe Asp
65                  70                  75                  80

Leu Gly Lys Gly Glu Val Ile Lys Ala Trp Asp Ile Ala Ile Ala Thr
                85                  90                  95

Met Lys Val Gly Glu Val Cys His Ile Thr Cys Lys Pro Glu Tyr Ala
            100                 105                 110

Tyr Gly Ser Ala Gly Ser Pro Pro Lys Ile Pro Pro Asn Ala Thr Leu
        115                 120                 125

Val Phe Glu Val Glu Leu Phe Glu Phe Lys Gly Glu Asp Leu Thr Glu
    130                 135                 140

Glu Glu Asp Gly Gly Ile Ile Arg Arg Ile Gln Thr Arg Gly Glu Gly
145                 150                 155                 160

Tyr Ala Lys Pro Asn Glu Gly Ala Ile Val Glu Val Ala Leu Glu Gly
                165                 170                 175

Tyr Tyr Lys Asp Lys Leu Phe Asp Gln Arg Glu Leu Arg Phe Glu Ile
            180                 185                 190

Gly Glu Gly Glu Asn Leu Asp Leu Pro Tyr Gly Leu Glu Arg Ala Ile
        195                 200                 205

Gln Arg Met Glu Lys Gly Glu His Ser Ile Val Tyr Leu Lys Pro Ser
    210                 215                 220

Tyr Ala Phe Gly Ser Val Gly Lys Glu Lys Phe Gln Ile Pro Pro Asn
225                 230                 235                 240

Ala Glu Leu Lys Tyr Glu Leu His Leu Lys Ser Phe Glu Lys Ala Lys
                245                 250                 255

Glu Ser Trp Glu Met Asn Ser Glu Glu Lys Leu Glu Gln Ser Thr Ile

            260                 265                 270

Val Lys Glu Arg Gly Thr Val Tyr Phe Lys Glu Gly Lys Tyr Lys Gln
        275                 280                 285

Ala Leu Leu Gln Tyr Lys Lys Ile Val Ser Trp Leu Glu Tyr Glu Ser
    290                 295                 300

Ser Phe Ser Asn Glu Glu Ala Gln Lys Ala Gln Ala Leu Arg Leu Ala
305                 310                 315                 320

Ser His Leu Asn Leu Ala Met Cys His Leu Lys Leu Gln Ala Phe Ser
                325                 330                 335

Ala Ala Ile Glu Ser Cys Asn Lys Ala Leu Glu Leu Asp Ser Asn Asn
            340                 345                 350

Glu Lys Gly Leu Phe Arg Arg Gly Glu Ala His Leu Ala Val Asn Asp
        355                 360                 365

Phe Glu Leu Ala Arg Ala Asp Phe Gln Lys Val Leu Gln Leu Tyr Pro
    370                 375                 380

Asn Asn Lys Ala Ala Lys Thr Gln Leu Ala Val Cys Gln Gln Arg Ile
385                 390                 395                 400

Arg Arg Gln Leu Ala Arg Glu Lys Lys Leu Tyr Ala Asn Met Phe Glu
                405                 410                 415

Arg Leu Ala Glu Glu Glu Asn Lys Ala Lys Ala Glu Ala Ser Ser Gly
            420                 425                 430

Asp His Pro Thr Asp Thr Glu Met Lys Glu Glu Gln Lys Ser Asn Thr
        435                 440                 445

Ala Gly Ser Gln Ser Gln Val Glu Thr Glu Ala
    450                 455

<210> 7
<211> 2562
<212> DNA
<213> Homo sapiens

<220>
<221> CDS
<222> (56)..(1651)
<223>

<400> 7
gccgcgccgg ctctgggcac tcagcatcgt ttccttttcc tccgctggag cagct atg 58
Met
1

gcg gcg gtg aag acc ctg aac ccc aag gcc gag gtg gcc cga gcg cag 106
Ala Ala Val Lys Thr Leu Asn Pro Lys Ala Glu Val Ala Arg Ala Gln
            5                   10                  15

gcg gcg ctg gcg gtc aac atc agc gca gcg cgg ggt ctg cag gac gtg 154
Ala Ala Leu Ala Val Asn Ile Ser Ala Ala Arg Gly Leu Gln Asp Val
        20                  25                  30

cta agg acc aac ctg ggg ccc aag ggc acc atg aag atg ctc gtt tct 202
Leu Arg Thr Asn Leu Gly Pro Lys Gly Thr Met Lys Met Leu Val Ser
    35                  40                  45

ggc gct gga gac atc aaa ctt act aaa gac ggc aat gtg ctg ctt cac 250
Gly Ala Gly Asp Ile Lys Leu Thr Lys Asp Gly Asn Val Leu Leu His
50                  55                  60                  65

gaa atg caa att caa cac cca aca gct tcc tta ata gca aag gta gca 298
Glu Met Gln Ile Gln His Pro Thr Ala Ser Leu Ile Ala Lys Val Ala
                70                  75                  80

aca gcc cag gat gat ata act ggt gat ggt acg act tct aat gtc cta 346
Thr Ala Gln Asp Asp Ile Thr Gly Asp Gly Thr Thr Ser Asn Val Leu
            85                  90                  95

atc att gga gag ctg ctg aaa cag gcg gat ctc tac att tct gaa ggc 394
Ile Ile Gly Glu Leu Leu Lys Gln Ala Asp Leu Tyr Ile Ser Glu Gly
        100                 105                 110

ctt cat cct aga ata atc act gaa gga ttt gaa gct gca aag gaa aag 442
Leu His Pro Arg Ile Ile Thr Glu Gly Phe Glu Ala Ala Lys Glu Lys
    115                 120                 125

gcc ctt cag ttt ttg gaa gaa gtc aaa gta agc aga gag atg gac agg 490
Ala Leu Gln Phe Leu Glu Glu Val Lys Val Ser Arg Glu Met Asp Arg

130                 135                 140                 145

gaa aca ctt ata gat gtg gcc aga aca tct ctt cgt act aaa gtt cat 538
Glu Thr Leu Ile Asp Val Ala Arg Thr Ser Leu Arg Thr Lys Val His
                150                 155                 160

gct gaa ctt gca gat gtc tta aca gag gct gta gtg gac tcc att ttg 586
Ala Glu Leu Ala Asp Val Leu Thr Glu Ala Val Val Asp Ser Ile Leu
            165                 170                 175

gcc att aaa aag caa gat gaa cct att gat ctc ttc atg att gag atc 634
Ala Ile Lys Lys Gln Asp Glu Pro Ile Asp Leu Phe Met Ile Glu Ile
        180                 185                 190

atg gag atg aaa cat aaa tct gaa act gat aca agc tta atc aga ggg 682
Met Glu Met Lys His Lys Ser Glu Thr Asp Thr Ser Leu Ile Arg Gly
    195                 200                 205

ctt gtt ttg gac cac gga gca cgg cat cct gat atg aag aaa agg gtg 730
Leu Val Leu Asp His Gly Ala Arg His Pro Asp Met Lys Lys Arg Val
210                 215                 220                 225

gag gat gca tac atc ctc act tgt aac gtg tca tta gag tat gag aaa 778
Glu Asp Ala Tyr Ile Leu Thr Cys Asn Val Ser Leu Glu Tyr Glu Lys
                230                 235                 240

aca gaa gtg aat tct ggc ttt ttt tac aag agt gca gaa gag aga gaa 826
Thr Glu Val Asn Ser Gly Phe Phe Tyr Lys Ser Ala Glu Glu Arg Glu
            245                 250                 255

aaa ctc gtg aaa gct gaa aga aaa ttc att gaa gat agg gtt aaa aaa 874
Lys Leu Val Lys Ala Glu Arg Lys Phe Ile Glu Asp Arg Val Lys Lys
        260                 265                 270

ata ata gaa ctg aaa agg aaa gtc tgt ggc gat tca gat aaa gga ttt 922
Ile Ile Glu Leu Lys Arg Lys Val Cys Gly Asp Ser Asp Lys Gly Phe
    275                 280                 285

gtt gtt att aat caa aag gga att gac ccc ttt tcc tta gat gct ctt 970
Val Val Ile Asn Gln Lys Gly Ile Asp Pro Phe Ser Leu Asp Ala Leu
290                 295                 300                 305

tca aaa gaa ggc ata gtc gct ctg cgc aga gct aaa agg aga aat atg 1018
Ser Lys Glu Gly Ile Val Ala Leu Arg Arg Ala Lys Arg Arg Asn Met
                310                 315                 320

gag agg ctg act ctt gct tgt ggt ggg gta gcc ctg aat tct ttt gac 1066
Glu Arg Leu Thr Leu Ala Cys Gly Gly Val Ala Leu Asn Ser Phe Asp
            325                 330                 335

gac cta agt cct gac tgc ttg gga cat gca gga ctt gta tat gag tat 1114
Asp Leu Ser Pro Asp Cys Leu Gly His Ala Gly Leu Val Tyr Glu Tyr
        340                 345                 350

aca ttg gga gaa gag aag ttt acc ttt att gag aaa tgt aac aac cct 1162
Thr Leu Gly Glu Glu Lys Phe Thr Phe Ile Glu Lys Cys Asn Asn Pro
    355                 360                 365

cgt tct gtc aca tta ttg atc aaa gga cca aat aag cac aca ctc act 1210
Arg Ser Val Thr Leu Leu Ile Lys Gly Pro Asn Lys His Thr Leu Thr
370                 375                 380                 385

cag atc aaa gat gca gtg agg gac ggc ttg agg gct gtc aaa aat gct 1258
Gln Ile Lys Asp Ala Val Arg Asp Gly Leu Arg Ala Val Lys Asn Ala
                390                 395                 400

att gat gat ggc tgt gtg gtt cca ggt gct ggt gcc gtg gaa gtg gca 1306
Ile Asp Asp Gly Cys Val Val Pro Gly Ala Gly Ala Val Glu Val Ala
            405                 410                 415

atg gca gaa gcc ctg att aaa cat aag ccc agt gta aag ggc agg gca 1354
Met Ala Glu Ala Leu Ile Lys His Lys Pro Ser Val Lys Gly Arg Ala
        420                 425                 430

cag ctt gga gtc caa gca ttt gct gat gca ttg ctc att att ccc aag 1402
Gln Leu Gly Val Gln Ala Phe Ala Asp Ala Leu Leu Ile Ile Pro Lys
    435                 440                 445

gtt ctt gct cag aac tct ggt ttt gac ctt cag gaa aca tta gtt aaa 1450
Val Leu Ala Gln Asn Ser Gly Phe Asp Leu Gln Glu Thr Leu Val Lys
450                 455                 460                 465

att caa gca gaa cat tca gaa tca ggt cag ctt gtg ggt gtg gac ctg 1498
Ile Gln Ala Glu His Ser Glu Ser Gly Gln Leu Val Gly Val Asp Leu
                470                 475                 480

aac aca ggt gag cca atg gtg gca gca gaa gta ggc gta tgg gat aac 1546
Asn Thr Gly Glu Pro Met Val Ala Ala Glu Val Gly Val Trp Asp Asn
            485                 490                 495

tat tgt gta aag aaa cag ctt ctt cac tcc tgc act gtg att gcc acc 1594
Tyr Cys Val Lys Lys Gln Leu Leu His Ser Cys Thr Val Ile Ala Thr
        500                 505                 510

aac att ctc ttg gtt gat gag atc atg cga gct gga atg tct tct ctg 1642
Asn Ile Leu Leu Val Asp Glu Ile Met Arg Ala Gly Met Ser Ser Leu
    515                 520                 525

aaa ggt tga attgaagctt cctctgtatc tgaatcttga agactgcaaa 1691
Lys Gly

530

gtgatcctga ggattacagc tgtggaattt ttgtccaagc ttcaaataat tttgaaagaa 1751

attttcccat atgaaaaaag gagagaacac tggcatctgt tgaaatttgg aagttctgaa 1811

attatagtat ttttaaaaat tgcactgaag tgtatacaca taaagcaggt cttttatcca 1871

gtgaacagga tgttttgctt tagcagcagt gacataaaat tccatgttag ataagcatat 1931

gttacttacc ttgttattaa atatttcttg aaaagcaaat tttaatggtt aattttatgt 1991

ggacgtatgt taaattatcc aaactaccct attgttaagc atttggtttt aaaattttta 2051

tgctaatata aatgctcaag taatttaaaa tattgaaagc atccctgttg gtataaattt 2111

ctgagtaaat gcattggatc agttggactt tgaacgccct ttgaaatggc tttgctaaaa 2171

tgctcccgcc acaaagttgt aggaaatggg aagaggagtc aactagaggc aagggagttg 2231

agagagctgc aactgtaaag ggcaagaaca ggcagaggta aaaagatgat ggaaggtgtg 2291

gtgactaagg gccacggtta ttgggtgaaa tttgagatgt aggccaactg tattttcaag 2351

cttctgaact taaggcaaaa tattcatcgc aaagtctcta gcgtcatatt tttctcaccc 2411

aaattacgtt tccacgagtt attatatata gttggtctat ctctgcagtc cttgaaggtg 2471

aagttgtgtg ttactaggct gtgttttggg atgtcagcag tggcctgaag tgagttgtgc 2531

aataaatgtt aagttgaaac ctcaaaaaaa a 2562

<210> 8
<211> 531
<212> PRT
<213> Homo sapiens

<400> 8

Met Ala Ala Val Lys Thr Leu Asn Pro Lys Ala Glu Val Ala Arg Ala
1               5                   10                  15

Gln Ala Ala Leu Ala Val Asn Ile Ser Ala Ala Arg Gly Leu Gln Asp
            20                  25                  30

Val Leu Arg Thr Asn Leu Gly Pro Lys Gly Thr Met Lys Met Leu Val
        35                  40                  45

Ser Gly Ala Gly Asp Ile Lys Leu Thr Lys Asp Gly Asn Val Leu Leu
    50                  55                  60

His Glu Met Gln Ile Gln His Pro Thr Ala Ser Leu Ile Ala Lys Val
65                  70                  75                  80

Ala Thr Ala Gln Asp Asp Ile Thr Gly Asp Gly Thr Thr Ser Asn Val
                85                  90                  95

Leu Ile Ile Gly Glu Leu Leu Lys Gln Ala Asp Leu Tyr Ile Ser Glu
            100                 105                 110

Gly Leu His Pro Arg Ile Ile Thr Glu Gly Phe Glu Ala Ala Lys Glu
        115                 120                 125

Lys Ala Leu Gln Phe Leu Glu Glu Val Lys Val Ser Arg Glu Met Asp
    130                 135                 140

Arg Glu Thr Leu Ile Asp Val Ala Arg Thr Ser Leu Arg Thr Lys Val
145                 150                 155                 160

His Ala Glu Leu Ala Asp Val Leu Thr Glu Ala Val Val Asp Ser Ile
                165                 170                 175

Leu Ala Ile Lys Lys Gln Asp Glu Pro Ile Asp Leu Phe Met Ile Glu
            180                 185                 190

Ile Met Glu Met Lys His Lys Ser Glu Thr Asp Thr Ser Leu Ile Arg
        195                 200                 205

Gly Leu Val Leu Asp His Gly Ala Arg His Pro Asp Met Lys Lys Arg
    210                 215                 220

Val Glu Asp Ala Tyr Ile Leu Thr Cys Asn Val Ser Leu Glu Tyr Glu
225                 230                 235                 240

Lys Thr Glu Val Asn Ser Gly Phe Phe Tyr Lys Ser Ala Glu Glu Arg
                245                 250                 255

Glu Lys Leu Val Lys Ala Glu Arg Lys Phe Ile Glu Asp Arg Val Lys
            260                 265                 270

Lys Ile Ile Glu Leu Lys Arg Lys Val Cys Gly Asp Ser Asp Lys Gly
        275                 280                 285

Phe Val Val Ile Asn Gln Lys Gly Ile Asp Pro Phe Ser Leu Asp Ala
    290                 295                 300

Leu Ser Lys Glu Gly Ile Val Ala Leu Arg Arg Ala Lys Arg Arg Asn
305                 310                 315                 320

Met Glu Arg Leu Thr Leu Ala Cys Gly Gly Val Ala Leu Asn Ser Phe
                325                 330                 335

Asp Asp Leu Ser Pro Asp Cys Leu Gly His Ala Gly Leu Val Tyr Glu
            340                 345                 350

Tyr Thr Leu Gly Glu Glu Lys Phe Thr Phe Ile Glu Lys Cys Asn Asn
        355                 360                 365

Pro Arg Ser Val Thr Leu Leu Ile Lys Gly Pro Asn Lys His Thr Leu
    370                 375                 380

Thr Gln Ile Lys Asp Ala Val Arg Asp Gly Leu Arg Ala Val Lys Asn
385                 390                 395                 400

Ala Ile Asp Asp Gly Cys Val Val Pro Gly Ala Gly Ala Val Glu Val
                405                 410                 415

Ala Met Ala Glu Ala Leu Ile Lys His Lys Pro Ser Val Lys Gly Arg
            420                 425                 430

Ala Gln Leu Gly Val Gln Ala Phe Ala Asp Ala Leu Leu Ile Ile Pro
        435                 440                 445

Lys Val Leu Ala Gln Asn Ser Gly Phe Asp Leu Gln Glu Thr Leu Val
    450                 455                 460

Lys Ile Gln Ala Glu His Ser Glu Ser Gly Gln Leu Val Gly Val Asp
465                 470                 475                 480

Leu Asn Thr Gly Glu Pro Met Val Ala Ala Glu Val Gly Val Trp Asp
                485                 490                 495

Asn Tyr Cys Val Lys Lys Gln Leu Leu His Ser Cys Thr Val Ile Ala
            500                 505                 510

Thr Asn Ile Leu Leu Val Asp Glu Ile Met Arg Ala Gly Met Ser Ser
        515                 520                 525

Leu Lys Gly
    530

<210>  9
<211>  2208
<212>  DNA
<213>  Homo sapiens

<220>
<221>  CDS
<222>  (230)..(1486)
<223>

<400>  9
tctttggctt tttttggcgg agctggggcg ccctccggaa gcgtttccaa ctttccagaa      60

gtttctcggg acgggcagga gggggtgggg actgccatat atagatcccg ggagcagggg     120

agcgggctaa gagtagaatc gtgtcgcggc tcgagagcga gagtcacgtc ccggcgctag     180

cccagcccga cccaggccca ccgtggtgca cgcaaaccac ttcctggcc atg cgc tcc     238
                                                      Met Arg Ser
                                                      1

ctc ctg ctt ctc agc gcc ttc tgc ctc ctg gag gcg gcc ctg gcc gcc       286
Leu Leu Leu Leu Ser Ala Phe Cys Leu Leu Glu Ala Ala Leu Ala Ala
    5                   10                  15

gag gtg aag aaa cct gca gcc gca gca gct cct ggc act gcg gag aag 334
Glu Val Lys Lys Pro Ala Ala Ala Ala Ala Pro Gly Thr Ala Glu Lys
20                  25                  30                  35

ttg agc ccc aag gcg gcc acg ctt gcc gag cgc agc gcc ggc ctg gcc 382
Leu Ser Pro Lys Ala Ala Thr Leu Ala Glu Arg Ser Ala Gly Leu Ala
                40                  45                  50

ttc agc ttg tac cag gcc atg gcc aag gac cag gca gtg gag aac atc 430
Phe Ser Leu Tyr Gln Ala Met Ala Lys Asp Gln Ala Val Glu Asn Ile
            55                  60                  65

ctg gtg tca ccc gtg gtg gtg gcc tcg tcg cta ggg ctc gtg tcg ctg 478
Leu Val Ser Pro Val Val Val Ala Ser Ser Leu Gly Leu Val Ser Leu
        70                  75                  80

ggc ggc aag gcg acc acg gcg tcg cag gcc aag gca gtg ctg agc gcc 526
Gly Gly Lys Ala Thr Thr Ala Ser Gln Ala Lys Ala Val Leu Ser Ala
    85                  90                  95

gag cag ctg cgc gac gag gag gtg cac gcc ggc ctg ggc gag ctg ctg 574
Glu Gln Leu Arg Asp Glu Glu Val His Ala Gly Leu Gly Glu Leu Leu
100                 105                 110                 115

cgc tca ctc agc aac tcc acg gcg cgc aac gtg acc tgg aag ctg ggc 622
Arg Ser Leu Ser Asn Ser Thr Ala Arg Asn Val Thr Trp Lys Leu Gly
                120                 125                 130

agc cga ctg tac gga ccc agc tca gtg agc ttc gct gat gac ttc gtg 670
Ser Arg Leu Tyr Gly Pro Ser Ser Val Ser Phe Ala Asp Asp Phe Val
            135                 140                 145

cgc agc agc aag cag cac tac aac tgc gag cac tcc aag atc aac ttc 718
Arg Ser Ser Lys Gln His Tyr Asn Cys Glu His Ser Lys Ile Asn Phe
        150                 155                 160

cgc gac aag cgc agc gcg ctg cag tcc atc aac gag tgg gcc gcg cag 766
Arg Asp Lys Arg Ser Ala Leu Gln Ser Ile Asn Glu Trp Ala Ala Gln
    165                 170                 175

acc acc gac ggc aag ctg ccc gag gtc acc aag gac gtg gag cgc acg 814
Thr Thr Asp Gly Lys Leu Pro Glu Val Thr Lys Asp Val Glu Arg Thr
180                 185                 190                 195

gac ggc gcc ctg cta gtc aac gcc atg ttc ttc aag cca cac tgg gat 862
Asp Gly Ala Leu Leu Val Asn Ala Met Phe Phe Lys Pro His Trp Asp
                200                 205                 210

gag aaa ttc cac cac aag atg gtg gac aac cgt ggc ttc atg gtg act 910

Glu Lys Phe His His Lys Met Val Asp Asn Arg Gly Phe Met Val Thr
            215                 220                 225

cgg tcc tat acc gtg ggt gtc atg atg atg cac cgg aca ggc ctc tac 958
Arg Ser Tyr Thr Val Gly Val Met Met Met His Arg Thr Gly Leu Tyr
        230                 235                 240

aac tac tac gac gac gag aag gaa aag ctg caa atc gtg gag atg ccc 1006
Asn Tyr Tyr Asp Asp Glu Lys Glu Lys Leu Gln Ile Val Glu Met Pro
    245                 250                 255

ctg gcc cac aag ctc tcc agc ctc atc atc ctc atg ccc cat cac gtg 1054
Leu Ala His Lys Leu Ser Ser Leu Ile Ile Leu Met Pro His His Val
260                 265                 270                 275

gag cct ctc gag cgc ctt gaa aag ctg cta acc aaa gag cag ctg aag 1102
Glu Pro Leu Glu Arg Leu Glu Lys Leu Leu Thr Lys Glu Gln Leu Lys
                280                 285                 290

atc tgg atg ggg aag atg cag aag aag gct gtt gcc atc tcc ttg ccc 1150
Ile Trp Met Gly Lys Met Gln Lys Lys Ala Val Ala Ile Ser Leu Pro
            295                 300                 305

aag ggt gtg gtg gag gtg acc cat gac ctg cag aaa cac ctg gct ggg 1198
Lys Gly Val Val Glu Val Thr His Asp Leu Gln Lys His Leu Ala Gly
        310                 315                 320

ctg ggc ctg act gag gcc att gac aag aac aag gcc gac ttg tca cgc 1246
Leu Gly Leu Thr Glu Ala Ile Asp Lys Asn Lys Ala Asp Leu Ser Arg
    325                 330                 335

atg tca ggc aag aag gac ctg tac ctg gcc agc gtg ttc cac gcc acc 1294
Met Ser Gly Lys Lys Asp Leu Tyr Leu Ala Ser Val Phe His Ala Thr
340                 345                 350                 355

gcc ttt gag ttg gac aca gat ggc aac ccc ttt gac cag gac atc tac 1342
Ala Phe Glu Leu Asp Thr Asp Gly Asn Pro Phe Asp Gln Asp Ile Tyr
                360                 365                 370

ggg cgc gag gag ctg cgc agc ccc aag ctg ttc tac gcc gac cac ccc 1390
Gly Arg Glu Glu Leu Arg Ser Pro Lys Leu Phe Tyr Ala Asp His Pro
            375                 380                 385

ttc atc ttc cta gtg cgg gac acc caa agc ggc tcc ctg cta ttc att 1438
Phe Ile Phe Leu Val Arg Asp Thr Gln Ser Gly Ser Leu Leu Phe Ile
        390                 395                 400

ggg cgc ctg gtc cgg cct aag ggt gac aag atg cga gac gag tta tag 1486
Gly Arg Leu Val Arg Pro Lys Gly Asp Lys Met Arg Asp Glu Leu
    405                 410                 415

ggcctcaggg tgcacacagg atggcaggag gcatccaaag gctcctgaga cacatgggtg 1546

ctattggggt tgggggggag gtgaggtacc agccttggat actccatggg gtgggggtgg 1606

aaaaacagac cggggttccc gtgtgcctga gcggaccttc ccagctagaa ttcactccac 1666

ttggacatgg gccccagata ccatgatgct gagcccggaa actccacatc ctgtgggacc 1726

tgggccatag tcattctgcc tgccctgaaa gtcccagatc aagcctgcct caatcagtat 1786

tcatatttat agccaggtac cttctcacct gtgagaccaa attgagctag gggggtcagc 1846

cagccctctt ctgacactaa aacacctcag ctgcctcccc agctctatcc caacctctcc 1906

caactataaa actaggtgct gcagcccctg ggaccaggca cccccagaat gacctggccg 1966

cagtgaggcg gattgagaag gagctcccag gaggggcttc tgggcagact ctggtcaaga 2026

agcatcgtgt ctggcgttgt ggggatgaac tttttgtttt gtttcttcct tttttagttc 2086

ttcaaagata gggagggaag ggggaacatg agcctttgtt gctatcaatc caagaactta 2146

tttgtacatt ttttttttca ataaaacttt tccaatgaca ttttgttgga gcgtggaaaa 2206

aa 2208

<210> 10
<211> 418
<212> PRT
<213> Homo sapiens

<400> 10

Met Arg Ser Leu Leu Leu Leu Ser Ala Phe Cys Leu Leu Glu Ala Ala
1               5                   10                  15

Leu Ala Ala Glu Val Lys Lys Pro Ala Ala Ala Ala Ala Pro Gly Thr
            20                  25                  30

Ala Glu Lys Leu Ser Pro Lys Ala Ala Thr Leu Ala Glu Arg Ser Ala
        35                  40                  45

Gly Leu Ala Phe Ser Leu Tyr Gln Ala Met Ala Lys Asp Gln Ala Val
    50                  55                  60

Glu Asn Ile Leu Val Ser Pro Val Val Val Ala Ser Ser Leu Gly Leu
65                  70                  75                  80

Val Ser Leu Gly Gly Lys Ala Thr Thr Ala Ser Gln Ala Lys Ala Val
                85                  90                  95

Leu Ser Ala Glu Gln Leu Arg Asp Glu Glu Val His Ala Gly Leu Gly
            100                 105                 110

Glu Leu Leu Arg Ser Leu Ser Asn Ser Thr Ala Arg Asn Val Thr Trp
        115                 120                 125

Lys Leu Gly Ser Arg Leu Tyr Gly Pro Ser Ser Val Ser Phe Ala Asp
    130                 135                 140

Asp Phe Val Arg Ser Ser Lys Gln His Tyr Asn Cys Glu His Ser Lys
145                 150                 155                 160

Ile Asn Phe Arg Asp Lys Arg Ser Ala Leu Gln Ser Ile Asn Glu Trp
                165                 170                 175

Ala Ala Gln Thr Thr Asp Gly Lys Leu Pro Glu Val Thr Lys Asp Val
            180                 185                 190

Glu Arg Thr Asp Gly Ala Leu Leu Val Asn Ala Met Phe Phe Lys Pro
        195                 200                 205

His Trp Asp Glu Lys Phe His His Lys Met Val Asp Asn Arg Gly Phe
    210                 215                 220

Met Val Thr Arg Ser Tyr Thr Val Gly Val Met Met Met His Arg Thr
225                 230                 235                 240

Gly Leu Tyr Asn Tyr Tyr Asp Asp Glu Lys Glu Lys Leu Gln Ile Val
                245                 250                 255

Glu Met Pro Leu Ala His Lys Leu Ser Ser Leu Ile Ile Leu Met Pro

260                 265                 270

His His Val Glu Pro Leu Glu Arg Leu Glu Lys Leu Leu Thr Lys Glu
        275                 280                 285

Gln Leu Lys Ile Trp Met Gly Lys Met Gln Lys Lys Ala Val Ala Ile
    290                 295                 300

Ser Leu Pro Lys Gly Val Val Glu Val Thr His Asp Leu Gln Lys His
305                 310                 315                 320

Leu Ala Gly Leu Gly Leu Thr Glu Ala Ile Asp Lys Asn Lys Ala Asp
                325                 330                 335

Leu Ser Arg Met Ser Gly Lys Lys Asp Leu Tyr Leu Ala Ser Val Phe
            340                 345                 350

His Ala Thr Ala Phe Glu Leu Asp Thr Asp Gly Asn Pro Phe Asp Gln
        355                 360                 365

Asp Ile Tyr Gly Arg Glu Glu Leu Arg Ser Pro Lys Leu Phe Tyr Ala
    370                 375                 380

Asp His Pro Phe Ile Phe Leu Val Arg Asp Thr Gln Ser Gly Ser Leu
385                 390                 395                 400

Leu Phe Ile Gly Arg Leu Val Arg Pro Lys Gly Asp Lys Met Arg Asp
                405                 410                 415

Glu Leu

<210>  11
<211>  1690
<212>  DNA
<213>  Homo sapiens

<220>
<221>  CDS

<222>  (77)..(1429)
<223>

<400>  11
ggcacgagga aggtttttgc tgcgccaacg cagtgaccga aggctccgct cacgcccggc      60

ctgatcctgc ctgaag atg gtg cca ctg gtg gct gtg gta tca ggg ccc cgt      112
                  Met Val Pro Leu Val Ala Val Val Ser Gly Pro Arg
                  1               5                   10

gcc cag ctc ttt gcc tgc ctg ctc agg ctg ggc act cag cag gtc ggc      160
Ala Gln Leu Phe Ala Cys Leu Leu Arg Leu Gly Thr Gln Gln Val Gly
        15                  20                  25

ccc ctt cag ctg cac acc ggg gcc agc cat gcg gcc agg aac cat tat      208
Pro Leu Gln Leu His Thr Gly Ala Ser His Ala Ala Arg Asn His Tyr
    30                  35                  40

gag gtg ctg gtg ctg ggt ggg ggc agt ggc gga atc acc atg gct gcc      256
Glu Val Leu Val Leu Gly Gly Gly Ser Gly Gly Ile Thr Met Ala Ala
45                  50                  55                  60

cgc atg aag agg aaa gtg ggt gca gag aat gtg gcc att gtt gag ccc      304
Arg Met Lys Arg Lys Val Gly Ala Glu Asn Val Ala Ile Val Glu Pro
                65                  70                  75

agt gag aga cat ttc tac cag cca atc tgg aca ctg gtg ggt gct ggt      352
Ser Glu Arg His Phe Tyr Gln Pro Ile Trp Thr Leu Val Gly Ala Gly
            80                  85                  90

gcc aaa caa ttg tcc tca tct ggt cgt ccc acg gca agt gtg att cca      400
Ala Lys Gln Leu Ser Ser Ser Gly Arg Pro Thr Ala Ser Val Ile Pro
        95                  100                 105

tct ggt gta gaa tgg atc aaa gct aga gtg act gag ttg aac cca gac      448
Ser Gly Val Glu Trp Ile Lys Ala Arg Val Thr Glu Leu Asn Pro Asp
    110                 115                 120

aag aac tgc att cac aca gat gac gac gag aag atc tcc tac cga tat      496
Lys Asn Cys Ile His Thr Asp Asp Asp Glu Lys Ile Ser Tyr Arg Tyr
125                 130                 135                 140

ctt att att gct ctc gga atc cag ctg gac tat gag aag att aaa ggc      544
Leu Ile Ile Ala Leu Gly Ile Gln Leu Asp Tyr Glu Lys Ile Lys Gly
                145                 150                 155

cta cct gaa ggt ttc gct cat ccc aaa ata ggg tcg aat tat tca gtt      592
Leu Pro Glu Gly Phe Ala His Pro Lys Ile Gly Ser Asn Tyr Ser Val
            160                 165                 170

aag act gta gag aag aca tgg aaa gct ctg cag gac ttc aaa gag ggc 640
Lys Thr Val Glu Lys Thr Trp Lys Ala Leu Gln Asp Phe Lys Glu Gly
        175                 180                 185

aat gcc atc ttc acc ttc cca aat act cca gtg aag tgt gct gga gcc 688
Asn Ala Ile Phe Thr Phe Pro Asn Thr Pro Val Lys Cys Ala Gly Ala
    190                 195                 200

cct cag aag atc atg tac tta tca gaa gcc tac ttc agg aag aca ggg 736
Pro Gln Lys Ile Met Tyr Leu Ser Glu Ala Tyr Phe Arg Lys Thr Gly
205                 210                 215                 220

aag cga tcc aag gcc aat atc att ttc aac act tct ctt gga gcc att 784
Lys Arg Ser Lys Ala Asn Ile Ile Phe Asn Thr Ser Leu Gly Ala Ile
                225                 230                 235

ttc ggg gtt aag aag tat gca gat gcc ctg cag gag atc atc cag gag 832
Phe Gly Val Lys Lys Tyr Ala Asp Ala Leu Gln Glu Ile Ile Gln Glu
            240                 245                 250

cgg aac ctc act gtt aac tac aag aaa aac ctc att gaa gtc cga gcc 880
Arg Asn Leu Thr Val Asn Tyr Lys Lys Asn Leu Ile Glu Val Arg Ala
        255                 260                 265

gat aaa caa gag gct gta ttt gag aac ctg gac aaa cca gga gag acc 928
Asp Lys Gln Glu Ala Val Phe Glu Asn Leu Asp Lys Pro Gly Glu Thr
    270                 275                 280

caa gtg att tca tat gaa atg ctt cat gtc aca cct cca atg agc cca 976
Gln Val Ile Ser Tyr Glu Met Leu His Val Thr Pro Pro Met Ser Pro
285                 290                 295                 300

cca gat gtc ctc aag acc agt cct gtg gct gat gct gct ggt tgg gtg 1024
Pro Asp Val Leu Lys Thr Ser Pro Val Ala Asp Ala Ala Gly Trp Val
                305                 310                 315

gat gtg gat aaa gaa act ctg caa cac agg agg tac cca aat gtg ttt 1072
Asp Val Asp Lys Glu Thr Leu Gln His Arg Arg Tyr Pro Asn Val Phe
            320                 325                 330

ggg att ggg gac tgc acc aac ctt cct acg tca aag acc gct gct gca 1120
Gly Ile Gly Asp Cys Thr Asn Leu Pro Thr Ser Lys Thr Ala Ala Ala
        335                 340                 345

gta gct gcc cag tca gga ata ctt gat agg aca att tct gta att atg 1168
Val Ala Ala Gln Ser Gly Ile Leu Asp Arg Thr Ile Ser Val Ile Met
    350                 355                 360

aag aat caa aca cca aca aag aag tat gat ggc tac aca tca tgt cca 1216
Lys Asn Gln Thr Pro Thr Lys Lys Tyr Asp Gly Tyr Thr Ser Cys Pro

365                 370                 375                 380

ctg gtg acc ggc tac aac cgt gtg att ctt gct gag ttt gac tac aaa      1264
Leu Val Thr Gly Tyr Asn Arg Val Ile Leu Ala Glu Phe Asp Tyr Lys
                385                 390                 395

gca gag ccg cta gaa acc ttc ccc ttt gat caa agc aaa gag cgc ctt      1312
Ala Glu Pro Leu Glu Thr Phe Pro Phe Asp Gln Ser Lys Glu Arg Leu
            400                 405                 410

tcc atg tat ctc atg aaa gct gac ctg atg cct ttc ctg tat tgg aat      1360
Ser Met Tyr Leu Met Lys Ala Asp Leu Met Pro Phe Leu Tyr Trp Asn
        415                 420                 425

atg atg cta agg ggt tac tgg gga gga cca gcg ttt ctg cgc aag ttg      1408
Met Met Leu Arg Gly Tyr Trp Gly Gly Pro Ala Phe Leu Arg Lys Leu
    430                 435                 440

ttt cat cta ggt atg agt taa ggatggctca gcacttgctc atcttggatg      1459
Phe His Leu Gly Met Ser
445                 450

gcttctgggc caaaactgca gtcactgaat gaccaagagc agcacgaagg acttggaacc      1519

tatccttgta aagagttcct tgatgggtaa tggtgaccaa atgcctccct tttcagtacc      1579

tttgaacagc aaccatgtgg gctactcatg atgggcttga ttctttggga ataataaaat      1639

gaaataatac ttttattttc tgaataaaag tttgtcactg aaaaaaaaaa a      1690

<210>  12
<211>  450
<212>  PRT
<213>  Homo sapiens

<400>  12

Met Val Pro Leu Val Ala Val Val Ser Gly Pro Arg Ala Gln Leu Phe
1               5                   10                  15

Ala Cys Leu Leu Arg Leu Gly Thr Gln Gln Val Gly Pro Leu Gln Leu
            20                  25                  30

His Thr Gly Ala Ser His Ala Ala Arg Asn His Tyr Glu Val Leu Val
        35                  40                  45

Leu Gly Gly Gly Ser Gly Gly Ile Thr Met Ala Ala Arg Met Lys Arg
    50                  55                  60

Lys Val Gly Ala Glu Asn Val Ala Ile Val Glu Pro Ser Glu Arg His
65                  70                  75                  80

Phe Tyr Gln Pro Ile Trp Thr Leu Val Gly Ala Gly Ala Lys Gln Leu
                85                  90                  95

Ser Ser Ser Gly Arg Pro Thr Ala Ser Val Ile Pro Ser Gly Val Glu
            100                 105                 110

Trp Ile Lys Ala Arg Val Thr Glu Leu Asn Pro Asp Lys Asn Cys Ile
        115                 120                 125

His Thr Asp Asp Asp Glu Lys Ile Ser Tyr Arg Tyr Leu Ile Ile Ala
    130                 135                 140

Leu Gly Ile Gln Leu Asp Tyr Glu Lys Ile Lys Gly Leu Pro Glu Gly
145                 150                 155                 160

Phe Ala His Pro Lys Ile Gly Ser Asn Tyr Ser Val Lys Thr Val Glu
                165                 170                 175

Lys Thr Trp Lys Ala Leu Gln Asp Phe Lys Glu Gly Asn Ala Ile Phe
            180                 185                 190

Thr Phe Pro Asn Thr Pro Val Lys Cys Ala Gly Ala Pro Gln Lys Ile
        195                 200                 205

Met Tyr Leu Ser Glu Ala Tyr Phe Arg Lys Thr Gly Lys Arg Ser Lys
    210                 215                 220

Ala Asn Ile Ile Phe Asn Thr Ser Leu Gly Ala Ile Phe Gly Val Lys
225                 230                 235                 240

Lys Tyr Ala Asp Ala Leu Gln Glu Ile Ile Gln Glu Arg Asn Leu Thr
                245                 250                 255

Val Asn Tyr Lys Lys Asn Leu Ile Glu Val Arg Ala Asp Lys Gln Glu
            260                 265                 270

Ala Val Phe Glu Asn Leu Asp Lys Pro Gly Glu Thr Gln Val Ile Ser
        275                 280                 285

Tyr Glu Met Leu His Val Thr Pro Pro Met Ser Pro Pro Asp Val Leu
    290                 295                 300

Lys Thr Ser Pro Val Ala Asp Ala Ala Gly Trp Val Asp Val Asp Lys
305                 310                 315                 320

Glu Thr Leu Gln His Arg Arg Tyr Pro Asn Val Phe Gly Ile Gly Asp
                325                 330                 335

Cys Thr Asn Leu Pro Thr Ser Lys Thr Ala Ala Ala Val Ala Ala Gln
            340                 345                 350

Ser Gly Ile Leu Asp Arg Thr Ile Ser Val Ile Met Lys Asn Gln Thr
        355                 360                 365

Pro Thr Lys Lys Tyr Asp Gly Tyr Thr Ser Cys Pro Leu Val Thr Gly
    370                 375                 380

Tyr Asn Arg Val Ile Leu Ala Glu Phe Asp Tyr Lys Ala Glu Pro Leu
385                 390                 395                 400

Glu Thr Phe Pro Phe Asp Gln Ser Lys Glu Arg Leu Ser Met Tyr Leu
                405                 410                 415

Met Lys Ala Asp Leu Met Pro Phe Leu Tyr Trp Asn Met Met Leu Arg
            420                 425                 430

Gly Tyr Trp Gly Gly Pro Ala Phe Leu Arg Lys Leu Phe His Leu Gly
        435                 440                 445

Met Ser
    450

<210>  13
<211>  2593
<212>  DNA
<213>  Homo sapiens

<220>
<221>  CDS
<222>  (49)..(2259)
<223>

<400>  13
ggccagcgcg tctgcttgtt cgtgtgtgtg tcgttgcagg ccttattc atg ggc tca      57
ccg ctg agg ttc gac ggg cgg gtg gta ctg gtc acc ggc gcg ggg gca      105
gga ttg ggc cga gcc tat gcc ctg gct ttt gca gaa aga gga gcg tta      153
gtt gtt gtg aat gat ttg gga ggg gac ttc aaa gga gtt ggt aaa ggc      201
tcc tta gct gct gat aag gtt gtt gaa gaa ata aga agg aga ggt gga      249
aaa gca gtg gcc aac tat gat tca gtg gaa gaa gga gag aag gtt gtg      297
aag aca gcc ctg gat gct ttt gga aga ata gat gtt gtg gtc aac aat      345
gct gga att ctg agg gat cgt tcc ttt gct agg ata agt gat gaa gac      393
tgg gat ata atc cac aga gtt cat ttg cgg ggt tca ttc caa gtg aca      441
cgg gca gca tgg gaa cac atg aag aaa cag aag tat gga agg att att 489
atg act tca tca gct tca gga ata tat ggc aac ttt ggc cag gcc aat 537
tat agt gct gca aag ttg ggt ctt ctg ggc ctt gca aat tct ctt gca 585
att gaa ggc agg aaa agc aac att cat tgt aac acc att gct cct aat 633
gcg gga tca cgg atg act cag aca gtt atg cct gaa gat ctt gtg gaa 681
gcc ctg aag cca gag tat gtg gca cct ctt gtc ctt tgg ctt tgt cac 729
gag agt tgt gag gag aat ggt ggc ttg ttt gag gtt gga gca gga tgg 777
att gga aaa tta cgc tgg gag cgg act ctt gga gct att gta aga caa 825
aag aat cac cca atg act cct gag gca gtc aag gct aac tgg aag aag 873
atc tgt gac ttt gag aat gcc agc aag cct cag agt atc caa gaa tca 921
act ggc agt ata att gaa gtt ctg agt aaa ata gat tca gaa gga gga 969
gtt tca gca aat cat act agt cgt gca acg tct aca gca aca tca gga 1017
ttt gct gga gct att ggc cag aaa ctc cct cca ttt tct tat gct tat 1065
acg gaa ctg gaa gct att atg tat gcc ctt gga gtg gga gcg tca atc 1113
aag gat cca aaa gat ttg aaa ttt att tat gaa gga agt tct gat ttc 1161
tcc tgt ttg ccc acc ttc gga gtt atc ata ggt cag aaa tct atg atg 1209
ggt gga gga tta gca gaa att cct gga ctt tca atc aac ttt gca aag 1257
gtt ctt cat gga gag cag tac tta gag tta tat aaa cca ctt ccc aga 1305
gca gga aaa tta aaa tgt gaa gca gtt gtt gct gat gtc cta gat aaa 1353
gga tcc ggt gta gtg att att atg gat gtc tat tct tat tct gag aag 1401
gaa ctt ata tgc cac aat cag ttc tct ctc ttt ctt gtt ggc tct gga 1449
ggc ttt ggt gga aaa cgg aca tca gac aaa gtc aag gta gct gta gcc 1497
ata cct aat aga cct cct gat gct gta ctt aca gat acc acc tct ctt 1545
aat cag gct gct ttg tac cgc ctc agt gga gac tgg aat ccc tta cac 1593
att gat cct aac ttt gct agt cta gca ggt ttt gac aag ccc ata tta 1641
cat gga tta tgt aca ttt gga ttt tct gcc agg cgt gtg tta cag cag 1689
ttt gca gat aat gat gtg tca aga ttc aag gca att aag gct cgt ttt 1737
gca aaa cca gta tat cca gga caa act cta caa act gag atg tgg aag 1785
gaa gga aac aga att cat ttt caa acc aag gtc caa gaa act gga gac 1833
att gtc att tca aat gca tat gtg gat ctt gca cca aca tct ggt act 1881
tca gct aag aca ccc tct gag ggc ggg aag ctt cag agt acc ttt gta 1929
ttt gag gaa ata gga cgc cgc cta aag gat att ggg cct gag gtg gtg 1977
aag aaa gta aat gct gta ttt gag tgg cat ata acc aaa ggc gga aat 2025
att ggg gct aag tgg act att gac ctg aaa agt ggt tct gga aaa gtg 2073
tac caa ggc cct gca aaa ggt gct gct gat aca aca atc ata ctt tca 2121
gat gaa gat ttc atg gag gtg gtc ctg ggc aag ctt gac cct cag aag 2169
gca ttc ttt agt ggc agg ctg aag gcc aga ggg aac atc atg ctg agc 2217
cag aaa ctt cag atg att ctt aaa gac tac gcc aag ctc tga 2259
agggcacact acactattaa taaaaatgga atcattaaat actctcttca cccaaatatg    2319
cttgattatt ctgcaaaagt gattagaact aagatgcagg ggaaattgct taacattttc    2379
agatatcaga taactgcaga ttttcatttt ctactaattt tcatgtatca ttatttttac    2439
aaggaactat atataagcta gcacatgatt atccttctgt tcttagatct gtatcttcat    2499
aataaaaaat tttgcccaag tcctgtttcc ttagaatttg tgatagcatt gataagttga    2559
aaggaaaatt aaatcaataa aggcctttga tacc                                2593

<210>  14
<211>  736
<212>  PRT
<213>  Homo sapiens

<400>  14

<210>  15
<211>  2113
<212>  DNA
<213>  Homo sapiens

<220>
<221>  CDS
<222>  (63)..(1694)
<223>

<400>  15
gtgcggttgg gaacgcggag cggacggatt cgattcaacg gggttccgga ccgcgctgcg      60

ct atg gag cag gtc aat gag ctg aag gag aaa ggc aac aag gcc ctg        107
   Met Glu Gln Val Asn Glu Leu Lys Glu Lys Gly Asn Lys Ala Leu
   1               5                   10                  15

agc gtg ggt aac atc gat gat gcc tta cag tgc tac tcc gaa gct att       155
Ser Val Gly Asn Ile Asp Asp Ala Leu Gln Cys Tyr Ser Glu Ala Ile
                20                  25                  30

aag ctg gat ccc cac aac cac gtg ctg tac agc aac cgt tct gct gcc       203
Lys Leu Asp Pro His Asn His Val Leu Tyr Ser Asn Arg Ser Ala Ala
            35                  40                  45

tat gcc aag aaa gga gac tac cag aag gct tat gag gat ggc tgc aag       251
Tyr Ala Lys Lys Gly Asp Tyr Gln Lys Ala Tyr Glu Asp Gly Cys Lys
        50                  55                  60

act gtc gac cta aag cct gac tgg ggc aag ggc tat tca cga aaa gca       299
Thr Val Asp Leu Lys Pro Asp Trp Gly Lys Gly Tyr Ser Arg Lys Ala
    65                  70                  75

gca gct cta gag ttc tta aac cgc ttt gaa gaa gcc aag cga acc tat       347
Ala Ala Leu Glu Phe Leu Asn Arg Phe Glu Glu Ala Lys Arg Thr Tyr
80                  85                  90                  95

gag gag ggc tta aaa cac gag gca aat aac cct caa ctg aaa gag ggt       395
Glu Glu Gly Leu Lys His Glu Ala Asn Asn Pro Gln Leu Lys Glu Gly
                100                 105                 110

tta cag aat atg gag gcc agg ttg gca gag aga aaa ttc atg aac cct       443
Leu Gln Asn Met Glu Ala Arg Leu Ala Glu Arg Lys Phe Met Asn Pro

            115                 120                 125

ttc aac atg cct aat ctg tat cag aag ttg gag agt gat ccc agg aca 491
Phe Asn Met Pro Asn Leu Tyr Gln Lys Leu Glu Ser Asp Pro Arg Thr
        130                 135                 140

agg aca cta ctc agt gat cct acc tac cgg gag ctg ata gag cag cta 539
Arg Thr Leu Leu Ser Asp Pro Thr Tyr Arg Glu Leu Ile Glu Gln Leu
    145                 150                 155

cga aac aag cct tct gac ctg ggc acg aaa cta caa gat ccc cgg atc 587
Arg Asn Lys Pro Ser Asp Leu Gly Thr Lys Leu Gln Asp Pro Arg Ile
160                 165                 170                 175

atg acc act ctc agc gtc ctc ctt ggg gtc gat ctg ggc agt atg gat 635
Met Thr Thr Leu Ser Val Leu Leu Gly Val Asp Leu Gly Ser Met Asp
                180                 185                 190

gag gag gaa gag att gca aca cct cca cca cca ccc cct ccc aaa aag 683
Glu Glu Glu Glu Ile Ala Thr Pro Pro Pro Pro Pro Pro Pro Lys Lys
            195                 200                 205

gag acc aag cca gag cca atg gaa gaa gat ctt cca gag aat aag aag 731
Glu Thr Lys Pro Glu Pro Met Glu Glu Asp Leu Pro Glu Asn Lys Lys
        210                 215                 220

cag gca ctg aaa gaa aaa gag ctg ggg aac gat gcc tac aag aag aaa 779
Gln Ala Leu Lys Glu Lys Glu Leu Gly Asn Asp Ala Tyr Lys Lys Lys
    225                 230                 235

gac ttt gac aca gcc ttg aag cat tac gac aaa gcc aag gag ctg gac 827
Asp Phe Asp Thr Ala Leu Lys His Tyr Asp Lys Ala Lys Glu Leu Asp
240                 245                 250                 255

ccc act aac atg act tac att acc aat caa gca gcg gta tac ttt gaa 875
Pro Thr Asn Met Thr Tyr Ile Thr Asn Gln Ala Ala Val Tyr Phe Glu
                260                 265                 270

aag ggc gac tac aat aag tgc cgg gag ctt tgt gag aag gcc att gaa 923
Lys Gly Asp Tyr Asn Lys Cys Arg Glu Leu Cys Glu Lys Ala Ile Glu
            275                 280                 285

gtg ggg aga gaa aac cga gaa gac tat cga cag att gcc aaa gca tat 971
Val Gly Arg Glu Asn Arg Glu Asp Tyr Arg Gln Ile Ala Lys Ala Tyr
        290                 295                 300

gct cga att ggc aac tcc tac ttc aaa gaa gaa aag tac aag gat gcc 1019
Ala Arg Ile Gly Asn Ser Tyr Phe Lys Glu Glu Lys Tyr Lys Asp Ala
    305                 310                 315

atc cat ttc tat aac aag tct ctg gca gag cac cga acc cca gat gtg 1067
Ile His Phe Tyr Asn Lys Ser Leu Ala Glu His Arg Thr Pro Asp Val
320                 325                 330                 335

ctc aag aaa tgc cag cag gca gag aaa atc ctg aag gag caa gag cgg 1115
Leu Lys Lys Cys Gln Gln Ala Glu Lys Ile Leu Lys Glu Gln Glu Arg
                340                 345                 350

ctg gcc tac ata aac ccc gac ctg gct ttg gag gag aag aac aaa ggc 1163
Leu Ala Tyr Ile Asn Pro Asp Leu Ala Leu Glu Glu Lys Asn Lys Gly
            355                 360                 365

aac gag tgt ttt cag aaa ggg gac tat ccc cag gcc atg aag cat tat 1211
Asn Glu Cys Phe Gln Lys Gly Asp Tyr Pro Gln Ala Met Lys His Tyr
        370                 375                 380

aca gaa gcc atc aaa agg aac ccg aaa gat gcc aaa tta tac agc aat 1259
Thr Glu Ala Ile Lys Arg Asn Pro Lys Asp Ala Lys Leu Tyr Ser Asn
    385                 390                 395

cga gct gcc tgc tac acc aaa ctc ctg gag ttc cag ctg gca ctc aag 1307
Arg Ala Ala Cys Tyr Thr Lys Leu Leu Glu Phe Gln Leu Ala Leu Lys
400                 405                 410                 415

gac tgt gag gaa tgt atc cag ctg gag ccg acc ttc atc aag ggt tat 1355
Asp Cys Glu Glu Cys Ile Gln Leu Glu Pro Thr Phe Ile Lys Gly Tyr
                420                 425                 430

aca cgg aaa gcc gct gcg ctg gaa gcg atg aag gac tac acc aaa gcc 1403
Thr Arg Lys Ala Ala Ala Leu Glu Ala Met Lys Asp Tyr Thr Lys Ala
            435                 440                 445

atg gat gtg tac cag aag gcg cta gac ctg gac tcc agc tgt aag gag 1451
Met Asp Val Tyr Gln Lys Ala Leu Asp Leu Asp Ser Ser Cys Lys Glu
        450                 455                 460

gcg gca gac ggc tac cag cgc tgt atg atg gcg cag tac aac cgg cac 1499
Ala Ala Asp Gly Tyr Gln Arg Cys Met Met Ala Gln Tyr Asn Arg His
    465                 470                 475

gac agc ccc gaa gat gtg aag cga cga gcc atg gcc gac cct gag gtg 1547
Asp Ser Pro Glu Asp Val Lys Arg Arg Ala Met Ala Asp Pro Glu Val
480                 485                 490                 495

cag cag atc atg agt gac cca gcc atg cgc ctt atc ctg gaa cag atg 1595
Gln Gln Ile Met Ser Asp Pro Ala Met Arg Leu Ile Leu Glu Gln Met
                500                 505                 510

cag aag gac ccc cag gca ctc agc gaa cac tta aag aat cct gta ata 1643
Gln Lys Asp Pro Gln Ala Leu Ser Glu His Leu Lys Asn Pro Val Ile

515                    520                    525

gca cag aag atc cag aag ctg atg gat gtg ggt ctg att gca att cgg      1691
Ala Gln Lys Ile Gln Lys Leu Met Asp Val Gly Leu Ile Ala Ile Arg
        530                    535                    540

tga tgacttgttc atcccccctt cccttcgccc tcatgtggaa agaggagctg           1744

ggaccgcggc gagcagcacg gagcggaagg gagagcaggg gagagaaggc ctcatctctc    1804

tatatttata cataaccccg gggaagacac agagactcgt acctgcgctg tttgtgccgc    1864

cgctgcctct gggccctccc agcacacgca tggtctcttc accgctgccc tcgagttcca    1924

tgtctctttc cctgccccct agttgctgtc tcggctgctc tcccatagtt ggtttttttt    1984

ttatttgggg cagtgggcat gttatgggga ggggaggggg ttcttccagc ctcaggtccc    2044

agctgtctca cgttgtttat tctgcgtccc cttctccaat aaaacaagcc agttgggcgt    2104

ggttataac                                                            2113

<210>  16
<211>  543
<212>  PRT
<213>  Homo sapiens

<400>  16

Met Glu Gln Val Asn Glu Leu Lys Glu Lys Gly Asn Lys Ala Leu Ser
1               5                   10                  15

Val Gly Asn Ile Asp Asp Ala Leu Gln Cys Tyr Ser Glu Ala Ile Lys
            20                  25                  30

Leu Asp Pro His Asn His Val Leu Tyr Ser Asn Arg Ser Ala Ala Tyr
        35                  40                  45

Ala Lys Lys Gly Asp Tyr Gln Lys Ala Tyr Glu Asp Gly Cys Lys Thr
    50                  55                  60

Val Asp Leu Lys Pro Asp Trp Gly Lys Gly Tyr Ser Arg Lys Ala Ala
65                  70                  75                  80

Ala Leu Glu Phe Leu Asn Arg Phe Glu Glu Ala Lys Arg Thr Tyr Glu
                85                  90                  95

Glu Gly Leu Lys His Glu Ala Asn Asn Pro Gln Leu Lys Glu Gly Leu
            100                 105                 110

Gln Asn Met Glu Ala Arg Leu Ala Glu Arg Lys Phe Met Asn Pro Phe
        115                 120                 125

Asn Met Pro Asn Leu Tyr Gln Lys Leu Glu Ser Asp Pro Arg Thr Arg
    130                 135                 140

Thr Leu Leu Ser Asp Pro Thr Tyr Arg Glu Leu Ile Glu Gln Leu Arg
145                 150                 155                 160

Asn Lys Pro Ser Asp Leu Gly Thr Lys Leu Gln Asp Pro Arg Ile Met
                165                 170                 175

Thr Thr Leu Ser Val Leu Leu Gly Val Asp Leu Gly Ser Met Asp Glu
            180                 185                 190

Glu Glu Glu Ile Ala Thr Pro Pro Pro Pro Pro Pro Pro Lys Lys Glu
        195                 200                 205

Thr Lys Pro Glu Pro Met Glu Glu Asp Leu Pro Glu Asn Lys Lys Gln
    210                 215                 220

Ala Leu Lys Glu Lys Glu Leu Gly Asn Asp Ala Tyr Lys Lys Lys Asp
225                 230                 235                 240

Phe Asp Thr Ala Leu Lys His Tyr Asp Lys Ala Lys Glu Leu Asp Pro
                245                 250                 255

Thr Asn Met Thr Tyr Ile Thr Asn Gln Ala Ala Val Tyr Phe Glu Lys
            260                 265                 270

Gly Asp Tyr Asn Lys Cys Arg Glu Leu Cys Glu Lys Ala Ile Glu Val
        275                 280                 285

Gly Arg Glu Asn Arg Glu Asp Tyr Arg Gln Ile Ala Lys Ala Tyr Ala
    290                 295                 300

Arg Ile Gly Asn Ser Tyr Phe Lys Glu Glu Lys Tyr Lys Asp Ala Ile
305                 310                 315                 320

His Phe Tyr Asn Lys Ser Leu Ala Glu His Arg Thr Pro Asp Val Leu
                325                 330                 335

Lys Lys Cys Gln Gln Ala Glu Lys Ile Leu Lys Glu Gln Glu Arg Leu
            340                 345                 350

Ala Tyr Ile Asn Pro Asp Leu Ala Leu Glu Glu Lys Asn Lys Gly Asn
        355                 360                 365

Glu Cys Phe Gln Lys Gly Asp Tyr Pro Gln Ala Met Lys His Tyr Thr
    370                 375                 380

Glu Ala Ile Lys Arg Asn Pro Lys Asp Ala Lys Leu Tyr Ser Asn Arg
385                 390                 395                 400

Ala Ala Cys Tyr Thr Lys Leu Leu Glu Phe Gln Leu Ala Leu Lys Asp
                405                 410                 415

Cys Glu Glu Cys Ile Gln Leu Glu Pro Thr Phe Ile Lys Gly Tyr Thr
            420                 425                 430

Arg Lys Ala Ala Ala Leu Glu Ala Met Lys Asp Tyr Thr Lys Ala Met
        435                 440                 445

Asp Val Tyr Gln Lys Ala Leu Asp Leu Asp Ser Ser Cys Lys Glu Ala
    450                 455                 460

Ala Asp Gly Tyr Gln Arg Cys Met Met Ala Gln Tyr Asn Arg His Asp
465                 470                 475                 480

Ser Pro Glu Asp Val Lys Arg Arg Ala Met Ala Asp Pro Glu Val Gln
                485                 490                 495

Gln Ile Met Ser Asp Pro Ala Met Arg Leu Ile Leu Glu Gln Met Gln
            500                 505                 510

Lys Asp Pro Gln Ala Leu Ser Glu His Leu Lys Asn Pro Val Ile Ala
        515                 520                 525

Gln Lys Ile Gln Lys Leu Met Asp Val Gly Leu Ile Ala Ile Arg
    530                 535                 540

<210>  17
<211>  2033
<212>  DNA
<213>  Homo sapiens

<220>
<221>  CDS
<222>  (29)..(1705)
<223>

<400>  17
ggacgagcag cggaggcggt cgggagcg atg gtg aag atg gcg gcg gcg ggc      52
                               Met Val Lys Met Ala Ala Ala Gly
                               1               5

ggc gga ggc ggc ggt ggc cgc tac tac ggc ggc ggc agt gag ggc ggc      100
Gly Gly Gly Gly Gly Gly Arg Tyr Tyr Gly Gly Gly Ser Glu Gly Gly
    10                  15                  20

cgg gcc cct aag cgg ctc aag act gac aac gcc ggc gac cag cac gga      148
Arg Ala Pro Lys Arg Leu Lys Thr Asp Asn Ala Gly Asp Gln His Gly
25                  30                  35                  40

ggc ggc ggc ggt ggc ggt gga gga gcc ggg gcg gcg ggc ggc ggc ggc      196
Gly Gly Gly Gly Gly Gly Gly Gly Ala Gly Ala Ala Gly Gly Gly Gly
                45                  50                  55

ggt ggg gag aac tac gat gac ccg cac aaa acc cct gcc tcc cca gtt      244
Gly Gly Glu Asn Tyr Asp Asp Pro His Lys Thr Pro Ala Ser Pro Val
            60                  65                  70

gtc cac atc agg ggc ctg att gac ggt gtg gtg gaa gca gac ctt gtg      292
Val His Ile Arg Gly Leu Ile Asp Gly Val Val Glu Ala Asp Leu Val
        75                  80                  85

gag gcc ttg cag gag ttt gga ccc atc agc tat gtg gtg gta atg cct 340
Glu Ala Leu Gln Glu Phe Gly Pro Ile Ser Tyr Val Val Val Met Pro
    90                  95                  100

aaa aag aga caa gca ctg gtg gag ttt gaa gat gtg ttg ggg gct tgc 388
Lys Lys Arg Gln Ala Leu Val Glu Phe Glu Asp Val Leu Gly Ala Cys
105                 110                 115                 120

aac gca gtg aac tac gca gcc gac aac caa ata tac att gct ggt cac 436
Asn Ala Val Asn Tyr Ala Ala Asp Asn Gln Ile Tyr Ile Ala Gly His
                125                 130                 135

cca gct ttt gtc aac tac tct acc agc cag aag atc tcc cgc cct ggg 484
Pro Ala Phe Val Asn Tyr Ser Thr Ser Gln Lys Ile Ser Arg Pro Gly
            140                 145                 150

gac tcg gat gac tcc cgg agc gtg aac agt gtg ctt ctc ttt acc atc 532
Asp Ser Asp Asp Ser Arg Ser Val Asn Ser Val Leu Leu Phe Thr Ile
        155                 160                 165

ctg aac ccc att tat tcg atc acc acg gat gtt ctt tac act atc tgt 580
Leu Asn Pro Ile Tyr Ser Ile Thr Thr Asp Val Leu Tyr Thr Ile Cys
    170                 175                 180

aat cct tgt ggc cct gtc cag aga att gtc att ttc agg aag aat gga 628
Asn Pro Cys Gly Pro Val Gln Arg Ile Val Ile Phe Arg Lys Asn Gly
185                 190                 195                 200

gtt cag gcg atg gtg gaa ttt gac tca gtt caa agt gcc cag cgg gcc 676
Val Gln Ala Met Val Glu Phe Asp Ser Val Gln Ser Ala Gln Arg Ala
                205                 210                 215

aag gcc tct ctc aat ggg gct gat atc tat tct ggc tgt tgc act ctg 724
Lys Ala Ser Leu Asn Gly Ala Asp Ile Tyr Ser Gly Cys Cys Thr Leu
            220                 225                 230

aag atc gaa tac gca aag cct aca cgc ttg aat gtg ttc aag aat gat 772
Lys Ile Glu Tyr Ala Lys Pro Thr Arg Leu Asn Val Phe Lys Asn Asp
        235                 240                 245

cag gat act tgg gac tac aca aac ccc aat ctc agt gga caa ggt gac 820
Gln Asp Thr Trp Asp Tyr Thr Asn Pro Asn Leu Ser Gly Gln Gly Asp
    250                 255                 260

cct ggc agc aac ccc aac aaa cgc cag agg cag ccc cct ctc ctg gga 868
Pro Gly Ser Asn Pro Asn Lys Arg Gln Arg Gln Pro Pro Leu Leu Gly
265                 270                 275                 280

gat cac ccc gca gaa tat gga ggg ccc cac ggt ggg tac cac agc cat 916

Asp His Pro Ala Glu Tyr Gly Gly Pro His Gly Gly Tyr His Ser His
                285                 290                 295

tac cat gat gag ggc tac ggg ccc ccc cca cct cac tac gaa ggg aga     964
Tyr His Asp Glu Gly Tyr Gly Pro Pro Pro Pro His Tyr Glu Gly Arg
            300                 305                 310

agg atg ggt cca cca gtg ggg ggt cac cgt cgg ggc cca agt cgc tac     1012
Arg Met Gly Pro Pro Val Gly Gly His Arg Arg Gly Pro Ser Arg Tyr
        315                 320                 325

ggc ccc cag tat ggg cac ccc cca ccc cct ccc cca cca ccc gag tat     1060
Gly Pro Gln Tyr Gly His Pro Pro Pro Pro Pro Pro Pro Pro Glu Tyr
    330                 335                 340

ggc cct cac gcc gac agc cct gtg ctc atg gtc tat ggc ttg gat caa     1108
Gly Pro His Ala Asp Ser Pro Val Leu Met Val Tyr Gly Leu Asp Gln
345                 350                 355                 360

tct aag atg aac ggt gac cga gtc ttc aat gtc ttc tgc tta tat ggc     1156
Ser Lys Met Asn Gly Asp Arg Val Phe Asn Val Phe Cys Leu Tyr Gly
                365                 370                 375

aat gtg gag aag gtg aaa ttc atg aaa agc aag ccg ggg gcc gcc atg     1204
Asn Val Glu Lys Val Lys Phe Met Lys Ser Lys Pro Gly Ala Ala Met
            380                 385                 390

gtg gag atg gct gat ggc tac gct gta gac cgg gcc att acc cac ctc     1252
Val Glu Met Ala Asp Gly Tyr Ala Val Asp Arg Ala Ile Thr His Leu
        395                 400                 405

aac aac aac ttc atg ttt ggg cag aag ctg aat gtc tgt gtc tcc aag     1300
Asn Asn Asn Phe Met Phe Gly Gln Lys Leu Asn Val Cys Val Ser Lys
    410                 415                 420

cag cca gcc atc atg cct ggt cag tca tac ggg ttg gaa gac ggg tct     1348
Gln Pro Ala Ile Met Pro Gly Gln Ser Tyr Gly Leu Glu Asp Gly Ser
425                 430                 435                 440

tgc agt tac aaa gac ttc agt gaa tcc cgg aac aat cgg ttc tcc acc     1396
Cys Ser Tyr Lys Asp Phe Ser Glu Ser Arg Asn Asn Arg Phe Ser Thr
                445                 450                 455

cca gag cag gca gcc aag aac cgc atc cag cac ccc agc aac gtg ctg     1444
Pro Glu Gln Ala Ala Lys Asn Arg Ile Gln His Pro Ser Asn Val Leu
            460                 465                 470

cac ttc ttc aac gcc ccg ctg gag gtg acc gag gag aac ttc ttt gag     1492
His Phe Phe Asn Ala Pro Leu Glu Val Thr Glu Glu Asn Phe Phe Glu
        475                 480                 485

atc tgc gat gag ctg gga gtg aag cgg cca tct tct gtg aaa gta ttc 1540
Ile Cys Asp Glu Leu Gly Val Lys Arg Pro Ser Ser Val Lys Val Phe
    490                 495                 500

tca ggc aaa agt gag cgc agc tcc tct gga ctg ctg gag tgg gaa tcc 1588
Ser Gly Lys Ser Glu Arg Ser Ser Ser Gly Leu Leu Glu Trp Glu Ser
505                 510                 515                 520

aag agc gat gcc ctg gag act ctg ggc ttc ctg aac cat tac cag atg 1636
Lys Ser Asp Ala Leu Glu Thr Leu Gly Phe Leu Asn His Tyr Gln Met
                525                 530                 535

aaa aac cca aat ggt cca tac cct tac act ctg aag ttg tgt ttc tcc 1684
Lys Asn Pro Asn Gly Pro Tyr Pro Tyr Thr Leu Lys Leu Cys Phe Ser
            540                 545                 550

act gct cag cac gcc tcc taa ttaggtgcct aggaagagtc ccatctgagc 1735
Thr Ala Gln His Ala Ser
        555

aggaagacat ttctctttcc tttatgccat tttttgtttt tgttatttgc aaaagatctt 1795

gtattccttt tttttttttt ttttttttaa atgctaggtt tgtagaggct tacttaacct 1855

taatggaaac gctggaaatc tgcaggggga gggagagggg aactgttatc tcccaagatt 1915

aaccttcact tttaaaaaat tattgtacat gtgatttttt tttttcctgt tcatacattt 1975

gtgctgccca tgtactcttg gcacatttca ataaaattgt ttggaaaata aacacagc 2033

<210> 18
<211> 558
<212> PRT
<213> Homo sapiens

<400> 18

Met Val Lys Met Ala Ala Ala Gly Gly Gly Gly Gly Gly Gly Arg Tyr
1               5                   10                  15

Tyr Gly Gly Gly Ser Glu Gly Gly Arg Ala Pro Lys Arg Leu Lys Thr
            20                  25                  30

Asp Asn Ala Gly Asp Gln His Gly Gly Gly Gly Gly Gly Gly Gly Gly
        35                  40                  45

Ala Gly Ala Ala Gly Gly Gly Gly Gly Gly Glu Asn Tyr Asp Asp Pro
    50                  55                  60

His Lys Thr Pro Ala Ser Pro Val Val His Ile Arg Gly Leu Ile Asp
65                  70                  75                  80

Gly Val Val Glu Ala Asp Leu Val Glu Ala Leu Gln Glu Phe Gly Pro
                85                  90                  95

Ile Ser Tyr Val Val Val Met Pro Lys Lys Arg Gln Ala Leu Val Glu
            100                 105                 110

Phe Glu Asp Val Leu Gly Ala Cys Asn Ala Val Asn Tyr Ala Ala Asp
        115                 120                 125

Asn Gln Ile Tyr Ile Ala Gly His Pro Ala Phe Val Asn Tyr Ser Thr
    130                 135                 140

Ser Gln Lys Ile Ser Arg Pro Gly Asp Ser Asp Asp Ser Arg Ser Val
145                 150                 155                 160

Asn Ser Val Leu Leu Phe Thr Ile Leu Asn Pro Ile Tyr Ser Ile Thr
                165                 170                 175

Thr Asp Val Leu Tyr Thr Ile Cys Asn Pro Cys Gly Pro Val Gln Arg
            180                 185                 190

Ile Val Ile Phe Arg Lys Asn Gly Val Gln Ala Met Val Glu Phe Asp
        195                 200                 205

Ser Val Gln Ser Ala Gln Arg Ala Lys Ala Ser Leu Asn Gly Ala Asp
    210                 215                 220

Ile Tyr Ser Gly Cys Cys Thr Leu Lys Ile Glu Tyr Ala Lys Pro Thr
225                 230                 235                 240

Arg Leu Asn Val Phe Lys Asn Asp Gln Asp Thr Trp Asp Tyr Thr Asn

                245                 250                 255

Pro Asn Leu Ser Gly Gln Gly Asp Pro Gly Ser Asn Pro Asn Lys Arg
            260                 265                 270

Gln Arg Gln Pro Pro Leu Leu Gly Asp His Pro Ala Glu Tyr Gly Gly
        275                 280                 285

Pro His Gly Gly Tyr His Ser His Tyr His Asp Glu Gly Tyr Gly Pro
    290                 295                 300

Pro Pro Pro His Tyr Glu Gly Arg Arg Met Gly Pro Pro Val Gly Gly
305                 310                 315                 320

His Arg Arg Gly Pro Ser Arg Tyr Gly Pro Gln Tyr Gly His Pro Pro
                325                 330                 335

Pro Pro Pro Pro Pro Pro Glu Tyr Gly Pro His Ala Asp Ser Pro Val
            340                 345                 350

Leu Met Val Tyr Gly Leu Asp Gln Ser Lys Met Asn Gly Asp Arg Val
        355                 360                 365

Phe Asn Val Phe Cys Leu Tyr Gly Asn Val Glu Lys Val Lys Phe Met
    370                 375                 380

Lys Ser Lys Pro Gly Ala Ala Met Val Glu Met Ala Asp Gly Tyr Ala
385                 390                 395                 400

Val Asp Arg Ala Ile Thr His Leu Asn Asn Asn Phe Met Phe Gly Gln
                405                 410                 415

Lys Leu Asn Val Cys Val Ser Lys Gln Pro Ala Ile Met Pro Gly Gln
            420                 425                 430

Ser Tyr Gly Leu Glu Asp Gly Ser Cys Ser Tyr Lys Asp Phe Ser Glu
        435                 440                 445

Ser Arg Asn Asn Arg Phe Ser Thr Pro Glu Gln Ala Ala Lys Asn Arg
    450                 455                 460

Ile Gln His Pro Ser Asn Val Leu His Phe Phe Asn Ala Pro Leu Glu
465                 470                 475                 480

Val Thr Glu Glu Asn Phe Phe Glu Ile Cys Asp Glu Leu Gly Val Lys
                485                 490                 495

Arg Pro Ser Ser Val Lys Val Phe Ser Gly Lys Ser Glu Arg Ser Ser
            500                 505                 510

Ser Gly Leu Leu Glu Trp Glu Ser Lys Ser Asp Ala Leu Glu Thr Leu
        515                 520                 525

Gly Phe Leu Asn His Tyr Gln Met Lys Asn Pro Asn Gly Pro Tyr Pro
    530                 535                 540

Tyr Thr Leu Lys Leu Cys Phe Ser Thr Ala Gln His Ala Ser
545                 550                 555

<210>  19
<211>  3465
<212>  DNA
<213>  Homo sapiens

<220>
<221>  CDS
<222>  (219)..(2639)
<223>

<400>  19
ctcgcgccag gcgagtctcc gcgtctccct cgcgaactcg gtgaaaggaa ttggcgccgt      60

tcgacaccag gcggatccgc tctgcagcac gaacccatct ccagccgcag ccgcagccgc     120

cgcccgggcc gaggagcagc cgcagcagcc gccaccagtg gccgagtgag cggagccgag     180

tttgaggcag cgcctagcgg tgaatcgggg ccctcacc atg agt tcc tcg cct gtt     236
                                          Met Ser Ser Ser Pro Val
                                          1               5

aat gta aaa aag ctg aag gtg tcg gag ctg aaa gag gag ctc aag aag 284
Asn Val Lys Lys Leu Lys Val Ser Glu Leu Lys Glu Glu Leu Lys Lys
            10                  15                  20

cga cgc ctt tct gac aag ggt ctc aag gcc gag ctc atg gag cga ctc 332
Arg Arg Leu Ser Asp Lys Gly Leu Lys Ala Glu Leu Met Glu Arg Leu
        25                  30                  35

cag gct gcg ctg gac gac gag gag gcc ggg ggc cgc ccc gcc atg gag 380
Gln Ala Ala Leu Asp Asp Glu Glu Ala Gly Gly Arg Pro Ala Met Glu
    40                  45                  50

ccc ggg aac ggc agc cta gac ctg ggc ggg gat tcc gct ggg cgc tcg 428
Pro Gly Asn Gly Ser Leu Asp Leu Gly Gly Asp Ser Ala Gly Arg Ser
55                  60                  65                  70

gga gca ggc ctc gag cag gag gcc gcg gcc ggc ggc gat gaa gag gag 476
Gly Ala Gly Leu Glu Gln Glu Ala Ala Ala Gly Gly Asp Glu Glu Glu
                75                  80                  85

gag gaa gag gaa gag gag gag gaa gga atc tcc gct ctg gac ggc gac 524
Glu Glu Glu Glu Glu Glu Glu Glu Gly Ile Ser Ala Leu Asp Gly Asp
            90                  95                  100

cag atg gag cta gga gag gag aac ggg gcc gcg ggg gcg gcc gac tcg 572
Gln Met Glu Leu Gly Glu Glu Asn Gly Ala Ala Gly Ala Ala Asp Ser
        105                 110                 115

ggc ccg atg gag gag gag gag gcc gcc tcg gaa gac gag aac ggc gac 620
Gly Pro Met Glu Glu Glu Glu Ala Ala Ser Glu Asp Glu Asn Gly Asp
    120                 125                 130

gat cag ggt ttc cag gaa ggg gaa gat gag ctc ggg gac gaa gag gaa 668
Asp Gln Gly Phe Gln Glu Gly Glu Asp Glu Leu Gly Asp Glu Glu Glu
135                 140                 145                 150

ggc gcg ggc gac gag aac ggg cac ggg gag cag cag cct caa ccg ccg 716
Gly Ala Gly Asp Glu Asn Gly His Gly Glu Gln Gln Pro Gln Pro Pro
                155                 160                 165

gcg acg cag cag caa cag ccc caa cag cag cgc ggg gcc gcc aag gag 764
Ala Thr Gln Gln Gln Gln Pro Gln Gln Gln Arg Gly Ala Ala Lys Glu
            170                 175                 180

gcc gcg ggg aag agc agc ggc ccc acc tcg ctg ttc gcg gtg acg gtg 812
Ala Ala Gly Lys Ser Ser Gly Pro Thr Ser Leu Phe Ala Val Thr Val
        185                 190                 195

gcg ccg ccc ggg gcg agg cag ggc cag cag cag gcg gga ggg gac ggc 860
Ala Pro Pro Gly Ala Arg Gln Gly Gln Gln Gln Ala Gly Gly Asp Gly

200                 205                 210

aaa aca gaa cag aaa ggc gga gat aaa aag agg ggt gtt aaa aga cca          908
Lys Thr Glu Gln Lys Gly Gly Asp Lys Lys Arg Gly Val Lys Arg Pro
215                 220                 225                 230

cga gaa gat cat ggc cgt gga tat ttt gag tac att gaa gag aac aag          956
Arg Glu Asp His Gly Arg Gly Tyr Phe Glu Tyr Ile Glu Glu Asn Lys
                235                 240                 245

tat agc aga gcc aaa tct cct cag cca cct gtt gaa gaa gaa gat gaa          1004
Tyr Ser Arg Ala Lys Ser Pro Gln Pro Pro Val Glu Glu Glu Asp Glu
            250                 255                 260

cac ttc gat gac aca gtg gtt tgt ctt gat act tat aat tgt gat cta          1052
His Phe Asp Asp Thr Val Val Cys Leu Asp Thr Tyr Asn Cys Asp Leu
        265                 270                 275

cat ttt aaa ata tca aga gat cgt ctc agt gct tct tcc ctt aca atg          1100
His Phe Lys Ile Ser Arg Asp Arg Leu Ser Ala Ser Ser Leu Thr Met
    280                 285                 290

gag agt ttt gct ttt ctt tgg gct gga gga aga gca tcc tat ggt gtg          1148
Glu Ser Phe Ala Phe Leu Trp Ala Gly Gly Arg Ala Ser Tyr Gly Val
295                 300                 305                 310

tca aaa ggc aaa gtg tgt ttt gag atg aag gtt aca gag aag atc cca          1196
Ser Lys Gly Lys Val Cys Phe Glu Met Lys Val Thr Glu Lys Ile Pro
                315                 320                 325

gta agg cat tta tat aca aaa gat att gac ata cat gaa gtt cgt att          1244
Val Arg His Leu Tyr Thr Lys Asp Ile Asp Ile His Glu Val Arg Ile
            330                 335                 340

ggc tgg tca cta act aca agt gga atg tta ctt ggt gaa gaa gaa ttt          1292
Gly Trp Ser Leu Thr Thr Ser Gly Met Leu Leu Gly Glu Glu Glu Phe
        345                 350                 355

tct tat ggg tat tct cta aaa gga ata aaa aca tgc aac tgt gag act          1340
Ser Tyr Gly Tyr Ser Leu Lys Gly Ile Lys Thr Cys Asn Cys Glu Thr
    360                 365                 370

gaa gat tat gga gaa aag ttt gat gaa aat gat gtg att aca tgt ttt          1388
Glu Asp Tyr Gly Glu Lys Phe Asp Glu Asn Asp Val Ile Thr Cys Phe
375                 380                 385                 390

gct aac ttt gaa agt gat gaa gta gaa ctc tcg tat gct aag aat gga          1436
Ala Asn Phe Glu Ser Asp Glu Val Glu Leu Ser Tyr Ala Lys Asn Gly
                395                 400                 405

caa gat ctt ggc gtt gcc ttc aaa atc agt aag gaa gtt ctt gct gga 1484
Gln Asp Leu Gly Val Ala Phe Lys Ile Ser Lys Glu Val Leu Ala Gly
            410                 415                 420

cgg cca ctg ttc ccg cat gtt ctc tgc cac aac tgt gca gtt gaa ttt 1532
Arg Pro Leu Phe Pro His Val Leu Cys His Asn Cys Ala Val Glu Phe
        425                 430                 435

aat ttt ggt cag aag gaa aag cca tat ttt cca ata cct gaa gag tat 1580
Asn Phe Gly Gln Lys Glu Lys Pro Tyr Phe Pro Ile Pro Glu Glu Tyr
    440                 445                 450

act ttc atc cag aac gtc ccc tta gag gat cga gtt aga gga cca aag 1628
Thr Phe Ile Gln Asn Val Pro Leu Glu Asp Arg Val Arg Gly Pro Lys
455                 460                 465                 470

ggg cct gaa gag aag aaa gat tgt gaa gtt gtg atg atg att ggc ttg 1676
Gly Pro Glu Glu Lys Lys Asp Cys Glu Val Val Met Met Ile Gly Leu
                475                 480                 485

cca gga gct gga aaa act acc tgg gtt act aaa cat gca gca gaa aat 1724
Pro Gly Ala Gly Lys Thr Thr Trp Val Thr Lys His Ala Ala Glu Asn
            490                 495                 500

cca ggg aaa tat aac att ctt ggc aca aat act att atg gat aag atg 1772
Pro Gly Lys Tyr Asn Ile Leu Gly Thr Asn Thr Ile Met Asp Lys Met
        505                 510                 515

atg gtg gca ggt ttt aag aag caa atg gca gat act gga aaa ctg aac 1820
Met Val Ala Gly Phe Lys Lys Gln Met Ala Asp Thr Gly Lys Leu Asn
    520                 525                 530

aca ctg ttg cag aga gcc ccc cag tgt ctt ggg aaa ttt att gag att 1868
Thr Leu Leu Gln Arg Ala Pro Gln Cys Leu Gly Lys Phe Ile Glu Ile
535                 540                 545                 550

gct gcc cga aag aag cga aat ttt att ctg gat cag aca aat gtg tct 1916
Ala Ala Arg Lys Lys Arg Asn Phe Ile Leu Asp Gln Thr Asn Val Ser
                555                 560                 565

gct gct gcc cag agg aga aaa atg tgc ctg ttt gca ggc ttc cag cga 1964
Ala Ala Ala Gln Arg Arg Lys Met Cys Leu Phe Ala Gly Phe Gln Arg
            570                 575                 580

aaa gct gtt gta gtt tgc cca aaa gat gaa gac tat aag caa aga aca 2012
Lys Ala Val Val Val Cys Pro Lys Asp Glu Asp Tyr Lys Gln Arg Thr
        585                 590                 595

cag aag aaa gca gaa gta gag ggg aaa gac cta cca gaa cat gcg gtc 2060
Gln Lys Lys Ala Glu Val Glu Gly Lys Asp Leu Pro Glu His Ala Val

600                 605                 610

ctc aaa atg aaa gga aac ttt acc ctc cca gag gta gct gag tgc ttt     2108
Leu Lys Met Lys Gly Asn Phe Thr Leu Pro Glu Val Ala Glu Cys Phe
615                 620                 625                 630

gat gaa ata acc tat gtt gaa ctt cag aag gaa gaa gcc caa aaa ctc     2156
Asp Glu Ile Thr Tyr Val Glu Leu Gln Lys Glu Glu Ala Gln Lys Leu
                635                 640                 645

ttg gag caa tat aag gaa gaa agc aaa aag gct ctt cca cca gaa aag     2204
Leu Glu Gln Tyr Lys Glu Glu Ser Lys Lys Ala Leu Pro Pro Glu Lys
            650                 655                 660

aaa cag aac act ggc tca aag aaa agc aat aaa aat aag agt ggc aag     2252
Lys Gln Asn Thr Gly Ser Lys Lys Ser Asn Lys Asn Lys Ser Gly Lys
        665                 670                 675

aac cag ttt aac aga ggt ggt ggc cat aga gga cgt gga gga ttc aat     2300
Asn Gln Phe Asn Arg Gly Gly Gly His Arg Gly Arg Gly Gly Phe Asn
    680                 685                 690

atg cgt ggt gga aat ttc aga gga gga gcc cct ggg aat cgt ggc gga     2348
Met Arg Gly Gly Asn Phe Arg Gly Gly Ala Pro Gly Asn Arg Gly Gly
695                 700                 705                 710

tat aat agg agg ggc aac atg cca cag aga ggt ggt ggc ggt gga gga     2396
Tyr Asn Arg Arg Gly Asn Met Pro Gln Arg Gly Gly Gly Gly Gly Gly
                715                 720                 725

agt ggt gga atc ggc tat cca tac cct cgt gcc cct gtt ttt cct ggc     2444
Ser Gly Gly Ile Gly Tyr Pro Tyr Pro Arg Ala Pro Val Phe Pro Gly
            730                 735                 740

cgt ggt agt tac tca aac aga ggg aac tac aac aga ggt gga atg ccc     2492
Arg Gly Ser Tyr Ser Asn Arg Gly Asn Tyr Asn Arg Gly Gly Met Pro
        745                 750                 755

aac aga ggg aac tac aac cag aac ttc aga gga cga gga aac aat cgt     2540
Asn Arg Gly Asn Tyr Asn Gln Asn Phe Arg Gly Arg Gly Asn Asn Arg
    760                 765                 770

ggc tac aaa aat caa tct cag ggc tac aac cag tgg cag cag ggt caa     2588
Gly Tyr Lys Asn Gln Ser Gln Gly Tyr Asn Gln Trp Gln Gln Gly Gln
775                 780                 785                 790

ttc tgg ggt cag aag cca tgg agt cag cat tat cac caa gga tat tat     2636
Phe Trp Gly Gln Lys Pro Trp Ser Gln His Tyr His Gln Gly Tyr Tyr
                795                 800                 805

tga atacccaaat aaaacgaact gatacatatt tctccaaaac cttcacaaga 2689

agtcgactgt tttctttagt aggctaactt tttaaacatt ccacaagagg aagtgcctgc 2749

gggttccttt tttagaagct ttgtgggttg attttttttc ttttcttttt tgtacatttt 2809

taattgcagt ttaaaagtga atcgtaagag aacctcagca ttgtgcacga taagagaatg 2869

tgtcagtatt tcagggttct acattttatc tgtaaaatgt gacttttttt tttttttatc 2929

acaacagaag taaaatgttg ctttgtacct ggtgtctttt attaagaatt tactcccccc 2989

atttctcaca gagaataaca gtcgggagtc attgtcacaa tataatagaa atgttagcaa 3049

ccagattcat gtaaggacta agtggtcctc atgaattgca ttaagactct gtactgctca 3109

tattacactc catcctctct gtagtttgct gggtagtgga gggggtaagc taaatcatag 3169

tttctgacaa taactgggaa ggttttttct taaaataaca atggaattgg tataattggg 3229

attgaaaact aaaacttgga actaagatag agaagatgga gtgtatgtag aagggctgtt 3289

aaaaatgtaa aacttggttg cattatttgt ggaggctcaa acttgtgaag gttaatacca 3349

taatttttcc atttgttctg cattttgatt ctgaaaagaa agctggcttt gcccatttct 3409

tattaaaaaa acttgttgta aaaaaaaaaa aaaaaaaaaa aaaaaaaaaa aaaaaa 3465

<210> 20
<211> 806
<212> PRT
<213> Homo sapiens

<400> 20

Met Ser Ser Ser Pro Val Asn Val Lys Lys Leu Lys Val Ser Glu Leu
1               5                   10                  15

Lys Glu Glu Leu Lys Lys Arg Arg Leu Ser Asp Lys Gly Leu Lys Ala
            20                  25                  30

Glu Leu Met Glu Arg Leu Gln Ala Ala Leu Asp Asp Glu Glu Ala Gly
        35                  40                  45

Gly Arg Pro Ala Met Glu Pro Gly Asn Gly Ser Leu Asp Leu Gly Gly
    50                  55                  60

Asp Ser Ala Gly Arg Ser Gly Ala Gly Leu Glu Gln Glu Ala Ala Ala
65                  70                  75                  80

Gly Gly Asp Glu Glu Glu Glu Glu Glu Glu Glu Glu Glu Glu Gly Ile
                85                  90                  95

Ser Ala Leu Asp Gly Asp Gln Met Glu Leu Gly Glu Glu Asn Gly Ala
            100                 105                 110

Ala Gly Ala Ala Asp Ser Gly Pro Met Glu Glu Glu Glu Ala Ala Ser
        115                 120                 125

Glu Asp Glu Asn Gly Asp Asp Gln Gly Phe Gln Glu Gly Glu Asp Glu
    130                 135                 140

Leu Gly Asp Glu Glu Glu Gly Ala Gly Asp Glu Asn Gly His Gly Glu
145                 150                 155                 160

Gln Gln Pro Gln Pro Pro Ala Thr Gln Gln Gln Gln Pro Gln Gln Gln
                165                 170                 175

Arg Gly Ala Ala Lys Glu Ala Ala Gly Lys Ser Ser Gly Pro Thr Ser
            180                 185                 190

Leu Phe Ala Val Thr Val Ala Pro Pro Gly Ala Arg Gln Gly Gln Gln
        195                 200                 205

Gln Ala Gly Gly Asp Gly Lys Thr Glu Gln Lys Gly Gly Asp Lys Lys
    210                 215                 220

Arg Gly Val Lys Arg Pro Arg Glu Asp His Gly Arg Gly Tyr Phe Glu
225                 230                 235                 240

Tyr Ile Glu Glu Asn Lys Tyr Ser Arg Ala Lys Ser Pro Gln Pro Pro
                245                 250                 255

Val Glu Glu Glu Asp Glu His Phe Asp Asp Thr Val Val Cys Leu Asp
            260                 265                 270

Thr Tyr Asn Cys Asp Leu His Phe Lys Ile Ser Arg Asp Arg Leu Ser
        275                 280                 285

Ala Ser Ser Leu Thr Met Glu Ser Phe Ala Phe Leu Trp Ala Gly Gly
    290                 295                 300

Arg Ala Ser Tyr Gly Val Ser Lys Gly Lys Val Cys Phe Glu Met Lys
305                 310                 315                 320

Val Thr Glu Lys Ile Pro Val Arg His Leu Tyr Thr Lys Asp Ile Asp
                325                 330                 335

Ile His Glu Val Arg Ile Gly Trp Ser Leu Thr Thr Ser Gly Met Leu
            340                 345                 350

Leu Gly Glu Glu Glu Phe Ser Tyr Gly Tyr Ser Leu Lys Gly Ile Lys
        355                 360                 365

Thr Cys Asn Cys Glu Thr Glu Asp Tyr Gly Glu Lys Phe Asp Glu Asn
    370                 375                 380

Asp Val Ile Thr Cys Phe Ala Asn Phe Glu Ser Asp Glu Val Glu Leu
385                 390                 395                 400

Ser Tyr Ala Lys Asn Gly Gln Asp Leu Gly Val Ala Phe Lys Ile Ser
                405                 410                 415

Lys Glu Val Leu Ala Gly Arg Pro Leu Phe Pro His Val Leu Cys His
            420                 425                 430

Asn Cys Ala Val Glu Phe Asn Phe Gly Gln Lys Glu Lys Pro Tyr Phe
        435                 440                 445

Pro Ile Pro Glu Glu Tyr Thr Phe Ile Gln Asn Val Pro Leu Glu Asp
    450                 455                 460

Arg Val Arg Gly Pro Lys Gly Pro Glu Glu Lys Lys Asp Cys Glu Val
465                 470                 475                 480

Val Met Met Ile Gly Leu Pro Gly Ala Gly Lys Thr Thr Trp Val Thr
                485                 490                 495

Lys His Ala Ala Glu Asn Pro Gly Lys Tyr Asn Ile Leu Gly Thr Asn
            500                 505                 510

Thr Ile Met Asp Lys Met Met Val Ala Gly Phe Lys Lys Gln Met Ala
        515                 520                 525

Asp Thr Gly Lys Leu Asn Thr Leu Leu Gln Arg Ala Pro Gln Cys Leu
    530                 535                 540

Gly Lys Phe Ile Glu Ile Ala Ala Arg Lys Lys Arg Asn Phe Ile Leu
545                 550                 555                 560

Asp Gln Thr Asn Val Ser Ala Ala Ala Gln Arg Arg Lys Met Cys Leu
                565                 570                 575

Phe Ala Gly Phe Gln Arg Lys Ala Val Val Val Cys Pro Lys Asp Glu
            580                 585                 590

Asp Tyr Lys Gln Arg Thr Gln Lys Lys Ala Glu Val Glu Gly Lys Asp
        595                 600                 605

Leu Pro Glu His Ala Val Leu Lys Met Lys Gly Asn Phe Thr Leu Pro
    610                 615                 620

Glu Val Ala Glu Cys Phe Asp Glu Ile Thr Tyr Val Glu Leu Gln Lys
625                 630                 635                 640

Glu Glu Ala Gln Lys Leu Leu Glu Gln Tyr Lys Glu Glu Ser Lys Lys
                645                 650                 655

Ala Leu Pro Pro Glu Lys Lys Gln Asn Thr Gly Ser Lys Lys Ser Asn
            660                 665                 670

Lys Asn Lys Ser Gly Lys Asn Gln Phe Asn Arg Gly Gly Gly His Arg
        675                 680                 685

Gly Arg Gly Gly Phe Asn Met Arg Gly Gly Asn Phe Arg Gly Gly Ala
    690                 695                 700

Pro Gly Asn Arg Gly Gly Tyr Asn Arg Arg Gly Asn Met Pro Gln Arg
705                 710                 715                 720

Gly Gly Gly Gly Gly Gly Ser Gly Gly Ile Gly Tyr Pro Tyr Pro Arg
                725                 730                 735

Ala Pro Val Phe Pro Gly Arg Gly Ser Tyr Ser Asn Arg Gly Asn Tyr
            740                 745                 750

Asn Arg Gly Gly Met Pro Asn Arg Gly Asn Tyr Asn Gln Asn Phe Arg
        755                 760                 765

Gly Arg Gly Asn Asn Arg Gly Tyr Lys Asn Gln Ser Gln Gly Tyr Asn
    770                 775                 780

Gln Trp Gln Gln Gly Gln Phe Trp Gly Gln Lys Pro Trp Ser Gln His
785                 790                 795                 800

Tyr His Gln Gly Tyr Tyr
                805

<210>  21
<211>  3933
<212>  DNA
<213>  Homo sapiens

<220>
<221>  CDS
<222>  (318)..(2861)
<223>

<400> 21
gcgagagtgg gtttgttctt gggctgcagc cgctgccgcc gcttctcgcc agcgccgttg 60
ctgcggggga ttgtgggagt ctccgcgtcc cgctcgctgg gagagaggta cctctccttt 120
tccctctccc tttccctaag agttgtctgc tggttctcag cttgaagaag attctgcagt 180
ccttattgat ccttttcctt ggcgttacca tttttgaagc aaagttaacc tagctttcta 240
gtttgagctt tctttttggc cgtctttaaa aaaaattttt tttttaatct ataaaataga 300
caagagctag ttctaca atg tcc aag tca ttc cag cag tca tct ctc agt 350
agg gac tca cag ggt cat ggg cgt gac ctg tct gcg gca gga ata ggc 398
ctt ctt gct gct gct acc cag tct tta agt atg cca gca tct ctt gga 446
agg atg aac cag ggt act gca cgc ctt gct agt tta atg aat ctt gga 494
atg agt tct tca ttg aat caa caa gga gct cat agt gca ctg tct tct 542
gct agt act tct tcc cat aat ttg cag tct ata ttt aac att gga agt 590
aga ggt cca ctc cct tta tct tct caa cac cgt gga gat gca gac cag 638
gcc agt aac att ttg gcc agc ttt ggt ctg tct gct aga gac tta gat 686
gaa ctg agt cgt tat cca gag gac aag att act cct gag aat ttg ccc 734
caa atc ctt cta cag ctt aaa agg agg aga act gaa gaa ggc cct acc 782
ttg agt tat ggt aga gat ggc aga tct gct aca cgg gag cca cca tac 830
aga gta cct agg gat gat tgg gaa gaa aaa agg cac ttt aga aga gat 878
agt ttt gat gat cgt ggt cct agt ctc aac cca gtg ctt gat tat gac 926
cat gga agt cgt tct caa gaa tct ggt tat tat gac aga atg gat tat 974
gaa gat gac aga tta aga gat gga gaa agg tgt agg gat gat tct ttt 1022
ttt ggt gag acc tcg cat aac tat cat aaa ttt gac agt gag tat gag 1070
aga atg gga cgt ggt cct ggc ccc tta caa gag aga tct ctc ttt gag 1118
aaa aag aga ggc gct cct cca agt agc aat att gaa gac ttc cat gga 1166
ctc tta ccg aag ggt tat ccc cat ctg tgc tct ata tgt gat ttg cca 1214
gtt cat tct aat aag gag tgg agt caa cat atc aat gga gca agt cac 1262
agt cgt cga tgc cag ctt ctt ctt gaa atc tac cca gaa tgg aat cct 1310
gac aat gat aca gga cac aca atg ggt gat cca ttc atg ttg cag cag 1358
tct aca aat cca gca cca gga att ctg gga cct cca cct ccc tca ttt 1406
cat ctt ggg gga cca gca gtt gga cca aga gga aat ctg ggt gct gga 1454
aat gga aac ctg caa gga cct aga cac atg cag aaa ggc aga gtg gaa 1502
act agc aga gtt gtt cac atc atg gat ttt caa cga ggg aaa aac ttg 1550
aga tac cag cta tta cag ctg gta gaa cca ttt gga gtc att tca aat 1598
cat ctg att cta aat aaa att aat gag gca ttt att gaa atg gca acc 1646
aca gag gat gct cag gcc gca gtg gat tat tac aca acc aca cca gcg 1694
tta gta ttt ggc aag cca gtg aga gtt cat tta tcc cag aag tat aaa 1742
aga ata aag aaa cct gaa gga aag cca gat cag aag ttt gat caa aag 1790
caa gag ctt gga cgt gtg ata cat ctc agc aat ttg ccg cat tct ggc 1838
tat tct gat agt gct gtt ctc aag ctt gct gag cct tat ggg aaa ata 1886
aag aat tac ata ttg atg agg atg aaa agt cag gct ttt att gag atg 1934
gag aca aga gaa gat gca atg gca atg gtt gac cat tgt ttg aaa aaa 1982
gcc ctt tgg ttt cag ggg aga tgt gtg aag gtt gac ctg tct gag aaa 2030
tat aaa aaa ctg gtt ctg agg att cca aac aga ggc att gat tta ctg 2078
aaa aaa gat aaa tcc cga aaa aga tct tac tct cca gat ggc aaa gaa 2126
tct cca agt gat aag aaa tcc aaa act gat ggt tcc cag aag act gag 2174
agt tca acc gaa ggt aaa gaa caa gaa gag aag tcc ggt gaa gat ggt 2222
gag aaa gac aca aag gat gac cag aca gag cag gaa cct aat atg ctt 2270
ctt gaa tct gaa gat gag cta ctt gta gat gaa gaa gaa gca gca gca 2318
ctg cta gaa agt ggc agt tca gtg gga gac gag acc gat ctt gct aat 2366
tta ggt gat gtg gct tct gat ggg aaa aag gaa cca tca gat aaa gct 2414
gtg aaa aaa gat gga agt gct tca gca gca gca aag aaa aag ctt aaa 2462
aag gtg gac aag atc gag gaa ctt gat caa gaa aac gaa gca gcg ttg 2510
gaa aat gga att aaa aat gag gaa aac aca gaa cca ggt gct gaa tct 2558
tct gag aac gct gat gat ccc aac aaa gat aca agt gaa aac gca gat 2606
ggt caa agt gat gag aac aag gac gac tat aca atc cca gat gag tat 2654
aga att gga cca tat cag ccc aat gtt cct gtt ggt ata gac tat gtg 2702
ata cct aaa aca ggg ttt tac tgt aag ctg tgt tca ctc ttt tat aca 2750
aat gaa gaa gtt gca aag aat act cat tgc agc agc ctt cct cat tat 2798
cag aaa tta aag aaa ttt ctg aat aaa ttg gca gaa gaa cgc aga cag 2846
aag aag gaa act taa gatgtgcaag gagatttaat gatttcaaag aaaataatgg 2901
ttctttgttt ttaatgttaa ccttttttaa atacaatact gatagttaga agaaaactat 2961
tgtactcttt tgttttagtg gagaaataat agatgtctgt tcatgtgtta agtgttatag 3021
caaaaaaaat acacatatgg ttaagttaat gaatagtttt tgttttatca gaatggcaac 3081
agacagaagt actttgtaga gattgacttc ctaagctact taagacaact tgcaccacta 3141
agaaaaaaat gtagaaccat ttggaaaaat gaaatttagt agttccaagt ttcaaagaaa 3201
tgtcaacatt ttattccatt caataaagaa caaaaccaat agtgttttta ttactttcat 3261
ctgaaacatt ccatgtttta atctgagcct tgcagacttt catttggagt ttgaacccgt 3321
tttggttgca tttcattttt ggagaactta attaacgtga gattggcaat tgaaatgcag 3381
gtgcagtttt ctgttaatgt catgctgttg tttaggtaat aagaaatatt aagtaattgg 3441
ctttagattt tgtaattttt ttccctgagt tcctgctaga tttcgtattc tagtagtcaa 3501
tgtattttca gtgaaatgca aaaatattcc cgttatcttt gaccagtatt aatttttgag 3561
atcttactgc ttgtcacttg aatcccgtga ttgtcataca tctctggtat aagcaacatt 3621
tgatttttga agtgtgtaga ccatctcttc atattttcaa gatgtaattt tacatttctg 3681
catttttaaa acagtttggc cataatccta gatgcacgct tctaattcat gtacctgcac 3741
atgtgacctt tgtgaacaga aatttgcatg tataatttgt gtttacttgt aactttctgg 3801
ttatatactg cttatatctg tggattcaag ttactgaagt gaataccaat aaaaagaaaa 3861
ccctaggcca tgttaattgg ttatacatgt ttggaatgtt aaaaaaaaaa aaaaaaaaaa 3921
aaaaaaaaaa aa 3933

<210> 22
<211> 847
<212> PRT
<213> Homo sapiens

<400> 22

<210>  23
<211>  1339
<212>  DNA
<213>  Homo sapiens

<220>
<221>  CDS
<222>  (47)..(1018)
<223>

<400>  23
gaattccgat tagtgtgatc tcagctcaag gcaaaggtgg gatatc atg gca tct        55
                                                   Met Ala Ser
                                                   1

atc tgg gtt gga cac cga gga aca gta aga gat tat cca gac ttt agc       103
Ile Trp Val Gly His Arg Gly Thr Val Arg Asp Tyr Pro Asp Phe Ser

5                   10                  15

cca tca gtg gat gct gaa gct att cag aaa gca atc aga gga att gga        151
Pro Ser Val Asp Ala Glu Ala Ile Gln Lys Ala Ile Arg Gly Ile Gly
20                  25                  30                  35

act gat gag aaa atg ctc atc agc att ctg act gag agg tca aat gca        199
Thr Asp Glu Lys Met Leu Ile Ser Ile Leu Thr Glu Arg Ser Asn Ala
                40                  45                  50

cag cgg cag ctg att gtt aag gaa tat caa gca gca tat gga aag gag        247
Gln Arg Gln Leu Ile Val Lys Glu Tyr Gln Ala Ala Tyr Gly Lys Glu
            55                  60                  65

ctg aaa gat gac ttg aag ggt gat ctc tct ggc cac ttt gag cat ctc        295
Leu Lys Asp Asp Leu Lys Gly Asp Leu Ser Gly His Phe Glu His Leu
        70                  75                  80

atg gtg gcc cta gtg act cca cca gca gtc ttt gat gca aag cag cta        343
Met Val Ala Leu Val Thr Pro Pro Ala Val Phe Asp Ala Lys Gln Leu
    85                  90                  95

aag aaa tcc atg aag ggc gcg gga aca aac gaa gat gcc ttg att gaa        391
Lys Lys Ser Met Lys Gly Ala Gly Thr Asn Glu Asp Ala Leu Ile Glu
100                 105                 110                 115

atc tta act acc agg aca agc agg caa atg aag gat atc tct caa gcc        439
Ile Leu Thr Thr Arg Thr Ser Arg Gln Met Lys Asp Ile Ser Gln Ala
                120                 125                 130

tat tat aca gta tac aag aag agt ctt gga gat gac att agt tcc gaa        487
Tyr Tyr Thr Val Tyr Lys Lys Ser Leu Gly Asp Asp Ile Ser Ser Glu
            135                 140                 145

aca tct ggt gac ttc cgg aaa gct ctg ttg act ttg gca gat ggc aga        535
Thr Ser Gly Asp Phe Arg Lys Ala Leu Leu Thr Leu Ala Asp Gly Arg
        150                 155                 160

aga gat gaa agt ctg aaa gtg gat gag cat ctg gcc aaa caa gat gcc        583
Arg Asp Glu Ser Leu Lys Val Asp Glu His Leu Ala Lys Gln Asp Ala
    165                 170                 175

cag att ctc tat aaa gct ggt gag aac aga tgg ggc acg gat gaa gac        631
Gln Ile Leu Tyr Lys Ala Gly Glu Asn Arg Trp Gly Thr Asp Glu Asp
180                 185                 190                 195

aaa ttc act gag atc ctg tgt tta agg agc ttt cct caa tta aaa cta        679
Lys Phe Thr Glu Ile Leu Cys Leu Arg Ser Phe Pro Gln Leu Lys Leu
                200                 205                 210

aca ttt gat gaa tac aga aat atc agc caa aag gac att gtg gac agc     727
Thr Phe Asp Glu Tyr Arg Asn Ile Ser Gln Lys Asp Ile Val Asp Ser
            215                 220                 225

ata aaa gga gaa tta tct ggg cat ttt gaa gac tta ctg ttg gcc ata     775
Ile Lys Gly Glu Leu Ser Gly His Phe Glu Asp Leu Leu Leu Ala Ile
        230                 235                 240

gtt aat tgt gtg agg aac acg ccg gcc ttt tta gcc gaa aga ctg cat     823
Val Asn Cys Val Arg Asn Thr Pro Ala Phe Leu Ala Glu Arg Leu His
    245                 250                 255

cga gcc ttg aag ggt att gga act gat gag ttt act ctg aac cga ata     871
Arg Ala Leu Lys Gly Ile Gly Thr Asp Glu Phe Thr Leu Asn Arg Ile
260                 265                 270                 275

atg gtg tcc aga tca gaa att gac ctt ttg gac att cga aca gag ttc     919
Met Val Ser Arg Ser Glu Ile Asp Leu Leu Asp Ile Arg Thr Glu Phe
                280                 285                 290

aag aag cat tat ggc tat tcc cta tat tca gca att aaa tcg gat act     967
Lys Lys His Tyr Gly Tyr Ser Leu Tyr Ser Ala Ile Lys Ser Asp Thr
            295                 300                 305

tct gga gac tat gaa atc aca ctc tta aaa atc tgt ggt gga gat gac     1015
Ser Gly Asp Tyr Glu Ile Thr Leu Leu Lys Ile Cys Gly Gly Asp Asp
        310                 315                 320

tga accaagaaga taatctccaa aggtccacga tgggctttcc caacagctcc     1068

accttacttc ttctcatact atttaagaga acaagcaaat ataaacagca acttgtgttc     1128

ctaacaggaa ttttcattgt tctataacaa caacaacaaa agcgattatt attttagagc     1188

atctcattta taatgtagca gctcataaat gaaattgaaa atggtattaa agatctgcaa     1248

ctactatcca acttatattt ctgctttcaa agttaagaat ctttatagtt ctactccatt     1308

aaatataaag caagataata aaacggaatt c     1339

<210>  24
<211>  323
<212>  PRT
<213>  Homo sapiens

<400>  24

Met Ala Ser Ile Trp Val Gly His Arg Gly Thr Val Arg Asp Tyr Pro
1               5                   10                  15

Asp Phe Ser Pro Ser Val Asp Ala Glu Ala Ile Gln Lys Ala Ile Arg
            20                  25                  30

Gly Ile Gly Thr Asp Glu Lys Met Leu Ile Ser Ile Leu Thr Glu Arg
        35                  40                  45

Ser Asn Ala Gln Arg Gln Leu Ile Val Lys Glu Tyr Gln Ala Ala Tyr
    50                  55                  60

Gly Lys Glu Leu Lys Asp Asp Leu Lys Gly Asp Leu Ser Gly His Phe
65                  70                  75                  80

Glu His Leu Met Val Ala Leu Val Thr Pro Pro Ala Val Phe Asp Ala
                85                  90                  95

Lys Gln Leu Lys Lys Ser Met Lys Gly Ala Gly Thr Asn Glu Asp Ala
            100                 105                 110

Leu Ile Glu Ile Leu Thr Thr Arg Thr Ser Arg Gln Met Lys Asp Ile
        115                 120                 125

Ser Gln Ala Tyr Tyr Thr Val Tyr Lys Lys Ser Leu Gly Asp Asp Ile
    130                 135                 140

Ser Ser Glu Thr Ser Gly Asp Phe Arg Lys Ala Leu Leu Thr Leu Ala
145                 150                 155                 160

Asp Gly Arg Arg Asp Glu Ser Leu Lys Val Asp Glu His Leu Ala Lys
                165                 170                 175

Gln Asp Ala Gln Ile Leu Tyr Lys Ala Gly Glu Asn Arg Trp Gly Thr
            180                 185                 190

Asp Glu Asp Lys Phe Thr Glu Ile Leu Cys Leu Arg Ser Phe Pro Gln
        195                 200                 205

Leu Lys Leu Thr Phe Asp Glu Tyr Arg Asn Ile Ser Gln Lys Asp Ile
    210                 215                 220

Val Asp Ser Ile Lys Gly Glu Leu Ser Gly His Phe Glu Asp Leu Leu
225                 230                 235                 240

Leu Ala Ile Val Asn Cys Val Arg Asn Thr Pro Ala Phe Leu Ala Glu
                245                 250                 255

Arg Leu His Arg Ala Leu Lys Gly Ile Gly Thr Asp Glu Phe Thr Leu
            260                 265                 270

Asn Arg Ile Met Val Ser Arg Ser Glu Ile Asp Leu Leu Asp Ile Arg
        275                 280                 285

Thr Glu Phe Lys Lys His Tyr Gly Tyr Ser Leu Tyr Ser Ala Ile Lys
    290                 295                 300

Ser Asp Thr Ser Gly Asp Tyr Glu Ile Thr Leu Leu Lys Ile Cys Gly
305                 310                 315                 320

Gly Asp Asp

<210>  25
<211>  1659
<212>  DNA
<213>  Homo sapiens

<220>
<221>  CDS
<222>  (152)..(1201)
<223>

<400>  25
gctgggggcg ggtcctgcgg caccgcccgg gaagctgcgc gagggtcgac agcctccgcc      60

acatcctcca cctctcttgg tccagcgagc gttgccgggc cagggtcaag cggagggctc     120

cgacggcgcg gacggagcga agcgccgagc c atg gcg cac caa acg ggc atc        172
                                   Met Ala His Gln Thr Gly Ile
                                   1               5

cac gcc acg gaa gag ctg aag gaa ttc ttt gcc aag gca cgg gct ggc 220
His Ala Thr Glu Glu Leu Lys Glu Phe Phe Ala Lys Ala Arg Ala Gly
        10                  15                  20

tct gtg cgg ctc atc aag gtt gtg att gag gac gag cag ctc gtg ctg 268
Ser Val Arg Leu Ile Lys Val Val Ile Glu Asp Glu Gln Leu Val Leu
    25                  30                  35

ggt gcc tcg cag gag cca gta ggc cgc tgg gat cag gac tat gac agg 316
Gly Ala Ser Gln Glu Pro Val Gly Arg Trp Asp Gln Asp Tyr Asp Arg
40                  45                  50                  55

gcc gtg ctg cca ctg ctg gac gcc cag cag ccc tgc tac ctg ctc tac 364
Ala Val Leu Pro Leu Leu Asp Ala Gln Gln Pro Cys Tyr Leu Leu Tyr
                60                  65                  70

cgc ctc gac tca cag aat gct cag ggc ttc gaa tgg ctc ttc ctc gcc 412
Arg Leu Asp Ser Gln Asn Ala Gln Gly Phe Glu Trp Leu Phe Leu Ala
            75                  80                  85

tgg tcg cct gat aac tcc ccc gtg cgg ctg aag atg ctg tac gcg gcc 460
Trp Ser Pro Asp Asn Ser Pro Val Arg Leu Lys Met Leu Tyr Ala Ala
        90                  95                  100

acg cgg gcc aca gtg aaa aag gag ttt gga ggt ggc cac atc aag gat 508
Thr Arg Ala Thr Val Lys Lys Glu Phe Gly Gly Gly His Ile Lys Asp
    105                 110                 115

gag ctc ttc ggg act gtg aag gat gac ctc tct ttt gct ggg tac cag 556
Glu Leu Phe Gly Thr Val Lys Asp Asp Leu Ser Phe Ala Gly Tyr Gln
120                 125                 130                 135

aaa cac ctg tcg tcc tgt gcg gca cct gcc ccg ctg acc tcg gct gag 604
Lys His Leu Ser Ser Cys Ala Ala Pro Ala Pro Leu Thr Ser Ala Glu
                140                 145                 150

aga gag ctc cag cag atc cgc att aac gag gtg aag aca gag atc agt 652
Arg Glu Leu Gln Gln Ile Arg Ile Asn Glu Val Lys Thr Glu Ile Ser
            155                 160                 165

gtg gaa agc aag cac cag acc ctg cag ggc ctc gcc ttc ccc ctg cag 700
Val Glu Ser Lys His Gln Thr Leu Gln Gly Leu Ala Phe Pro Leu Gln
        170                 175                 180

cct gag gcc cag cgg gca ctc cag cag ctc aag cag aaa atg gtc aac 748
Pro Glu Ala Gln Arg Ala Leu Gln Gln Leu Lys Gln Lys Met Val Asn
    185                 190                 195

tac atc cag atg aag ctg gac cta gag cgg gaa acc att gag ctg gtg 796

Tyr Ile Gln Met Lys Leu Asp Leu Glu Arg Glu Thr Ile Glu Leu Val
200                 205                 210                 215

cac aca gag ccc acg gat gtg gcc cag ctg ccc tcc cgg gtg ccc cga     844
His Thr Glu Pro Thr Asp Val Ala Gln Leu Pro Ser Arg Val Pro Arg
                220                 225                 230

gat gct gcc cgc tac cac ttc ttc ctc tac aag cac acc cat gag ggc     892
Asp Ala Ala Arg Tyr His Phe Phe Leu Tyr Lys His Thr His Glu Gly
            235                 240                 245

gac ccc ctt gag tct gta gtg ttc atc tac tcc atg ccg ggg tac aag     940
Asp Pro Leu Glu Ser Val Val Phe Ile Tyr Ser Met Pro Gly Tyr Lys
        250                 255                 260

tgc agc atc aag gag cga atg ctc tac tcc agc tgc aag agc cgc ctc     988
Cys Ser Ile Lys Glu Arg Met Leu Tyr Ser Ser Cys Lys Ser Arg Leu
    265                 270                 275

ctc gac tcc gtg gag cag gac ttc cat ctg gag atc gcc aag aaa att     1036
Leu Asp Ser Val Glu Gln Asp Phe His Leu Glu Ile Ala Lys Lys Ile
280                 285                 290                 295

gag att ggc gat ggg gca gag ctg acg gca gag ttc ctc tac gac gag     1084
Glu Ile Gly Asp Gly Ala Glu Leu Thr Ala Glu Phe Leu Tyr Asp Glu
                300                 305                 310

gtg cac ccc aag caa cac gcc ttc aag cag gcc ttc gcc aag ccc aag     1132
Val His Pro Lys Gln His Ala Phe Lys Gln Ala Phe Ala Lys Pro Lys
            315                 320                 325

ggc cca ggg ggc aag cgg ggc cat aag cgc ctc atc cgc ggc ccg ggt     1180
Gly Pro Gly Gly Lys Arg Gly His Lys Arg Leu Ile Arg Gly Pro Gly
        330                 335                 340

gaa aat ggg gat gac agc tag gaggctggag cagggccggc cacgtgtgga     1231
Glu Asn Gly Asp Asp Ser
    345

ctgtggggct gcccaccttc cgctccctgc caccatcctc cttcctgggc tccaggaaag     1291

tgtttctggg aggtcaggag ggctggcagc tgaacgcact tgcagcgtcc gagggccacc     1351

gggctggcat tttgtgaccc ttccctgttg ctgtccctgc atctcgtctg tgtgcccagg     1411

gtgtccgggg accctgcctg gctggcttaa gggggctggg tcaggggcct ggcatgaacc     1471

tggcctcccg gggagctgag actagggtcc cagcacagcc cagaaacctt tggccacaag     1531

aagtggggtc agtcagggct ggggcagggg tcactgcagt ttgggatggt tgaatgctgt     1591

attttctaaa gaataaaata tttttaaatc aagaaaaaaa aaaaaaaaaa aaaaaaaaaa 1651

aaaaaaaa 1659

<210> 26
<211> 349
<212> PRT
<213> Homo sapiens

<400> 26

Met Ala His Gln Thr Gly Ile His Ala Thr Glu Glu Leu Lys Glu Phe
1               5                   10                  15

Phe Ala Lys Ala Arg Ala Gly Ser Val Arg Leu Ile Lys Val Val Ile
            20                  25                  30

Glu Asp Glu Gln Leu Val Leu Gly Ala Ser Gln Glu Pro Val Gly Arg
        35                  40                  45

Trp Asp Gln Asp Tyr Asp Arg Ala Val Leu Pro Leu Leu Asp Ala Gln
    50                  55                  60

Gln Pro Cys Tyr Leu Leu Tyr Arg Leu Asp Ser Gln Asn Ala Gln Gly
65                  70                  75                  80

Phe Glu Trp Leu Phe Leu Ala Trp Ser Pro Asp Asn Ser Pro Val Arg
                85                  90                  95

Leu Lys Met Leu Tyr Ala Ala Thr Arg Ala Thr Val Lys Lys Glu Phe
            100                 105                 110

Gly Gly Gly His Ile Lys Asp Glu Leu Phe Gly Thr Val Lys Asp Asp
        115                 120                 125

Leu Ser Phe Ala Gly Tyr Gln Lys His Leu Ser Ser Cys Ala Ala Pro
    130                 135                 140

Ala Pro Leu Thr Ser Ala Glu Arg Glu Leu Gln Gln Ile Arg Ile Asn

145                 150                 155                 160

Glu Val Lys Thr Glu Ile Ser Val Glu Ser Lys His Gln Thr Leu Gln
                165                 170                 175

Gly Leu Ala Phe Pro Leu Gln Pro Glu Ala Gln Arg Ala Leu Gln Gln
            180                 185                 190

Leu Lys Gln Lys Met Val Asn Tyr Ile Gln Met Lys Leu Asp Leu Glu
        195                 200                 205

Arg Glu Thr Ile Glu Leu Val His Thr Glu Pro Thr Asp Val Ala Gln
    210                 215                 220

Leu Pro Ser Arg Val Pro Arg Asp Ala Ala Arg Tyr His Phe Phe Leu
225                 230                 235                 240

Tyr Lys His Thr His Glu Gly Asp Pro Leu Glu Ser Val Val Phe Ile
                245                 250                 255

Tyr Ser Met Pro Gly Tyr Lys Cys Ser Ile Lys Glu Arg Met Leu Tyr
            260                 265                 270

Ser Ser Cys Lys Ser Arg Leu Leu Asp Ser Val Glu Gln Asp Phe His
        275                 280                 285

Leu Glu Ile Ala Lys Lys Ile Glu Ile Gly Asp Gly Ala Glu Leu Thr
    290                 295                 300

Ala Glu Phe Leu Tyr Asp Glu Val His Pro Lys Gln His Ala Phe Lys
305                 310                 315                 320

Gln Ala Phe Ala Lys Pro Lys Gly Pro Gly Gly Lys Arg Gly His Lys
                325                 330                 335

Arg Leu Ile Arg Gly Pro Gly Glu Asn Gly Asp Asp Ser
            340                 345

<210> 27
<211> 2765
<212> DNA
<213> Homo sapiens

<220>
<221> CDS
<222> (91)..(837)
<223>

<400> 27
   gggagacgtg gtgccgctgc gggctcgctc tgccgtgcgc taggcttggt gggaaggcct 60
   gttctcgagt ccgcgctttt cgtcaccgcc atg tcg gga ggt ggt gtg att cgt 114
   Met Ser Gly Gly Gly Val Ile Arg
   1 5
   ggc ccc gca ggg aac aac gat tgc cgc atc tac gtg ggt aac tta cct 162
   Gly Pro Ala Gly Asn Asn Asp Cys Arg Ile Tyr Val Gly Asn Leu Pro
   10 15 20
   cca gac atc cga acc aag gac att gag gac gtg ttc tac aaa tac ggc 210
   Pro Asp Ile Arg Thr Lys Asp Ile Glu Asp Val Phe Tyr Lys Tyr Gly
   25 30 35 40
   gct atc cgc gac atc gac ctc aag aat cgc cgc ggg gga ccg ccc ttc 258
   Ala Ile Arg Asp Ile Asp Leu Lys Asn Arg Arg Gly Gly Pro Pro Phe
   45 50 55
   gcc ttc gtt gag ttc gag gac ccg cga gac gcg gaa gac gcg gtg tat 306
   Ala Phe Val Glu Phe Glu Asp Pro Arg Asp Ala Glu Asp Ala Val Tyr
   60 65 70
   ggt cgc gac ggc tat gat tac gat ggg tac cgt ctg cgg gtg gag ttt 354
   Gly Arg Asp Gly Tyr Asp Tyr Asp Gly Tyr Arg Leu Arg Val Glu Phe
   75 80 85
   cct cga agc ggc cgt gga aca ggc cga ggc ggc ggc ggg ggt gga ggt 402
   Pro Arg Ser Gly Arg Gly Thr Gly Arg Gly Gly Gly Gly Gly Gly Gly
   90 95 100
   ggc gga gct ccc cga ggt cgc tat ggc ccc cca tcc agg cgg tct gaa 450
   Gly Gly Ala Pro Arg Gly Arg Tyr Gly Pro Pro Ser Arg Arg Ser Glu
   105 110 115 120
   aac aga gtg gtt gtc tct gga ctg cct cca agt gga agt tgg cag gat 498
   Asn Arg Val Val Val Ser Gly Leu Pro Pro Ser Gly Ser Trp Gln Asp
   125 130 135
   tta aag gat cac atg cgt gaa gca ggt gat gta tgt tat gct gat gtt 546
   Leu Lys Asp His Met Arg Glu Ala Gly Asp Val Cys Tyr Ala Asp Val
   140 145 150
   tac cga gat ggc act ggt gtc gtg gag ttt gta cgg aaa gaa gat atg 594
   Tyr Arg Asp Gly Thr Gly Val Val Glu Phe Val Arg Lys Glu Asp Met
   155 160 165
   acc tat gca gtt cga aaa ctg gat aac act aag ttt aga tct cat gag 642
   Thr Tyr Ala Val Arg Lys Leu Asp Asn Thr Lys Phe Arg Ser His Glu
   170 175 180
   gga gaa act gcc tac atc cgg gtt aaa gtt gat ggg ccc aga agt cca 690
   Gly Glu Thr Ala Tyr Ile Arg Val Lys Val Asp Gly Pro Arg Ser Pro
   185 190 195 200
   agt tat gga aga tct cga tct cga agc cgt agt cgt agc aga agc cgt 738
   Ser Tyr Gly Arg Ser Arg Ser Arg Ser Arg Ser Arg Ser Arg Ser Arg
   205 210 215
   agc aga agc aac agc agg agt cgc agt tac tcc cca agg aga agc aga 786
   Ser Arg Ser Asn Ser Arg Ser Arg Ser Tyr Ser Pro Arg Arg Ser Arg
   220 225 230
   gga tca cca cgc tat tct ccc cgt cat agc aga tct cgc tct cgt aca 834
   Gly Ser Pro Arg Tyr Ser Pro Arg His Ser Arg Ser Arg Ser Arg Thr
   235 240 245
   taa gatgattggt gacacttttt gtagaaccca tgttgtatac agttttcctt 887
   tattcagtac aatcttttca ttttttaatt caaactgttt tgttcagaat gggctaaagt 947
   gttgaattgc attcttgtaa tatccccttg ctcctaacat ctacattccc ttcgtgtctt 1007
   tgataaattg tattttaagt gatgtcatag acaggattgt ttaaatttag ttaactccat 1067
   actcttcaga ctgtgatatt gtgtaaatgt ctatctgccc tggtttgtgt gaactgggat 1127
   gttgggggtg tttgtggtta tcttacctgg ggaagttctt atgtttatct tgcttttcat 1187
   gtgtctttct gtagacatat ctgaagagat ggattaagaa tgctttggat taaggattgt 1247
   ggagcacatt tcaatcattt taggattgtc aaaaggagga ttgaggagga tcagatcaat 1307
   aatggaggca atggtatgac tccaagtgct attgtcacag atgaaattgg cagtattgac 1367
   cttatactaa aaggcagggg ttaaaaatga ttatatacat tttccttaaa acacttgcaa 1427
   acattttatt cagttgtctt tagctacaat tgctttgctt tttaaacctt ggcaattgtg 1487
   gcaaaattat attgcccatt ttgtagcaac ttattttgct cccttccccc catttttgtt 1547
   ttaataggga ctaatgtggg aagaactggc taatttgtca cagtgcttag ttacaactgt 1607
   taatgtgtga cctgctgttg gtgtacatgt gggtacaggg tgtttttaaa tccaacaaga 1667
   tagagtataa tatcaatact gctaaatctg catgtcctct gtgtgactga tagagcgttg 1727
   ctatttcatt tttttaagac aaaatgaaag caaaatatag agttccaatg tattggtgta 1787
   gataatctag ttgggaatac ttttaagtct caccttcccc tttaaactaa tattcataat 1847
   tggttcatat gtttaaaaga ctttaattta caaattaaat tgcaaatggg agcattagat 1907
   ttagttttag acttaggtgg gtagcaatgc cagtaaactt aaattacgta acttcttgca 1967
   accacgaaac ctgtaatacg ctgtacagta acaagtgttg gcattatcag ttgaactgta 2027
   aatacaaaat gcttcttcca attagtctct atgatgatta agtttctaaa atttatctga 2087
   acaccattca gaaacttgtt ttggggaatt tgatagttat tgatgtgcat ctgttaaact 2147
   gatgacagac ataactcatc attccccaga aacctttttt gattacagta tctaacattt 2207
   tgcctcctct tttttggttt tgctggttat aaaggtttgg attggagagg gctcactgga 2267
   tcccaatcct tggagctgga tcattggatt caaatcataa tgtggatagg atagggagga 2327
   tgaattaccc aggattcatg gagcgggatc agattaccag gaacatagga gtggattcct 2387
   gccccaacca aaccgcattc gtgtggattt ttttattcaa cttaattggc tattccaaag 2447
   attttttttt tcctattttt gacgattgga gcccttaaga tgcacgatgg aattgtgttt 2507
   tgcgtttttt ggtaaaggga gcaaagcgag gacctggaga taaacgctgg agcaatctcc 2567
   ttggaaggat tcagcacgag tagatggtaa acatttaaag gggaaagggg gggtttgttt 2627
   aaaatagtaa atcagtaagt cacttctaaa tttaaagaaa acaaaattgg agttgaagaa 2687
   taagtaggtt tccaattggc tattgccgtt ttctttgaaa aaataaacat tttttaaaaa 2747
   actaaaaaaa aaaaaaaa 2765

<210> 28
<211> 248
<212> PRT
<213> Homo sapiens

<400> 28

Met Ser Gly Gly Gly Val Ile Arg Gly Pro Ala Gly Asn Asn Asp Cys
1               5                   10                  15

Arg Ile Tyr Val Gly Asn Leu Pro Pro Asp Ile Arg Thr Lys Asp Ile
            20                  25                  30

Glu Asp Val Phe Tyr Lys Tyr Gly Ala Ile Arg Asp Ile Asp Leu Lys
        35                  40                  45

Asn Arg Arg Gly Gly Pro Pro Phe Ala Phe Val Glu Phe Glu Asp Pro
    50                  55                  60

Arg Asp Ala Glu Asp Ala Val Tyr Gly Arg Asp Gly Tyr Asp Tyr Asp
65                  70                  75                  80

Gly Tyr Arg Leu Arg Val Glu Phe Pro Arg Ser Gly Arg Gly Thr Gly
                85                  90                  95

Arg Gly Gly Gly Gly Gly Gly Gly Gly Gly Ala Pro Arg Gly Arg Tyr
            100                 105                 110

Gly Pro Pro Ser Arg Arg Ser Glu Asn Arg Val Val Val Ser Gly Leu
        115                 120                 125

Pro Pro Ser Gly Ser Trp Gln Asp Leu Lys Asp His Met Arg Glu Ala
    130                 135                 140

Gly Asp Val Cys Tyr Ala Asp Val Tyr Arg Asp Gly Thr Gly Val Val
145                 150                 155                 160

Glu Phe Val Arg Lys Glu Asp Met Thr Tyr Ala Val Arg Lys Leu Asp
                165                 170                 175

Asn Thr Lys Phe Arg Ser His Glu Gly Glu Thr Ala Tyr Ile Arg Val
            180                 185                 190

Lys Val Asp Gly Pro Arg Ser Pro Ser Tyr Gly Arg Ser Arg Ser Arg
        195                 200                 205

Ser Arg Ser Arg Ser Arg Ser Arg Ser Arg Ser Asn Ser Arg Ser Arg
    210                 215                 220

Ser Tyr Ser Pro Arg Arg Ser Arg Gly Ser Pro Arg Tyr Ser Pro Arg
225                 230                 235                 240

His Ser Arg Ser Arg Ser Arg Thr
                245

<210> 29
<211> 1167
<212> DNA
<213> Homo sapiens

<220>
<221> CDS
<222> (73)..(966)
<223>

<400> 29
gccaacttct ccagccggcc ggggcgagcg ccagcgcggc gtccggggcg agtgacacgc 60

agagctgaag cc atg gtt cat cag gtg ctc tac cgg gcg ctg gtc tcc acc 111
Met Val His Gln Val Leu Tyr Arg Ala Leu Val Ser Thr
1               5                   10

aag tgg ctg gcg gag tcc atc agg act ggc aag ctg ggg ccc ggc ctg 159
Lys Trp Leu Ala Glu Ser Ile Arg Thr Gly Lys Leu Gly Pro Gly Leu
    15                  20                  25

cgg gtg ctg gac gcg tcc tgg tac tca cca ggc acc cga gag gcc cgc 207
Arg Val Leu Asp Ala Ser Trp Tyr Ser Pro Gly Thr Arg Glu Ala Arg
30                  35                  40                  45

aag gag tac ctc gag cgc cac gta ccc ggc gcc tct ttc ttt gac ata 255
Lys Glu Tyr Leu Glu Arg His Val Pro Gly Ala Ser Phe Phe Asp Ile
                50                  55                  60

gaa gag tgc cgg gac acg gcg tcg ccc tac gag atg atg ctg ccc agc 303
Glu Glu Cys Arg Asp Thr Ala Ser Pro Tyr Glu Met Met Leu Pro Ser
            65                  70                  75

gag gct ggc ttc gcc gag tat gtg ggc cgc ctg ggc atc agc aac cac 351

Glu Ala Gly Phe Ala Glu Tyr Val Gly Arg Leu Gly Ile Ser Asn His
        80                  85                  90

acg cac gtg gtg gtg tat gat ggt gaa cac ctg ggc agc ttc tat gct 399
Thr His Val Val Val Tyr Asp Gly Glu His Leu Gly Ser Phe Tyr Ala
    95                  100                 105

ccc cgg gtc tgg tgg atg ttc cgt gtg ttt ggc cac cgc acc gta tca 447
Pro Arg Val Trp Trp Met Phe Arg Val Phe Gly His Arg Thr Val Ser
110                 115                 120                 125

gtg ctc aat ggt ggc ttc cgg aac tgg ctg aag gag ggc cac ccg gtg 495
Val Leu Asn Gly Gly Phe Arg Asn Trp Leu Lys Glu Gly His Pro Val
                130                 135                 140

aca tcc gag ccc tca cgc cca gaa ccg gcc gtc ttc aaa gcc aca ctg 543
Thr Ser Glu Pro Ser Arg Pro Glu Pro Ala Val Phe Lys Ala Thr Leu
            145                 150                 155

gac cgc tcc ctg ctc aag acc tac gag cag gtg ctg gag aac ctt gaa 591
Asp Arg Ser Leu Leu Lys Thr Tyr Glu Gln Val Leu Glu Asn Leu Glu
        160                 165                 170

tct aag agg ttc cag ctg gtg gat tca agg tct caa ggg cgg ttc ctg 639
Ser Lys Arg Phe Gln Leu Val Asp Ser Arg Ser Gln Gly Arg Phe Leu
    175                 180                 185

ggc acc gag ccg gag ccg gat gca gta gga ctg gac tcg ggc cat atc 687
Gly Thr Glu Pro Glu Pro Asp Ala Val Gly Leu Asp Ser Gly His Ile
190                 195                 200                 205

cgt ggt gcc gtc aac atg cct ttc atg gac ttc ctg act gag gat ggc 735
Arg Gly Ala Val Asn Met Pro Phe Met Asp Phe Leu Thr Glu Asp Gly
                210                 215                 220

ttc gag aag ggc cca gaa gag ctc cgt gct ctg ttc cag acc aag aag 783
Phe Glu Lys Gly Pro Glu Glu Leu Arg Ala Leu Phe Gln Thr Lys Lys
            225                 230                 235

gtg gat ctc tcg cag cct ctc att gcc acg tgc cgc aag gga gtc acc 831
Val Asp Leu Ser Gln Pro Leu Ile Ala Thr Cys Arg Lys Gly Val Thr
        240                 245                 250

gcc tgc cac gtg gcc ttg gct gcc tac ctc tgc ggc aag cct gat gtg 879
Ala Cys His Val Ala Leu Ala Ala Tyr Leu Cys Gly Lys Pro Asp Val
    255                 260                 265

gcc gtg tac gat ggc tcc tgg tcc gag tgg ttt cgc cgg gcc ccc cca 927
Ala Val Tyr Asp Gly Ser Trp Ser Glu Trp Phe Arg Arg Ala Pro Pro
270                 275                 280                 285

gag agc cgt gtg tcc cag gga aag tct gag aag gcc tga gccgtgacct 976
Glu Ser Arg Val Ser Gln Gly Lys Ser Glu Lys Ala
                290                 295

cttctgctta ctgtaactgc ggccggttta gtgaccccat gacttacagc cggttcttac 1036

ctcttaggtg aaggagatga catgttttta gaattgctgt gcaaggctca ccctctctct 1096

gtcaacactg gaataaactt tgccttttct gaaaaaaaaa aaaaaaaaaa aaaaaaaaaa 1156

aaaaaaaaaa a 1167

<210> 30
<211> 297
<212> PRT
<213> Homo sapiens

<400> 30

Met Val His Gln Val Leu Tyr Arg Ala Leu Val Ser Thr Lys Trp Leu
1               5                   10                  15

Ala Glu Ser Ile Arg Thr Gly Lys Leu Gly Pro Gly Leu Arg Val Leu
            20                  25                  30

Asp Ala Ser Trp Tyr Ser Pro Gly Thr Arg Glu Ala Arg Lys Glu Tyr
        35                  40                  45

Leu Glu Arg His Val Pro Gly Ala Ser Phe Phe Asp Ile Glu Glu Cys
    50                  55                  60

Arg Asp Thr Ala Ser Pro Tyr Glu Met Met Leu Pro Ser Glu Ala Gly
65                  70                  75                  80

Phe Ala Glu Tyr Val Gly Arg Leu Gly Ile Ser Asn His Thr His Val
                85                  90                  95

Val Val Tyr Asp Gly Glu His Leu Gly Ser Phe Tyr Ala Pro Arg Val
            100                 105                 110

Trp Trp Met Phe Arg Val Phe Gly His Arg Thr Val Ser Val Leu Asn

115                 120                 125

Gly Gly Phe Arg Asn Trp Leu Lys Glu Gly His Pro Val Thr Ser Glu
    130                 135                 140

Pro Ser Arg Pro Glu Pro Ala Val Phe Lys Ala Thr Leu Asp Arg Ser
145                 150                 155                 160

Leu Leu Lys Thr Tyr Glu Gln Val Leu Glu Asn Leu Glu Ser Lys Arg
                165                 170                 175

Phe Gln Leu Val Asp Ser Arg Ser Gln Gly Arg Phe Leu Gly Thr Glu
            180                 185                 190

Pro Glu Pro Asp Ala Val Gly Leu Asp Ser Gly His Ile Arg Gly Ala
        195                 200                 205

Val Asn Met Pro Phe Met Asp Phe Leu Thr Glu Asp Gly Phe Glu Lys
    210                 215                 220

Gly Pro Glu Glu Leu Arg Ala Leu Phe Gln Thr Lys Lys Val Asp Leu
225                 230                 235                 240

Ser Gln Pro Leu Ile Ala Thr Cys Arg Lys Gly Val Thr Ala Cys His
                245                 250                 255

Val Ala Leu Ala Ala Tyr Leu Cys Gly Lys Pro Asp Val Ala Val Tyr
            260                 265                 270

Asp Gly Ser Trp Ser Glu Trp Phe Arg Arg Ala Pro Pro Glu Ser Arg
        275                 280                 285

Val Ser Gln Gly Lys Ser Glu Lys Ala
    290                 295

<210>  31
<211>  2110
<212>  DNA

<213>  Homo sapiens

<220>
<221>  CDS
<222>  (48)..(1346)
<223>

<400>  31
   ctgaggccca gcccccttcg cccgtttcca tcacgagtgc cgccagc atg tct gac      56
   aaa ctg ccc tac aaa gtc gcc gac atc ggc ctg gct gcc tgg gga cgc      104
   aag gcc ctg gac att gct gag aac gag atg ccg ggc ctg atg cgt atg      152
   cgg gag cgg tac tcg gcc tcc aag cca ctg aag ggc gcc cgc atc gct      200
   ggc tgc ctg cac atg acc gtg gag acg gcc gtc ctc att gag acc ctc      248
   gtc acc ctg ggt gct gag gtg cag tgg tcc agc tgc aac atc ttc tcc      296
   acc cag gac cat gcg gcg gct gcc att gcc aag gct ggc att ccg gtg      344
   tat gcc tgg aag ggc gaa acg gac gag gag tac ctg tgg tgc att gag      392
   cag acc ctg tac ttc aag gac ggg ccc ctc aac atg att ctg gac gac      440
   ggg ggc gac ctc acc aac ctc atc cac acc aag tac ccg cag ctt ctg      488
   cca ggc atc cga ggc atc tct gag gag acc acg act ggg gtc cac aac      536
   ctc tac aag atg atg gcc aat ggg atc ctc aag gtg cct gcc atc aat        584
   gtc aat gac tcc gtc acc aag agc aag ttt gac aac ctc tat ggc tgc        632
   cgg gag tcc ctc ata gat ggc atc aag cgg gcc aca gat gtg atg att        680
   gcc ggc aag gta gcg gtg gta gca ggc tat ggt gat gtg ggc aag ggc        728
   tgt gcc cag gcc ctg cgg ggt ttc gga gcc cgc gtc atc atc acc gag        776
   att gac ccc atc aac gca ctg cag gct gcc atg gag ggc tat gag gtg        824
   acc acc atg gat gag gcc tgt cag gag ggc aac atc ttt gtc acc acc        872
   aca ggc tgt att gac atc atc ctt ggc cgg cac ttt gag cag atg aag        920
   gat gat gcc att gtg tgt aac att gga cac ttt gac gtg gag atc gat        968
   gtc aag tgg ctc aac gag aac gcc gtg gag aag gtg aac atc aag ccg       1016
   cag gtg gac cgg tat cgg ttg aag aat ggg cgc cgc atc atc ctg ctg       1064
   gcc gag ggt cgg ctg gtc aac ctg ggt tgt gcc atg ggc cac ccc agc       1112
   ttc gtg atg agt aac tcc ttc acc aac cag gtg atg gcg cag atc gag 1160
   ctg tgg acc cat cca gac aag tac ccc gtt ggg gtt cat ttc ctg ccc 1208
   aag aag ctg gat gag gca gtg gct gaa gcc cac ctg ggc aag ctg aat 1256
   gtg aag ttg acc aag cta act gag aag caa gcc cag tac ctg ggc atg 1304
   tcc tgt gat ggc ccc ttc aag ccg gat cac tac cgc tac tga 1346
   gagccaggtc tgcgtttcac cctccagctg ctgtccttgc ccaggcccca cctctcctcc 1406
   ctaagagcta atggcaccaa ctttgtgact ggtttgtcag tgtcccccat cgactctctg 1466
   gggctgatca cttagttttt ggcctctgct gcagccgtca tactgttcca aatgtggcag 1526
   cgggaacaga gtaccctctt caagccccgg tcatgatgga ggtcccagcc acagggaacc 1586
   atgagctcag tggtcttgga acagctcact aagtcagtcc ttccttagcc tggaagccag 1646
   tagtggagtc acaaagccca tgtgttttgc catctaggcc ttcacctggt ctgtggactt 1706
   atacctgtgt gcttggttta caggtccagt ggttcttcag cccatgacag atgagaaggg 1766
   gctatattga agggcaaaga ggaactgttg tttgaatttt cctgagagcc tggcttagtg 1826
   ctgggccttc tcttaaacct cattacaatg aggttagtac ttttagtccc tgttttacag 1886
   gggttagaat agactgttaa ggggcaactg agaaagaaca gagaagtgac agctaggggt 1946
   tgagaggggc cagaaaaaca tgaatgcagg cagatttcgt gaaatctgcc accactttat 2006
   aaccagatgg ttcctttcac aaccctgggt caaaaagaga ataatttggc ctataatgtt 2066
   aaaagaaagc aggaaggtgg gtaaataaaa atcttggtgc ctgg 2110

<210> 32
<211> 432
<212> PRT

<213>  Homo sapiens

<400>  32

<210>  33
<211>  1698
<212>  DNA
<213>  Homo sapiens

<220>
<221>  CDS
<222>  (191)..(1309)
<223>

<400>  33
cccggccctc cctgcacggc ctcccgtgcg cccctgtcag actgtggcgg ccggtcgcgc      60

ggtgcgctct ccctccctgc ccgcagcctg gagaggcgct tcgtgctgca cacccccgcg     120

ttcctgccgg caccgcgcct gccctctgcc gcgctccgcc ctgccgccga ccgcacgccc     180

gccgcgggac atg gca cac gca ccg gca cgc tgc ccc agc gcc cgg ggc        229
           Met Ala His Ala Pro Ala Arg Cys Pro Ser Ala Arg Gly
           1               5                   10

tcc ggg gac ggc gag atg ggc aag ccc agg aac gtg gcg ctc atc acc       277
Ser Gly Asp Gly Glu Met Gly Lys Pro Arg Asn Val Ala Leu Ile Thr
    15                  20                  25

ggt atc aca ggc cag gat ggt tcc tac ctg gct gag ttc ctg ctg gag       325
Gly Ile Thr Gly Gln Asp Gly Ser Tyr Leu Ala Glu Phe Leu Leu Glu
30                  35                  40                  45

aaa ggc tat gag gtc cat gga att gta cgg cgg tcc agt tca ttt aat       373
Lys Gly Tyr Glu Val His Gly Ile Val Arg Arg Ser Ser Ser Phe Asn
                50                  55                  60

acg ggt cga att gag cat ctg tat aag aat ccc cag gct cac att gaa       421
Thr Gly Arg Ile Glu His Leu Tyr Lys Asn Pro Gln Ala His Ile Glu
            65                  70                  75

gga aac atg aag ttg cac tat ggc gat ctc act gac agt acc tgc ctt       469

Gly Asn Met Lys Leu His Tyr Gly Asp Leu Thr Asp Ser Thr Cys Leu
        80                  85                  90

gtg aag atc att aat gaa gta aag ccc aca gag atc tac aac ctt gga 517
Val Lys Ile Ile Asn Glu Val Lys Pro Thr Glu Ile Tyr Asn Leu Gly
    95                  100                 105

gcc cag agc cac gtc aaa att tcc ttt gac ctc gct gag tac act gcg 565
Ala Gln Ser His Val Lys Ile Ser Phe Asp Leu Ala Glu Tyr Thr Ala
110                 115                 120                 125

gac gtt gac gga gtt ggc act cta cga ctt cta gat gca gtt aag act 613
Asp Val Asp Gly Val Gly Thr Leu Arg Leu Leu Asp Ala Val Lys Thr
                130                 135                 140

tgt ggc ctt atc aac tct gtg aag ttc tac caa gcc tca aca agt gaa 661
Cys Gly Leu Ile Asn Ser Val Lys Phe Tyr Gln Ala Ser Thr Ser Glu
            145                 150                 155

ctt tat ggg aaa gtg cag gaa ata ccc cag aag gag acc acc cct ttc 709
Leu Tyr Gly Lys Val Gln Glu Ile Pro Gln Lys Glu Thr Thr Pro Phe
        160                 165                 170

tat ccc cgg tca ccc tat ggg gca gca aaa ctc tat gcc tat tgg att 757
Tyr Pro Arg Ser Pro Tyr Gly Ala Ala Lys Leu Tyr Ala Tyr Trp Ile
    175                 180                 185

gtg gtg aac ttc cgt gag gcg tat aat ctc ttt gca gtg aac ggc att 805
Val Val Asn Phe Arg Glu Ala Tyr Asn Leu Phe Ala Val Asn Gly Ile
190                 195                 200                 205

ctc ttc aat cat gag agt ccc aga aga gga gct aat ttc gtt act cga 853
Leu Phe Asn His Glu Ser Pro Arg Arg Gly Ala Asn Phe Val Thr Arg
                210                 215                 220

aaa att agc cgg tca gta gct aag att tac ctt gga caa ctg gaa tgt 901
Lys Ile Ser Arg Ser Val Ala Lys Ile Tyr Leu Gly Gln Leu Glu Cys
            225                 230                 235

ttc agt ttg gga aat ctg gat gcc aaa cga gat tgg ggc cat gcc aag 949
Phe Ser Leu Gly Asn Leu Asp Ala Lys Arg Asp Trp Gly His Ala Lys
        240                 245                 250

gac tat gtg gag gct atg tgg ttg atg ttg cag aat gat gag ccg gag 997
Asp Tyr Val Glu Ala Met Trp Leu Met Leu Gln Asn Asp Glu Pro Glu
    255                 260                 265

gac ttc gtt ata gct act ggg gag gtc cat agt gtc cgg gaa ttt gtc 1045
Asp Phe Val Ile Ala Thr Gly Glu Val His Ser Val Arg Glu Phe Val
270                 275                 280                 285

gag aaa tca ttc ttg cac att gga aaa acc att gtg tgg gaa gga aag     1093
Glu Lys Ser Phe Leu His Ile Gly Lys Thr Ile Val Trp Glu Gly Lys
                290                 295                 300

aat gaa aat gaa gtg ggc aga tgt aaa gag acc ggc aaa gtt cac gtg     1141
Asn Glu Asn Glu Val Gly Arg Cys Lys Glu Thr Gly Lys Val His Val
            305                 310                 315

act gtg gat ctc aag tac tac cgg cca act gaa gtg gac ttt ctg cag     1189
Thr Val Asp Leu Lys Tyr Tyr Arg Pro Thr Glu Val Asp Phe Leu Gln
        320                 325                 330

ggc gac tgc acc aaa gcg aaa cag aag ctg aac tgg aag ccc cgg gtc     1237
Gly Asp Cys Thr Lys Ala Lys Gln Lys Leu Asn Trp Lys Pro Arg Val
    335                 340                 345

gct ttc gat gag ctg gtg agg gag atg gtg cac gcc gac gtg gag ctc     1285
Ala Phe Asp Glu Leu Val Arg Glu Met Val His Ala Asp Val Glu Leu
350                 355                 360                 365

atg agg aca aac ccc aat gcc tga gcagcgcctc ggagcccggc ccgccctccg     1339
Met Arg Thr Asn Pro Asn Ala
                370

gctacaatcc ccgcagagtc tccggtgcag acgcgctgcg gggatgggga gcggcgtgcc     1399

aatctgcggg tcccctgcgg cccctgctgc cgctgcgctg tcccggccgc aagagcgggg     1459

ccgccccgcc gaggtttgta gcagccggga tgtgaccctc cagggtttgg gtcgctttgc     1519

gtttgtcgaa gcctcctctg aatggctttg tgaaatcaag atgttttaat cacattcact     1579

ttacttgaaa ttatgttgtt acacaacaaa ttgtggggcc ttcaaattgt ttttctcttt     1639

tcatattaaa aatggtcttt ctgtgaacta gcaaaaaaaa aaaaaaaaaa aaaaaaaaa     1698

<210>  34
<211>  372
<212>  PRT
<213>  Homo sapiens

<400>  34

Met Ala His Ala Pro Ala Arg Cys Pro Ser Ala Arg Gly Ser Gly Asp
1               5                   10                  15

Gly Glu Met Gly Lys Pro Arg Asn Val Ala Leu Ile Thr Gly Ile Thr

            20                  25                  30

Gly Gln Asp Gly Ser Tyr Leu Ala Glu Phe Leu Leu Glu Lys Gly Tyr
        35                  40                  45

Glu Val His Gly Ile Val Arg Arg Ser Ser Ser Phe Asn Thr Gly Arg
    50                  55                  60

Ile Glu His Leu Tyr Lys Asn Pro Gln Ala His Ile Glu Gly Asn Met
65                  70                  75                  80

Lys Leu His Tyr Gly Asp Leu Thr Asp Ser Thr Cys Leu Val Lys Ile
                85                  90                  95

Ile Asn Glu Val Lys Pro Thr Glu Ile Tyr Asn Leu Gly Ala Gln Ser
            100                 105                 110

His Val Lys Ile Ser Phe Asp Leu Ala Glu Tyr Thr Ala Asp Val Asp
        115                 120                 125

Gly Val Gly Thr Leu Arg Leu Leu Asp Ala Val Lys Thr Cys Gly Leu
    130                 135                 140

Ile Asn Ser Val Lys Phe Tyr Gln Ala Ser Thr Ser Glu Leu Tyr Gly
145                 150                 155                 160

Lys Val Gln Glu Ile Pro Gln Lys Glu Thr Thr Pro Phe Tyr Pro Arg
                165                 170                 175

Ser Pro Tyr Gly Ala Ala Lys Leu Tyr Ala Tyr Trp Ile Val Val Asn
            180                 185                 190

Phe Arg Glu Ala Tyr Asn Leu Phe Ala Val Asn Gly Ile Leu Phe Asn
        195                 200                 205

His Glu Ser Pro Arg Arg Gly Ala Asn Phe Val Thr Arg Lys Ile Ser
    210                 215                 220

Arg Ser Val Ala Lys Ile Tyr Leu Gly Gln Leu Glu Cys Phe Ser Leu
225                 230                 235                 240

Gly Asn Leu Asp Ala Lys Arg Asp Trp Gly His Ala Lys Asp Tyr Val
                245                 250                 255

Glu Ala Met Trp Leu Met Leu Gln Asn Asp Glu Pro Glu Asp Phe Val
            260                 265                 270

Ile Ala Thr Gly Glu Val His Ser Val Arg Glu Phe Val Glu Lys Ser
        275                 280                 285

Phe Leu His Ile Gly Lys Thr Ile Val Trp Glu Gly Lys Asn Glu Asn
    290                 295                 300

Glu Val Gly Arg Cys Lys Glu Thr Gly Lys Val His Val Thr Val Asp
305                 310                 315                 320

Leu Lys Tyr Tyr Arg Pro Thr Glu Val Asp Phe Leu Gln Gly Asp Cys
                325                 330                 335

Thr Lys Ala Lys Gln Lys Leu Asn Trp Lys Pro Arg Val Ala Phe Asp
            340                 345                 350

Glu Leu Val Arg Glu Met Val His Ala Asp Val Glu Leu Met Arg Thr
        355                 360                 365

Asn Pro Asn Ala
    370

<210>  35
<211>  2963
<212>  DNA
<213>  Homo sapiens

<220>
<221>  CDS
<222>  (26)..(2317)
<223>

<400> 35
ccactgctgt cctcttcagc tcaag atg gtg gcc tgc cgg gcg att ggc atc 52
ctc agc cgc ttt tct gcc ttc agg atc ctc cgc tcc cga ggt tat ata 100
tgc cgc aat ttt aca ggg tct tct gct ttg ctg acc aga acc cat att 148
aac tat gga gtc aaa ggg gat gtg gca gtt gtt cga att aac tct ccc 196
aat tca aag gta aat aca ctg agt aaa gag cta cat tca gag ttc tca 244
gaa gtt atg aat gaa atc tgg gct agt gat caa atc aga agt gcc gtc 292
ctt atc tca tca aag cca ggc tgc ttt att gca ggt gct gat atc aac 340
atg tta gcc gct tgc aag acc ctt caa gaa gta aca cag cta tca caa 388
gaa gca cag aga ata gtt gag aaa ctt gaa aag tcc aca aag cct att 436
gtg gct gcc atc aat gga tcc tgc ctg gga gga gga ctt gag gtt gcc 484
att tca tgc caa tac aga ata gca aca aaa gac aga aaa aca gta tta 532
ggt acc cct gaa gtt ttg ctg ggg gcc tta cca gga gca gga ggc aca 580
caa agg ctg ccc aaa atg gtg ggt gtg cct gct gct ttg gac atg atg 628
ctg act ggt aga agc att cgt gca gac agg gca aag aaa atg gga ctg 676
gtt gac caa ctg gtg gaa ccc ctg gga cca gga cta aaa cct cca gag 724
gaa cgg aca ata gaa tac cta gaa gaa gtt gca att act ttt gcc aaa 772
gga cta gct gat aag aag atc tct cca aag aga gac aag gga ttg gtg 820
gaa aaa ttg aca gcg tat gcc atg act att cca ttt gtc agg caa cag 868
gtt tac aaa aaa gtg gaa gaa aaa gtg cga aag cag act aaa ggc ctt 916
tat cct gca cct ctg aaa ata att gat gtg gta aag act gga att gag 964
caa ggg agt gat gcc ggt tat ctc tgt gaa tct cag aaa ttt gga gag 1012
ctt gta atg acc aaa gaa tca aag gcc ttg atg gga ctc tac cat ggt 1060
cag gtc ctg tgc aag aag aat aaa ttt gga gct cca cag aag gat gtt 1108
aag cat ctg gct att ctt ggt gca ggg ctg atg gga gca ggc atc gcc 1156
caa gtc tcc gtg gat aag ggg cta aag act ata ctt aaa gat gcc acc 1204
ctc act gcg cta gac cga gga cag caa caa gtg ttc aaa gga ttg aat        1252
gac aaa gtg aag aag aaa gct cta aca tca ttt gaa agg gat tcc atc        1300
ttc agc aac ttg act ggg cag ctt gat tac caa ggt ttt gaa aag gcc        1348
gac atg gtg att gaa gct gtg ttt gag gac ctt agt ctt aag cac aga        1396
gtg cta aag gaa gta gaa gcg gtg att cca gat cac tgt atc ttt gcc        1444
agt aac aca tct gct ctc cca atc agt gaa atc gct gct gtc agc aaa        1492
aga cct gag aag gtg att ggc atg cac tac ttc tct ccc gtg gac aag        1540
atg cag ctg ctg gag att atc acg acc gag aaa act tcc aaa gac acc        1588
agt gct tca gct gta gca gtt ggt ctc aag cag ggg aag gtc atc att        1636
gtg gtt aag gat gga cct ggc ttc tat act acc agg tgt ctt gcg ccc        1684
atg atg tct gaa gtc atc cga atc ctc cag gaa gga gtt gac ccg aag        1732
aag ctg gat tcc ctg acc aca agc ttt ggc ttt cct gtg ggt gcc gcc        1780
aca ctg gtg gat gaa gtt ggt gtg gat gta gcg aaa cat gtg gcg gaa 1828
gat ctg ggc aaa gtc ttt ggg gag cgg ttt gga ggt gga aac cca gaa 1876
ctg ctg aca cag atg gtg tcc aag ggc ttc cta ggt cgt aaa tct ggg 1924
aag ggc ttt tac atc tat cag gag ggt gtg aag agg aag gat ttg aat 1972
tct gac atg gat agt att tta gcg agt ctg aag ctg cct cct aag tct 2020
gaa gtc tca tca gac gaa gac atc cag ttc cgc ctg gtg aca aga ttt 2068
gtg aat gag gca gtc atg tgc ctg caa gag ggg atc ttg gcc aca cct 2116
gca gag gga gac atc gga gcc gtc ttt ggg ctt ggc ttc ccg cct tgt 2164
ctg gga ggg cct ttc cgc ttt gtg gat ctg tat ggc gcc cag aag ata 2212
gtg gac cgg ctc aag aaa tat gaa gct gcc tat gga aaa cag ttc acc 2260
cca tgc cag ctg cta gct gac cat gct aac agc cct aac aag aag ttc 2308
tac cag tga gcaggcctca tgcctcgctc agtcagtgca ctaaccccag 2357
ctgccggcag tgctggttct ccaacagagt ggtgtctaga tttatcagag taacgagaag 2417
acaaactccg gcactgggtt tgctccctga ttaaagtgcc ttcagccaag accatctctc 2477
cctcctggtg aagtgtgact tcgaattagt ttgcacttcc tattggaagg tagagcccac 2537
tgctcattgt ataagccccg aggcctagag tggcagccaa gagccatctg aagccacctc 2597
tctgcctgtt cctcccaaga ggccagggtg gccaggggtg gtgagggcag ttctgcaccc 2657
agccaaacac ataacaataa aaaccaaact ctgtgtcagc atctttgccc ttctggttta 2717
aacgcctcct tcaaaaagca atctggaaga aagccctgtg ctttggggga gtaagaatgt 2777
gtgtgcagaa ttctaggcag caccttaggg agggactggg atgagagaaa gtgggacctg 2837
gtgggctcaa ccacacacac ctgtctgtgc agatgctttg cccaggcttc tcaccacggt 2897
gtaccgggat attaaacctc tttccccagc aaaaaaaaaa aaaaaaaaaa aaaaaaaaaa 2957
aaaaaa 2963

<210> 36
<211> 763
<212> PRT
<213> Homo sapiens

<400> 36

<210>  37
<211>  2438
<212>  DNA
<213>  Homo sapiens

<220>
<221>  CDS
<222>  (45)..(1571)
<223>

<400>  37
cccggcggcg ccaaccgaag cgccccgcct gatccgtgtc cgac atg ctg cgc cgc      56
gct ctg ctg tgc ctg gcc gtg gcc gcc ctg gtg cgc gcc gac gcc ccc      104
gag gag gag gac cac gtc ctg gtg ctg cgg aaa agc aac ttc gcg gag      152
gcg ctg gcg gcc cac aag tac ctg ctg gtg gag ttc tat gcc cct tgg      200
tgt ggc cac tgc aag gct ctg gcc cct gag tat gcc aaa gcc gct ggg      248
aag ctg aag gca gaa ggt tcc gag atc agg ttg gcc aag gtg gac gcc 296
acg gag gag tct gac ctg gcc cag cag tac ggc gtg cgc ggc tat ccc 344
acc atc aag ttc ttc agg aat gga gac acg gct tcc ccc aag gaa tat 392
aca gct ggc aga gag gct gat gac atc gtg aac tgg ctg aag aag cgc 440
acg ggc ccg gct gcc acc acc ctg cct gac ggc gca gct gca gag tcc 488
ttg gtg gag tcc agc gag gtg gct gtc atc ggc ttc ttc aag gac gtg 536
gag tcg gac tct gcc aag cag ttt ttg cag gca gca gag gcc atc gat 584
gac ata cca ttt ggg atc act tcc aac agt gac gtg ttc tcc aaa tac 632
cag ctc gac aaa gat ggg gtt gtc ctc ttt aag aag ttt gat gaa ggc 680
cgg aac aac ttt gaa ggg gag gtc acc aag gag aac ctg ctg gac ttt 728
atc aaa cac aac cag ctg ccc ctt gtc atc gag ttc acc gag cag aca 776
gcc ccg aag att ttt gga ggt gaa atc aag act cac atc ctg ctg ttc 824
ttg ccc aag agt gtg tct gac tat gac ggc aaa ctg agc aac ttc aaa 872
aca gca gcc gag agc ttc aag ggc aag atc ctg ttc atc ttc atc gac 920
agc gac cac acc gac aac cag cgc atc ctc gag ttc ttt ggc ctg aag 968
aag gaa gag tgc ccg gcc gtg cgc ctc atc acc ctg gag gag gag atg 1016
acc aag tac aag ccc gaa tcg gag gag ctg acg gca gag agg atc aca 1064
gag ttc tgc cac cgc ttc ctg gag ggc aaa atc aag ccc cac ctg atg 1112
agc cag gag ctg ccg gag gac tgg gac aag cag cct gtc aag gtg ctt 1160
gtt ggg aag aac ttt gaa gac gtg gct ttt gat gag aaa aaa aac gtc 1208
ttt gtg gag ttc tat gcc cca tgg tgt ggt cac tgc aaa cag ttg gct 1256
ccc att tgg gat aaa ctg gga gag acg tac aag gac cat gag aac atc 1304
gtc atc gcc aag atg gac tcg act gcc aac gag gtg gag gcc gtc aaa 1352
gtg cac agc ttc ccc aca ctc aag ttc ttt cct gcc agt gcc gac agg 1400
acg gtc att gat tac aac ggg gaa cgc acg ctg gat ggt ttt aag aaa 1448
ttc ctg gag agc ggt ggc cag gat ggg gca ggg gat gat gac gat ctc 1496
gag gac ctg gaa gaa gca gag gag cca gac atg gag gaa gac gat gat 1544
cag aaa gct gtg aaa gat gaa ctg taa tacgcaaagc cagacccggg 1591
cgctgccgag acccctcggg ggctgcacac ccagcagcag cgcacgcctc cgaagcctgc 1651
ggcctcgctt gaaggagggc gtcgccggaa acccagggaa cctctctgaa gtgacacctc 1711
acccctacac accgtccgtt cacccccgtc tcttccttct gcttttcggt ttttggaaag 1771
ggatccatct ccaggcagcc caccctggtg gggcttgttt cctgaaacca tgatgtactt 1831
tttcatacat gagtctgtcc agagtgcttg ctaccgtgtt cggagtctcg ctgcctccct 1891
cccgcgggag gtttctcctc tttttgaaaa ttccgtctgt gggattttta gacatttttc 1951
gacatcaggg tatttgttcc accttggcca ggcctcctcg gagaagcttg tcccccgtgt 2011
gggagggacg gagccggact ggacatggtc actcagtacc gcctgcagtg tcgccatgac 2071
tgatcatggc tcttgcattt ttgggtaaat ggagacttcc ggatcctgtc agggtgtccc 2131
ccatgcctgg aagaggagct ggtggctgcc agccctgggg cccggcacag gcctgggcct 2191
tccccttccc tcaagccagg gctcctcctc ctgtcgtggg ctcattgtga ccactggcct 2251
ctctacagca cggcctgtgg cctgttcaag gcagaaccac gacccttgac tcccgggtgg 2311
ggaggtggcc aaggatgctg gagctgaatc agacgctgac agttcttcag gcatttctat 2371
ttcacaatcg aattgaacac attggccaaa taaagttgaa attttaccac caaaaaaaaa 2431
aaaaaaa 2438

<210> 38
<211> 508
<212> PRT
<213> Homo sapiens

<400> 38

<210> 39
<211> 959
<212> DNA
<213> Homo sapiens

<220>
<221> CDS
<222> (120)..(764)
<223>

<400> 39
gcagtggagg cggcccaggc ccgccttccg cagggtgtcg ccgctgtgcc gctagcggtg 60

ccccgcctgc tgcggtggca ccagccagga ggcggagtgg aagtggccgt ggggcgggt 119

atg gga cta gct ggc gtg tgc gcc ctg aga cgc tca gcg ggc tat ata 167
Met Gly Leu Ala Gly Val Cys Ala Leu Arg Arg Ser Ala Gly Tyr Ile
1               5                   10                  15

ctc gtc ggt ggg gcc ggc ggt cag tct gcg gca gcg gca gca aga cgg 215
Leu Val Gly Gly Ala Gly Gly Gln Ser Ala Ala Ala Ala Ala Arg Arg

20                    25                    30

tgc agt gaa gga gag tgg gcg tct ggc ggg gtc cgc agt ttc agc aga          263
Cys Ser Glu Gly Glu Trp Ala Ser Gly Gly Val Arg Ser Phe Ser Arg
        35                  40                  45

gcc gct gca gcc atg gcc cca atc aag gtg gga gat gcc atc cca gca          311
Ala Ala Ala Ala Met Ala Pro Ile Lys Val Gly Asp Ala Ile Pro Ala
    50                  55                  60

gtg gag gtg ttt gaa ggg gag cca ggg aac aag gtg aac ctg gca gag          359
Val Glu Val Phe Glu Gly Glu Pro Gly Asn Lys Val Asn Leu Ala Glu
65                  70                  75                  80

ctg ttc aag ggc aag aag ggt gtg ctg ttt gga gtt cct ggg gcc ttc          407
Leu Phe Lys Gly Lys Lys Gly Val Leu Phe Gly Val Pro Gly Ala Phe
                85                  90                  95

acc cct gga tgt tcc aag aca cac ctg cca ggg ttt gtg gag cag gct          455
Thr Pro Gly Cys Ser Lys Thr His Leu Pro Gly Phe Val Glu Gln Ala
            100                 105                 110

gag gct ctg aag gcc aag gga gtc cag gtg gtg gcc tgt ctg agt gtt          503
Glu Ala Leu Lys Ala Lys Gly Val Gln Val Val Ala Cys Leu Ser Val
        115                 120                 125

aat gat gcc ttt gtg act ggc gag tgg ggc cga gcc cac aag gcg gaa          551
Asn Asp Ala Phe Val Thr Gly Glu Trp Gly Arg Ala His Lys Ala Glu
    130                 135                 140

ggc aag gtt cgg ctc ctg gct gat ccc act ggg gcc ttt ggg aag gag          599
Gly Lys Val Arg Leu Leu Ala Asp Pro Thr Gly Ala Phe Gly Lys Glu
145                 150                 155                 160

aca gac tta tta cta gat gat tcg ctg gtg tcc atc ttt ggg aat cga          647
Thr Asp Leu Leu Leu Asp Asp Ser Leu Val Ser Ile Phe Gly Asn Arg
                165                 170                 175

cgt ctc aag agg ttc tcc atg gtg gta cag gat ggc ata gtg aag gcc          695
Arg Leu Lys Arg Phe Ser Met Val Val Gln Asp Gly Ile Val Lys Ala
            180                 185         .       190

ctg aat gtg gaa cca gat ggc aca ggc ctc acc tgc agc ctg gca ccc          743
Leu Asn Val Glu Pro Asp Gly Thr Gly Leu Thr Cys Ser Leu Ala Pro
        195                 200                 205

aat atc atc tca cag ctc tga ggccctgggc cagattactt cctccacccc          794
Asn Ile Ile Ser Gln Leu
    210

tccctatctc acctgcccag ccctgtgctg gggccctgca attggaatgt tggccagatt 854

tctgcaataa acacttgtgg tttgcggcca aaaaaaaaaa aaaaaaaaaa aaaaaaaaaa 914

aaaaaaaaaa aaaaaaaaaa aaaaaaaaaa aaaaaaaaaa aaaaa 959

<210> 40
<211> 214
<212> PRT
<213> Homo sapiens

<400> 40

Met Gly Leu Ala Gly Val Cys Ala Leu Arg Arg Ser Ala Gly Tyr Ile
1               5                   10                  15

Leu Val Gly Gly Ala Gly Gly Gln Ser Ala Ala Ala Ala Ala Arg Arg
            20                  25                  30

Cys Ser Glu Gly Glu Trp Ala Ser Gly Gly Val Arg Ser Phe Ser Arg
        35                  40                  45

Ala Ala Ala Ala Met Ala Pro Ile Lys Val Gly Asp Ala Ile Pro Ala
    50                  55                  60

Val Glu Val Phe Glu Gly Glu Pro Gly Asn Lys Val Asn Leu Ala Glu
65                  70                  75                  80

Leu Phe Lys Gly Lys Lys Gly Val Leu Phe Gly Val Pro Gly Ala Phe
                85                  90                  95

Thr Pro Gly Cys Ser Lys Thr His Leu Pro Gly Phe Val Glu Gln Ala
            100                 105                 110

Glu Ala Leu Lys Ala Lys Gly Val Gln Val Val Ala Cys Leu Ser Val
        115                 120                 125

Asn Asp Ala Phe Val Thr Gly Glu Trp Gly Arg Ala His Lys Ala Glu
    130                 135                 140

Gly Lys Val Arg Leu Leu Ala Asp Pro Thr Gly Ala Phe Gly Lys Glu
145                 150                 155                 160

Thr Asp Leu Leu Leu Asp Asp Ser Leu Val Ser Ile Phe Gly Asn Arg
                165                 170                 175

Arg Leu Lys Arg Phe Ser Met Val Val Gln Asp Gly Ile Val Lys Ala
            180                 185                 190

Leu Asn Val Glu Pro Asp Gly Thr Gly Leu Thr Cys Ser Leu Ala Pro
        195                 200                 205

Asn Ile Ile Ser Gln Leu
    210

<210>  41
<211>  1874
<212>  DNA
<213>  Homo sapiens

<220>
<221>  CDS
<222>  (7)..(678)
<223>

<400>  41
ccggtg atg gcg gct ggt gat ggg gac gtg aag cta ggc acc ctg ggg        48
       Met Ala Ala Gly Asp Gly Asp Val Lys Leu Gly Thr Leu Gly
       1               5                   10

agt ggc agc gag agc agc aac gac ggc ggc agc gag agt cca ggc gac        96
Ser Gly Ser Glu Ser Ser Asn Asp Gly Gly Ser Glu Ser Pro Gly Asp
15                  20                  25                  30

gcg gga gcg gca gcg gaa ggg gga ggc tgg gcg gcg gcg gcg ttg gcg       144
Ala Gly Ala Ala Ala Glu Gly Gly Gly Trp Ala Ala Ala Ala Leu Ala
                35                  40                  45

ctt ctg acg ggg ggc ggg gaa atg ctg ctg aac gtg gcg ctg gtg gct       192
Leu Leu Thr Gly Gly Gly Glu Met Leu Leu Asn Val Ala Leu Val Ala
            50                  55                  60

ctg gtg ctg ctg ggg gcc tac cgg ctg tgg gtg cgc tgg ggg cgg cgg       240
Leu Val Leu Leu Gly Ala Tyr Arg Leu Trp Val Arg Trp Gly Arg Arg
        65                  70                  75

ggt ctg ggg gcc ggg gcc ggg gcg ggc gag gag agc ccc gcc acc tct 288
Gly Leu Gly Ala Gly Ala Gly Ala Gly Glu Glu Ser Pro Ala Thr Ser
    80                  85                  90

ctg cct cgc atg aag aag cgg gac ttc agc ttg gag cag ctg cgc cag 336
Leu Pro Arg Met Lys Lys Arg Asp Phe Ser Leu Glu Gln Leu Arg Gln
95                  100                 105                 110

tac gac ggc tcc cgc aac ccg cgc atc ctg ctc gcg gtc aat ggg aaa 384
Tyr Asp Gly Ser Arg Asn Pro Arg Ile Leu Leu Ala Val Asn Gly Lys
                115                 120                 125

gtc ttc gac gtg acc aaa ggc agc aag ttc tac ggc ccg gcg ggt cca 432
Val Phe Asp Val Thr Lys Gly Ser Lys Phe Tyr Gly Pro Ala Gly Pro
            130                 135                 140

tat gga ata ttt gct ggt agg gat gcc tcc aga gga ctg gcc aca ttt 480
Tyr Gly Ile Phe Ala Gly Arg Asp Ala Ser Arg Gly Leu Ala Thr Phe
        145                 150                 155

tgc cta gat aaa gat gca ctt aga gat gaa tat gat gat ctc tca gat 528
Cys Leu Asp Lys Asp Ala Leu Arg Asp Glu Tyr Asp Asp Leu Ser Asp
    160                 165                 170

ttg aat gca gta caa atg gag agt gtt cga gaa tgg gaa atg cag ttt 576
Leu Asn Ala Val Gln Met Glu Ser Val Arg Glu Trp Glu Met Gln Phe
175                 180                 185                 190

aaa gaa aaa tat gat tat gta ggc aga ctc cta aaa cca gga gaa gaa 624
Lys Glu Lys Tyr Asp Tyr Val Gly Arg Leu Leu Lys Pro Gly Glu Glu
                195                 200                 205

cca tca gaa tat aca gat gaa gaa gat acc aag gat cac aat aaa cag 672
Pro Ser Glu Tyr Thr Asp Glu Glu Asp Thr Lys Asp His Asn Lys Gln
            210                 215                 220

gat tga actttgtaaa caaccaaagt caggggcctt cagaactgca attcttactc 728
Asp

cctttcacag actgtccgga gtctttgggt ttgattcacc tgctgcgaaa aacattcaac 788

aaattgtgta caagataaat taatctcact atgaagattt gaataactag acattattta 848

tgctgccaaa ctcatttgtt gcagttgttt gtaatgtcta gtggggcttc atcatcctga 908

aaagaaggag acagggattt ttttaaagag caagaaagtc acaatattac ttctttcctt 968

ccttttttcc ttctttcctt tcttctttct ctttctttct ttttaaaata tattgaagac 1028

aaccagatat gtatttgcta ctcaagtgta cagatctcct caagaaacat caagggactc 1088

ctgtgtcaca tactgtgttt ttattttaac atgggtgagg gaggcgacct gatcagggga 1148

ggtgggggta cacatcaatt tgagttgttc aggctactga aacattaaaa tgtgaattcc 1208

caaacttttc tttttggctt tgtcagggaa aagaaaaata tctttataaa gaaatctttg 1268

gaaattagga gaaggaattt caggtgggtt taagtcagag ctagttcccc aacagaaaga 1328

tcatttgaaa ccagttttta tcccttctct ttccttccct ttccctaaat caaatcaata 1388

ttaattgtgc cttatttcac ttaacataga cttgaattat ttttagggaa agcccctata 1448

atgaattcag aaatcactac aagcagcatt aagactgaag ttggaatatt ctgttgacca 1508

taaaaccttg atatcattct gtgtatatag aatgtaaaag gaatattaca gtgttaactg 1568

ccatatatgt aatatacaca aactcaatta gcattgtaat ggccaaatgc attcccccat 1628

gcttttctgt tttcaaaaaa attgaaaaac aaatcaactc ttatccccaa cagctgccta 1688

attttaggag tctgaccctc cacatctcac tggtgtgggt gcatggggct gtggagtggg 1748

tgtcagtatg gatgtgtctg aatgtgtgag gccttggaag ggactctttc tgcagatact 1808

gtaaatacaa gtaccatttt aataaagcat gtacaataaa ccaaaaaaaa aaaaaaaaaa 1868

aaaaaa 1874

<210> 42
<211> 223
<212> PRT
<213> Homo sapiens

<400> 42

Met Ala Ala Gly Asp Gly Asp Val Lys Leu Gly Thr Leu Gly Ser Gly
1               5                   10                  15

Ser Glu Ser Ser Asn Asp Gly Gly Ser Glu Ser Pro Gly Asp Ala Gly
            20                  25                  30

Ala Ala Ala Glu Gly Gly Gly Trp Ala Ala Ala Ala Leu Ala Leu Leu
        35                  40                  45

Thr Gly Gly Gly Glu Met Leu Leu Asn Val Ala Leu Val Ala Leu Val
    50                  55                  60

Leu Leu Gly Ala Tyr Arg Leu Trp Val Arg Trp Gly Arg Arg Gly Leu
65                  70                  75                  80

Gly Ala Gly Ala Gly Ala Gly Glu Glu Ser Pro Ala Thr Ser Leu Pro
                85                  90                  95

Arg Met Lys Lys Arg Asp Phe Ser Leu Glu Gln Leu Arg Gln Tyr Asp
            100                 105                 110

Gly Ser Arg Asn Pro Arg Ile Leu Leu Ala Val Asn Gly Lys Val Phe
        115                 120                 125

Asp Val Thr Lys Gly Ser Lys Phe Tyr Gly Pro Ala Gly Pro Tyr Gly
    130                 135                 140

Ile Phe Ala Gly Arg Asp Ala Ser Arg Gly Leu Ala Thr Phe Cys Leu
145                 150                 155                 160

Asp Lys Asp Ala Leu Arg Asp Glu Tyr Asp Asp Leu Ser Asp Leu Asn
                165                 170                 175

Ala Val Gln Met Glu Ser Val Arg Glu Trp Glu Met Gln Phe Lys Glu
            180                 185                 190

Lys Tyr Asp Tyr Val Gly Arg Leu Leu Lys Pro Gly Glu Glu Pro Ser
        195                 200                 205

Glu Tyr Thr Asp Glu Glu Asp Thr Lys Asp His Asn Lys Gln Asp
    210                 215                 220

<210>  43
<211>  3795
<212>  DNA
<213>  Homo sapiens

<220>
<221> CDS
<222> (236)..(1633)
<223>

<400> 43
cagcggcggt ggcccaggta gaggggtccg cgctggcggc ggcggcggcg ctgttccccg 60

cgcggtccgc ggagcggggt ccgggctgcg cgacgtgggg cggcggcggc actgcggccc 120

cggcccaagc ccgaccccgg gtcccctcct cggccgcccc ccgcccggcc gcccgccctc 180

gggcctcccc ccgggccctc ggtcccctcc cccgctggcg gggcccggac agaag atg 238
Met
1

gtg cag aag aaa cca gcc gaa ctt cag ggt ttc cac cgt tcg ttc aag 286
Val Gln Lys Lys Pro Ala Glu Leu Gln Gly Phe His Arg Ser Phe Lys
            5                   10                  15

ggg cag aac ccc ttc gag ctg gcc ttc tcc cta gac cag ccc gac cac 334
Gly Gln Asn Pro Phe Glu Leu Ala Phe Ser Leu Asp Gln Pro Asp His
        20                  25                  30

gga gac tct gac ttt ggc ctg cag tgc tca gcc cgc cct gac atg ccc 382
Gly Asp Ser Asp Phe Gly Leu Gln Cys Ser Ala Arg Pro Asp Met Pro
    35                  40                  45

gcc agc cag ccc att gac atc ccg gac gcc aag aag agg ggc aag aag 430
Ala Ser Gln Pro Ile Asp Ile Pro Asp Ala Lys Lys Arg Gly Lys Lys
50                  55                  60                  65

aag aag cgc ggc cgg gcc acc gac agc ttc tcg ggc agg ttt gaa gac 478
Lys Lys Arg Gly Arg Ala Thr Asp Ser Phe Ser Gly Arg Phe Glu Asp
                70                  75                  80

gtc tac cag ctg cag gaa gat gtg ctg ggg gag ggc gct cat gcc cga 526
Val Tyr Gln Leu Gln Glu Asp Val Leu Gly Glu Gly Ala His Ala Arg
            85                  90                  95

gtg cag acc tgc atc aac ctg atc acc agc cag gag tac gcc gtc aag 574
Val Gln Thr Cys Ile Asn Leu Ile Thr Ser Gln Glu Tyr Ala Val Lys
        100                 105                 110

atc att gag aag cag cca ggc cac att cgg agc agg gtt ttc agg gag 622
Ile Ile Glu Lys Gln Pro Gly His Ile Arg Ser Arg Val Phe Arg Glu
    115                 120                 125

gtg gag atg ctg tac cag tgc cag gga cac agg aac gtc cta gag ctg 670
Val Glu Met Leu Tyr Gln Cys Gln Gly His Arg Asn Val Leu Glu Leu

130                 135                 140                 145

att gag ttc ttc gag gag gag gac cgc ttc tac ctg gtg ttt gag aag     718
Ile Glu Phe Phe Glu Glu Glu Asp Arg Phe Tyr Leu Val Phe Glu Lys
                150                 155                 160

atg cgg gga ggc tcc atc ctg agc cac atc cac aag cgc cgg cac ttc     766
Met Arg Gly Gly Ser Ile Leu Ser His Ile His Lys Arg Arg His Phe
            165                 170                 175

aac gag ctg gag gcc agc gtg gtg gtg cag gac gtg gcc agc gcc ttg     814
Asn Glu Leu Glu Ala Ser Val Val Val Gln Asp Val Ala Ser Ala Leu
        180                 185                 190

gac ttt ctg cat aac aaa ggc atc gcc cac agg gac cta aag ccg gaa     862
Asp Phe Leu His Asn Lys Gly Ile Ala His Arg Asp Leu Lys Pro Glu
    195                 200                 205

aac atc ctc tgt gag cac ccc aac cag gtc tcc ccc gtg aag atc tgt     910
Asn Ile Leu Cys Glu His Pro Asn Gln Val Ser Pro Val Lys Ile Cys
210                 215                 220                 225

gac ttc gac ctg ggc agc ggc atc aaa ctc aac ggg gac tgc tcc cct     958
Asp Phe Asp Leu Gly Ser Gly Ile Lys Leu Asn Gly Asp Cys Ser Pro
                230                 235                 240

atc tcc acc ccg gag ctg ctc act ccg tgc ggc tcg gcg gag tac atg     1006
Ile Ser Thr Pro Glu Leu Leu Thr Pro Cys Gly Ser Ala Glu Tyr Met
            245                 250                 255

gcc ccg gag gta gtg gag gcc ttc agc gag gag gct agc atc tac gac     1054
Ala Pro Glu Val Val Glu Ala Phe Ser Glu Glu Ala Ser Ile Tyr Asp
        260                 265                 270

aag cgc tgc gac ctg tgg agc ctg ggc gtc atc ttg tat atc cta ctc     1102
Lys Arg Cys Asp Leu Trp Ser Leu Gly Val Ile Leu Tyr Ile Leu Leu
    275                 280                 285

agc ggc tac ccg ccc ttc gtg ggc cgc tgt ggc agc gac tgc ggc tgg     1150
Ser Gly Tyr Pro Pro Phe Val Gly Arg Cys Gly Ser Asp Cys Gly Trp
290                 295                 300                 305

gac cgc ggc gag gcc tgc cct gcc tgc cag aac atg ctg ttt gag agc     1198
Asp Arg Gly Glu Ala Cys Pro Ala Cys Gln Asn Met Leu Phe Glu Ser
                310                 315                 320

atc cag gag ggc aag tac gag ttc ccc gac aag gac tgg gcc cac atc     1246
Ile Gln Glu Gly Lys Tyr Glu Phe Pro Asp Lys Asp Trp Ala His Ile
            325                 330                 335

tcc tgc gct gcc aaa gac ctc atc tcc aag ctg ctg gtc cgt gac gcc     1294
Ser Cys Ala Ala Lys Asp Leu Ile Ser Lys Leu Leu Val Arg Asp Ala
        340                 345                 350

aag cag agg ctg agt gcc gcc caa gtc ctg cag cac ccc tgg gtt cag     1342
Lys Gln Arg Leu Ser Ala Ala Gln Val Leu Gln His Pro Trp Val Gln
    355                 360                 365

ggg tgc gcc ccg gag aac acc ttg ccc act ccc atg gtc ctg cag agg     1390
Gly Cys Ala Pro Glu Asn Thr Leu Pro Thr Pro Met Val Leu Gln Arg
370                 375                 380                 385

aac agc tgt gcc aaa gac ctc acg tcc ttc gcg gct gag gcc att gcc     1438
Asn Ser Cys Ala Lys Asp Leu Thr Ser Phe Ala Ala Glu Ala Ile Ala
                390                 395                 400

atg aac cgg cag ctg gcc cag cac gac gag gac ctg gct gag gag gag     1486
Met Asn Arg Gln Leu Ala Gln His Asp Glu Asp Leu Ala Glu Glu Glu
            405                 410                 415

gcc gcg ggg cag ggc cag ccc gtc ctg gtc cga gct acc tca cgc tgc     1534
Ala Ala Gly Gln Gly Gln Pro Val Leu Val Arg Ala Thr Ser Arg Cys
        420                 425                 430

ctg cag ctg tct cca ccc tcc cag tcc aag ctg gcg cag cgg cgg caa     1582
Leu Gln Leu Ser Pro Pro Ser Gln Ser Lys Leu Ala Gln Arg Arg Gln
    435                 440                 445

agg gcc agt ctg tcc tcg gcc cca gtg gtc ctg gtg gga gac cac gcc     1630
Arg Ala Ser Leu Ser Ser Ala Pro Val Val Leu Val Gly Asp His Ala
450                 455                 460                 465

tga ccctcccatc tcccctctgt acataggtca cccgtccccc aatcaaatct     1683

aaaggttttt taagctatcg ccagccggtg tccagcgggc tgcccctcct ctgcctggat     1743

tcccaggcac taagctcagc tgaggggggt gttttataga aggtttttgc ttttgggttt     1803

tttttttcct gtttccaccc ctccccgtta ttttttcctt tggatggtta aaagcattgc     1863

aggcacccgg gaaggtgagc agagggtagg tgggtgggct tgtcccctcc ccggtccccc     1923

gccctgctca cctctactat gaaggtgccc ccaggtcacc tgtgctgccc gccatctgcc     1983

cacgtggctt gcagtgactc aggagagcag gcccacagcg tttgccatct tgcagagctg     2043

gggaggggca caggaccctg ccctcgtgtt ccctcccagc ccgcagtatt tcagggacag     2103

gctcttcccc tctatccctc accctgagag cacccctggt ggcttggttg gggaagggag     2163

gggctgcctg tctctggagg tgtcaggcag gcaggtggca ggcagctcac ccacccaccc 2223
catgggatcc cccagccctt cacccgcgcc tgccttgtcc ccatgatagt tgacaatcgg 2283
ggcttcctgc aaggcccgtc tgtctgtcca ggactcctgg tggccagatt cggcctccga 2343
ccttgacctt aaactgcagc tgaccccagg ggctcgccgc tgcccctccc ctccacacca 2403
aggcctgaga cagcaggagc cccgcctggc ccgaagccgt ttccaccgca gcaggcagag 2463
gggctggaca ggcactgtca gccaatgtgg ggggtcctga agacaccccc ttggggcacc 2523
cgagtgcccc ttctcagggc tcagtctgac cgtagccacg tcctgcctcg cgccgcccct 2583
cgggcctgac ctggaagctc cgtcagctcc gtccttgtcc ttagagctga gcccagaccc 2643
cggggtctgg ccgaatcctc acccccaggg cagtgttttt ggtctgccac cttcaggaaa 2703
acggctgcgg cctcggcctc ccttcgggca cccaggaatg cgggggtctg ctcagtcccc 2763
ccaccctcca tgctccaacc cccgggggct gcggagcctg ctgccccctc cccgcgggtg 2823
gggacgttct atgcaataca gggttccact ttagaagtgc gcgcggctag ggtcaccgcc 2883
cgcccttccc ggcgcagccc ccgagctcca cagctggggc agcccctctg gcttctaaat 2943
ccgcggtcgg gattcttcct cctgtttagt tttttagttt ttccttaaaa aaaaacaaca 3003
catcgatgga ctttgcttcc ctgttcttga agaatacttg aatgtcgggg ggcctggggg 3063
tgggggcctc ggagaccgtc tgcctggccc tgctgcccct cctgaatctc gtatgatggt 3123
cacagtccgg tggccgtggg ggtgctctgc cttccctggt ccccactgcc catatctgtg 3183
gactgcccct tccaaagacc cctggggggg gtggggcatt ccgcccaccc ctttccccca 3243
tcacttctcg cctgtcagtg attccatgtt tcgtaacggg ggattctctg cctttttgta 3303
tcaaagaaca agcaaatgga cccccgcccg ctgcaggcgc ccatagccat cgggtctcta 3363
aagctgagtg gctagcagcg tttgtttgtt tgtttttttt tttttttctg aaggtgggac 3423
agtcacttcc tcctccctcc ccacccctgt cgcatccacg tgcgacctgg aggactggtc 3483
agaaccgtta ctgtgaatga gtgaagatcc tggaggaccc tgggccccag gccagctccc 3543
atcgctgggg gacggtgaac ggccatgtgt taatgttacg atgtttttaa aagacaaaaa 3603
aaaaaaaaaa acctcaaaag tttttttaaa gtgggggaaa aacatccaag cactttaatt 3663

ccaatgtacc aggtgaactg acggagctca gaagttttcc tttacaccaa ctgtcaatgc 3723

cggaattttg tattctgttt tgtaaagatt taataaaagt caaaaaactt gcaaaaaaaa 3783

aaaaaaaaaa aa 3795

<210> 44
<211> 465
<212> PRT
<213> Homo sapiens

<400> 44

Met Val Gln Lys Lys Pro Ala Glu Leu Gln Gly Phe His Arg Ser Phe
1               5                   10                  15

Lys Gly Gln Asn Pro Phe Glu Leu Ala Phe Ser Leu Asp Gln Pro Asp
            20                  25                  30

His Gly Asp Ser Asp Phe Gly Leu Gln Cys Ser Ala Arg Pro Asp Met
        35                  40                  45

Pro Ala Ser Gln Pro Ile Asp Ile Pro Asp Ala Lys Lys Arg Gly Lys
    50                  55                  60

Lys Lys Lys Arg Gly Arg Ala Thr Asp Ser Phe Ser Gly Arg Phe Glu
65                  70                  75                  80

Asp Val Tyr Gln Leu Gln Glu Asp Val Leu Gly Glu Gly Ala His Ala
                85                  90                  95

Arg Val Gln Thr Cys Ile Asn Leu Ile Thr Ser Gln Glu Tyr Ala Val
            100                 105                 110

Lys Ile Ile Glu Lys Gln Pro Gly His Ile Arg Ser Arg Val Phe Arg
        115                 120                 125

Glu Val Glu Met Leu Tyr Gln Cys Gln Gly His Arg Asn Val Leu Glu
    130                 135                 140

Leu Ile Glu Phe Phe Glu Glu Glu Asp Arg Phe Tyr Leu Val Phe Glu
145                 150                 155                 160

Lys Met Arg Gly Gly Ser Ile Leu Ser His Ile His Lys Arg Arg His
                165                 170                 175

Phe Asn Glu Leu Glu Ala Ser Val Val Val Gln Asp Val Ala Ser Ala
            180                 185                 190

Leu Asp Phe Leu His Asn Lys Gly Ile Ala His Arg Asp Leu Lys Pro
        195                 200                 205

Glu Asn Ile Leu Cys Glu His Pro Asn Gln Val Ser Pro Val Lys Ile
    210                 215                 220

Cys Asp Phe Asp Leu Gly Ser Gly Ile Lys Leu Asn Gly Asp Cys Ser
225                 230                 235                 240

Pro Ile Ser Thr Pro Glu Leu Leu Thr Pro Cys Gly Ser Ala Glu Tyr
                245                 250                 255

Met Ala Pro Glu Val Val Glu Ala Phe Ser Glu Glu Ala Ser Ile Tyr
            260                 265                 270

Asp Lys Arg Cys Asp Leu Trp Ser Leu Gly Val Ile Leu Tyr Ile Leu
        275                 280                 285

Leu Ser Gly Tyr Pro Pro Phe Val Gly Arg Cys Gly Ser Asp Cys Gly
    290                 295                 300

Trp Asp Arg Gly Glu Ala Cys Pro Ala Cys Gln Asn Met Leu Phe Glu
305                 310                 315                 320

Ser Ile Gln Glu Gly Lys Tyr Glu Phe Pro Asp Lys Asp Trp Ala His
                325                 330                 335

Ile Ser Cys Ala Ala Lys Asp Leu Ile Ser Lys Leu Leu Val Arg Asp
            340                 345                 350

Ala Lys Gln Arg Leu Ser Ala Ala Gln Val Leu Gln His Pro Trp Val
        355                 360                 365

Gln Gly Cys Ala Pro Glu Asn Thr Leu Pro Thr Pro Met Val Leu Gln
    370                 375                 380

Arg Asn Ser Cys Ala Lys Asp Leu Thr Ser Phe Ala Ala Glu Ala Ile
385                 390                 395                 400

Ala Met Asn Arg Gln Leu Ala Gln His Asp Glu Asp Leu Ala Glu Glu
                405                 410                 415

Glu Ala Ala Gly Gln Gly Gln Pro Val Leu Val Arg Ala Thr Ser Arg
            420                 425                 430

Cys Leu Gln Leu Ser Pro Pro Ser Gln Ser Lys Leu Ala Gln Arg Arg
        435                 440                 445

Gln Arg Ala Ser Leu Ser Ser Ala Pro Val Val Leu Val Gly Asp His
    450                 455                 460

Ala
465

<210>  45
<211>  631
<212>  DNA
<213>  Homo sapiens

<400>  45
gaggtgaggg gcgcctctgc ccggccgccc ctactgggaa gtgaggagcc cctctgcctg      60

gccagccgcc ccgtccggga gggcggtggg ggggtcagcc cccctcccgg ccagccgccc     120

catctgggag gtgaggggca cttctgccgg gccgccccta ctgggaagtg aggagcccct     180

ctgcccggcc acgaccccgt ctgggaggtg tgcccagcgg ctcattgggg atgggccatg     240

atgacaatgg cggttttgtg gaatagaaag gcgggaaggg tggggaaaaa attgagaaat     300

cggatggttg cggggtctgt gtggatagaa gtagacatgg gagacttttc attttgttct     360

gtactaagaa aaattcttct gccttgggat cctgttgatc tgtgacctta tccccaaccc     420

tgtgctctct gaaacatgtg ctgtgtccac tcagggttaa atggattaag ggcggtgcaa     480

gatgtgcttt gttaaacaga tgcttgaagg cagcatgctc gttaagagtc atcaccactc     540

cctaatctta agtacccagg gacacaaaca ctgcggaagg ccgcagggtc ctctgcctag     600

gaaaaccaga gacctttgtt cacttgttat c                                    631

<210>  46
<211>  472
<212>  DNA
<213>  Homo sapiens

<400>  46
tactgggaag tgaggaaccc ctctgcccgg ccagccgccc cgtccgggag ggaggtgggg      60

ggatcagccc cccgcctggc agccgccccg tccgggaggt gaggggcgcc tctgcccggc     120

cgcccctact gggaagtgag gagcccctct gcccggccag ccgccccgtc cgggagggag     180

gtgggggggt cagccgccgc ccgtccggga cctagctggg caccgatggg ccggcacggg     240

tacggtggca ggaagcgtca ggcgagcgaa gtgcgcgtaa cagtgccagc gacgacgaga     300

cgagcaaagg tagtgccgag tgccagtaca tgatgccatc ctcagacgat gggaggcggt     360

gtcgacgacc cgcgcgctgt actgagcaac agggtacact aagaatacaa cagtgggtcg     420

cagtgcctat gagagaataa gtgatgacgc gtgtcgagcg attgtaccca ga             472

<210>  47
<211>  7026
<212>  DNA
<213>  Homo sapiens

<220>
<221>  CDS
<222>  (420)..(5045)
<223>

<400>  47
gacacgcacg cacccgggcg ccgaagggaa agccgcgtct cgccctcccg ccccgccgtc      60

ggtcctgtct cagtccctca gcagagcggg aaagcggagg ccggagccgt gacctctgac     120

cccgtggtta tgcggagccg ccgcattcct tagcgatcgc ggggcagccg ccgctgccgc 180

cgtgggcgac tgacgcagcg cgggcgcgtg gagccgccgc cgcccctccc ccaccgccgc 240

tctcgcgcca gccggtcccc gcgtgcccgc cccttctccc cggccgcacc cgagacctcg 300

cgcgccgccg ctgccacgcg ccccccccac cgccgccgcc gccccagccc cgcgccaccg 360

ccccagcccg cccagcccgg aggtcccgcg tggagctgcc gccgccgccg gggagaagg 419

atg aag gac aaa cag aag aag aag aag gag cgc acg tgg gcc gag gcc 467
Met Lys Asp Lys Gln Lys Lys Lys Lys Glu Arg Thr Trp Ala Glu Ala
1               5                   10                  15

gcg cgc ctg gta tta gaa aac tac tcg gat gct cca atg aca cca aaa 515
Ala Arg Leu Val Leu Glu Asn Tyr Ser Asp Ala Pro Met Thr Pro Lys
            20                  25                  30

cag att ctg cag gtc ata gag gca gaa gga cta aag gaa atg aga agt 563
Gln Ile Leu Gln Val Ile Glu Ala Glu Gly Leu Lys Glu Met Arg Ser
        35                  40                  45

ggg act tcc cct ctc gca tgc ctc aat gct atg cta cat tcc aat tca 611
Gly Thr Ser Pro Leu Ala Cys Leu Asn Ala Met Leu His Ser Asn Ser
    50                  55                  60

aga gga gga gag ggg ttg ttt tat aaa ctg cct ggc cga atc agc ctt 659
Arg Gly Gly Glu Gly Leu Phe Tyr Lys Leu Pro Gly Arg Ile Ser Leu
65                  70                  75                  80

ttc acg ctc aag aag gat gcc ctg cag tgg tct cgc cat cca gct aca 707
Phe Thr Leu Lys Lys Asp Ala Leu Gln Trp Ser Arg His Pro Ala Thr
                85                  90                  95

gtg gag gga gag gag cca gag gac acg gct gat gtg gag agc tgt ggg 755
Val Glu Gly Glu Glu Pro Glu Asp Thr Ala Asp Val Glu Ser Cys Gly
            100                 105                 110

tct aat gaa gcc agc act gtg agt ggt gaa aac gat gta tct ctt gat 803
Ser Asn Glu Ala Ser Thr Val Ser Gly Glu Asn Asp Val Ser Leu Asp
        115                 120                 125

gaa aca tct tcg aac gca tcc tgt tct aca gaa tct cag agt cga cct 851
Glu Thr Ser Ser Asn Ala Ser Cys Ser Thr Glu Ser Gln Ser Arg Pro
    130                 135                 140

ctt tcc aat ccc agg gac agc tac aga gct tcc tca cag gcg aac aaa 899
Leu Ser Asn Pro Arg Asp Ser Tyr Arg Ala Ser Ser Gln Ala Asn Lys
145                 150                 155                 160

caa aag aaa aag act ggg gtg atg ctg cct cga gtt gtc ctg act cct 947
Gln Lys Lys Lys Thr Gly Val Met Leu Pro Arg Val Val Leu Thr Pro
                165                 170                 175

ctg aag gta aac ggg gcc cac gtg gaa tct gca tca ggg ttc tcg ggc 995
Leu Lys Val Asn Gly Ala His Val Glu Ser Ala Ser Gly Phe Ser Gly
            180                 185                 190

tgc cac gcc gat ggc gag agc ggc agc ccg tcc agc agc agc agc ggc 1043
Cys His Ala Asp Gly Glu Ser Gly Ser Pro Ser Ser Ser Ser Ser Gly
        195                 200                 205

tct ctg gcc ctg ggc agc gct gct att cgt ggc cag gcc gag gtc acc 1091
Ser Leu Ala Leu Gly Ser Ala Ala Ile Arg Gly Gln Ala Glu Val Thr
    210                 215                 220

cag gac cct gcc ccg ctc ctg aga ggc ttc cgg aag cca gcc aca ggt 1139
Gln Asp Pro Ala Pro Leu Leu Arg Gly Phe Arg Lys Pro Ala Thr Gly
225                 230                 235                 240

caa atg aag cgc aac aga ggg gaa gaa ata gat ttt gag aca cct ggg 1187
Gln Met Lys Arg Asn Arg Gly Glu Glu Ile Asp Phe Glu Thr Pro Gly
                245                 250                 255

tcc att ctt gtc aac acc aac ctc cgt gcc ctg atc aac tct cgg acc 1235
Ser Ile Leu Val Asn Thr Asn Leu Arg Ala Leu Ile Asn Ser Arg Thr
            260                 265                 270

ttc cat gcc tta cca tca cac ttc cag cag cag ctc ctc ttc ctc ctg 1283
Phe His Ala Leu Pro Ser His Phe Gln Gln Gln Leu Leu Phe Leu Leu
        275                 280                 285

cct gaa gta gac aga cag gtg ggg acg gat ggc ctg ttg cgt ctc agc 1331
Pro Glu Val Asp Arg Gln Val Gly Thr Asp Gly Leu Leu Arg Leu Ser
    290                 295                 300

agc agt gca cta aat aac gag ttt ttt acc cat gcg gct cag agc tgg 1379
Ser Ser Ala Leu Asn Asn Glu Phe Phe Thr His Ala Ala Gln Ser Trp
305                 310                 315                 320

cgg gag cgc ctg gct gat ggt gaa ttt act cat gag atg caa gtc agg 1427
Arg Glu Arg Leu Ala Asp Gly Glu Phe Thr His Glu Met Gln Val Arg
                325                 330                 335

ata cga cag gaa atg gag aag gaa aag aag gtg gaa caa tgg aaa gaa 1475
Ile Arg Gln Glu Met Glu Lys Glu Lys Lys Val Glu Gln Trp Lys Glu
            340                 345                 350

aag ttc ttt gaa gac tac tat gga cag aag ctg ggt ttg acc aaa gaa 1523

Lys Phe Phe Glu Asp Tyr Tyr Gly Gln Lys Leu Gly Leu Thr Lys Glu
        355                 360                 365

gag tca ttg cag cag aac gtg ggc cag gag gag gct gaa atc aaa agt 1571
Glu Ser Leu Gln Gln Asn Val Gly Gln Glu Glu Ala Glu Ile Lys Ser
    370                 375                 380

ggc ttg tgt gtc cca gga gaa tca gtg cgt ata cag cgt ggt cca gcc 1619
Gly Leu Cys Val Pro Gly Glu Ser Val Arg Ile Gln Arg Gly Pro Ala
385                 390                 395                 400

acc cga cag cga gat ggg cat ttt aag aaa cgc tct cgg cca gat ctc 1667
Thr Arg Gln Arg Asp Gly His Phe Lys Lys Arg Ser Arg Pro Asp Leu
                405                 410                 415

cga acc aga gcc aga agg aat ctg tac aaa aaa cag gag tca gaa caa 1715
Arg Thr Arg Ala Arg Arg Asn Leu Tyr Lys Lys Gln Glu Ser Glu Gln
            420                 425                 430

gca ggg gtt gct aag gat gca aaa tct gtg gcc tca gat gtt ccc ctc 1763
Ala Gly Val Ala Lys Asp Ala Lys Ser Val Ala Ser Asp Val Pro Leu
        435                 440                 445

tac aag gat ggg gag gct aag act gac cca gca ggg ctg agc agt ccc 1811
Tyr Lys Asp Gly Glu Ala Lys Thr Asp Pro Ala Gly Leu Ser Ser Pro
    450                 455                 460

cat ctg cca ggc aca tcc tct gca gca ccc gac ctg gag ggt ccc gaa 1859
His Leu Pro Gly Thr Ser Ser Ala Ala Pro Asp Leu Glu Gly Pro Glu
465                 470                 475                 480

ttc cca gtt gag tct gtg gct tct cgg atc cag gct gag cca gac aac 1907
Phe Pro Val Glu Ser Val Ala Ser Arg Ile Gln Ala Glu Pro Asp Asn
                485                 490                 495

ttg gca cgt gcc tct gca tct cca gac aga att cct agc ctg cct cag 1955
Leu Ala Arg Ala Ser Ala Ser Pro Asp Arg Ile Pro Ser Leu Pro Gln
            500                 505                 510

gaa act gtg gat cag gaa ccc aag gat cag aag agg aaa tcc ttt gag 2003
Glu Thr Val Asp Gln Glu Pro Lys Asp Gln Lys Arg Lys Ser Phe Glu
        515                 520                 525

cag gcg gcc tct gca tcc ttt ccc gaa aag aag ccc cgg ctt gaa gat 2051
Gln Ala Ala Ser Ala Ser Phe Pro Glu Lys Lys Pro Arg Leu Glu Asp
    530                 535                 540

cgt cag tcc ttt cgt aac aca att gaa agt gtt cac acc gaa aag cca 2099
Arg Gln Ser Phe Arg Asn Thr Ile Glu Ser Val His Thr Glu Lys Pro
545                 550                 555                 560

cag ccc act aaa gag gag ccc aaa gtc ccg ccc atc cgg att caa ctt 2147
Gln Pro Thr Lys Glu Glu Pro Lys Val Pro Pro Ile Arg Ile Gln Leu
                565                 570                 575

tca cgt atc aaa cca ccc tgg gtg gtt aaa ggt cag ccc act tac cag 2195
Ser Arg Ile Lys Pro Pro Trp Val Val Lys Gly Gln Pro Thr Tyr Gln
            580                 585                 590

ata tgc ccc cgg atc atc ccc acc acg gag tcc tcc tgc cgg ggt tgg 2243
Ile Cys Pro Arg Ile Ile Pro Thr Thr Glu Ser Ser Cys Arg Gly Trp
        595                 600                 605

act ggc gcc agg acc ctc gca gac att aaa gcc cgt gct ctg cag gtc 2291
Thr Gly Ala Arg Thr Leu Ala Asp Ile Lys Ala Arg Ala Leu Gln Val
    610                 615                 620

cga ggg gcg aga ggt cac cac tgc cat aga gag gcg gcc acc act gcc 2339
Arg Gly Ala Arg Gly His His Cys His Arg Glu Ala Ala Thr Thr Ala
625                 630                 635                 640

atc gga ggg ggg ggt ggc ccg ggt gga ggt ggc ggc ggg gcc acc gat 2387
Ile Gly Gly Gly Gly Gly Pro Gly Gly Gly Gly Gly Gly Ala Thr Asp
                645                 650                 655

gag gga ggt ggc aga ggc agc agc agt ggt gat ggt ggt gag gcc tgt 2435
Glu Gly Gly Gly Arg Gly Ser Ser Ser Gly Asp Gly Gly Glu Ala Cys
            660                 665                 670

ggc cac cct gag ccc agg gga ggc ccg agc acc cct gga aag tgt acg 2483
Gly His Pro Glu Pro Arg Gly Gly Pro Ser Thr Pro Gly Lys Cys Thr
        675                 680                 685

tca gat cta cag cga aca caa cta ctg ccg cct tat cct cta aat ggg 2531
Ser Asp Leu Gln Arg Thr Gln Leu Leu Pro Pro Tyr Pro Leu Asn Gly
    690                 695                 700

gag cat acc cag gcc gga act gcc atg tcc aga gct agg aga gag gac 2579
Glu His Thr Gln Ala Gly Thr Ala Met Ser Arg Ala Arg Arg Glu Asp
705                 710                 715                 720

ctg cct tct ctg aga aag gag gaa agc tgc cta cta cag agg gct aca 2627
Leu Pro Ser Leu Arg Lys Glu Glu Ser Cys Leu Leu Gln Arg Ala Thr
                725                 730                 735

gtt gga ctc aca gat ggg cta gga gat gcc tcc caa ctc ccc gtt gct 2675
Val Gly Leu Thr Asp Gly Leu Gly Asp Ala Ser Gln Leu Pro Val Ala
            740                 745                 750

ccc act ggg gac cag cca tgc cag gcc ttg ccc cta ctg tcc tcc caa 2723

Pro Thr Gly Asp Gln Pro Cys Gln Ala Leu Pro Leu Leu Ser Ser Gln
        755                 760                 765

acc tca gta gct gag aga tta gtg gag cag cct cag ttg cat ccg gat 2771
Thr Ser Val Ala Glu Arg Leu Val Glu Gln Pro Gln Leu His Pro Asp
    770                 775                 780

gtt aga act gaa tgt gag tct ggc acc act tcc tgg gaa agt gat gat 2819
Val Arg Thr Glu Cys Glu Ser Gly Thr Thr Ser Trp Glu Ser Asp Asp
785                 790                 795                 800

gag gag caa gga ccc acc gtt cct gca gac aat ggt ccc att ccg tct 2867
Glu Glu Gln Gly Pro Thr Val Pro Ala Asp Asn Gly Pro Ile Pro Ser
                805                 810                 815

cta gtg gga gat gat aca tta gag aaa gga act ggc caa gct ctt gac 2915
Leu Val Gly Asp Asp Thr Leu Glu Lys Gly Thr Gly Gln Ala Leu Asp
            820                 825                 830

agt cat ccc act atg aag gat cct gta aat gtg acc ccc agt tcc aca 2963
Ser His Pro Thr Met Lys Asp Pro Val Asn Val Thr Pro Ser Ser Thr
        835                 840                 845

cct gaa tcc tca ccg act gat tgc ctg cag aac aga gca ttt gat gac 3011
Pro Glu Ser Ser Pro Thr Asp Cys Leu Gln Asn Arg Ala Phe Asp Asp
    850                 855                 860

gaa tta ggg ctt ggt ggc tca tgc cct cct atg agg gaa agt gat act 3059
Glu Leu Gly Leu Gly Gly Ser Cys Pro Pro Met Arg Glu Ser Asp Thr
865                 870                 875                 880

aga caa gaa aac ttg aaa acc aag gct ctc gtt tct aac agt tct ttg 3107
Arg Gln Glu Asn Leu Lys Thr Lys Ala Leu Val Ser Asn Ser Ser Leu
                885                 890                 895

cat tgg ata ccc atc cca tcg aat gat gag gta gtg aaa cag ccc aaa 3155
His Trp Ile Pro Ile Pro Ser Asn Asp Glu Val Val Lys Gln Pro Lys
            900                 905                 910

cca gaa tcc aga gaa cac ata cca tct gtt gag ccc cag gtt gga gag 3203
Pro Glu Ser Arg Glu His Ile Pro Ser Val Glu Pro Gln Val Gly Glu
        915                 920                 925

gag tgg gag aaa gct gct ccc acc cct cct gca ttg cct ggg gat ttg 3251
Glu Trp Glu Lys Ala Ala Pro Thr Pro Pro Ala Leu Pro Gly Asp Leu
    930                 935                 940

aca gct gag gag ggt cta gat cct ctt gac agc ctt act tca ctc tgg 3299
Thr Ala Glu Glu Gly Leu Asp Pro Leu Asp Ser Leu Thr Ser Leu Trp
945                 950                 955                 960

act gtg cca tct cga gga ggc agt gac agc aat ggc agt tac tgt caa 3347
Thr Val Pro Ser Arg Gly Gly Ser Asp Ser Asn Gly Ser Tyr Cys Gln
                965                 970                 975

cag gtg gac att gaa aag ctg aaa atc aac gga gac tct gaa gca ctg 3395
Gln Val Asp Ile Glu Lys Leu Lys Ile Asn Gly Asp Ser Glu Ala Leu
            980                 985                 990

agt cct cac ggt gag tcc acg gat aca gcc tct gac ttt gaa ggt cac 3443
Ser Pro His Gly Glu Ser Thr Asp Thr Ala Ser Asp Phe Glu Gly His
        995                 1000                1005

ctc acg gag gac agc agt gag gct gac act aga gaa gct gca gtg 3488
Leu Thr Glu Asp Ser Ser Glu Ala Asp Thr Arg Glu Ala Ala Val
    1010                1015                1020

aca aag gga tct tcg gtg gac aag gat gag aaa ccc aat tgg aac 3533
Thr Lys Gly Ser Ser Val Asp Lys Asp Glu Lys Pro Asn Trp Asn
    1025                1030                1035

caa tct gcc cca ctg tcc aag gtg aat ggt gac atg cgt ctg gtt 3578
Gln Ser Ala Pro Leu Ser Lys Val Asn Gly Asp Met Arg Leu Val
    1040                1045                1050

aca agg aca gat ggg atg gtt gct cct cag agc tgg gtg tct cga 3623
Thr Arg Thr Asp Gly Met Val Ala Pro Gln Ser Trp Val Ser Arg
    1055                1060                1065

gta tgt gcg gtc cgc caa aag atc cca gat tcc cta ctg ctg gcc 3668
Val Cys Ala Val Arg Gln Lys Ile Pro Asp Ser Leu Leu Leu Ala
    1070                1075                1080

agt act gag tac cag cca aga gcc gtg tgc ctg tcc atg cct ggg 3713
Ser Thr Glu Tyr Gln Pro Arg Ala Val Cys Leu Ser Met Pro Gly
    1085                1090                1095

tcc tca gtg gag gcc act aac cca ctt gtg atg cag ttg ctg cag 3758
Ser Ser Val Glu Ala Thr Asn Pro Leu Val Met Gln Leu Leu Gln
    1100                1105                1110

ggt agc ttg ccc cta gag aag gtt ctt cca cca gcc cac gat gac 3803
Gly Ser Leu Pro Leu Glu Lys Val Leu Pro Pro Ala His Asp Asp
    1115                1120                1125

agc atg tca gaa tcc cca caa gta cca ctt aca aaa gac cag agc 3848
Ser Met Ser Glu Ser Pro Gln Val Pro Leu Thr Lys Asp Gln Ser
    1130                1135                1140

cat ggc tcg cta cgc atg gga tct tta cat ggt ctt gga aaa aac 3893

His Gly Ser Leu Arg Met Gly Ser Leu His Gly Leu Gly Lys Asn
    1145                1150                1155

agt ggc atg gtt gat gga agc agc ccc agt tct tta agg gct ttg 3938
Ser Gly Met Val Asp Gly Ser Ser Pro Ser Ser Leu Arg Ala Leu
    1160                1165                1170

aag gag cct ctt ctg cca gat agc tgt gaa aca ggc act ggt ctt 3983
Lys Glu Pro Leu Leu Pro Asp Ser Cys Glu Thr Gly Thr Gly Leu
    1175                1180                1185

gcc agg att gag gcc acc cag gct cct gga gca ccc caa aag aat 4028
Ala Arg Ile Glu Ala Thr Gln Ala Pro Gly Ala Pro Gln Lys Asn
    1190                1195                1200

tgc aag gca gtc cca agt ttt gac tcc ctc cat cca gtg aca aat 4073
Cys Lys Ala Val Pro Ser Phe Asp Ser Leu His Pro Val Thr Asn
    1205                1210                1215

ccc att aca tcc tct agg aaa ctg gaa gaa atg gat tcc aaa gag 4118
Pro Ile Thr Ser Ser Arg Lys Leu Glu Glu Met Asp Ser Lys Glu
    1220                1225                1230

cag ttc tct tcc ttt agt tgt gaa gat cag aag gaa gtc cgt gct 4163
Gln Phe Ser Ser Phe Ser Cys Glu Asp Gln Lys Glu Val Arg Ala
    1235                1240                1245

atg tca cag gac agt aat tca aat gct gct cca gga aag agc cca 4208
Met Ser Gln Asp Ser Asn Ser Asn Ala Ala Pro Gly Lys Ser Pro
    1250                1255                1260

gga gat ctt act acc tcg aga aca cct cgt ttc tca tct cca aat 4253
Gly Asp Leu Thr Thr Ser Arg Thr Pro Arg Phe Ser Ser Pro Asn
    1265                1270                1275

gtg atc tcc ttt ggt cca gag cag aca ggt cgg gcc ctg ggt gat 4298
Val Ile Ser Phe Gly Pro Glu Gln Thr Gly Arg Ala Leu Gly Asp
    1280                1285                1290

cag agc aat gtt aca ggc caa ggg aag aag ctt ttt ggc tct ggg 4343
Gln Ser Asn Val Thr Gly Gln Gly Lys Lys Leu Phe Gly Ser Gly
    1295                1300                1305

aat gtg gct gca acc ctt cag cgc ccc agg cct gcg gac ccg atg 4388
Asn Val Ala Ala Thr Leu Gln Arg Pro Arg Pro Ala Asp Pro Met
    1310                1315                1320

cct ctt cct gct gag atc cct cca gtt ttt ccc agt ggg aag ttg 4433
Pro Leu Pro Ala Glu Ile Pro Pro Val Phe Pro Ser Gly Lys Leu
    1325                1330                1335

gga cca agc aca aac tcc atg tct ggt ggg gta cag act cca agg 4478
Gly Pro Ser Thr Asn Ser Met Ser Gly Gly Val Gln Thr Pro Arg
    1340                1345                1350

gaa gac tgg gct cca aag cca cat gcc ttt gtt ggc agc gtc aag 4523
Glu Asp Trp Ala Pro Lys Pro His Ala Phe Val Gly Ser Val Lys
    1355                1360                1365

aat gag aag act ttt gtg ggg ggt cct ctt aag gca aat gcc gag 4568
Asn Glu Lys Thr Phe Val Gly Gly Pro Leu Lys Ala Asn Ala Glu
    1370                1375                1380

aac agg aaa gct act ggg cat agt ccc ctg gaa ctg gtg ggt cac 4613
Asn Arg Lys Ala Thr Gly His Ser Pro Leu Glu Leu Val Gly His
    1385                1390                1395

ttg gaa ggg atg ccc ttt gtc atg gac ttg ccc ttc tgg aaa tta 4658
Leu Glu Gly Met Pro Phe Val Met Asp Leu Pro Phe Trp Lys Leu
    1400                1405                1410

ccc cga gag cca ggg aag ggg ctc agt gag cct ctg gag cct tct 4703
Pro Arg Glu Pro Gly Lys Gly Leu Ser Glu Pro Leu Glu Pro Ser
    1415                1420                1425

tct ctc ccc tcc caa ctc agc atc aag cag gca ttt tat ggg aag 4748
Ser Leu Pro Ser Gln Leu Ser Ile Lys Gln Ala Phe Tyr Gly Lys
    1430                1435                1440

ctt tct aaa ctc caa ctg agt tcc acc agc ttt aat tat tcc tct 4793
Leu Ser Lys Leu Gln Leu Ser Ser Thr Ser Phe Asn Tyr Ser Ser
    1445                1450                1455

agc tct ccc acc ttt ccc aaa ggc ctt gct gga agt gtg gtg cag 4838
Ser Ser Pro Thr Phe Pro Lys Gly Leu Ala Gly Ser Val Val Gln
    1460                1465                1470

ctg agc cac aaa gca aac ttt ggt gcg agc cac agt gca tca ctt 4883
Leu Ser His Lys Ala Asn Phe Gly Ala Ser His Ser Ala Ser Leu
    1475                1480                1485

tcc ttg caa atg ttc act gac agc agc acg gtg gaa agc atc tcg 4928
Ser Leu Gln Met Phe Thr Asp Ser Ser Thr Val Glu Ser Ile Ser
    1490                1495                1500

ctc cag tgt gcg tgc agc ctg aaa gcc atg atc atg tgc caa ggc 4973
Leu Gln Cys Ala Cys Ser Leu Lys Ala Met Ile Met Cys Gln Gly
    1505                1510                1515

tgc ggt gcg ttc tgt cac gat gac tgt att gga ccc tca aag ctc 5018

Cys Gly Ala Phe Cys His Asp Asp Cys Ile Gly Pro Ser Lys Leu
    1520                1525                1530

tgt gta ttg tgc ctt gtg gtg aga taa taaattatgg ccatgggaaa 5065
Cys Val Leu Cys Leu Val Val Arg
    1535                1540

cattgtatat ttagtgtgtg tattttgata atgattgatc ttaaatctgt atacagaata 5125

tcattgatat aatactcttt aggcaggagc actcttgcct tcccccaaaa tttacactgc 5185

taaagccctc tgtcacttgg cgacccttct ggtcttgctg gaggggtttc ctgggtataa 5245

cccattgggc tgcccaaggc cagccagcct gagctctcct gcaagacaga gcctgatgtg 5305

gcacggagtg gggttgcggg gggtgggggg actgcctgac tcccagaggg acttgaaact 5365

gaagcaagaa ggttgcattc tccaccaagg gagttaacct acctgaacta agtagaaatg 5425

ccagtcttcc actaccccct ccctgccatc ttttcttctg ctactttggg gagttgatgg 5485

ccaggaaaga agccagcaca gggttaaagt aactcctggc attgcccacc agggggctgg 5545

tgcacctgct gacctcaggg tcacagttga gtcatttgcc agttgacgga gcaagtttga 5605

ccttggttct gttgctgaag caaatttgga acttttctgt ctcagtgtga tccactaacc 5665

cacaggatca tttggaacct tgaatagctc tgcttggaca atggggttgg ggaatagggt 5725

tgtctttcct atgaaaatgc catctgtaga ccttgtgagt cagccgtcca gatgtttgca 5785

ggtgaattcc tctgcttgac atcctccctg tcactttgga ccctatggga gtgggcatct 5845

ccacgcacct gtgtatgtga aagtcatttt acatttcaaa gcagtgtgtg tttcttattt 5905

ttatattttt aactctttat tcttggatgt ataaagtgaa ctttttggct tctgtaagta 5965

tgctctatgc acctctaatg ttttatcatg tatttatatg ttgtacacag tactggctga 6025

ttctgtaaat ggatgtattg tacagagaac atgaacgtct cttcctaatt ttacatcttc 6085

agcatcattg cattaaagtg gtgtaatctc cttctctaca tctgttgtca gagccactga 6145

gtgctgtgct gctcgacgtg agggtgaaat gattgacttg tgacctgcca ggttgcccga 6205

tgccctgttg ggtcaccggc tggacctgct gcagcctgca gagccacagt cagcctgccc 6265

acatgccacc gagcaaacgc atcttgcttt tcacatctct cctcctacag ccttaatggc 6325

tgcttgctgc catatgtgac aaatcaccac caccagtgtt aagtgcttct ggattcatgg 6385

gtgagttccc tgggcagccc ccaggaaggc cttccagatc tggctccagg gtcaccacct 6445

gtcacagcaa tacctgggac catgctctcc tgggactgtg aggctccttt tgacgtactt 6505

ttgacatcag gcaggtttgg gaagaaacaa agccatgcct gctcctgcct ctctcccaac 6565

atgtttccag caagtagatg cccctgtgtg tgttttccct tgccttgttt cctgccttat 6625

atcttgtatt tcgacttatt acagagttga gggttcttgc ttaatttaga tcaagtataa 6685

aatttgtatg acttcaagtc tcattttatc tgaaaggttt ttttctcatt taatctgatg 6745

tggcattttc gtcatctgaa gcatgagtga caagttggga atgatgtggt gatttagaat 6805

gcagtattgg ccaagtccaa gttgtcaact taagcgtctg tttaccaaag accgggaaca 6865

ggggcccaaa catgtccagt cctcttcttc cctctgctgg aacctttggg gacactcaag 6925

ggtacagttt gacactgatc tggtccatga ggctgcccag agaaagcact gcttctgtat 6985

gtctcttgtg gtattggaac aataaacccg tacaacctgc a 7026

<210> 48
<211> 1541
<212> PRT
<213> Homo sapiens

<400> 48

Met Lys Asp Lys Gln Lys Lys Lys Lys Glu Arg Thr Trp Ala Glu Ala
1               5                   10                  15

Ala Arg Leu Val Leu Glu Asn Tyr Ser Asp Ala Pro Met Thr Pro Lys
            20                  25                  30

Gln Ile Leu Gln Val Ile Glu Ala Glu Gly Leu Lys Glu Met Arg Ser
        35                  40                  45

Gly Thr Ser Pro Leu Ala Cys Leu Asn Ala Met Leu His Ser Asn Ser
    50                  55                  60

Arg Gly Gly Glu Gly Leu Phe Tyr Lys Leu Pro Gly Arg Ile Ser Leu
65                  70                  75                  80

Phe Thr Leu Lys Lys Asp Ala Leu Gln Trp Ser Arg His Pro Ala Thr
                85                  90                  95

Val Glu Gly Glu Glu Pro Glu Asp Thr Ala Asp Val Glu Ser Cys Gly
            100                 105                 110

Ser Asn Glu Ala Ser Thr Val Ser Gly Glu Asn Asp Val Ser Leu Asp
        115                 120                 125

Glu Thr Ser Ser Asn Ala Ser Cys Ser Thr Glu Ser Gln Ser Arg Pro
    130                 135                 140

Leu Ser Asn Pro Arg Asp Ser Tyr Arg Ala Ser Ser Gln Ala Asn Lys
145                 150                 155                 160

Gln Lys Lys Lys Thr Gly Val Met Leu Pro Arg Val Val Leu Thr Pro
                165                 170                 175

Leu Lys Val Asn Gly Ala His Val Glu Ser Ala Ser Gly Phe Ser Gly
            180                 185                 190

Cys His Ala Asp Gly Glu Ser Gly Ser Pro Ser Ser Ser Ser Ser Gly
        195                 200                 205

Ser Leu Ala Leu Gly Ser Ala Ala Ile Arg Gly Gln Ala Glu Val Thr
    210                 215                 220

Gln Asp Pro Ala Pro Leu Leu Arg Gly Phe Arg Lys Pro Ala Thr Gly
225                 230                 235                 240

Gln Met Lys Arg Asn Arg Gly Glu Glu Ile Asp Phe Glu Thr Pro Gly
                245                 250                 255

Ser Ile Leu Val Asn Thr Asn Leu Arg Ala Leu Ile Asn Ser Arg Thr
            260                 265                 270

Phe His Ala Leu Pro Ser His Phe Gln Gln Gln Leu Leu Phe Leu Leu

        275                 280                 285

Pro Glu Val Asp Arg Gln Val Gly Thr Asp Gly Leu Leu Arg Leu Ser
    290                 295                 300

Ser Ser Ala Leu Asn Asn Glu Phe Phe Thr His Ala Ala Gln Ser Trp
305                 310                 315                 320

Arg Glu Arg Leu Ala Asp Gly Glu Phe Thr His Glu Met Gln Val Arg
                325                 330                 335

Ile Arg Gln Glu Met Glu Lys Glu Lys Lys Val Glu Gln Trp Lys Glu
            340                 345                 350

Lys Phe Phe Glu Asp Tyr Tyr Gly Gln Lys Leu Gly Leu Thr Lys Glu
        355                 360                 365

Glu Ser Leu Gln Gln Asn Val Gly Gln Glu Glu Ala Glu Ile Lys Ser
    370                 375                 380

Gly Leu Cys Val Pro Gly Glu Ser Val Arg Ile Gln Arg Gly Pro Ala
385                 390                 395                 400

Thr Arg Gln Arg Asp Gly His Phe Lys Lys Arg Ser Arg Pro Asp Leu
                405                 410                 415

Arg Thr Arg Ala Arg Arg Asn Leu Tyr Lys Lys Gln Glu Ser Glu Gln
            420                 425                 430

Ala Gly Val Ala Lys Asp Ala Lys Ser Val Ala Ser Asp Val Pro Leu
        435                 440                 445

Tyr Lys Asp Gly Glu Ala Lys Thr Asp Pro Ala Gly Leu Ser Ser Pro
    450                 455                 460

His Leu Pro Gly Thr Ser Ser Ala Ala Pro Asp Leu Glu Gly Pro Glu
465                 470                 475                 480

Phe Pro Val Glu Ser Val Ala Ser Arg Ile Gln Ala Glu Pro Asp Asn
                485                 490                 495

Leu Ala Arg Ala Ser Ala Ser Pro Asp Arg Ile Pro Ser Leu Pro Gln
            500                 505                 510

Glu Thr Val Asp Gln Glu Pro Lys Asp Gln Lys Arg Lys Ser Phe Glu
        515                 520                 525

Gln Ala Ala Ser Ala Ser Phe Pro Glu Lys Lys Pro Arg Leu Glu Asp
    530                 535                 540

Arg Gln Ser Phe Arg Asn Thr Ile Glu Ser Val His Thr Glu Lys Pro
545                 550                 555                 560

Gln Pro Thr Lys Glu Glu Pro Lys Val Pro Pro Ile Arg Ile Gln Leu
                565                 570                 575

Ser Arg Ile Lys Pro Pro Trp Val Val Lys Gly Gln Pro Thr Tyr Gln
            580                 585                 590

Ile Cys Pro Arg Ile Ile Pro Thr Thr Glu Ser Ser Cys Arg Gly Trp
        595                 600                 605

Thr Gly Ala Arg Thr Leu Ala Asp Ile Lys Ala Arg Ala Leu Gln Val
    610                 615                 620

Arg Gly Ala Arg Gly His His Cys His Arg Glu Ala Ala Thr Thr Ala
625                 630                 635                 640

Ile Gly Gly Gly Gly Gly Pro Gly Gly Gly Gly Gly Gly Ala Thr Asp
                645                 650                 655

Glu Gly Gly Gly Arg Gly Ser Ser Ser Gly Asp Gly Gly Glu Ala Cys
            660                 665                 670

Gly His Pro Glu Pro Arg Gly Gly Pro Ser Thr Pro Gly Lys Cys Thr

        675                 680                 685

Ser Asp Leu Gln Arg Thr Gln Leu Leu Pro Pro Tyr Pro Leu Asn Gly
    690                 695                 700

Glu His Thr Gln Ala Gly Thr Ala Met Ser Arg Ala Arg Arg Glu Asp
705                 710                 715                 720

Leu Pro Ser Leu Arg Lys Glu Glu Ser Cys Leu Leu Gln Arg Ala Thr
                725                 730                 735

Val Gly Leu Thr Asp Gly Leu Gly Asp Ala Ser Gln Leu Pro Val Ala
            740                 745                 750

Pro Thr Gly Asp Gln Pro Cys Gln Ala Leu Pro Leu Leu Ser Ser Gln
        755                 760                 765

Thr Ser Val Ala Glu Arg Leu Val Glu Gln Pro Gln Leu His Pro Asp
    770                 775                 780

Val Arg Thr Glu Cys Glu Ser Gly Thr Thr Ser Trp Glu Ser Asp Asp
785                 790                 795                 800

Glu Glu Gln Gly Pro Thr Val Pro Ala Asp Asn Gly Pro Ile Pro Ser
                805                 810                 815

Leu Val Gly Asp Asp Thr Leu Glu Lys Gly Thr Gly Gln Ala Leu Asp
            820                 825                 830

Ser His Pro Thr Met Lys Asp Pro Val Asn Val Thr Pro Ser Ser Thr
        835                 840                 845

Pro Glu Ser Ser Pro Thr Asp Cys Leu Gln Asn Arg Ala Phe Asp Asp
    850                 855                 860

Glu Leu Gly Leu Gly Gly Ser Cys Pro Pro Met Arg Glu Ser Asp Thr
865                 870                 875                 880

Arg Gln Glu Asn Leu Lys Thr Lys Ala Leu Val Ser Asn Ser Ser Leu
                885                 890                 895

His Trp Ile Pro Ile Pro Ser Asn Asp Glu Val Val Lys Gln Pro Lys
            900                 905                 910

Pro Glu Ser Arg Glu His Ile Pro Ser Val Glu Pro Gln Val Gly Glu
        915                 920                 925

Glu Trp Glu Lys Ala Ala Pro Thr Pro Pro Ala Leu Pro Gly Asp Leu
    930                 935                 940

Thr Ala Glu Glu Gly Leu Asp Pro Leu Asp Ser Leu Thr Ser Leu Trp
945                 950                 955                 960

Thr Val Pro Ser Arg Gly Gly Ser Asp Ser Asn Gly Ser Tyr Cys Gln
                965                 970                 975

Gln Val Asp Ile Glu Lys Leu Lys Ile Asn Gly Asp Ser Glu Ala Leu
            980                 985                 990

Ser Pro His Gly Glu Ser Thr Asp  Thr Ala Ser Asp Phe  Glu Gly His
        995                 1000                1005

Leu Thr  Glu Asp Ser Ser Glu  Ala Asp Thr Arg Glu  Ala Ala Val
    1010                1015                1020

Thr Lys  Gly Ser Ser Val Asp  Lys Asp Glu Lys Pro  Asn Trp Asn
    1025                1030                1035

Gln Ser  Ala Pro Leu Ser Lys  Val Asn Gly Asp Met  Arg Leu Val
    1040                1045                1050

Thr Arg  Thr Asp Gly Met Val  Ala Pro Gln Ser Trp  Val Ser Arg
    1055                1060                1065

Val Cys  Ala Val Arg Gln Lys  Ile Pro Asp Ser Leu  Leu Leu Ala

1070                    1075                    1080

Ser Thr Glu Tyr Gln Pro Arg Ala Val Cys Leu Ser Met Pro Gly
    1085                    1090                    1095

Ser Ser Val Glu Ala Thr Asn Pro Leu Val Met Gln Leu Leu Gln
    1100                    1105                    1110

Gly Ser Leu Pro Leu Glu Lys Val Leu Pro Pro Ala His Asp Asp
    1115                    1120                    1125

Ser Met Ser Glu Ser Pro Gln Val Pro Leu Thr Lys Asp Gln Ser
    1130                    1135                    1140

His Gly Ser Leu Arg Met Gly Ser Leu His Gly Leu Gly Lys Asn
    1145                    1150                    1155

Ser Gly Met Val Asp Gly Ser Ser Pro Ser Ser Leu Arg Ala Leu
    1160                    1165                    1170

Lys Glu Pro Leu Leu Pro Asp Ser Cys Glu Thr Gly Thr Gly Leu
    1175                    1180                    1185

Ala Arg Ile Glu Ala Thr Gln Ala Pro Gly Ala Pro Gln Lys Asn
    1190                    1195                    1200

Cys Lys Ala Val Pro Ser Phe Asp Ser Leu His Pro Val Thr Asn
    1205                    1210                    1215

Pro Ile Thr Ser Ser Arg Lys Leu Glu Glu Met Asp Ser Lys Glu
    1220                    1225                    1230

Gln Phe Ser Ser Phe Ser Cys Glu Asp Gln Lys Glu Val Arg Ala
    1235                    1240                    1245

Met Ser Gln Asp Ser Asn Ser Asn Ala Ala Pro Gly Lys Ser Pro
    1250                    1255                    1260

Gly Asp Leu Thr Thr Ser Arg Thr Pro Arg Phe Ser Ser Pro Asn
    1265                1270                1275

Val Ile Ser Phe Gly Pro Glu Gln Thr Gly Arg Ala Leu Gly Asp
    1280                1285                1290

Gln Ser Asn Val Thr Gly Gln Gly Lys Lys Leu Phe Gly Ser Gly
    1295                1300                1305

Asn Val Ala Ala Thr Leu Gln Arg Pro Arg Pro Ala Asp Pro Met
    1310                1315                1320

Pro Leu Pro Ala Glu Ile Pro Pro Val Phe Pro Ser Gly Lys Leu
    1325                1330                1335

Gly Pro Ser Thr Asn Ser Met Ser Gly Gly Val Gln Thr Pro Arg
    1340                1345                1350

Glu Asp Trp Ala Pro Lys Pro His Ala Phe Val Gly Ser Val Lys
    1355                1360                1365

Asn Glu Lys Thr Phe Val Gly Gly Pro Leu Lys Ala Asn Ala Glu
    1370                1375                1380

Asn Arg Lys Ala Thr Gly His Ser Pro Leu Glu Leu Val Gly His
    1385                1390                1395

Leu Glu Gly Met Pro Phe Val Met Asp Leu Pro Phe Trp Lys Leu
    1400                1405                1410

Pro Arg Glu Pro Gly Lys Gly Leu Ser Glu Pro Leu Glu Pro Ser
    1415                1420                1425

Ser Leu Pro Ser Gln Leu Ser Ile Lys Gln Ala Phe Tyr Gly Lys
    1430                1435                1440

Leu Ser Lys Leu Gln Leu Ser Ser Thr Ser Phe Asn Tyr Ser Ser

1445                1450                1455

Ser Ser Pro Thr Phe Pro Lys Gly Leu Ala Gly Ser Val Val Gln
    1460                1465                1470

Leu Ser His Lys Ala Asn Phe Gly Ala Ser His Ser Ala Ser Leu
    1475                1480                1485

Ser Leu Gln Met Phe Thr Asp Ser Ser Thr Val Glu Ser Ile Ser
    1490                1495                1500

Leu Gln Cys Ala Cys Ser Leu Lys Ala Met Ile Met Cys Gln Gly
    1505                1510                1515

Cys Gly Ala Phe Cys His Asp Asp Cys Ile Gly Pro Ser Lys Leu
    1520                1525                1530

Cys Val Leu Cys Leu Val Val Arg
    1535                1540

<210>  49
<211>  3124
<212>  DNA
<213>  Homo sapiens

<220>
<221>  CDS
<222>  (313)..(1731)
<223>

<400>  49
taagatccac atcagctcaa ctgcacttgc ctcgcagagg cagcccgctc acttcccgcg      60

gaggcgctcc ccggcgccgc gctccgcggc agccgcctgc ccccggcgct gcccccgccc      120

gccgcgccgc cgccgccgcc gcgcacgccg cgccccgcag ctctgggctt cctcttcgcc      180

cgggtggcgt tgggcccgcg cgggcgctcg ggtgactgca gctgctcagc tcccctcccc      240

cgccccgcgc cgcgcggccg cccgtcgctt cgcacagggc tggatggttg tattgggcag      300

ggtggctcca gg atg tta gga act gtg aag atg gaa ggg cat gaa acc agc      351
              Met Leu Gly Thr Val Lys Met Glu Gly His Glu Thr Ser

1                    5                    10

gac tgg aac agc tac tac gca gac acg cag gag gcc tac tcc tcc gtc          399
Asp Trp Asn Ser Tyr Tyr Ala Asp Thr Gln Glu Ala Tyr Ser Ser Val
    15                  20                  25

ccg gtc agc aac atg aac tca ggc ctg ggc tcc atg aac tcc atg aac          447
Pro Val Ser Asn Met Asn Ser Gly Leu Gly Ser Met Asn Ser Met Asn
30                  35                  40                  45

acc tac atg acc atg aac acc atg act acg agc ggc aac atg acc ccg          495
Thr Tyr Met Thr Met Asn Thr Met Thr Thr Ser Gly Asn Met Thr Pro
                50                  55                  60

gcg tcc ttc aac atg tcc tat gcc aac ccg ggc cta ggg gcc ggc ctg          543
Ala Ser Phe Asn Met Ser Tyr Ala Asn Pro Gly Leu Gly Ala Gly Leu
            65                  70                  75

agt ccc ggc gca gta gcc ggc atg ccg ggg ggc tcg gcg ggc gcc atg          591
Ser Pro Gly Ala Val Ala Gly Met Pro Gly Gly Ser Ala Gly Ala Met
        80                  85                  90

aac agc atg act gcg gcc ggc gtg acg gcc atg ggt acg gcg ctg agc          639
Asn Ser Met Thr Ala Ala Gly Val Thr Ala Met Gly Thr Ala Leu Ser
    95                  100                 105

ccg agc ggc atg ggc gcc atg ggt gcg cag cag gcg gcc tcc atg aat          687
Pro Ser Gly Met Gly Ala Met Gly Ala Gln Gln Ala Ala Ser Met Asn
110                 115                 120                 125

ggc ctg ggc ccc tac gcg gcc gcc atg aac ccg tgc atg agc ccc atg          735
Gly Leu Gly Pro Tyr Ala Ala Ala Met Asn Pro Cys Met Ser Pro Met
                130                 135                 140

gcg tac gcg ccg tcc aac ctg ggc cgc agc cgc gcg ggc ggc ggc ggc          783
Ala Tyr Ala Pro Ser Asn Leu Gly Arg Ser Arg Ala Gly Gly Gly Gly
            145                 150                 155

gac gcc aag acg ttc aag cgc agc tac ccg cac gcc aag ccg ccc tac          831
Asp Ala Lys Thr Phe Lys Arg Ser Tyr Pro His Ala Lys Pro Pro Tyr
        160                 165                 170

tcg tac atc tcg ctc atc acc atg gcc atc cag cag gcg ccc agc aag          879
Ser Tyr Ile Ser Leu Ile Thr Met Ala Ile Gln Gln Ala Pro Ser Lys
    175                 180                 185

atg ctc acg ctg agc gag atc tac cag tgg atc atg gac ctc ttc ccc          927
Met Leu Thr Leu Ser Glu Ile Tyr Gln Trp Ile Met Asp Leu Phe Pro
190                 195                 200                 205

tat tac cgg cag aac cag cag cgc tgg cag aac tcc atc cgc cac tcg     975
Tyr Tyr Arg Gln Asn Gln Gln Arg Trp Gln Asn Ser Ile Arg His Ser
                210                 215                 220

ctg tcc ttc aat gac tgc ttc gtc aag gtg gca cgc tcc ccg gac aag     1023
Leu Ser Phe Asn Asp Cys Phe Val Lys Val Ala Arg Ser Pro Asp Lys
            225                 230                 235

ccg ggc aag ggc tcc tac tgg acg ctg cac ccg gac tcc ggc aac atg     1071
Pro Gly Lys Gly Ser Tyr Trp Thr Leu His Pro Asp Ser Gly Asn Met
        240                 245                 250

ttc gag aac ggc tgc tac ttg cgc cgc cag aag cgc ttc aag tgc gag     1119
Phe Glu Asn Gly Cys Tyr Leu Arg Arg Gln Lys Arg Phe Lys Cys Glu
    255                 260                 265

aag cag ccg ggg gcc ggc ggc ggg ggc ggg agc gga agc ggg ggc agc     1167
Lys Gln Pro Gly Ala Gly Gly Gly Gly Gly Ser Gly Ser Gly Gly Ser
270                 275                 280                 285

ggc gcc aag ggc ggc cct gag agc cgc aag gac ccc tct ggc gcc tct     1215
Gly Ala Lys Gly Gly Pro Glu Ser Arg Lys Asp Pro Ser Gly Ala Ser
                290                 295                 300

aac ccc agc gcc gac tcg ccc ctc cat cgg ggt gtg cac ggg aag acc     1263
Asn Pro Ser Ala Asp Ser Pro Leu His Arg Gly Val His Gly Lys Thr
            305                 310                 315

ggc cag cta gag ggc gcg ccg gcc ccc ggg ccc gcc gcc agc ccc cag     1311
Gly Gln Leu Glu Gly Ala Pro Ala Pro Gly Pro Ala Ala Ser Pro Gln
        320                 325                 330

act ctg gac cac agt ggg gcg acg gcg aca ggg ggc gcc tcg gag ttg     1359
Thr Leu Asp His Ser Gly Ala Thr Ala Thr Gly Gly Ala Ser Glu Leu
    335                 340                 345

aag act cca gcc tcc tca act gcg ccc ccc ata agc tcc ggg ccc ggg     1407
Lys Thr Pro Ala Ser Ser Thr Ala Pro Pro Ile Ser Ser Gly Pro Gly
350                 355                 360                 365

gcg ctg gcc tct gtg ccc gcc tct cac ccg gca cac ggc ttg gca ccc     1455
Ala Leu Ala Ser Val Pro Ala Ser His Pro Ala His Gly Leu Ala Pro
                370                 375                 380

cac gag tcc cag ctg cac ctg aaa ggg gac ccc cac tac tcc ttc aac     1503
His Glu Ser Gln Leu His Leu Lys Gly Asp Pro His Tyr Ser Phe Asn
            385                 390                 395

cac ccg ttc tcc atc aac aac ctc atg tcc tcc tcg gag cag cag cat     1551
His Pro Phe Ser Ile Asn Asn Leu Met Ser Ser Ser Glu Gln Gln His

400                 405                 410

aag ctg gac ttc aag gca tac gaa cag gca ctg caa tac tcg cct tac     1599
Lys Leu Asp Phe Lys Ala Tyr Glu Gln Ala Leu Gln Tyr Ser Pro Tyr
    415                 420                 425

ggc tct acg ttg ccc gcc agc ctg cct cta ggc agc gcc tcg gtg acc     1647
Gly Ser Thr Leu Pro Ala Ser Leu Pro Leu Gly Ser Ala Ser Val Thr
430                 435                 440                 445

acc agg agc ccc atc gag ccc tca gcc ctg gag ccg gcg tac tac caa     1695
Thr Arg Ser Pro Ile Glu Pro Ser Ala Leu Glu Pro Ala Tyr Tyr Gln
                450                 455                 460

ggt gtg tat tcc aga ccc gtc cta aac act tcc tag ctcccgggac          1741
Gly Val Tyr Ser Arg Pro Val Leu Asn Thr Ser
            465                 470

tggggggttt gtctggcata gccatgctgg tagcaagaga gaaaaaatca acagcaaaca   1801

aaaccacaca aaccaaaccg tcaacagcat aataaaatcc caacaactat ttttatttca    1861

tttttcatgc acaacctttc ccccagtgca aaagactgtt actttattat tgtattcaaa   1921

attcattgtg tatattacta caaagacaac cccaaaccaa tttttttcct gcgaagttta  1981

atgatccaca agtgtatata tgaaattctc ctccttcctt gcccccctct ctttcttccc   2041

tctttcccct ccagacattc tagtttgtgg agggttattt aaaaaaacaa aaaaggaaga    2101

tggtcaagtt tgtaaaatat ttgtttgtgc tttttccccc tccttacctg accccctacg   2161

agtttacagg tctgtggcaa tactcttaac cataagaatt gaaatggtga agaaacaagt   2221

atacactaga ggctcttaaa agtattgaaa gacaatactg ctgttatata gcaagacata   2281

aacagattat aaacatcaga gccatttgct tctcagttta catttctgat acatgcagat   2341

agcagatgtc tttaaatgaa atacatgtat attgtgtatg gacttaatta tgcacatgct   2401

cagatgtgta gacatcctcc gtatatttac ataacatata gaggtaatag ataggtgata   2461

tacatgatac attctcaaga gttgcttgac cgaaagttac aaggacccca acccctttgt  2521

cctctctacc cacagatggc cctgggaatc aattcctcag gaattgccct caagaactct   2581

gcttcttgct ttgcagagtg ccatggtcat gtcattctga ggtcacataa cacataaaat   2641

tagtttctat gagtgtatac catttaaaga attttttttt cagtaaaagg gaatattaca    2701

atgttggagg agagataagt tatagggagc tggatttcaa aacgtggtcc aagattcaaa    2761

aatcctattg atagtggcca ttttaatcat tgccatcgtg tgcttgtttc atccagtgtt    2821

atgcactttc cacagttgga catggtgtta gtatagccag acgggtttca ttattatttc    2881

tctttgcttt ctcaatgtta atttattgca tggtttattc tttttcttta cagctgaaat    2941

tgctttaaat gatggttaaa attacaaatt aaattgttaa tttttatcaa tgtgattgta    3001

attaaaaata ttttgattta aataacaaaa ataataccag attttaagcc gtggaaaatg    3061

ttcttgatca tttgcagtta aggactttaa ataaatcaaa tgttaacaaa aaaaaaaaaa    3121

aaa                                                                  3124

<210>  50
<211>  472
<212>  PRT
<213>  Homo sapiens

<400>  50

Met Leu Gly Thr Val Lys Met Glu Gly His Glu Thr Ser Asp Trp Asn
1               5                   10                  15

Ser Tyr Tyr Ala Asp Thr Gln Glu Ala Tyr Ser Ser Val Pro Val Ser
            20                  25                  30

Asn Met Asn Ser Gly Leu Gly Ser Met Asn Ser Met Asn Thr Tyr Met
        35                  40                  45

Thr Met Asn Thr Met Thr Thr Ser Gly Asn Met Thr Pro Ala Ser Phe
    50                  55                  60

Asn Met Ser Tyr Ala Asn Pro Gly Leu Gly Ala Gly Leu Ser Pro Gly
65                  70                  75                  80

Ala Val Ala Gly Met Pro Gly Gly Ser Ala Gly Ala Met Asn Ser Met
                85                  90                  95

Thr Ala Ala Gly Val Thr Ala Met Gly Thr Ala Leu Ser Pro Ser Gly
            100                 105                 110

Met Gly Ala Met Gly Ala Gln Gln Ala Ala Ser Met Asn Gly Leu Gly
        115                 120                 125

Pro Tyr Ala Ala Ala Met Asn Pro Cys Met Ser Pro Met Ala Tyr Ala
    130                 135                 140

Pro Ser Asn Leu Gly Arg Ser Arg Ala Gly Gly Gly Gly Asp Ala Lys
145                 150                 155                 160

Thr Phe Lys Arg Ser Tyr Pro His Ala Lys Pro Pro Tyr Ser Tyr Ile
                165                 170                 175

Ser Leu Ile Thr Met Ala Ile Gln Gln Ala Pro Ser Lys Met Leu Thr
            180                 185                 190

Leu Ser Glu Ile Tyr Gln Trp Ile Met Asp Leu Phe Pro Tyr Tyr Arg
        195                 200                 205

Gln Asn Gln Gln Arg Trp Gln Asn Ser Ile Arg His Ser Leu Ser Phe
    210                 215                 220

Asn Asp Cys Phe Val Lys Val Ala Arg Ser Pro Asp Lys Pro Gly Lys
225                 230                 235                 240

Gly Ser Tyr Trp Thr Leu His Pro Asp Ser Gly Asn Met Phe Glu Asn
                245                 250                 255

Gly Cys Tyr Leu Arg Arg Gln Lys Arg Phe Lys Cys Glu Lys Gln Pro
            260                 265                 270

Gly Ala Gly Gly Gly Gly Gly Ser Gly Ser Gly Gly Ser Gly Ala Lys
        275                 280                 285

Gly Gly Pro Glu Ser Arg Lys Asp Pro Ser Gly Ala Ser Asn Pro Ser
    290                 295                 300

Ala Asp Ser Pro Leu His Arg Gly Val His Gly Lys Thr Gly Gln Leu
305                 310                 315                 320

Glu Gly Ala Pro Ala Pro Gly Pro Ala Ala Ser Pro Gln Thr Leu Asp
                325                 330                 335

His Ser Gly Ala Thr Ala Thr Gly Gly Ala Ser Glu Leu Lys Thr Pro
            340                 345                 350

Ala Ser Ser Thr Ala Pro Pro Ile Ser Ser Gly Pro Gly Ala Leu Ala
        355                 360                 365

Ser Val Pro Ala Ser His Pro Ala His Gly Leu Ala Pro His Glu Ser
    370                 375                 380

Gln Leu His Leu Lys Gly Asp Pro His Tyr Ser Phe Asn His Pro Phe
385                 390                 395                 400

Ser Ile Asn Asn Leu Met Ser Ser Ser Glu Gln Gln His Lys Leu Asp
                405                 410                 415

Phe Lys Ala Tyr Glu Gln Ala Leu Gln Tyr Ser Pro Tyr Gly Ser Thr
            420                 425                 430

Leu Pro Ala Ser Leu Pro Leu Gly Ser Ala Ser Val Thr Thr Arg Ser
        435                 440                 445

Pro Ile Glu Pro Ser Ala Leu Glu Pro Ala Tyr Tyr Gln Gly Val Tyr
    450                 455                 460

Ser Arg Pro Val Leu Asn Thr Ser
465                 470

<210>  51
<211>  3780
<212>  DNA
<213>  Homo sapiens

<220>

<221>  CDS
<222>  (960)..(2282)
<223>

<400>  51
agagcgctgc cgccgccgct ttcgcccggg agccgggggc cgggcgccat catgctgagc      60

cggctcgggg cgctgctgca ggaagccgtg ggggcgcgcg agcccagcat tgacctgctg     120

caggccttcg tggagcactg gaagggcatc acgcactact acatcgagag cacagatgaa     180

agcacccccg ccaagaagac agacattccc tggcggctga agcagatgct ggatatcctg     240

gtgtatgaag agcagcagca ggcggccgcg ggtgaggcag ggccctgcct ggagtacctg     300

ctgcagcaca agatcctgga gactctctgc acgctgggca aggccgagta ccccccaggc     360

atgcggcagc aggtgttcca gttcttcagc aaggttctgg cgcaggtgca gcaccccctg     420

ctgcattacc tcagcgtcca caggcctgtg cagaaactcc tccgacttgg tgggactgct     480

tccggatccg ttacagaaaa ggaggaggtg cagttcacca ccgtcctctg ctccaagatc     540

cagcaggacc cagagctgct cgcctacatc ctggaaggta aaaagattgt aggtaggaag     600

aaagcatgcg gagaacccac tgccctgcct aaggacacaa ccagccacgg ggacaaggac     660

tgctcccacg atggtgctcc tgccaggccc cagctggacg gggagtcctg tggggcccag     720

gccttgaaca gccacatgcc tgctgagacc gaggagctgg acggtgggac cacagagagc     780

aacctgatta cctccctgct tgggctgtgc cagagcaaga agagtcgggt ggccttgaag     840

gcccaggaga acctgctgct cctggtgagc atggcctccc cagcagctgc cacctacctg     900

gtacagagca gcgcctgctg ccctgcgatc gtccggcact ttgccagttg taccggtcc      959

atg cct gtc ttc ctg gac ccc gca gac att gcc acc tta gag ggc atc      1007
Met Pro Val Phe Leu Asp Pro Ala Asp Ile Ala Thr Leu Glu Gly Ile
1               5                   10                  15

agc tgg agg tta ccc agt gcc ccg tct gat gag gct tcc ttc cct ggc      1055
Ser Trp Arg Leu Pro Ser Ala Pro Ser Asp Glu Ala Ser Phe Pro Gly
            20                  25                  30

aag gag gcc ttg gct gcc ttc ttg ggc tgg ttt gat tac tgc gac cac      1103
Lys Glu Ala Leu Ala Ala Phe Leu Gly Trp Phe Asp Tyr Cys Asp His
        35                  40                  45

ctc atc aca gag gca cac acg gtg gtt gcg gac gcc ttg gcg aag gct      1151

Leu Ile Thr Glu Ala His Thr Val Val Ala Asp Ala Leu Ala Lys Ala
    50                  55                  60

gtg gct gag aac ttc ttc gtg gag acc ctg cag ccc cag ctc ctg cac      1199
Val Ala Glu Asn Phe Phe Val Glu Thr Leu Gln Pro Gln Leu Leu His
65                  70                  75                  80

gtg tcc gag cag agc atc ttg acc tcc acc gcc ctc ctc aca gcc atg      1247
Val Ser Glu Gln Ser Ile Leu Thr Ser Thr Ala Leu Leu Thr Ala Met
                85                  90                  95

ctg cgc cag ctt cgc tcc cct gcg ctg ctg cgg gag gcc gtg gct ttc      1295
Leu Arg Gln Leu Arg Ser Pro Ala Leu Leu Arg Glu Ala Val Ala Phe
            100                 105                 110

ctc ctg ggc aca gac cgg cag cct gaa gcc ccc ggg gac aac ccc cac      1343
Leu Leu Gly Thr Asp Arg Gln Pro Glu Ala Pro Gly Asp Asn Pro His
        115                 120                 125

acc ctg tat gct cat ctc atc ggg cat tgt gac cac ctc tct gat gag      1391
Thr Leu Tyr Ala His Leu Ile Gly His Cys Asp His Leu Ser Asp Glu
    130                 135                 140

atc agc atc acc aca ctc cgg ctg ttt gag gag ctg ctg cag aag ccc      1439
Ile Ser Ile Thr Thr Leu Arg Leu Phe Glu Glu Leu Leu Gln Lys Pro
145                 150                 155                 160

cac gag ggg atc atc cac agc ctg gtc ctg cgc aac ctt gag ggc cgc      1487
His Glu Gly Ile Ile His Ser Leu Val Leu Arg Asn Leu Glu Gly Arg
                165                 170                 175

cct tac gtg gcc tgg ggc tca cca gag cct gag agc tat gag gac acc      1535
Pro Tyr Val Ala Trp Gly Ser Pro Glu Pro Glu Ser Tyr Glu Asp Thr
            180                 185                 190

cta gac ctg gag gaa gac ccc tac ttc acc gac agc ttc ctg gat tcc      1583
Leu Asp Leu Glu Glu Asp Pro Tyr Phe Thr Asp Ser Phe Leu Asp Ser
        195                 200                 205

ggc ttt caa act ccc gca aag cct cgc cta gct cct gct acc agt tac      1631
Gly Phe Gln Thr Pro Ala Lys Pro Arg Leu Ala Pro Ala Thr Ser Tyr
    210                 215                 220

gat ggc aaa aca gca gtg acc gag atc gtc aac agt ttc ctg tgc ctg      1679
Asp Gly Lys Thr Ala Val Thr Glu Ile Val Asn Ser Phe Leu Cys Leu
225                 230                 235                 240

gtc ccc gag gaa gcc aag acc tct gcc ttc ctg gag gag aca ggc tat      1727
Val Pro Glu Glu Ala Lys Thr Ser Ala Phe Leu Glu Glu Thr Gly Tyr
                245                 250                 255

gac aca tac gtc cac gat gct tat ggc ctg ttc cag gag tgc agc tcc 1775
Asp Thr Tyr Val His Asp Ala Tyr Gly Leu Phe Gln Glu Cys Ser Ser
            260                 265                 270

cgc gtc gcc tcc tgg ggc tgg cct ctg acc ccc aca cct ttg gac ccc 1823
Arg Val Ala Ser Trp Gly Trp Pro Leu Thr Pro Thr Pro Leu Asp Pro
        275                 280                 285

cat gag ccc gag cga cct ttc ttc gag ggc cac ttc ctc cga gtg ctg 1871
His Glu Pro Glu Arg Pro Phe Phe Glu Gly His Phe Leu Arg Val Leu
    290                 295                 300

ttt gac cgc atg tcc cgg att ctg gat cag cca tac agc ctg aac ctg 1919
Phe Asp Arg Met Ser Arg Ile Leu Asp Gln Pro Tyr Ser Leu Asn Leu
305                 310                 315                 320

cag gtg acc tcg gtc ctg tcc cgg ctt gcc ctc ttc ccc cac ccc cat 1967
Gln Val Thr Ser Val Leu Ser Arg Leu Ala Leu Phe Pro His Pro His
                325                 330                 335

att cat gag tac ctg ctg gat ccg tac atc agc ctg gcc ccc ggc tgc 2015
Ile His Glu Tyr Leu Leu Asp Pro Tyr Ile Ser Leu Ala Pro Gly Cys
            340                 345                 350

agg agc cta ttc tcc gtg ttg gtg agg gtg atc ggg gac ttg atg cag 2063
Arg Ser Leu Phe Ser Val Leu Val Arg Val Ile Gly Asp Leu Met Gln
        355                 360                 365

aga atc cag agg gta ccc cag ttc cca ggc aag ctg ctc ctg gtg cgc 2111
Arg Ile Gln Arg Val Pro Gln Phe Pro Gly Lys Leu Leu Leu Val Arg
    370                 375                 380

aag cag ttg acg ggc cag gct cct ggg gag cag ctg gac cac cag acc 2159
Lys Gln Leu Thr Gly Gln Ala Pro Gly Glu Gln Leu Asp His Gln Thr
385                 390                 395                 400

ctc ctc cag ggc gtg gtg gtg ctg gag gag ttc tgc aag gag ctg gct 2207
Leu Leu Gln Gly Val Val Val Leu Glu Glu Phe Cys Lys Glu Leu Ala
                405                 410                 415

gcc att gcc ttc gtc aag ttt ccc cca cat gat cct cgc cag aac gtc 2255
Ala Ile Ala Phe Val Lys Phe Pro Pro His Asp Pro Arg Gln Asn Val
            420                 425                 430

tcc cca gcc ccg gaa ggg cag gtc tga gccagcacca gggcggtggg 2302
Ser Pro Ala Pro Glu Gly Gln Val
        435                 440

agactcctgt ccacacctct gccccagagc tgcctcctgc ctggcactgc cgccacactc 2362

ccctcctggg atggggcttc tgctcccggg ctcactcaag gagactgcgg catgttgacc 2422

acaccagact gggtttcagg gaatgggcat gccaggtgcc aaggagccaa acagatggct 2482

ttccaggcag caaggtcctt ggggccttct tggaggagct tgggtgacag ccaggtgagc 2542

acccagaccc cagaccctca tgtgctgtgt gcctggcccc ttctgtactg gccatttgtg 2602

gccagggcca agcctgtgac tcaactccag gggcaagatg gggagtgagc tgatggctcc 2662

gagactggtc aggagcccag gccagtgaga tggggcctgg agccttgtct gtgtcacatt 2722

aggtaccatg ggagctgctg agacctgaca ttttgtcccc tgcctacatg gcttggccca 2782

tggagaagga gcagtgaatg ggatcgtcgg ggaagcccct cttcctgctc tgctcccctg 2842

gaaactgttg caaaactccc agccgcctca tggcaaatgc ccaaagcatg ttccgcaccc 2902

aggcgggggc ccctgctaat gagaaccttg gtgcagctgc agccaggagg ggagcgggcc 2962

caggagccag gctcaggtcc agctggttcc tctctggcgc cttctgaacc cgtctcagca 3022

ggtccacagc acctgggcag aggtcagaga ccaggggagg ccgggccttg ccctcccttc 3082

tgcccagggc ccagtgttct tgatagaaga cccttctggg gagccaggga gctcagggga 3142

cagataaggg aaggacgccc cctgactcca ggcccctgag cctggcggga agtggctgcg 3202

gcccaggcag ccagtcctgg tggtgttctc cctgcatgcc ctccgtggct gggctgccac 3262

cccacccggc ccgaatctgt cttgacctgc aggaatacac gggcggcgcc aggcattacc 3322

tcacagcggg actacacagt tgctggcttt gctcctgggc aaggaggagc aggccagagc 3382

ctcttttgct tccttttctt gcccatgccg cttctagaag ccaggcacag gttgccaaga 3442

ggtgacacga aacaggagga aactcagtga cctctgcctc tcccacattc ctccccgcgg 3502

gggaggacct cgccgctctg aagagcaccg tgcacatgtg ggtgcacaaa cgtgggtgtt 3562

ggtgtggacg gggcgcagat ctccgtggat gaactgcgtc tggactctta gattcataaa 3622

atattcgagg gtttgggagt cacagaccct cccctctcct cagtgcactt tggcatttgc 3682

acggtgtctt ccccggacag cacagcaata aatggtgtga ttgcgtggaa aaaaaaaaaa 3742

aaaaaaaaaa aaaaaaaaaa aaaaaaaaaa aaaaaaaa 3780

<210> 52
<211> 440
<212> PRT
<213> Homo sapiens

<400> 52

Met Pro Val Phe Leu Asp Pro Ala Asp Ile Ala Thr Leu Glu Gly Ile
1               5                   10                  15

Ser Trp Arg Leu Pro Ser Ala Pro Ser Asp Glu Ala Ser Phe Pro Gly
            20                  25                  30

Lys Glu Ala Leu Ala Ala Phe Leu Gly Trp Phe Asp Tyr Cys Asp His
        35                  40                  45

Leu Ile Thr Glu Ala His Thr Val Val Ala Asp Ala Leu Ala Lys Ala
    50                  55                  60

Val Ala Glu Asn Phe Phe Val Glu Thr Leu Gln Pro Gln Leu Leu His
65                  70                  75                  80

Val Ser Glu Gln Ser Ile Leu Thr Ser Thr Ala Leu Leu Thr Ala Met
                85                  90                  95

Leu Arg Gln Leu Arg Ser Pro Ala Leu Leu Arg Glu Ala Val Ala Phe
            100                 105                 110

Leu Leu Gly Thr Asp Arg Gln Pro Glu Ala Pro Gly Asp Asn Pro His
        115                 120                 125

Thr Leu Tyr Ala His Leu Ile Gly His Cys Asp His Leu Ser Asp Glu
    130                 135                 140

Ile Ser Ile Thr Thr Leu Arg Leu Phe Glu Glu Leu Leu Gln Lys Pro
145                 150                 155                 160

His Glu Gly Ile Ile His Ser Leu Val Leu Arg Asn Leu Glu Gly Arg
                165                 170                 175

Pro Tyr Val Ala Trp Gly Ser Pro Glu Pro Glu Ser Tyr Glu Asp Thr
            180                 185                 190

Leu Asp Leu Glu Glu Asp Pro Tyr Phe Thr Asp Ser Phe Leu Asp Ser
        195                 200                 205

Gly Phe Gln Thr Pro Ala Lys Pro Arg Leu Ala Pro Ala Thr Ser Tyr
    210                 215                 220

Asp Gly Lys Thr Ala Val Thr Glu Ile Val Asn Ser Phe Leu Cys Leu
225                 230                 235                 240

Val Pro Glu Glu Ala Lys Thr Ser Ala Phe Leu Glu Glu Thr Gly Tyr
                245                 250                 255

Asp Thr Tyr Val His Asp Ala Tyr Gly Leu Phe Gln Glu Cys Ser Ser
            260                 265                 270

Arg Val Ala Ser Trp Gly Trp Pro Leu Thr Pro Thr Pro Leu Asp Pro
        275                 280                 285

His Glu Pro Glu Arg Pro Phe Phe Glu Gly His Phe Leu Arg Val Leu
    290                 295                 300

Phe Asp Arg Met Ser Arg Ile Leu Asp Gln Pro Tyr Ser Leu Asn Leu
305                 310                 315                 320

Gln Val Thr Ser Val Leu Ser Arg Leu Ala Leu Phe Pro His Pro His
                325                 330                 335

Ile His Glu Tyr Leu Leu Asp Pro Tyr Ile Ser Leu Ala Pro Gly Cys
            340                 345                 350

Arg Ser Leu Phe Ser Val Leu Val Arg Val Ile Gly Asp Leu Met Gln
        355                 360                 365

Arg Ile Gln Arg Val Pro Gln Phe Pro Gly Lys Leu Leu Leu Val Arg

370                 375                 380

Lys Gln Leu Thr Gly Gln Ala Pro Gly Glu Gln Leu Asp His Gln Thr
385                 390                 395                 400

Leu Leu Gln Gly Val Val Val Leu Glu Glu Phe Cys Lys Glu Leu Ala
                405                 410                 415

Ala Ile Ala Phe Val Lys Phe Pro Pro His Asp Pro Arg Gln Asn Val
            420                 425                 430

Ser Pro Ala Pro Glu Gly Gln Val
        435                 440

<210>  53
<211>  1175
<212>  DNA
<213>  Homo sapiens

<220>
<221>  CDS
<222>  (68)..(895)
<223>

<400>  53
cccggtcgcc gcggctgccg cgccgccgct gcaggatagc tagcggccag gagaaataca      60

gtggaaa atg caa aac aac gaa att ata aag cct gcc aaa tac ttc tca      109
        Met Gln Asn Asn Glu Ile Ile Lys Pro Ala Lys Tyr Phe Ser
        1               5                   10

gaa ttg gaa aag agc atc ctg ctg gct tta gta gaa aag tat aaa tat      157
Glu Leu Glu Lys Ser Ile Leu Leu Ala Leu Val Glu Lys Tyr Lys Tyr
15                  20                  25                  30

gtg ctg gaa tgt aag aaa agt gat gcg cga act att gcc ctt aag cag      205
Val Leu Glu Cys Lys Lys Ser Asp Ala Arg Thr Ile Ala Leu Lys Gln
                35                  40                  45

cgt acc tgg cag gcg ctg gcc cac gaa tac aac tct cag ccc agc gtg      253
Arg Thr Trp Gln Ala Leu Ala His Glu Tyr Asn Ser Gln Pro Ser Val
            50                  55                  60

tcc ctg cgg gat ttc aaa cag ctg aag aag tgc tgg gag aac atc aag      301
Ser Leu Arg Asp Phe Lys Gln Leu Lys Lys Cys Trp Glu Asn Ile Lys

        65                  70                  75

gct cgg acc aaa aaa att atg gcc cat gaa agg aga gag aaa gtg aaa 349
Ala Arg Thr Lys Lys Ile Met Ala His Glu Arg Arg Glu Lys Val Lys
    80                  85                  90

cgg agc gtc agc cct ctc ctg agt acc cac gtc cta ggg aag gag aag 397
Arg Ser Val Ser Pro Leu Leu Ser Thr His Val Leu Gly Lys Glu Lys
95                  100                 105                 110

atc gcc agc atg ctg ccg gag cag ctc tac ttc ctg cag agc ccc ccg 445
Ile Ala Ser Met Leu Pro Glu Gln Leu Tyr Phe Leu Gln Ser Pro Pro
                115                 120                 125

gag gag gag ccc gaa tac cac ccc gac gcc tca gcc caa gaa tca ttt 493
Glu Glu Glu Pro Glu Tyr His Pro Asp Ala Ser Ala Gln Glu Ser Phe
            130                 135                 140

gct gtt tca aat aga gaa ctg tgc gat gat gag aaa gag ttc ata cat 541
Ala Val Ser Asn Arg Glu Leu Cys Asp Asp Glu Lys Glu Phe Ile His
        145                 150                 155

ttt cca gta tgt gag ggg acc tct caa cct gaa ccc tcg tgt tca gct 589
Phe Pro Val Cys Glu Gly Thr Ser Gln Pro Glu Pro Ser Cys Ser Ala
    160                 165                 170

gtc aga ata aca gcc aat aaa aac tac agg agc aaa acc tct cag gaa 637
Val Arg Ile Thr Ala Asn Lys Asn Tyr Arg Ser Lys Thr Ser Gln Glu
175                 180                 185                 190

ggt gct tta aaa aag atg cat gag gaa gaa cac cat caa caa atg tcc 685
Gly Ala Leu Lys Lys Met His Glu Glu Glu His His Gln Gln Met Ser
                195                 200                 205

atc tta caa ctg caa ctg ata caa atg aat gag gtg cat gtg gcc aaa 733
Ile Leu Gln Leu Gln Leu Ile Gln Met Asn Glu Val His Val Ala Lys
            210                 215                 220

atc cag cag ata gag cga gag tgt gag atg gca gag gag gaa cac agg 781
Ile Gln Gln Ile Glu Arg Glu Cys Glu Met Ala Glu Glu Glu His Arg
        225                 230                 235

ata aaa atg gaa gtt ctc aat aaa aag aag atg tat tgg gaa aga aaa 829
Ile Lys Met Glu Val Leu Asn Lys Lys Lys Met Tyr Trp Glu Arg Lys
    240                 245                 250

cta caa act ttt acc aag gaa tgg cct gtt tcc tca ttt aac cgg ccc 877
Leu Gln Thr Phe Thr Lys Glu Trp Pro Val Ser Ser Phe Asn Arg Pro
255                 260                 265                 270

ttt ccc aat tcg ccc taa gactttgggg gtggctctct tgtaattaat          925
Phe Pro Asn Ser Pro
                275

ctgtgttggc aaagaatgtc tggaacatgg acttggcggt cagtaacctg taacagagct    985

acaactagga aaattagagt ggtagtagtc acttatttaa gaattcattc aggtaaacag   1045

ctgcaccctc tgtacccctt aagtggcaaa gaagctgtta tagtcttctg aaaattatca   1105

ctatgagtgc tataattctg aatataatgt ctcttaatta gaattcatac aagaaaaaaa   1165

aaaaaaaaaa                                                          1175

<210>  54
<211>  275
<212>  PRT
<213>  Homo sapiens

<400>  54

Met Gln Asn Asn Glu Ile Ile Lys Pro Ala Lys Tyr Phe Ser Glu Leu
1               5                   10                  15

Glu Lys Ser Ile Leu Leu Ala Leu Val Glu Lys Tyr Lys Tyr Val Leu
            20                  25                  30

Glu Cys Lys Lys Ser Asp Ala Arg Thr Ile Ala Leu Lys Gln Arg Thr
        35                  40                  45

Trp Gln Ala Leu Ala His Glu Tyr Asn Ser Gln Pro Ser Val Ser Leu
    50                  55                  60

Arg Asp Phe Lys Gln Leu Lys Lys Cys Trp Glu Asn Ile Lys Ala Arg
65                  70                  75                  80

Thr Lys Lys Ile Met Ala His Glu Arg Arg Glu Lys Val Lys Arg Ser
                85                  90                  95

Val Ser Pro Leu Leu Ser Thr His Val Leu Gly Lys Glu Lys Ile Ala
            100                 105                 110

Ser Met Leu Pro Glu Gln Leu Tyr Phe Leu Gln Ser Pro Pro Glu Glu
        115                 120                 125

Glu Pro Glu Tyr His Pro Asp Ala Ser Ala Gln Glu Ser Phe Ala Val
    130                 135                 140

Ser Asn Arg Glu Leu Cys Asp Asp Glu Lys Glu Phe Ile His Phe Pro
145                 150                 155                 160

Val Cys Glu Gly Thr Ser Gln Pro Glu Pro Ser Cys Ser Ala Val Arg
                165                 170                 175

Ile Thr Ala Asn Lys Asn Tyr Arg Ser Lys Thr Ser Gln Glu Gly Ala
            180                 185                 190

Leu Lys Lys Met His Glu Glu Glu His His Gln Gln Met Ser Ile Leu
        195                 200                 205

Gln Leu Gln Leu Ile Gln Met Asn Glu Val His Val Ala Lys Ile Gln
    210                 215                 220

Gln Ile Glu Arg Glu Cys Glu Met Ala Glu Glu Glu His Arg Ile Lys
225                 230                 235                 240

Met Glu Val Leu Asn Lys Lys Lys Met Tyr Trp Glu Arg Lys Leu Gln
                245                 250                 255

Thr Phe Thr Lys Glu Trp Pro Val Ser Ser Phe Asn Arg Pro Phe Pro
            260                 265                 270

Asn Ser Pro
        275

<210>  55
<211>  1988
<212>  DNA
<213>  Homo sapiens

<220>

<221>  CDS
<222>  (12)..(1805)
<223>

<400>  55
cctccgggaa g atg gcg gcc gtg cag gcg gcc gag gtg aaa gtg gat ggc      50
             Met Ala Ala Val Gln Ala Ala Glu Val Lys Val Asp Gly
             1               5                   10

agc gag ccg aaa ctg agc aag aat gag ctg aag aga cgc ctg aaa gct      98
Ser Glu Pro Lys Leu Ser Lys Asn Glu Leu Lys Arg Arg Leu Lys Ala
    15                  20                  25

gag aag aaa gta gca gag aag gag gcc aaa cag aag gag ctc agt gag      146
Glu Lys Lys Val Ala Glu Lys Glu Ala Lys Gln Lys Glu Leu Ser Glu
30                  35                  40                  45

aaa cag cta agc caa gcc act gct gct gcc acc aac cac acc act gat      194
Lys Gln Leu Ser Gln Ala Thr Ala Ala Ala Thr Asn His Thr Thr Asp
                50                  55                  60

aat ggt gtg ggt cct gag gaa gag agc gtg gac cca aat caa tac tac      242
Asn Gly Val Gly Pro Glu Glu Glu Ser Val Asp Pro Asn Gln Tyr Tyr
            65                  70                  75

aaa atc cgc agt caa gca att cat cag ctg aag gtc aat ggg gaa gac      290
Lys Ile Arg Ser Gln Ala Ile His Gln Leu Lys Val Asn Gly Glu Asp
        80                  85                  90

cca tac cca cac aag ttc cat gta gac atc tca ctc act gac ttc atc      338
Pro Tyr Pro His Lys Phe His Val Asp Ile Ser Leu Thr Asp Phe Ile
    95                  100                 105

caa aaa tat agt cac ctg cag cct ggg gat cac ctg act gac atc acc      386
Gln Lys Tyr Ser His Leu Gln Pro Gly Asp His Leu Thr Asp Ile Thr
110                 115                 120                 125

tta aag gtg gca ggt agg atc cat gcc aaa aga gct tct ggg gga aag      434
Leu Lys Val Ala Gly Arg Ile His Ala Lys Arg Ala Ser Gly Gly Lys
                130                 135                 140

ctc atc ttc tat gat ctt cga gga gag ggg gtg aag ttg caa gtc atg      482
Leu Ile Phe Tyr Asp Leu Arg Gly Glu Gly Val Lys Leu Gln Val Met
            145                 150                 155

gcc aat tcc aga aat tat aaa tca gaa gaa gaa ttt att cat att aat      530
Ala Asn Ser Arg Asn Tyr Lys Ser Glu Glu Glu Phe Ile His Ile Asn
        160                 165                 170

aac aaa ctg cgt cgg gga gac ata att gga gtt cag ggg aat cct ggt      578

Asn Lys Leu Arg Arg Gly Asp Ile Ile Gly Val Gln Gly Asn Pro Gly
    175                 180                 185

aaa acc aag aag ggt gag ctg agc atc att ccg tat gag atc aca ctg         626
Lys Thr Lys Lys Gly Glu Leu Ser Ile Ile Pro Tyr Glu Ile Thr Leu
190                 195                 200                 205

ctg tct ccc tgt ttg cat atg tta cct cat ctt cac ttt ggc ctc aaa         674
Leu Ser Pro Cys Leu His Met Leu Pro His Leu His Phe Gly Leu Lys
                210                 215                 220

gac aag gaa aca agg tat cgc cag aga tac ttg gac ttg atc ctg aat         722
Asp Lys Glu Thr Arg Tyr Arg Gln Arg Tyr Leu Asp Leu Ile Leu Asn
            225                 230                 235

gac ttt gtg agg cag aaa ttt atc atc cgc tct aag atc atc aca tat         770
Asp Phe Val Arg Gln Lys Phe Ile Ile Arg Ser Lys Ile Ile Thr Tyr
        240                 245                 250

ata aga agt ttc tta gat gag ctg gga ttc cta gag att gaa act ccc         818
Ile Arg Ser Phe Leu Asp Glu Leu Gly Phe Leu Glu Ile Glu Thr Pro
    255                 260                 265

atg atg aac atc atc cca ggg gga gcc gtg gcc aag cct ttc atc act         866
Met Met Asn Ile Ile Pro Gly Gly Ala Val Ala Lys Pro Phe Ile Thr
270                 275                 280                 285

tat cac aac gag ctg gac atg aac tta tat atg aga att gct cca gaa         914
Tyr His Asn Glu Leu Asp Met Asn Leu Tyr Met Arg Ile Ala Pro Glu
                290                 295                 300

ctc tat cat aag atg ctt gtg gtt ggt ggc atc gac cgg gtt tat gaa         962
Leu Tyr His Lys Met Leu Val Val Gly Gly Ile Asp Arg Val Tyr Glu
            305                 310                 315

att gga cgc cag ttc cgg aat gag ggg att gat ttg acg cac aat cct        1010
Ile Gly Arg Gln Phe Arg Asn Glu Gly Ile Asp Leu Thr His Asn Pro
        320                 325                 330

gag ttc acc acc tgt gag ttc tac atg gcc tat gca gac tat cac gat        1058
Glu Phe Thr Thr Cys Glu Phe Tyr Met Ala Tyr Ala Asp Tyr His Asp
    335                 340                 345

ctc atg gaa atc acg gag aag atg gtt tca ggg atg gtg aag cat att        1106
Leu Met Glu Ile Thr Glu Lys Met Val Ser Gly Met Val Lys His Ile
350                 355                 360                 365

aca ggc agt tac aag gtc acc tac cac cca gat ggc cca gag ggc caa        1154
Thr Gly Ser Tyr Lys Val Thr Tyr His Pro Asp Gly Pro Glu Gly Gln
                370                 375                 380

gcc tac gat gtt gac ttc acc cca ccc ttc cgg cga atc aac atg gta     1202
Ala Tyr Asp Val Asp Phe Thr Pro Pro Phe Arg Arg Ile Asn Met Val
            385                 390                 395

gaa gag ctt gag aaa gcc ctg ggg atg aag ctg cca gaa acg aac ctc     1250
Glu Glu Leu Glu Lys Ala Leu Gly Met Lys Leu Pro Glu Thr Asn Leu
        400                 405                 410

ttt gaa act gaa gaa act cgc aaa att ctt gat gat atc tgt gtg gca     1298
Phe Glu Thr Glu Glu Thr Arg Lys Ile Leu Asp Asp Ile Cys Val Ala
    415                 420                 425

aaa gct gtt gaa tgc cct cca cct cgg acc aca gcc agg ctc ctt gac     1346
Lys Ala Val Glu Cys Pro Pro Pro Arg Thr Thr Ala Arg Leu Leu Asp
430                 435                 440                 445

aag ctt gtt ggg gag ttc ctg gaa gtg act tgc atc aat cct aca ttc     1394
Lys Leu Val Gly Glu Phe Leu Glu Val Thr Cys Ile Asn Pro Thr Phe
                450                 455                 460

atc tgt gat cac cca cag ata atg agc cct ttg gct aaa tgg cac cgc     1442
Ile Cys Asp His Pro Gln Ile Met Ser Pro Leu Ala Lys Trp His Arg
            465                 470                 475

tct aaa gag ggt ctg act gag cgc ttt gag ctg ttt gtc atg aag aaa     1490
Ser Lys Glu Gly Leu Thr Glu Arg Phe Glu Leu Phe Val Met Lys Lys
        480                 485                 490

gag ata tgc aat gcg tat act gag ctg aat gat ccc atg cgg cag cgg     1538
Glu Ile Cys Asn Ala Tyr Thr Glu Leu Asn Asp Pro Met Arg Gln Arg
    495                 500                 505

cag ctt ttt gaa gaa cag gcc aag gcc aag gct gca ggt gat gat gag     1586
Gln Leu Phe Glu Glu Gln Ala Lys Ala Lys Ala Ala Gly Asp Asp Glu
510                 515                 520                 525

gcc atg ttc ata gat gaa aac ttc tgt act gcc ctg gaa tat ggg ctg     1634
Ala Met Phe Ile Asp Glu Asn Phe Cys Thr Ala Leu Glu Tyr Gly Leu
                530                 535                 540

ccc ccc aca gct ggc tgg ggc atg ggc att gat cga gtc gcc atg ttt     1682
Pro Pro Thr Ala Gly Trp Gly Met Gly Ile Asp Arg Val Ala Met Phe
            545                 550                 555

ctc acg gac tcc aac aac atc aag gaa gta ctt ctg ttt cct gcc atg     1730
Leu Thr Asp Ser Asn Asn Ile Lys Glu Val Leu Leu Phe Pro Ala Met
        560                 565                 570

aaa ccc gaa gac aag aag gag aat gta gca acc act gat aca ctg gaa     1778

Lys Pro Glu Asp Lys Lys Glu Asn Val Ala Thr Thr Asp Thr Leu Glu
    575                 580                 585

agc aca aca gtt ggc act tct gtc tag aaaataataa ttgcaagttg          1825
Ser Thr Thr Val Gly Thr Ser Val
590                 595

tataactcag gcgtctttgc atttctgcga aagatcaagg tctgcaaggg aattcttgtg   1885

tgctgctttc catttgacac cgcagttctg ttcagccatc agaagagaga caaggaatta   1945

aaaatttctt tttaatcctg ttaccaaaaa aaaaaaaaaa aaa                     1988

<210>  56
<211>  597
<212>  PRT
<213>  Homo sapiens

<400>  56

Met Ala Ala Val Gln Ala Ala Glu Val Lys Val Asp Gly Ser Glu Pro
1               5                   10                  15

Lys Leu Ser Lys Asn Glu Leu Lys Arg Arg Leu Lys Ala Glu Lys Lys
            20                  25                  30

Val Ala Glu Lys Glu Ala Lys Gln Lys Glu Leu Ser Glu Lys Gln Leu
        35                  40                  45

Ser Gln Ala Thr Ala Ala Ala Thr Asn His Thr Thr Asp Asn Gly Val
    50                  55                  60

Gly Pro Glu Glu Glu Ser Val Asp Pro Asn Gln Tyr Tyr Lys Ile Arg
65                  70                  75                  80

Ser Gln Ala Ile His Gln Leu Lys Val Asn Gly Glu Asp Pro Tyr Pro
                85                  90                  95

His Lys Phe His Val Asp Ile Ser Leu Thr Asp Phe Ile Gln Lys Tyr
            100                 105                 110

Ser His Leu Gln Pro Gly Asp His Leu Thr Asp Ile Thr Leu Lys Val

        115                 120                 125

Ala Gly Arg Ile His Ala Lys Arg Ala Ser Gly Gly Lys Leu Ile Phe
    130                 135                 140

Tyr Asp Leu Arg Gly Glu Gly Val Lys Leu Gln Val Met Ala Asn Ser
145                 150                 155                 160

Arg Asn Tyr Lys Ser Glu Glu Glu Phe Ile His Ile Asn Asn Lys Leu
                165                 170                 175

Arg Arg Gly Asp Ile Ile Gly Val Gln Gly Asn Pro Gly Lys Thr Lys
            180                 185                 190

Lys Gly Glu Leu Ser Ile Ile Pro Tyr Glu Ile Thr Leu Leu Ser Pro
        195                 200                 205

Cys Leu His Met Leu Pro His Leu His Phe Gly Leu Lys Asp Lys Glu
    210                 215                 220

Thr Arg Tyr Arg Gln Arg Tyr Leu Asp Leu Ile Leu Asn Asp Phe Val
225                 230                 235                 240

Arg Gln Lys Phe Ile Ile Arg Ser Lys Ile Ile Thr Tyr Ile Arg Ser
                245                 250                 255

Phe Leu Asp Glu Leu Gly Phe Leu Glu Ile Glu Thr Pro Met Met Asn
            260                 265                 270

Ile Ile Pro Gly Gly Ala Val Ala Lys Pro Phe Ile Thr Tyr His Asn
        275                 280                 285

Glu Leu Asp Met Asn Leu Tyr Met Arg Ile Ala Pro Glu Leu Tyr His
    290                 295                 300

Lys Met Leu Val Val Gly Gly Ile Asp Arg Val Tyr Glu Ile Gly Arg
305                 310                 315                 320

Gln Phe Arg Asn Glu Gly Ile Asp Leu Thr His Asn Pro Glu Phe Thr
                325                 330                 335

Thr Cys Glu Phe Tyr Met Ala Tyr Ala Asp Tyr His Asp Leu Met Glu
            340                 345                 350

Ile Thr Glu Lys Met Val Ser Gly Met Val Lys His Ile Thr Gly Ser
        355                 360                 365

Tyr Lys Val Thr Tyr His Pro Asp Gly Pro Glu Gly Gln Ala Tyr Asp
    370                 375                 380

Val Asp Phe Thr Pro Pro Phe Arg Arg Ile Asn Met Val Glu Glu Leu
385                 390                 395                 400

Glu Lys Ala Leu Gly Met Lys Leu Pro Glu Thr Asn Leu Phe Glu Thr
                405                 410                 415

Glu Glu Thr Arg Lys Ile Leu Asp Asp Ile Cys Val Ala Lys Ala Val
            420                 425                 430

Glu Cys Pro Pro Pro Arg Thr Thr Ala Arg Leu Leu Asp Lys Leu Val
        435                 440                 445

Gly Glu Phe Leu Glu Val Thr Cys Ile Asn Pro Thr Phe Ile Cys Asp
    450                 455                 460

His Pro Gln Ile Met Ser Pro Leu Ala Lys Trp His Arg Ser Lys Glu
465                 470                 475                 480

Gly Leu Thr Glu Arg Phe Glu Leu Phe Val Met Lys Lys Glu Ile Cys
                485                 490                 495

Asn Ala Tyr Thr Glu Leu Asn Asp Pro Met Arg Gln Arg Gln Leu Phe
            500                 505                 510

Glu Glu Gln Ala Lys Ala Lys Ala Ala Gly Asp Asp Glu Ala Met Phe

515                 520                 525

Ile Asp Glu Asn Phe Cys Thr Ala Leu Glu Tyr Gly Leu Pro Pro Thr
    530                 535                 540

Ala Gly Trp Gly Met Gly Ile Asp Arg Val Ala Met Phe Leu Thr Asp
545                 550                 555                 560

Ser Asn Asn Ile Lys Glu Val Leu Leu Phe Pro Ala Met Lys Pro Glu
                565                 570                 575

Asp Lys Lys Glu Asn Val Ala Thr Thr Asp Thr Leu Glu Ser Thr Thr
            580                 585                 590

Val Gly Thr Ser Val
        595

<210>  57
<211>  763
<212>  DNA
<213>  Homo sapiens

<400>  57
ggggctaaac ctagccccaa acccactcca ccttactacc agacaacctt agccaaacca      60

tttacccaaa taaagtatag gcgatagaaa ttgaaacctg gcgcaataga tatagtaccg     120

caagggaaag atgaaaaatt ataaccaagc ataatatagc aaggactaac ccctatacct     180

tctgcataat gaattaacta gaaataactt tgcaaggaga gccaaagcta agacccccga     240

aaccagacga gctacctaag aacagctaaa agagcacacc cgtctatgta gcaaaatagt     300

gggaagattt ataggtagag gcgacaaacc taccgagcct ggtgatagct ggttgtccaa     360

gatagaatct tagttcaact ttaaatttgc ccacagaacc ctctaaatcc ccttgtaaat     420

ttaactgtta gtccaaagag gaacagctct ttggacacta ggaaaaaacc ttgtagagag     480

agtaaaaaat ttaacaccca tagtaggcct aaaagcagcc accaattaag aaagcgttca     540

agctcaacac ccactaccta aaaaatccca aacatataac tgaactcctc acacccaatt     600

ggaccaatct atcaccctat agaagaacta atgttagtat aagtaacatg aaaacattct     660

cctccgcata agcctgcgtc agattaaaac actgaactga caattaacag cccaatatct     720

acaatcaacc cacaagtcat tattaccctc actgtcaacc caa                      763

<210>  58
<211>  1575
<212>  DNA
<213>  Homo sapiens

<220>
<221>  CDS
<222>  (196)..(834)
<223>

<400>  58
ctcttcccgg ctccagctcc gccgccagct ccagcctttg ctccccctcc caaagtcccc      60

tccccggagc ggagcgcacc tagggtccct cttccgtccc cccagcccag ctacccgttc     120

agaccagcag cctcgggggg cacccccccg ccagcctgcc tccctcccgc tcagccctgc     180

cagggttccc cagcc atg aat ctc ttc cga ttc ctg gga gac ctc tcc cac     231
                 Met Asn Leu Phe Arg Phe Leu Gly Asp Leu Ser His
                 1               5                   10

ctc ctc gcc atc atc ttg cta ctg ctc aaa atc tgg aag tcc cgc tcg     279
Leu Leu Ala Ile Ile Leu Leu Leu Leu Lys Ile Trp Lys Ser Arg Ser
        15                  20                  25

tgc gcc gga att tca ggg aag agc cag gtc ctg ttt gct gtg gtg ttc     327
Cys Ala Gly Ile Ser Gly Lys Ser Gln Val Leu Phe Ala Val Val Phe
    30                  35                  40

act gcc cga tat ctg gac ctc ttc acc aac tac atc tca ctc tac aac     375
Thr Ala Arg Tyr Leu Asp Leu Phe Thr Asn Tyr Ile Ser Leu Tyr Asn
45                  50                  55                  60

acg tgt atg aag gtg gtc tac ata gcc tgc tcc ttc acc acg gtc tgg     423
Thr Cys Met Lys Val Val Tyr Ile Ala Cys Ser Phe Thr Thr Val Trp
                65                  70                  75

ttg att tat agc aag ttc aaa gct act tac gat ggg aac cat gac acg     471
Leu Ile Tyr Ser Lys Phe Lys Ala Thr Tyr Asp Gly Asn His Asp Thr
            80                  85                  90

ttc aga gtg gag ttc ctg gtc gtt ccc aca gcc att ctg gcg ttc ctg     519
Phe Arg Val Glu Phe Leu Val Val Pro Thr Ala Ile Leu Ala Phe Leu
        95                  100                 105

gtc aat cat gac ttc acc cct ctg gag atc ctc tgg acc ttc tcc atc     567
Val Asn His Asp Phe Thr Pro Leu Glu Ile Leu Trp Thr Phe Ser Ile
    110                 115                 120

tac ctg gag tca gtg gcc atc ttg ccg cag ctg ttc atg gtg agc aag     615
Tyr Leu Glu Ser Val Ala Ile Leu Pro Gln Leu Phe Met Val Ser Lys
125                 130                 135                 140

acc ggc gag gcg gag acc atc acc agc cac tac ttg ttt gcg cta ggc     663
Thr Gly Glu Ala Glu Thr Ile Thr Ser His Tyr Leu Phe Ala Leu Gly
                145                 150                 155

gtt tac cgc acg ctc tat ctc ttc aac tgg atc tgg cgc tac cat ttc     711
Val Tyr Arg Thr Leu Tyr Leu Phe Asn Trp Ile Trp Arg Tyr His Phe
            160                 165                 170

gag ggc ttc ttc gac ctc atc gcc att gtg gca ggc ctg gtc cag aca     759
Glu Gly Phe Phe Asp Leu Ile Ala Ile Val Ala Gly Leu Val Gln Thr
        175                 180                 185

gtc ctc tac tgc gat ttc ttc tac ctc tat atc acc aaa gtc cta aag     807
Val Leu Tyr Cys Asp Phe Phe Tyr Leu Tyr Ile Thr Lys Val Leu Lys
    190                 195                 200

ggg aag aag ttg agt ttg ccg gca tag ccccggtcct ctccatctct           854
Gly Lys Lys Leu Ser Leu Pro Ala
205                 210

ctcctcggca gcagcgggag gcagaggaag gcggcagaag atgaagagct ttcccatcca   914

ggggtgactt ttttaagaac ccacctcttg tgctccccat cccgcctcct gccgggtttc   974

agggggacag tggaggatcc aggtcttggg gagctcagga cttgggctgt ttgtagtttt   1034

ttgcctttta gacaagaaaa aaaaatcttt ccactcttta gtttttgatt ctgatgactc   1094

gtttttcttc tactctgtgg ccccaatttt tataaagtgt ttttgagtgt cctatgggcc    1154

ggggcagggt ccaagatctt ttcccttccc caggcccctc ggctccctcc cagatcccac   1214

ccccagcccc actggttgcc aaacactaaa tctgccgaca cccatctgcc ccacctcctg   1274

ccatggccat gaaccgcgac ccccactaaa tttctagatt ggggataggg agaaagggag  1334

gcccaggaag gtctcccctg attttttttc atagtaattt ttttccccag agtttgaatt    1394

ttttggtctt ctcctggttt tttggcaaat taggggggcc cggggctcaa gtgcgggaag  1454

ggggctggcc cgaggatccc atggctctca caccatgttt ttgtacagaa ctgatggttg   1514

aatctttgtt ctcttgaaat aaacagaaga aaatgaaacc tttaaaaaaa aaaaaaaaaa 1574

a 1575

<210> 59
<211> 212
<212> PRT
<213> Homo sapiens

<400> 59

Met Asn Leu Phe Arg Phe Leu Gly Asp Leu Ser His Leu Leu Ala Ile
1               5                   10                  15

Ile Leu Leu Leu Leu Lys Ile Trp Lys Ser Arg Ser Cys Ala Gly Ile
            20                  25                  30

Ser Gly Lys Ser Gln Val Leu Phe Ala Val Val Phe Thr Ala Arg Tyr
        35                  40                  45

Leu Asp Leu Phe Thr Asn Tyr Ile Ser Leu Tyr Asn Thr Cys Met Lys
    50                  55                  60

Val Val Tyr Ile Ala Cys Ser Phe Thr Thr Val Trp Leu Ile Tyr Ser
65                  70                  75                  80

Lys Phe Lys Ala Thr Tyr Asp Gly Asn His Asp Thr Phe Arg Val Glu
                85                  90                  95

Phe Leu Val Val Pro Thr Ala Ile Leu Ala Phe Leu Val Asn His Asp
            100                 105                 110

Phe Thr Pro Leu Glu Ile Leu Trp Thr Phe Ser Ile Tyr Leu Glu Ser
        115                 120                 125

Val Ala Ile Leu Pro Gln Leu Phe Met Val Ser Lys Thr Gly Glu Ala
    130                 135                 140

Glu Thr Ile Thr Ser His Tyr Leu Phe Ala Leu Gly Val Tyr Arg Thr

145                 150                 155                 160

Leu Tyr Leu Phe Asn Trp Ile Trp Arg Tyr His Phe Glu Gly Phe Phe
                165                 170                 175

Asp Leu Ile Ala Ile Val Ala Gly Leu Val Gln Thr Val Leu Tyr Cys
            180                 185                 190

Asp Phe Phe Tyr Leu Tyr Ile Thr Lys Val Leu Lys Gly Lys Lys Leu
        195                 200                 205

Ser Leu Pro Ala
    210

<210>  60
<211>  2245
<212>  DNA
<213>  Homo sapiens

<220>
<221>  CDS
<222>  (157)..(1110)
<223>

<400>  60
gaatctcgac ccttgaatgg agttacacga acggccagat gaaagaagga aggcccggac      60

ctccactcag ggccgactag gggactggcg gagggtgcac gctgatggat ttactcaccg      120

ggtgcttgga gctccagcag ctggctggag cccgcg atg acg tca cgg act cgg      174
                                        Met Thr Ser Arg Thr Arg
                                        1               5

gtc aca tgg ccg agt ccg ccc cgc ccc ctc ccc gtc ccc gcc gct gca      222
Val Thr Trp Pro Ser Pro Pro Arg Pro Leu Pro Val Pro Ala Ala Ala
            10                  15                  20

gcc gtc gcc ttc gga gcg aag ggt acc gac ccg gca gaa gct cgg agc      270
Ala Val Ala Phe Gly Ala Lys Gly Thr Asp Pro Ala Glu Ala Arg Ser
        25                  30                  35

tct cgg ggt atc gag gag gca ggc ccg cgg gcg cac ggg cga gcg ggc      318
Ser Arg Gly Ile Glu Glu Ala Gly Pro Arg Ala His Gly Arg Ala Gly
    40                  45                  50

cgg gag ccg gag cgg cgg agg agc cgg cag cag cgg cgc ggc ggg ctc 366
Arg Glu Pro Glu Arg Arg Arg Ser Arg Gln Gln Arg Arg Gly Gly Leu
55                  60                  65                  70

cag gcg agg cgg tcg acg ctc ctg aaa act tgc gcg cgc gct cgc gcc 414
Gln Ala Arg Arg Ser Thr Leu Leu Lys Thr Cys Ala Arg Ala Arg Ala
                75                  80                  85

act gcg ccc gga gcg atg aag atg gtc gcg ccc tgg acg cgg ttc tac 462
Thr Ala Pro Gly Ala Met Lys Met Val Ala Pro Trp Thr Arg Phe Tyr
            90                  95                  100

tcc aac agc tgc tgc ttg tgc tgc cat gtc cgc acc ggc acc atc ctg 510
Ser Asn Ser Cys Cys Leu Cys Cys His Val Arg Thr Gly Thr Ile Leu
        105                 110                 115

ctc ggc gtc tgg tat ctg atc atc aat gct gtg gta ctg ttg att tta 558
Leu Gly Val Trp Tyr Leu Ile Ile Asn Ala Val Val Leu Leu Ile Leu
    120                 125                 130

ttg agt gcc ctg gct gat ccg gat cag tat aac ttt tca agt tct gaa 606
Leu Ser Ala Leu Ala Asp Pro Asp Gln Tyr Asn Phe Ser Ser Ser Glu
135                 140                 145                 150

ctg gga ggt gac ttt gag ttc atg gat gat gcc aac atg tgc att gcc 654
Leu Gly Gly Asp Phe Glu Phe Met Asp Asp Ala Asn Met Cys Ile Ala
                155                 160                 165

att gcg att tct ctt ctc atg atc ctg ata tgt gct atg gct act tac 702
Ile Ala Ile Ser Leu Leu Met Ile Leu Ile Cys Ala Met Ala Thr Tyr
            170                 175                 180

gga gcg tac aag caa cgc gca gcc tgg atc atc cca ttc ttc tgt tac 750
Gly Ala Tyr Lys Gln Arg Ala Ala Trp Ile Ile Pro Phe Phe Cys Tyr
        185                 190                 195

cag atc ttt gac ttt gcc ctg aac atg ttg gtt gca atc act gtg ctt 798
Gln Ile Phe Asp Phe Ala Leu Asn Met Leu Val Ala Ile Thr Val Leu
    200                 205                 210

att tat cca aac tcc att cag gaa tac ata cgg caa ctg cct cct aat 846
Ile Tyr Pro Asn Ser Ile Gln Glu Tyr Ile Arg Gln Leu Pro Pro Asn
215                 220                 225                 230

ttt ccc tac aga gat gat gtc atg tca gtg aat cct acc tgt ttg gtc 894
Phe Pro Tyr Arg Asp Asp Val Met Ser Val Asn Pro Thr Cys Leu Val
                235                 240                 245

ctt att att ctt ctg ttt att agc att atc ttg act ttt aag ggt tac 942
Leu Ile Ile Leu Leu Phe Ile Ser Ile Ile Leu Thr Phe Lys Gly Tyr

250                 255                 260

ttg att agc tgt gtt tgg aac tgc tac cga tac atc aat ggt agg aac      990
Leu Ile Ser Cys Val Trp Asn Cys Tyr Arg Tyr Ile Asn Gly Arg Asn
        265                 270                 275

tcc tct gat gtc ctg gtt tat gtt acc agc aat gac act acg gtg ctg      1038
Ser Ser Asp Val Leu Val Tyr Val Thr Ser Asn Asp Thr Thr Val Leu
    280                 285                 290

cta ccc ccg tat gat gat gcc act gtg aat ggt gct gcc aag gag cca      1086
Leu Pro Pro Tyr Asp Asp Ala Thr Val Asn Gly Ala Ala Lys Glu Pro
295                 300                 305                 310

ccg cca cct tac gtg tct gcc taa gccttcaagt gggcggagct gagggcagca      1140
Pro Pro Pro Tyr Val Ser Ala
                315

gcttgacttt gcagacatct gagcaatagt tctgttattt cacttttgcc atgagcctct      1200

ctgagcttgt ttgttgctga aatgctactt tttaaaattt agatgttaga ttgaaaactg      1260

tagttttcaa catatgcttt gctggaacac tgtgatagat taactgtaga attcttcctg      1320

tacgattggg gatataatgg gcttcactaa ccttccctag gcattgaaac ttcccccaaa      1380

tctgatggac ctagaagtct gcttttgtac ctgctgggcc ccaaagttgg gcatttttct      1440

ctctgttccc tctcttttga aaatgtaaaa taaaaccaaa aatagacaac tttttcttca      1500

gccattccag catagagaac aaaaccttat ggaaacagga atgtcaattg tgtaatcatt      1560

gttctaatta ggtaaataga agtccttatg tatgtgttac aagaatttcc cccacaacat      1620

cctttatgac tgaagttcaa tgacagtttg tgtttggtgg taaaggattt tctccatggc      1680

ctgaattaag accattagaa agcaccaggc cgtgggagca gtgaccatct gctgactgtt      1740

cttgtggatc ttgtgtccag ggacatgggg tgacatgcct cgtatgtgtt agagggtgga      1800

atggatgtgt ttggcgctgc atgggatctg gtgcccctct tctcctggat tcacatcccc      1860

acccagggcc cgcttttact aagtgttctg ccctagattg gttcaaggag gtcatccaac      1920

tgactttatc gagtggaatt gggatatatt tgatatactt ctgcctaaca acatggaaaa      1980

gggttttctt ttccctgcaa gctacatcct actgctttga acttccaagt atgtctagtc      2040

accttttaaa atgtaaacat tttcagaaaa atgaggattg ccttccttgt atgcgctttt      2100

taccttgact acctgaattg caagggattt ttatatattc atatgttaca aagtcagcaa 2160

ctctcctgtt ggttcattat tgaatgtgct gtaaattaag ttgtttgcaa ttaaaacaag 2220

gtttgcccac aaaaaaaaaa aaaaa 2245

<210> 61
<211> 317
<212> PRT
<213> Homo sapiens

<400> 61

Met Thr Ser Arg Thr Arg Val Thr Trp Pro Ser Pro Pro Arg Pro Leu
1               5                   10                  15

Pro Val Pro Ala Ala Ala Ala Val Ala Phe Gly Ala Lys Gly Thr Asp
            20                  25                  30

Pro Ala Glu Ala Arg Ser Ser Arg Gly Ile Glu Glu Ala Gly Pro Arg
        35                  40                  45

Ala His Gly Arg Ala Gly Arg Glu Pro Glu Arg Arg Arg Ser Arg Gln
    50                  55                  60

Gln Arg Arg Gly Gly Leu Gln Ala Arg Arg Ser Thr Leu Leu Lys Thr
65                  70                  75                  80

Cys Ala Arg Ala Arg Ala Thr Ala Pro Gly Ala Met Lys Met Val Ala
                85                  90                  95

Pro Trp Thr Arg Phe Tyr Ser Asn Ser Cys Cys Leu Cys Cys His Val
            100                 105                 110

Arg Thr Gly Thr Ile Leu Leu Gly Val Trp Tyr Leu Ile Ile Asn Ala
        115                 120                 125

Val Val Leu Leu Ile Leu Leu Ser Ala Leu Ala Asp Pro Asp Gln Tyr
    130                 135                 140

Asn Phe Ser Ser Ser Glu Leu Gly Gly Asp Phe Glu Phe Met Asp Asp
145                 150                 155                 160

Ala Asn Met Cys Ile Ala Ile Ala Ile Ser Leu Leu Met Ile Leu Ile
                165                 170                 175

Cys Ala Met Ala Thr Tyr Gly Ala Tyr Lys Gln Arg Ala Ala Trp Ile
            180                 185                 190

Ile Pro Phe Phe Cys Tyr Gln Ile Phe Asp Phe Ala Leu Asn Met Leu
        195                 200                 205

Val Ala Ile Thr Val Leu Ile Tyr Pro Asn Ser Ile Gln Glu Tyr Ile
    210                 215                 220

Arg Gln Leu Pro Pro Asn Phe Pro Tyr Arg Asp Asp Val Met Ser Val
225                 230                 235                 240

Asn Pro Thr Cys Leu Val Leu Ile Ile Leu Leu Phe Ile Ser Ile Ile
                245                 250                 255

Leu Thr Phe Lys Gly Tyr Leu Ile Ser Cys Val Trp Asn Cys Tyr Arg
            260                 265                 270

Tyr Ile Asn Gly Arg Asn Ser Ser Asp Val Leu Val Tyr Val Thr Ser
        275                 280                 285

Asn Asp Thr Thr Val Leu Leu Pro Pro Tyr Asp Asp Ala Thr Val Asn
    290                 295                 300

Gly Ala Ala Lys Glu Pro Pro Pro Pro Tyr Val Ser Ala
305                 310                 315

<210>  62
<211>  1453
<212>  DNA
<213>  Homo sapiens

<220>

<221>  CDS
<222>  (65)..(1057)
<223>

<400>  62
   ggctgccgga gggcgggagg caggagcggg ccaggagctg ctgggctgga gcggcggcgc      60
   cgcc atg tcc gac agc gag aag ctc aac ctg gac tcg atc atc ggg cgc      109
        Met Ser Asp Ser Glu Lys Leu Asn Leu Asp Ser Ile Ile Gly Arg
        1               5                   10                  15
   ctg ctg gaa gtg cag ggc tcg cgg cct ggc aag aat gta cag ctg aca      157
   Leu Leu Glu Val Gln Gly Ser Arg Pro Gly Lys Asn Val Gln Leu Thr
                   20                  25                  30
   gag aac gag atc cgc ggt ctg tgc ctg aaa tcc cgg gag att ttt ctg      205
   Glu Asn Glu Ile Arg Gly Leu Cys Leu Lys Ser Arg Glu Ile Phe Leu
               35                  40                  45
   agc cag ccc att ctt ctg gag ctg gag gca ccc ctc aag atc tgc ggt      253
   Ser Gln Pro Ile Leu Leu Glu Leu Glu Ala Pro Leu Lys Ile Cys Gly
           50                  55                  60
   gac ata cac ggc cag tac tac gac ctt ctg cga cta ttt gag tat ggc      301
   Asp Ile His Gly Gln Tyr Tyr Asp Leu Leu Arg Leu Phe Glu Tyr Gly
       65                  70                  75
   ggt ttc cct ccc gag agc aac tac ctc ttt ctg ggg gac tat gtg gac      349
   Gly Phe Pro Pro Glu Ser Asn Tyr Leu Phe Leu Gly Asp Tyr Val Asp
   80                  85                  90                  95
   agg ggc aag cag tcc ttg gag acc atc tgc ctg ctg ctg gcc tat aag      397
   Arg Gly Lys Gln Ser Leu Glu Thr Ile Cys Leu Leu Leu Ala Tyr Lys
                   100                 105                 110
   atc aag tac ccc gag aac ttc ttc ctg ctc cgt ggg aac cac gag tgt      445
   Ile Lys Tyr Pro Glu Asn Phe Phe Leu Leu Arg Gly Asn His Glu Cys
               115                 120                 125
   gcc agc atc aac cgc atc tat ggt ttc tac gat gag tgc aag aga cgc      493
   Ala Ser Ile Asn Arg Ile Tyr Gly Phe Tyr Asp Glu Cys Lys Arg Arg
           130                 135                 140
   tac aac atc aaa ctg tgg aaa acc ttc act gac tgc ttc aac tgc ctg      541
   Tyr Asn Ile Lys Leu Trp Lys Thr Phe Thr Asp Cys Phe Asn Cys Leu
       145                 150                 155
   ccc atc gcg gcc ata gtg gac gaa aag atc ttc tgc tgc cac gga ggc      589
   Pro Ile Ala Ala Ile Val Asp Glu Lys Ile Phe Cys Cys His Gly Gly
   160                 165                 170                 175
   ctg tcc ccg gac ctg cag tct atg gag cag att cgg cgg atc atg cgg 637
   Leu Ser Pro Asp Leu Gln Ser Met Glu Gln Ile Arg Arg Ile Met Arg
   180 185 190
   ccc aca gat gtg cct gac cag ggc ctg ctg tgt gac ctg ctg tgg tct 685
   Pro Thr Asp Val Pro Asp Gln Gly Leu Leu Cys Asp Leu Leu Trp Ser
   195 200 205
   gac cct gac aag gac gtg cag ggc tgg ggc gag aac gac cgt ggc gtc 733
   Asp Pro Asp Lys Asp Val Gln Gly Trp Gly Glu Asn Asp Arg Gly Val
   210 215 220
   tct ttt acc ttt gga gcc gag gtg gtg gcc aag ttc ctc cac aag cac 781
   Ser Phe Thr Phe Gly Ala Glu Val Val Ala Lys Phe Leu His Lys His
   225 230 235
   gac ttg gac ctc atc tgc cga gca cac cag gtg gta gaa gac ggc tac 829
   Asp Leu Asp Leu Ile Cys Arg Ala His Gln Val Val Glu Asp Gly Tyr
   240 245 250 255
   gag ttc ttt gcc aag cgg cag ctg gtg aca ctt ttc tca gct ccc aac 877
   Glu Phe Phe Ala Lys Arg Gln Leu Val Thr Leu Phe Ser Ala Pro Asn
   260 265 270
   tac tgt ggc gag ttt gac aat gct ggc gcc atg atg agt gtg gac gag 925
   Tyr Cys Gly Glu Phe Asp Asn Ala Gly Ala Met Met Ser Val Asp Glu
   275 280 285
   acc ctc atg tgc tct ttc cag atc ctc aag ccc gcc gac aag aac aag 973
   Thr Leu Met Cys Ser Phe Gln Ile Leu Lys Pro Ala Asp Lys Asn Lys
   290 295 300
   ggg aag tac ggg cag ttc agt ggc ctg aac cct gga ggc cga ccc atc 1021
   Gly Lys Tyr Gly Gln Phe Ser Gly Leu Asn Pro Gly Gly Arg Pro Ile
   305 310 315
   acc cca ccc cgc aat tcc gcc aaa gcc aag aaa tag cccccgcaca 1067
   Thr Pro Pro Arg Asn Ser Ala Lys Ala Lys Lys
   320 325 330
   ccaccctgtg ccccagatga tggattgatt gtacagaaat catgctgcca tgctgggggg 1127
   gggtcacccc gacccctcag gcccacctgt cacggggaac atggagcctt ggtgtatttt 1187
   tcttttcttt ttttaatgaa tcaatagcag cgtccagtcc cccagggctg cttcctgcct 1247
   gcacctgcgg tgactgtgag caggatcctg gggccgaggc tgcagctcag ggcaacggca 1307
   ggccaggtcg tgggtctcca gccgtgcttg gcctcagggc tggcagccgg atcctggggc 1367
   aacccatctg gtctcttgaa taaaggtcaa agctggattc tcaaaaaaaa aaaaaaaaaa 1427
   aaaaaaaaaa aaaaaaaaaa aaaaaa 1453

<210> 63
<211> 330
<212> PRT
<213> Homo sapiens

<400> 63

Met Ser Asp Ser Glu Lys Leu Asn Leu Asp Ser Ile Ile Gly Arg Leu
1               5                   10                  15

Leu Glu Val Gln Gly Ser Arg Pro Gly Lys Asn Val Gln Leu Thr Glu
            20                  25                  30

Asn Glu Ile Arg Gly Leu Cys Leu Lys Ser Arg Glu Ile Phe Leu Ser
        35                  40                  45

Gln Pro Ile Leu Leu Glu Leu Glu Ala Pro Leu Lys Ile Cys Gly Asp
    50                  55                  60

Ile His Gly Gln Tyr Tyr Asp Leu Leu Arg Leu Phe Glu Tyr Gly Gly
65                  70                  75                  80

Phe Pro Pro Glu Ser Asn Tyr Leu Phe Leu Gly Asp Tyr Val Asp Arg
                85                  90                  95

Gly Lys Gln Ser Leu Glu Thr Ile Cys Leu Leu Leu Ala Tyr Lys Ile
            100                 105                 110

Lys Tyr Pro Glu Asn Phe Phe Leu Leu Arg Gly Asn His Glu Cys Ala
        115                 120                 125

Ser Ile Asn Arg Ile Tyr Gly Phe Tyr Asp Glu Cys Lys Arg Arg Tyr
    130                 135                 140

Asn Ile Lys Leu Trp Lys Thr Phe Thr Asp Cys Phe Asn Cys Leu Pro

145                 150                 155                 160

Ile Ala Ala Ile Val Asp Glu Lys Ile Phe Cys Cys His Gly Gly Leu
                165                 170                 175

Ser Pro Asp Leu Gln Ser Met Glu Gln Ile Arg Arg Ile Met Arg Pro
            180                 185                 190

Thr Asp Val Pro Asp Gln Gly Leu Leu Cys Asp Leu Leu Trp Ser Asp
        195                 200                 205

Pro Asp Lys Asp Val Gln Gly Trp Gly Glu Asn Asp Arg Gly Val Ser
    210                 215                 220

Phe Thr Phe Gly Ala Glu Val Val Ala Lys Phe Leu His Lys His Asp
225                 230                 235                 240

Leu Asp Leu Ile Cys Arg Ala His Gln Val Val Glu Asp Gly Tyr Glu
                245                 250                 255

Phe Phe Ala Lys Arg Gln Leu Val Thr Leu Phe Ser Ala Pro Asn Tyr
            260                 265                 270

Cys Gly Glu Phe Asp Asn Ala Gly Ala Met Met Ser Val Asp Glu Thr
        275                 280                 285

Leu Met Cys Ser Phe Gln Ile Leu Lys Pro Ala Asp Lys Asn Lys Gly
    290                 295                 300

Lys Tyr Gly Gln Phe Ser Gly Leu Asn Pro Gly Gly Arg Pro Ile Thr
305                 310                 315                 320

Pro Pro Arg Asn Ser Ala Lys Ala Lys Lys
                325                 330

<210>  64
<211>  1591
<212>  DNA

<213>  Homo sapiens

<220>
<221>  CDS
<222>  (44)..(814)
<223>

<400>  64
ccctgcgtct ctgcccgccc cgtggcgccc gagtgcactg aag atg gcg gct gct      55
                                                Met Ala Ala Ala
                                                1

gta gga cgg ttg ctc cga gcg tcg gtt gcc cga cat gtg agt gcc att      103
Val Gly Arg Leu Leu Arg Ala Ser Val Ala Arg His Val Ser Ala Ile
5                   10                  15                  20

cct tgg ggc att tct gcc act gca gcc ctc agg cct gct gca tgt gga      151
Pro Trp Gly Ile Ser Ala Thr Ala Ala Leu Arg Pro Ala Ala Cys Gly
                25                  30                  35

aga acg agc ttg aca aat tta ttg tgt tct ggt tcc agt caa gca aaa      199
Arg Thr Ser Leu Thr Asn Leu Leu Cys Ser Gly Ser Ser Gln Ala Lys
            40                  45                  50

tta ttc agc acc agt tcc tca tgc cat gca cct gct gtc acc cag cat      247
Leu Phe Ser Thr Ser Ser Ser Cys His Ala Pro Ala Val Thr Gln His
        55                  60                  65

gca ccc tat ttt aag ggt aca gcc gtt gtc aat gga gag ttc aaa gac      295
Ala Pro Tyr Phe Lys Gly Thr Ala Val Val Asn Gly Glu Phe Lys Asp
    70                  75                  80

cta agc ctt gat gac ttt aag ggg aaa tat ttg gtg ctt ttc ttc tat      343
Leu Ser Leu Asp Asp Phe Lys Gly Lys Tyr Leu Val Leu Phe Phe Tyr
85                  90                  95                  100

cct ttg gat ttc acc ttt gtg tgt cct aca gaa att gtt gct ttt agt      391
Pro Leu Asp Phe Thr Phe Val Cys Pro Thr Glu Ile Val Ala Phe Ser
                105                 110                 115

gac aaa gct aac gaa ttt cac gac gtg aac tgt gaa gtt gtc gca gtc      439
Asp Lys Ala Asn Glu Phe His Asp Val Asn Cys Glu Val Val Ala Val
            120                 125                 130

tca gtg gat tcc cac ttt agc cat ctt gcc tgg ata aat aca cca agg      487
Ser Val Asp Ser His Phe Ser His Leu Ala Trp Ile Asn Thr Pro Arg
        135                 140                 145

aag aat ggt ggt ttg ggc cac atg aac atc gca ctc ttg tca gac tta      535
Lys Asn Gly Gly Leu Gly His Met Asn Ile Ala Leu Leu Ser Asp Leu

150                 155                 160

act aag cag att tcc cga gac tac ggt gtg ctg tta gaa ggt tct ggt     583
Thr Lys Gln Ile Ser Arg Asp Tyr Gly Val Leu Leu Glu Gly Ser Gly
165                 170                 175                 180

ctt gca cta aga ggt ctc ttc ata att gac ccc aat gga gtc atc aag     631
Leu Ala Leu Arg Gly Leu Phe Ile Ile Asp Pro Asn Gly Val Ile Lys
                185                 190                 195

cat ttg agc gtc aac gat ctc cca gtg ggc cga agc gtg gaa gaa acc     679
His Leu Ser Val Asn Asp Leu Pro Val Gly Arg Ser Val Glu Glu Thr
            200                 205                 210

ctc cgc ttg gtg aag gcg ttc cag tat gta gaa aca cat gga gaa gtc     727
Leu Arg Leu Val Lys Ala Phe Gln Tyr Val Glu Thr His Gly Glu Val
        215                 220                 225

tgc cca gcg aac tgg aca ccg gat tct cct acg atc aag cca agt cca     775
Cys Pro Ala Asn Trp Thr Pro Asp Ser Pro Thr Ile Lys Pro Ser Pro
    230                 235                 240

gct gct tcc aaa gag tac ttt cag aag gta aat cag tag atcacccatg     824
Ala Ala Ser Lys Glu Tyr Phe Gln Lys Val Asn Gln
245                 250                 255

tgtatctgca ccttctcaac tgagagaaga accacagttg aaacctgctt ttatcatttt     884

caagatggtt atttgtagaa ggcaaggaac caattatgct tgtattcata agtattactc     944

taaatgtttt gtttttgtaa ttctggctaa gaccttttaa acatggttag ttgctagtac     1004

aaggaatcct ttattggtaa catcttggtg gctggctagc tagtttctac agaacataat     1064

ttgcctctat agaaggctat tcttagatca tgtctcaatg gaaacactct tctttcttag     1124

ccttacttga atcttgccta taataaagta gagcaacaca cattgaaagc ttctgatcaa     1184

cggtcctgaa attttcatct tgaatgtctt tgtattaaac tgaattttct tttaagctaa     1244

caaagatcat aattttcaat gattagccgt gtaactcctg caatgaatgt ttatgtgatt     1304

gaagcaaatg tgaatcgtat tattttaaaa agtggcagag tgacttaact gatcatgcat     1364

gatccctcat ccctgaaatt gagtttatgt agtcatttta cttattttat tcattagcta     1424

actttgtcta tgtatatttc tagatattga ttagtgtaat cgattataaa ggatatttat     1484

caaatccagg gattgcattt tgaaattata attattttct ttgctgaagt attcattgta     1544

aaacatacaa aataaacata ttttaaaaca tttgcatttt accacca 1591

<210> 65
<211> 256
<212> PRT
<213> Homo sapiens

<400> 65

Met Ala Ala Ala Val Gly Arg Leu Leu Arg Ala Ser Val Ala Arg His
1               5                   10                  15

Val Ser Ala Ile Pro Trp Gly Ile Ser Ala Thr Ala Ala Leu Arg Pro
            20                  25                  30

Ala Ala Cys Gly Arg Thr Ser Leu Thr Asn Leu Leu Cys Ser Gly Ser
        35                  40                  45

Ser Gln Ala Lys Leu Phe Ser Thr Ser Ser Ser Cys His Ala Pro Ala
    50                  55                  60

Val Thr Gln His Ala Pro Tyr Phe Lys Gly Thr Ala Val Val Asn Gly
65                  70                  75                  80

Glu Phe Lys Asp Leu Ser Leu Asp Asp Phe Lys Gly Lys Tyr Leu Val
                85                  90                  95

Leu Phe Phe Tyr Pro Leu Asp Phe Thr Phe Val Cys Pro Thr Glu Ile
            100                 105                 110

Val Ala Phe Ser Asp Lys Ala Asn Glu Phe His Asp Val Asn Cys Glu
        115                 120                 125

Val Val Ala Val Ser Val Asp Ser His Phe Ser His Leu Ala Trp Ile
    130                 135                 140

Asn Thr Pro Arg Lys Asn Gly Gly Leu Gly His Met Asn Ile Ala Leu
145                 150                 155                 160

Leu Ser Asp Leu Thr Lys Gln Ile Ser Arg Asp Tyr Gly Val Leu Leu
                165                 170                 175

Glu Gly Ser Gly Leu Ala Leu Arg Gly Leu Phe Ile Ile Asp Pro Asn
            180                 185                 190

Gly Val Ile Lys His Leu Ser Val Asn Asp Leu Pro Val Gly Arg Ser
        195                 200                 205

Val Glu Glu Thr Leu Arg Leu Val Lys Ala Phe Gln Tyr Val Glu Thr
    210                 215                 220

His Gly Glu Val Cys Pro Ala Asn Trp Thr Pro Asp Ser Pro Thr Ile
225                 230                 235                 240

Lys Pro Ser Pro Ala Ala Ser Lys Glu Tyr Phe Gln Lys Val Asn Gln
                245                 250                 255

<210>  66
<211>  1224
<212>  DNA
<213>  Homo sapiens

<220>
<221>  CDS
<222>  (70)..(1041)
<223>

<400>  66
gcccattgtt tttgtaatct ctgaggagaa gcagcagcaa acatttgcta gtcagacaag      60

tgacaggga atg gat tcc aaa cac cag tgt gta aag cta aat gat ggc cac      111
          Met Asp Ser Lys His Gln Cys Val Lys Leu Asn Asp Gly His
          1               5                   10

ttc atg cct gta ttg gga ttt ggc acc tat gca cct cca gag gtt ccg      159
Phe Met Pro Val Leu Gly Phe Gly Thr Tyr Ala Pro Pro Glu Val Pro
15                  20                  25                  30

aga agt aaa gct ttg gag gtc aca aaa tta gca ata gaa gct ggg ttc      207
Arg Ser Lys Ala Leu Glu Val Thr Lys Leu Ala Ile Glu Ala Gly Phe
                35                  40                  45

cgc cat ata gat tct gct cat tta tac aat aat gag gag cag gtt gga      255

Arg His Ile Asp Ser Ala His Leu Tyr Asn Asn Glu Glu Gln Val Gly
            50                  55                  60

ctg gcc atc cga agc aag att gca gat ggc agt gtg aag aga gaa gac 303
Leu Ala Ile Arg Ser Lys Ile Ala Asp Gly Ser Val Lys Arg Glu Asp
        65                  70                  75

ata ttc tac act tca aag ctt tgg tcc act ttt cat cga cca gag ttg 351
Ile Phe Tyr Thr Ser Lys Leu Trp Ser Thr Phe His Arg Pro Glu Leu
    80                  85                  90

gtc cga cca gcc ttg gaa aac tca ctg aag aaa gct caa ttg gac tat 399
Val Arg Pro Ala Leu Glu Asn Ser Leu Lys Lys Ala Gln Leu Asp Tyr
95                  100                 105                 110

gtt gac ctc tat ctt att cat tct cca atg tct cta aag cca ggt gag 447
Val Asp Leu Tyr Leu Ile His Ser Pro Met Ser Leu Lys Pro Gly Glu
                115                 120                 125

gaa ctt tca cca aca gat gaa aat gga aaa gta ata ttt gac ata gtg 495
Glu Leu Ser Pro Thr Asp Glu Asn Gly Lys Val Ile Phe Asp Ile Val
            130                 135                 140

gat ctc tgt acc acc tgg gag gcc atg gag aag tgt aag gat gca gga 543
Asp Leu Cys Thr Thr Trp Glu Ala Met Glu Lys Cys Lys Asp Ala Gly
        145                 150                 155

ttg gcc aag tcc att ggg gtg tca aac ttc aac cgc agg cag ctg gag 591
Leu Ala Lys Ser Ile Gly Val Ser Asn Phe Asn Arg Arg Gln Leu Glu
    160                 165                 170

atg atc ctc aac aag cca gga ctc aag tac aag cct gtc tgc aac cag 639
Met Ile Leu Asn Lys Pro Gly Leu Lys Tyr Lys Pro Val Cys Asn Gln
175                 180                 185                 190

gta gaa tgt cat ccg tat ttc aac cgg agt aaa ttg cta gat ttc tgc 687
Val Glu Cys His Pro Tyr Phe Asn Arg Ser Lys Leu Leu Asp Phe Cys
                195                 200                 205

aag tcg aaa gat att gtt ctg gtt gcc tat agt gct ctg gga tct caa 735
Lys Ser Lys Asp Ile Val Leu Val Ala Tyr Ser Ala Leu Gly Ser Gln
            210                 215                 220

cga gac aaa cga tgg gtg gac ccg aac tcc ccg gtg ctc ttg gag gac 783
Arg Asp Lys Arg Trp Val Asp Pro Asn Ser Pro Val Leu Leu Glu Asp
        225                 230                 235

cca gtc ctt tgt gcc ttg gca aaa aag cac aag cga acc cca gcc ctg 831
Pro Val Leu Cys Ala Leu Ala Lys Lys His Lys Arg Thr Pro Ala Leu
    240                 245                 250

att gcc ctg cgc tac cag ctg cag cgt ggg gtt gtg gtc ctg gcc aag          879
Ile Ala Leu Arg Tyr Gln Leu Gln Arg Gly Val Val Val Leu Ala Lys
255                 260                 265                 270

agc tac aat gag cag cgc atc aga cag aac gtg cag gtt ttt gag ttc          927
Ser Tyr Asn Glu Gln Arg Ile Arg Gln Asn Val Gln Val Phe Glu Phe
                275                 280                 285

cag ttg act gca gag gac atg aaa gcc ata gat ggc cta gac aga aat          975
Gln Leu Thr Ala Glu Asp Met Lys Ala Ile Asp Gly Leu Asp Arg Asn
            290                 295                 300

ctc cac tat ttt aac agt gat agt ttt gct agc cac cct aat tat cca          1023
Leu His Tyr Phe Asn Ser Asp Ser Phe Ala Ser His Pro Asn Tyr Pro
        305                 310                 315

tat tca gat gaa tat taa catggagggc tttgcctgat gtctaccaga                 1071
Tyr Ser Asp Glu Tyr
    320

agccctgtgt gtggatggtg acgcagagga cgtctctatg ccggtgactg gacatatcac        1131

ctctacttaa atccgtcctg tttagcgact tcagtcaact acagctgagt ccataggcca        1191

gaaagacaat aaatttttat cattttgaaa taa                                     1224

<210>  67
<211>  323
<212>  PRT
<213>  Homo sapiens

<400>  67

Met Asp Ser Lys His Gln Cys Val Lys Leu Asn Asp Gly His Phe Met
1               5                   10                  15

Pro Val Leu Gly Phe Gly Thr Tyr Ala Pro Pro Glu Val Pro Arg Ser
            20                  25                  30

Lys Ala Leu Glu Val Thr Lys Leu Ala Ile Glu Ala Gly Phe Arg His
        35                  40                  45

Ile Asp Ser Ala His Leu Tyr Asn Asn Glu Glu Gln Val Gly Leu Ala
    50                  55                  60

Ile Arg Ser Lys Ile Ala Asp Gly Ser Val Lys Arg Glu Asp Ile Phe
65                  70                  75                  80

Tyr Thr Ser Lys Leu Trp Ser Thr Phe His Arg Pro Glu Leu Val Arg
                85                  90                  95

Pro Ala Leu Glu Asn Ser Leu Lys Lys Ala Gln Leu Asp Tyr Val Asp
            100                 105                 110

Leu Tyr Leu Ile His Ser Pro Met Ser Leu Lys Pro Gly Glu Glu Leu
        115                 120                 125

Ser Pro Thr Asp Glu Asn Gly Lys Val Ile Phe Asp Ile Val Asp Leu
    130                 135                 140

Cys Thr Thr Trp Glu Ala Met Glu Lys Cys Lys Asp Ala Gly Leu Ala
145                 150                 155                 160

Lys Ser Ile Gly Val Ser Asn Phe Asn Arg Arg Gln Leu Glu Met Ile
                165                 170                 175

Leu Asn Lys Pro Gly Leu Lys Tyr Lys Pro Val Cys Asn Gln Val Glu
            180                 185                 190

Cys His Pro Tyr Phe Asn Arg Ser Lys Leu Leu Asp Phe Cys Lys Ser
        195                 200                 205

Lys Asp Ile Val Leu Val Ala Tyr Ser Ala Leu Gly Ser Gln Arg Asp
    210                 215                 220

Lys Arg Trp Val Asp Pro Asn Ser Pro Val Leu Leu Glu Asp Pro Val
225                 230                 235                 240

Leu Cys Ala Leu Ala Lys Lys His Lys Arg Thr Pro Ala Leu Ile Ala
                245                 250                 255

Leu Arg Tyr Gln Leu Gln Arg Gly Val Val Val Leu Ala Lys Ser Tyr

260                 265                 270

Asn Glu Gln Arg Ile Arg Gln Asn Val Gln Val Phe Glu Phe Gln Leu
        275                 280                 285

Thr Ala Glu Asp Met Lys Ala Ile Asp Gly Leu Asp Arg Asn Leu His
    290                 295                 300

Tyr Phe Asn Ser Asp Ser Phe Ala Ser His Pro Asn Tyr Pro Tyr Ser
305                 310                 315                 320

Asp Glu Tyr

<210>  68
<211>  1715
<212>  DNA
<213>  Homo sapiens

<220>
<221>  CDS
<222>  (37)..(591)
<223>

<400>  68
ccgtgctctg cctcctgtgt tccagggact ttgaac atg tcg ggg atc gcc ctc      54
                                        Met Ser Gly Ile Ala Leu
                                        1               5

agc aga ctc gcc cag gag agg aaa gca tgg agg aaa gac cac cca ttt      102
Ser Arg Leu Ala Gln Glu Arg Lys Ala Trp Arg Lys Asp His Pro Phe
            10                  15                  20

ggt ttc gtg gct gtc cca aca aaa aat ccc gat ggc acg atg aac ctc      150
Gly Phe Val Ala Val Pro Thr Lys Asn Pro Asp Gly Thr Met Asn Leu
        25                  30                  35

atg aac tgg gag tgc gcc att cca gga aag aaa ggg act ccg tgg gaa      198
Met Asn Trp Glu Cys Ala Ile Pro Gly Lys Lys Gly Thr Pro Trp Glu
    40                  45                  50

gga ggc ttg ttt aaa cta cgg atg ctt ttc aaa gat gat tat cca tct      246
Gly Gly Leu Phe Lys Leu Arg Met Leu Phe Lys Asp Asp Tyr Pro Ser
55                  60                  65                  70

tcg cca cca aaa tgt aaa ttc gaa cca cca tta ttt cac ccg aat gtg 294
Ser Pro Pro Lys Cys Lys Phe Glu Pro Pro Leu Phe His Pro Asn Val
                75                  80                  85

tac cct tcg ggg aca gtg tgc ctg tcc atc tta gag gag gac aag gac 342
Tyr Pro Ser Gly Thr Val Cys Leu Ser Ile Leu Glu Glu Asp Lys Asp
            90                  95                  100

tgg agg cca gcc atc aca atc aaa cag atc cta tta gga ata cag gaa 390
Trp Arg Pro Ala Ile Thr Ile Lys Gln Ile Leu Leu Gly Ile Gln Glu
        105                 110                 115

ctt cta aat gaa cca aat atc caa gac cca gct caa gca gag gcc tac 438
Leu Leu Asn Glu Pro Asn Ile Gln Asp Pro Ala Gln Ala Glu Ala Tyr
    120                 125                 130

acg att tac tgg tta gta gca gcc ctg gcc ccg ctg gtg gca gct cct 486
Thr Ile Tyr Trp Leu Val Ala Ala Leu Ala Pro Leu Val Ala Ala Pro
135                 140                 145                 150

ccc cgt ccc agc caa ggc cgc ctg gca gga cgg gag tgg agc aca cag 534
Pro Arg Pro Ser Gln Gly Arg Leu Ala Gly Arg Glu Trp Ser Thr Gln
                155                 160                 165

gct cac cct agg gac agc cag ggt ccg cgc ctc tgt ggg gaa ggt cgg 582
Ala His Pro Arg Asp Ser Gln Gly Pro Arg Leu Cys Gly Glu Gly Arg
            170                 175                 180

ggg gca taa accctgtggg cagcaggcac cgttgcacac cgtctgcggt 631
Gly Ala

gggcatggat ggcgccggag ccgtgtccag gggagggagc aggccaggtg acaggggctg 691

tcttgggcca ctgccccagt gagttgtggc cagctaggag gggaaggacc ctgggcgtgg 751

gtgccagagg aggccatgct gagaggcctc tccaccggct cagcctcccg tggaccccag 811

cctcccgcgc cgcccttgct gcttgtcccc atcccctcta caacttggtt ccttctgtgg 871

gggggcccag cccagcaggg ctcagtgacg tgatgctgtc acacggaccc tgggcttcca 931

ccctgtccct gtgaggtgct gctgtggcta gaagggtccc cgtgggcctg gggtcctgtc 991

tgcaccgagg agggcctggt gtgagttagg atgcagacga cagccaaaca gtgctgggga 1051

aagcagggag gaccctgccc tgagcagagg ccatgcgccc gcctgtcctg ttccctctgc 1111

agctgttctg ggcaggtcca taagaaggtg ggttggggcc tccgaccccc actgctccgc 1171

ctgcagggtt gagaaaccca gccccatctc accacgggtc cccacacctc tgaggaggtc 1231

tcagggcccc caatggccag ggacccagaa ggctcacctg cagccacccc acacatccct 1291

gccactgtgg gcatcagagg ccctctccct ccagggtgca tctcctggtc aggagggggg 1351

aggcccggtt gccttaggcc cggcctgcac aggtcgtgag gaaaagcacc tggtttcggc 1411

cgggcgcggt ggctcacgct tgtaatccca gcactttggg aggccgaggt gggcgaatca 1471

tgcggtcagg agttcgagac cagcctggcc aacatggcga aaccctcgtc cctactaaaa 1531

atacaaaaaa ttagttgggc gtagtggtgg gtgcctctaa tcccagctac ctgggaggct 1591

gagacaggag aatcacttga acccgggagg cggaggttgc agtgagccga gatcctgcca 1651

ctgctctcca gcctggcgac ggaggaatgc tgtctcaaaa aaaaaaaaaa aaaaaaaaaa 1711

aaaa 1715

<210> 69
<211> 184
<212> PRT
<213> Homo sapiens

<400> 69

Met Ser Gly Ile Ala Leu Ser Arg Leu Ala Gln Glu Arg Lys Ala Trp
1               5                   10                  15

Arg Lys Asp His Pro Phe Gly Phe Val Ala Val Pro Thr Lys Asn Pro
            20                  25                  30

Asp Gly Thr Met Asn Leu Met Asn Trp Glu Cys Ala Ile Pro Gly Lys
        35                  40                  45

Lys Gly Thr Pro Trp Glu Gly Gly Leu Phe Lys Leu Arg Met Leu Phe
    50                  55                  60

Lys Asp Asp Tyr Pro Ser Ser Pro Pro Lys Cys Lys Phe Glu Pro Pro
65                  70                  75                  80

Leu Phe His Pro Asn Val Tyr Pro Ser Gly Thr Val Cys Leu Ser Ile
                85                  90                  95

Leu Glu Glu Asp Lys Asp Trp Arg Pro Ala Ile Thr Ile Lys Gln Ile
            100                 105                 110

Leu Leu Gly Ile Gln Glu Leu Leu Asn Glu Pro Asn Ile Gln Asp Pro
        115                 120                 125

Ala Gln Ala Glu Ala Tyr Thr Ile Tyr Trp Leu Val Ala Ala Leu Ala
    130                 135                 140

Pro Leu Val Ala Ala Pro Pro Arg Pro Ser Gln Gly Arg Leu Ala Gly
145                 150                 155                 160

Arg Glu Trp Ser Thr Gln Ala His Pro Arg Asp Ser Gln Gly Pro Arg
                165                 170                 175

Leu Cys Gly Glu Gly Arg Gly Ala
            180

<210> 70
<211> 1327
<212> DNA
<213> Homo sapiens

<220>
<221> CDS
<222> (100)..(966)
<223>

<400> 70
ctcctctccg cgcggggcgg gctccgcgcc acgtgactcc gcggccgggc cgggacgcga 60

cgggacgcgc tgggaccggc gtcgggggtc gcggggacc atg cag cgg agg tgg 114
Met Gln Arg Arg Trp
1               5

gtc ttc gtg ctg ctc gac gtg ctg tgc tta ctg gtc gcc tcc ctg ccc 162
Val Phe Val Leu Leu Asp Val Leu Cys Leu Leu Val Ala Ser Leu Pro
                10                  15                  20

ttc gct atc ctg acg ctg gtg aac gcc ccg tac aag cga gga ttt tac 210
Phe Ala Ile Leu Thr Leu Val Asn Ala Pro Tyr Lys Arg Gly Phe Tyr
            25                  30                  35

tgc ggg gat gac tcc atc cgg tac ccc tac cgt cca gat acc atc acc 258
Cys Gly Asp Asp Ser Ile Arg Tyr Pro Tyr Arg Pro Asp Thr Ile Thr
        40                  45                  50

cac ggg ctc atg gct ggg gtc acc atc acg gcc acc gtc atc ctt gtc 306
His Gly Leu Met Ala Gly Val Thr Ile Thr Ala Thr Val Ile Leu Val
    55                  60                  65

tcg gcc ggg gaa gcc tac ctg gtg tac aca gac cgg ctc tat tct cgc 354
Ser Ala Gly Glu Ala Tyr Leu Val Tyr Thr Asp Arg Leu Tyr Ser Arg
70                  75                  80                  85

tcg gac ttc aac aac tac gtg gct gct gta tac aag gtg ctg ggg acc 402
Ser Asp Phe Asn Asn Tyr Val Ala Ala Val Tyr Lys Val Leu Gly Thr
                90                  95                  100

ttc ctg ttt ggg gct gcc gtg agc cag tct ctg aca gac ctg gcc aag 450
Phe Leu Phe Gly Ala Ala Val Ser Gln Ser Leu Thr Asp Leu Ala Lys
            105                 110                 115

tac atg att ggg cgt ctg agg ccc aac ttc cta gcc gtc tgc gac ccc 498
Tyr Met Ile Gly Arg Leu Arg Pro Asn Phe Leu Ala Val Cys Asp Pro
        120                 125                 130

gac tgg agc cgg gtc aac tgc tcg gtc tat gtg cag ctg gag aag gtg 546
Asp Trp Ser Arg Val Asn Cys Ser Val Tyr Val Gln Leu Glu Lys Val
    135                 140                 145

tgc agg gga aac cct gct gat gtc acc gag gcc agg ttg tct ttc tac 594
Cys Arg Gly Asn Pro Ala Asp Val Thr Glu Ala Arg Leu Ser Phe Tyr
150                 155                 160                 165

tcg gga cac tct tcc ttt ggg atg tac tgc atg gtg ttc ttg gcg ctg 642
Ser Gly His Ser Ser Phe Gly Met Tyr Cys Met Val Phe Leu Ala Leu
                170                 175                 180

tat gtg cag gca cga ctc tgt tgg aag tgg gca cgg ctg ctg cga ccc 690
Tyr Val Gln Ala Arg Leu Cys Trp Lys Trp Ala Arg Leu Leu Arg Pro
            185                 190                 195

aca gtc cag ttc ttc ctg gtg gcc ttt gcc ctc tac gtg ggc tac acc 738
Thr Val Gln Phe Phe Leu Val Ala Phe Ala Leu Tyr Val Gly Tyr Thr
        200                 205                 210

cgc gtg tct gat tac aaa cac cac tgg agc gat gtc ctt gtt ggc ctc 786
Arg Val Ser Asp Tyr Lys His His Trp Ser Asp Val Leu Val Gly Leu
    215                 220                 225

ctg cag ggg gca ctg gtg gct gcc ctc act gtc tgc tac atc tca gac 834

Leu Gln Gly Ala Leu Val Ala Ala Leu Thr Val Cys Tyr Ile Ser Asp
230                 235                 240                 245

ttc ttc aaa gcc cga ccc cca cag cac tgt ctg aag gag gag gag ctg        882
Phe Phe Lys Ala Arg Pro Pro Gln His Cys Leu Lys Glu Glu Glu Leu
                250                 255                 260

gaa cgg aag ccc agc ctg tca ctg acg ttg acc ctg ggc gag gct gac        930
Glu Arg Lys Pro Ser Leu Ser Leu Thr Leu Thr Leu Gly Glu Ala Asp
            265                 270                 275

cac aac cac tat gga tac ccg cac tcc tcc tcc tga ggccggaccc             976
His Asn His Tyr Gly Tyr Pro His Ser Ser Ser
        280                 285

cgcccaggca gggagctgct gtgagtccag ctgaggccca cccaggtggt ccctccagcc    1036

ctggttaggc actgagggct ctggacgggc tccaggaacc ctgggctgat gggagcagtg    1096

agcgggctcc gctgccccct gccctgcact ggaccaggag tctggagatg cctgggtagc    1156

cctcagcatt tggaggggaa cctgttcccg tcggtcccca aatatcccct tctttttatg    1216

gggttaagga agggaccgag agatcagata gttgctgttt tgtaaaatgt aatgtatatg    1276

tggtttttag taaaataggg cacctgtttc acaaaaaaaa aaaaaaaaaa a             1327

<210>  71
<211>  288
<212>  PRT
<213>  Homo sapiens

<400>  71

Met Gln Arg Arg Trp Val Phe Val Leu Leu Asp Val Leu Cys Leu Leu
1               5                   10                  15

Val Ala Ser Leu Pro Phe Ala Ile Leu Thr Leu Val Asn Ala Pro Tyr
            20                  25                  30

Lys Arg Gly Phe Tyr Cys Gly Asp Asp Ser Ile Arg Tyr Pro Tyr Arg
        35                  40                  45

Pro Asp Thr Ile Thr His Gly Leu Met Ala Gly Val Thr Ile Thr Ala
    50                  55                  60

Thr Val Ile Leu Val Ser Ala Gly Glu Ala Tyr Leu Val Tyr Thr Asp
65                  70                  75                  80

Arg Leu Tyr Ser Arg Ser Asp Phe Asn Asn Tyr Val Ala Ala Val Tyr
                85                  90                  95

Lys Val Leu Gly Thr Phe Leu Phe Gly Ala Ala Val Ser Gln Ser Leu
            100                 105                 110

Thr Asp Leu Ala Lys Tyr Met Ile Gly Arg Leu Arg Pro Asn Phe Leu
        115                 120                 125

Ala Val Cys Asp Pro Asp Trp Ser Arg Val Asn Cys Ser Val Tyr Val
    130                 135                 140

Gln Leu Glu Lys Val Cys Arg Gly Asn Pro Ala Asp Val Thr Glu Ala
145                 150                 155                 160

Arg Leu Ser Phe Tyr Ser Gly His Ser Ser Phe Gly Met Tyr Cys Met
                165                 170                 175

Val Phe Leu Ala Leu Tyr Val Gln Ala Arg Leu Cys Trp Lys Trp Ala
            180                 185                 190

Arg Leu Leu Arg Pro Thr Val Gln Phe Phe Leu Val Ala Phe Ala Leu
        195                 200                 205

Tyr Val Gly Tyr Thr Arg Val Ser Asp Tyr Lys His His Trp Ser Asp
    210                 215                 220

Val Leu Val Gly Leu Leu Gln Gly Ala Leu Val Ala Ala Leu Thr Val
225                 230                 235                 240

Cys Tyr Ile Ser Asp Phe Phe Lys Ala Arg Pro Pro Gln His Cys Leu
                245                 250                 255

Lys Glu Glu Glu Leu Glu Arg Lys Pro Ser Leu Ser Leu Thr Leu Thr

260                 265                 270

Leu Gly Glu Ala Asp His Asn His Tyr Gly Tyr Pro His Ser Ser Ser
        275                 280                 285

<210>  72
<211>  2980
<212>  DNA
<213>  Homo sapiens

<220>
<221>  CDS
<222>  (274)..(519)
<223>

<220>
<221>  misc_feature
<222>  (2974)..(2974)
<223>  n = a, t, g or c

<400>  72
   ggccgctcct gctgctgcta ctgccgccgc cgcagcggct gctcgggctg agcacgcccc      60
   ggaacaggcc gccgcgcgct gcgcgccgga cccgctgccc ctgccggccc ggccgggtcg     120
   ggcggcccag ggaccgacag acttgacaac ggtgacagca ctggggcggc accttcctac     180
   ttctgcccag ccacagccct cccctcacag ttgagcacct gtttgcctga agttaatttc     240
   cagaagcagg agtccccaga gccaggcagg ggg atg aac cgc gag gga gct ccc      294
                                        Met Asn Arg Glu Gly Ala Pro
                                        1               5
   ggg aag agt ccg gag gag atg tac att cag cag aag gtc cga gtg ctg      342
   Gly Lys Ser Pro Glu Glu Met Tyr Ile Gln Gln Lys Val Arg Val Leu
           10                  15                  20
   ctc atg ctg cgg aag atg gga tca aac ctg aca gcc agc gag gag gag      390
   Leu Met Leu Arg Lys Met Gly Ser Asn Leu Thr Ala Ser Glu Glu Glu
       25                  30                  35
   ttc ctg cgc acc tat gca ggg gtg gtc aac agc cag ctc agc cag ctg      438
   Phe Leu Arg Thr Tyr Ala Gly Val Val Asn Ser Gln Leu Ser Gln Leu
   40                  45                  50                  55
   cct ccg cac tcc atc gac cag ggt gca gag gac gtg gtg atg gcg ttt      486
   Pro Pro His Ser Ile Asp Gln Gly Ala Glu Asp Val Val Met Ala Phe
   60 65 70
   tcc agg tcg gag acg gaa gac cgg agg cag tag ctgcaaagcc cttggaacac 539
   Ser Arg Ser Glu Thr Glu Asp Arg Arg Gln
   75 80
   cctggatgct gttgaagggc caagagatct gtgtggctcc tgggccggct gaatggcagc 599
   agcccccctt gccccacctc ccccttccct acccaaccct gccctgcccc accccacctc 659
   acagctactc agtggggctg gcatcaaggg agacaccagt ggtgcgttta taattggctt 719
   aaagggatgg acttgtgatt ggctgcagga agaaactttt ttatttttta aatcttgacc 779
   aacagaaacc ttttattttt atttctgact cttatttttt aaaaaatttg cgcctcggta 839
   tctggcttcc ctggaagctc tccgagctct ggtgctttag ttaggtcatt tttttagaaa 899
   tgtgaagagg tctgattggc tgcttaaact ggaaagggac tgtgattggc tggttaatgg 959
   gaaacggttt ttttctttgg ctgcaggtgt tctgctgata tcaacagctt ccctattttg 1019
   aatgcagaaa acagggtctg ggacattagt cgttatattt gacttgaaaa gaaagaaacc 1079
   aagtgcgctt tgcaatattt attacacaaa gaacttgctg ctgccttcac atttggggtt 1139
   tgtgtttgat tggctttcga tgcgtgtgtt tggtttccca ttggttcacc tgtgactcct 1199
   gttgccatgg attcaccccc ctctgctgcc ggctctgggc ctgagggtcc acctggagag 1259
   tacatttgct ttaatgagtg cacctgcctc caccagcaag gggaccccga gaaccctgag 1319
   cagggtccac agctggaaag ttgggcccct gaggagcttt gtgtcgtctt gaacgagcag 1379
   cccagggcct agaggtaacc gttaggcggg atttatgtgc actgcctgca tgagctggca 1439
   accagccagc gtcccttggt gagaaaggga ttgctgaggc accgtccagg ccccaccggc 1499
   caggccgcgc ccagcagagg cgtactaccc agctctgtcc tcttggccat ccttctgtgt 1559
   accacttcct gaggcctcat tttgggggtc atcttggaaa ggggaggagc ttctcccagt 1619
   gtgagacccc aaagactctg gaggtcatct ggcggaggtc tctgggagcc cagaacccac 1679
   ataaaagccc cagcttggct cacaaggccc aggagacctc cagctaaaca ccaacccctg 1739
   acctacccca gccaggctcc tacctgtctg ctgccagcac agtaggtccc ggccagctct 1799
   ggagttctct catcggaggc ccatgccctc cactccactg cctttggaag ggtctctctc 1859
   caggtcagcc tggaagggac agtatcgttt gtttatgaaa tgccactggg acagctggct 1919
   gggccttcac caagcaagtc ccttcagact ggcccttaag ccaaactcag gcccagaatt 1979
   gcagttcaga atggcagtcc tggaggcagg gggtgagggg caggtctagt gttcctgcac 2039
   caaacctaag tccttccacc tgccaccccc ttccctggga gggaggtggt cctcctatct 2099
   ccctggctca ctggcaggtg tgggatctgg ggagagcggc tggagaaaga tgcagtcctc 2159
   aggaaggggg ccgccaccct cccctatgct ggtagatgct gaggccccta ggtgcccagg 2219
   gccagtggga ccctctcaga accaaatctt tccccttctc cggggcttgg ggctcgggcc 2279
   gtaggggctc ctgagtgtca tgaagtgcac aggagccaaa tgaccgagcc ctggagagcc 2339
   ccatggtggg taggtggttc gtgctgtgct ctggcaccat cagcctgttc cagaaggagg 2399
   attcgagcat caggctaaga ccctgtgtcc tccaccatgc actcacccct agccctggtt 2459
   agctgacagt cagctgtggg gaacacagct acaaccctac cctggcaggg acctgagagc 2519
   atctcaggag gggcagcgca tgtgtgcatg tgctgtgtga gtgagcacac ccgtgtgcac 2579
   actcatacac atgtgcacac acacgcactc tccccgctca ggggcctgga ggtctggctg 2639
   agcccctggg gaaaggtgag ttctttcatc tccctcctcc aggtcggagt gcctggagtc 2699
   aggtgtcgag gccacattgc tggctgcccc ctctttgtag ctcctataaa gggcccacac 2759
   ctggtggata cctggttgag cgtgtggtct ctgccccagc ctgtccttgt cacgatcaca 2819
   ggccttgctt ttgtaacaat gatgaccccg gcctgtctca tcttctgaag aggaaaagtc 2879
   aaagtgttgc tgtggctcca tatttcaact aaaaatatat ctgttggaga aagaaattaa 2939
   caataaagaa ttttcatagg ttaaaaaaaa aaaanaaaaa g 2980

<210> 73
<211> 81
<212> PRT
<213> Homo sapiens

<400> 73

Met Asn Arg Glu Gly Ala Pro Gly Lys Ser Pro Glu Glu Met Tyr Ile
1               5                   10                  15

Gln Gln Lys Val Arg Val Leu Leu Met Leu Arg Lys Met Gly Ser Asn
            20                  25                  30

Leu Thr Ala Ser Glu Glu Glu Phe Leu Arg Thr Tyr Ala Gly Val Val
        35                  40                  45

Asn Ser Gln Leu Ser Gln Leu Pro Pro His Ser Ile Asp Gln Gly Ala
    50                  55                  60

Glu Asp Val Val Met Ala Phe Ser Arg Ser Glu Thr Glu Asp Arg Arg
65                  70                  75                  80

Gln

<210>  74
<211>  2153
<212>  DNA
<213>  Homo sapiens

<220>
<221>  CDS
<222>  (589)..(1356)
<223>

<400>  74
cagcggccgg gagggggctc cggggaccat ggggctcctg accattctga agaagatgaa      60

gcagaaagag cgggagctgc gactgctcat gcttggcctg gacaatgctg gaaagacaac     120

catcctgaag aagttcaatg gggaggacat cgacaccatc tccccaacgc tgggcttcaa     180

catcaagacc ctggagcacc gaggattcaa gctgaacatc tgggatgtgg gtggccagaa     240

gtccctgcgg tcctactggc ggaactactt tgagagcacc gatggcctca tctgggtagt     300

ggacagcgca gaccgccagc gcatgcagga ctgccagcgg gagctccaga gcctgctggt     360

ggaggagtga cctgggttta cgcttcacca cagagaagga acttttcaga cgctcccttg     420

gccgcgcacc cgggatcgga acagcgctgt cacttgcgac aaaatcccta ggccagagga     480

agaagagcgc aggcctgggc gaggcggcgg cgggcggagg ctgggccgga ggggtgggga     540

cggcgaggag gtggaggccg gcgctccgct ccgctccagc tcggtttc atg tcc cgc      597

Met Ser Arg
1

cag gcg aag gat gac ttc ctg cgg cac tac aca gtg tcg gac ccc agg 645
Gln Ala Lys Asp Asp Phe Leu Arg His Tyr Thr Val Ser Asp Pro Arg
    5                   10                  15

act cac ccc aag ggc tac acc gag tac aaa gta acc gcg cag ttc atc 693
Thr His Pro Lys Gly Tyr Thr Glu Tyr Lys Val Thr Ala Gln Phe Ile
20                  25                  30                  35

tca aag aag gac cca gag gat gtc aaa gag gtg gtg gtc tgg aag cgg 741
Ser Lys Lys Asp Pro Glu Asp Val Lys Glu Val Val Val Trp Lys Arg
                40                  45                  50

tac agc gac ttc cgc aag ctg cat gga gac ctg gcc tac acc cac cgc 789
Tyr Ser Asp Phe Arg Lys Leu His Gly Asp Leu Ala Tyr Thr His Arg
            55                  60                  65

aac ctc ttc cgc cgc ctc gag gag ttc cct gct ttc ccc cgg gcc cag 837
Asn Leu Phe Arg Arg Leu Glu Glu Phe Pro Ala Phe Pro Arg Ala Gln
        70                  75                  80

gtg ttt ggc cgg ttt gaa gcc tca gtg atc gag gag cgg cga aag ggg 885
Val Phe Gly Arg Phe Glu Ala Ser Val Ile Glu Glu Arg Arg Lys Gly
    85                  90                  95

gca gag gac ctg ctt cgc ttc act gtg cac ata cct gcg ctc aac aac 933
Ala Glu Asp Leu Leu Arg Phe Thr Val His Ile Pro Ala Leu Asn Asn
100                 105                 110                 115

agc ccc cag ctc aag gag ttc ttc cgg ggt ggg gag gtg acc cga ccc 981
Ser Pro Gln Leu Lys Glu Phe Phe Arg Gly Gly Glu Val Thr Arg Pro
                120                 125                 130

ttg gag gtg tcc agg gac cta cac atc ctg cca ccc cct ctg atc ccc 1029
Leu Glu Val Ser Arg Asp Leu His Ile Leu Pro Pro Pro Leu Ile Pro
            135                 140                 145

acc ccg ccc cct gat gac ccc cgg cta tcc caa ctg ctc cct gca gaa 1077
Thr Pro Pro Pro Asp Asp Pro Arg Leu Ser Gln Leu Leu Pro Ala Glu
        150                 155                 160

agg agg ggc ctc gag gaa ttg gag gtg cca gtg gac ccc cca cca tcc 1125
Arg Arg Gly Leu Glu Glu Leu Glu Val Pro Val Asp Pro Pro Pro Ser
    165                 170                 175

agc cct gcc cag gag gcc ctg gat ctc ctc ttt aac tgt gag agc acc 1173
Ser Pro Ala Gln Glu Ala Leu Asp Leu Leu Phe Asn Cys Glu Ser Thr
180                 185                 190                 195

gag gag gca tct ggt tcc cct gcc cga ggc ccc ctc acc gag gct gag 1221
Glu Glu Ala Ser Gly Ser Pro Ala Arg Gly Pro Leu Thr Glu Ala Glu
                200                 205                 210

ctt gcc ctc ttc gac ccc ttc tcc aag ggt gac ccg ttg cct gcc cgc 1269
Leu Ala Leu Phe Asp Pro Phe Ser Lys Gly Asp Pro Leu Pro Ala Arg
            215                 220                 225

cag gaa ggt gtg aag aag aag gca gct gag tac ctg aag cgg gca gag 1317
Gln Glu Gly Val Lys Lys Lys Ala Ala Glu Tyr Leu Lys Arg Ala Glu
        230                 235                 240

gag atc ctg cgc ctg cac ctg tct caa ctc cca ccc taa cagggagtgg 1366
Glu Ile Leu Arg Leu His Leu Ser Gln Leu Pro Pro
    245                 250                 255

gccattccct gggactctca ctcctgcact gccagcccct tctcctctcc ccagggcctg 1426

gccctacctc ctggtcttgt aattacagga gccatttctg taggtaactg gaccaagaat 1486

gagaaaaata atgaattctt agctccctga ttacacctgc caccttggaa tccaggactc 1546

acacttctga ccctgcctgt ctttttgggg tttttttgag ttggagtctc gctgtgtcgc 1606

ccagactgga gtgcagtggt gggatcgcgg ctcactgcaa cctccacctc ccaggttcaa 1666

gcagttctcc tgtctcagcc tccccagtag ctgagattgc aggcacatgc caccacgccc 1726

agctaatatt ttgtattttc agtagggacg gggttacacc atgttggcca ggctggtctc 1786

gaactcctga cctcaagtga tccacccgcc tcagtctccc aaagtgctga gattacaggc 1846

atgagtcact acgcccggcc catgtctgtc tgtcttgatg tgtgagcagc agctgtggtc 1906

attaaaccat tagtttaccc ctctagaact ggggtctgca aactcccacc tgcagccaaa 1966

tctggcccac ctccttttta atgtaagggc tgtgagagtg gtttttactt tttttaatga 2026

ttaaaaaaat caaaataata ttctgtgaca atgacaggtg aaatttatat gtgacaagtg 2086

aaaattatat gaaatttaag agtccataaa taaaatttgt tggaacacaa aaaaaaaaaa 2146

aaaaaaa 2153

<210> 75
<211> 255
<212> PRT
<213> Homo sapiens

<400> 75

Met Ser Arg Gln Ala Lys Asp Asp Phe Leu Arg His Tyr Thr Val Ser
1               5                   10                  15

Asp Pro Arg Thr His Pro Lys Gly Tyr Thr Glu Tyr Lys Val Thr Ala
            20                  25                  30

Gln Phe Ile Ser Lys Lys Asp Pro Glu Asp Val Lys Glu Val Val Val
        35                  40                  45

Trp Lys Arg Tyr Ser Asp Phe Arg Lys Leu His Gly Asp Leu Ala Tyr
    50                  55                  60

Thr His Arg Asn Leu Phe Arg Arg Leu Glu Glu Phe Pro Ala Phe Pro
65                  70                  75                  80

Arg Ala Gln Val Phe Gly Arg Phe Glu Ala Ser Val Ile Glu Glu Arg
                85                  90                  95

Arg Lys Gly Ala Glu Asp Leu Leu Arg Phe Thr Val His Ile Pro Ala
            100                 105                 110

Leu Asn Asn Ser Pro Gln Leu Lys Glu Phe Phe Arg Gly Gly Glu Val
        115                 120                 125

Thr Arg Pro Leu Glu Val Ser Arg Asp Leu His Ile Leu Pro Pro Pro
    130                 135                 140

Leu Ile Pro Thr Pro Pro Pro Asp Asp Pro Arg Leu Ser Gln Leu Leu
145                 150                 155                 160

Pro Ala Glu Arg Arg Gly Leu Glu Glu Leu Glu Val Pro Val Asp Pro
                165                 170                 175

Pro Pro Ser Ser Pro Ala Gln Glu Ala Leu Asp Leu Leu Phe Asn Cys
            180                 185                 190

Glu Ser Thr Glu Glu Ala Ser Gly Ser Pro Ala Arg Gly Pro Leu Thr
        195                 200                 205

Glu Ala Glu Leu Ala Leu Phe Asp Pro Phe Ser Lys Gly Asp Pro Leu
    210                 215                 220

Pro Ala Arg Gln Glu Gly Val Lys Lys Lys Ala Ala Glu Tyr Leu Lys
225                 230                 235                 240

Arg Ala Glu Glu Ile Leu Arg Leu His Leu Ser Gln Leu Pro Pro
                245                 250                 255